# EUROPEAN PATENT SPECIFICATION

(11) **EP 3 528 302 B1**
(45) Date of publication and mention of the grant of the patent: **01.04.2026**
(21) Application number: 18248288.5
(22) Date of filing: 28.12.2018
(51) Int. Cl.: H10K 50/15, H10K 85/10

(54) **CROSS-LINKABLE ARYLAMINE-BASED COMPOUND, POLYMER OBTAINED THEREFROM, AND ORGANIC LIGHT-EMITTING DEVICE INCLUDING THE POLYMER**
VERNETZBARE ARYLAMINBASIERTE VERBINDUNG, DARAUS ERHALTENES POLYMER UND ORGANISCHE LICHTEMITTIERENDE VORRICHTUNG MIT DEM POLYMER
COMPOSÉ RÉTICULABLE À BASE D'ARYLAMINE, POLYMÈRE OBTENU À PARTIR DE CELUI-CI ET DISPOSITIF ÉLECTROLUMINESCENT ORGANIQUE COMPRENANT LE POLYMÈRE

(30) Priority: 19.02.2018 KR 20180019525; 19.02.2018 KR 20180019526
(43) Date of publication of application: 21.08.2019
(73) Proprietor: Samsung Display Co., Ltd., Yongin-si, Gyeonggi-do (KR)
(72) Inventor: SHIN, Dongwoo, Gyeonggi-do (KR); KIM, Dukki, Gyeonggi-do (KR); KIM, Sehun, Gyeonggi-do (KR); KIM, Jaeyun, Gyeonggi-do (KR); PARK, Jongwon, Gyeonggi-do (KR); LEE, Seungmook, Gyeonggi-do (KR); HA, Jaekook, Gyeonggi-do (KR); ITO, Naoyuki, Gyeonggi-do (KR)
(74) Representative: Marks & Clerk LLP

(56) References cited:
- KR-A- 20120 052 063
- US-A1- 2013 248 848
- US-A1- 2017 077 462
- YABIN SONG ET AL: "New semiconductors based on triphenylamine with macrocyclic architecture: synthesis, properties and applications in OFETs", JOURNAL OF MATERIALS CHEMISTRY, vol. 17, no. 42, 1 January 2007 (2007-01-01), GB, pages 4483, XP055600308, ISSN: 0959-9428, DOI: 10.1039/b708887f

## Description

### BACKGROUND

### 1. Field

One or more aspects of embodiments of the present disclosure relate to a cross-linkable arylamine-based compound, a polymer obtained therefrom, and an organic light-emitting device including the polymer.

### 2. Description of the Related Art

Organic light-emitting devices (OLEDs) are self-emission devices that produce full-color images and have wide viewing angles, high contrast ratios, short response times, and excellent brightness, driving voltage, and response speed characteristics, compared with devices in the related art.

An organic light-emitting device (OLED) may include a first electrode on a substrate, and a hole transport region, an emission layer, an electron transport region, and a second electrode sequentially stacked on the first electrode. Holes provided from the first electrode may move toward the emission layer through the hole transport region, and electrons provided from the second electrode may move toward the emission layer through the electron transport region. Carriers (such as holes and/or electrons) may recombine in the emission layer to produce excitons. The excitons may transition from an excited state to the ground state to thereby generate light.

US 2017/077462 A1 discloses an organic electroluminescence device. US 2013/248848 A1 discloses a process and materials for making contained layers and devices.

### SUMMARY

One or more aspects of embodiments of the present disclosure are directed to a cross-linkable arylamine-based compound, a polymer obtained therefrom, and an organic light-emitting device including the polymer.

Additional aspects will be set forth in part in the following description and, in part, will be apparent from the description, or may be learned by practice of the presented embodiments.

One or more embodiments of the present disclosure provide a cross-linkable arylamine-based compound represented by Formula 1a or 1b:

**Formula 1a** A₁-(B)ₚ-A₂,

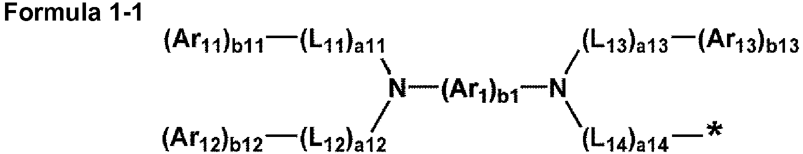

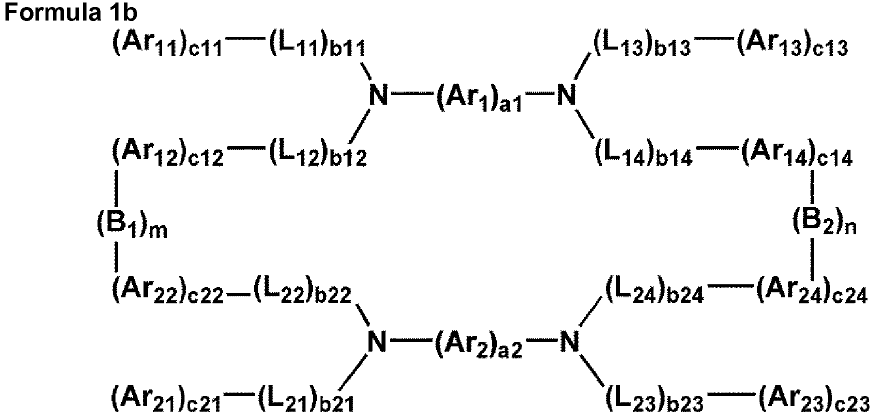

In Formula 1a, A₁ and A₂ are each a group represented by Formula 1-1, where A₁ and A₂ are identical to or different from each other,
in Formula 1a, B is selected from a substituted or unsubstituted C₅-C₆₀ carbocyclic group, a substituted or unsubstituted C₁-C₆₀ heterocyclic group, and *'-Si(Q₄₁)(Q₄₂)-*", and p is an integer of 1 to 10,
in Formula 1-1, * indicates a binding site to (B)ₚ in Formula 1a,
in Formula 1-1, Ar1 is selected from a benzene group, a pyridine group, a pyrimidine group, a pyrazine group, a triazine group, a furan group, a thiophene group, an imidazole group, a thiazole group, an isoxazole group, and an oxazole group,
in Formula 1-1, b1 is an integer of four or more, and four or more Ar₁(s) may be identical to or different from each other,
in Formula 1-1, L₁₁ to L₁₄ are each independently a substituted or unsubstituted C₅-C₆₀ carbocyclic group or a substituted or unsubstituted C₁-C₆₀ heterocyclic group,
in Formula 1-1, a11 to a14 are each independently 0, 1, 2, 3, or 4,
in Formula 1-1, when a11 is 0, *'-(L₁₁)ₐ₁₁-*" is equivalent to a single bond; when a12 is 0, *'-(L₁₂)ₐ₁₂-*" is equivalent to a single bond; when a13 is 0, *'-(L₁₃)ₐ₁₃-*" is equivalent to a single bond; when a14 is 0, *'-(L₁₄)ₐ₁₄-*" is equivalent to a single bond; when a11 is two or more, two or more L₁₁(s) are identical to or different from each other; when a12 is two or more, two or more L₁₂(s) are identical to or different from each other; when a13 is two or more, two or more L₁₃(s) are identical to or different from each other; and when a14 is two or more, two or more L₁₄(s) are identical to or different from each other,
in Formula 1-1, Ar₁₁ to Ar₁₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein at least one of Ar₁₁ to Ar₁₃ is substituted with a cross-linkable group,
in Formula 1-1, b11 to b13 are each independently 1, 2, 3, 4, or 5, wherein, when b11 is two or more, two or more Ar₁₁(s) are identical to or different from each other; when b12 is two or more, two or more Ar₁₂(s) are identical to or different from each other; and when b13 is two or more, two or more Ar₁₃(s) are identical to or different from each other,
in Formula 1b, Ar₁ and Ar₂ are each independently a substituted or unsubstituted C₅-C₆₀ carbocyclic group or a substituted or unsubstituted C₁-C₆₀ heterocyclic group, wherein Ar₁ and Ar₂ are identical to or different from each other,
in Formula 1b, a1 and a2 are each independently an integer of 1 to 5, wherein, when a1 is two or more, two or more Ar₁(s) are identical to or different from each other; and when a2 is two or more, two or more Ar₂(s) are identical to or different from each other,
in Formula 1b, L₁₁ to L₁₄ and L₂₁ to L₂₄ are each independently a substituted or unsubstituted C₅-C₆₀ carbocyclic group or a substituted or unsubstituted C₁-C₆₀ heterocyclic group,
in Formula 1b, b11 to b14 and b21 to b24 are each independently an integer of 0 to 3, wherein, when b11 is 0, *-(L₁₁)_{b11}-*' is equivalent to a single bond; when b12 is 0, *-(L₁₂)_{b12}-*' is equivalent to a single bond; when b13 is 0, *-(L₁₃)_{b13}-*' is equivalent to a single bond; when b14 is 0, *-(L₁₄)_{b14}-*' is equivalent to a single bond; when b21 is 0, *-(L₂₁)_{b21}-*' is equivalent to a single bond; when b22 is 0, *-(L₂₂)_{b22}-*' is equivalent to a single bond; when b23 is 0, *-(L₂₃)_{b23}-*' is equivalent to a single bond; when b24 is 0, *-(L₂₄)_{b24}-*' is equivalent to a single bond; when b11 is two or more, two or more L₁₁(s) are identical to or different from each other; when b12 is two or more, two or more L₁₂(s) are identical to or different from each other; when b13 is two or more, two or more L₁₃(s) are identical to or different from each other; when b14 is two or more, two or more L₁₄(s) are identical to or different from each other; when b21 is two or more, two or more L₂₁(s) are identical to or different from each other; when b22 is two or more, two or more L₂₂(s) are identical to or different from each other; when b23 is two or more, two or more L₂₃(s) are identical to or different from each other; and when b24 is two or more, two or more L₂₄(s) are identical to or different from each other,
in Formula 1b, B₁ and B₂ are each independently selected from a single bond, a substituted or unsubstituted C₁-C₂₀ alkylene group, a substituted or unsubstituted C₂-C₂₀ alkenylene group, and a substituted or unsubstituted C₂-C₂₀ alkynylene group,
in Formula 1b, m and n are each independently an integer of 1 to 3, wherein, when m is two or more, two or more B₁(s) are identical to or different from each other; and when n is two or more, two or more B₂(s) are identical to or different from each other,
in Formula 1b, Ar₁₁, Ar₁₃, Ar₂₁, and Ar₂₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group,
in Formula 1b, Ar₁₂, Ar₁₄, Ar₂₂, and Ar₂₄ are each independently selected from a single bond, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
in Formula 1b, c11 to c14 and c21 to c24 are each independently an integer of 1 to 5, wherein, when c11 is two or more, two or more Ar₁₁(s) are identical to or different from each other; when c12 is two or more, two or more Ar₁₂(s) are identical to or different from each other; when c13 is two or more, two or more Ar₁₃(s) are identical to or different from each other; when c14 is two or more, two or more Ar₁₄(s) are identical to or different from each other; when c21 is two or more, two or more Ar₂₁(s) are identical to or different from each other; when c22 is two or more, two or more Ar₂₂(s) are identical to or different from each other; when c23 is two or more, two or more Ar₂₃(s) are identical to or different from each other; and when c24 is two or more, two or more Ar₂₄(s) are identical to or different from each other,
in Formula 1b, at least one substituent in Ar₁, Ar₂, Ar₁₁ to Ar₁₄, and Ar₂₁ to Ar₂₄ includes a cross-linkable group,
*'and *" each indicate a binding site to a neighboring atom,
at least one substituent of the substituted C₅-C₆₀ carbocyclic group, the substituted C₁-C₆₀ heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, the substituted monovalent non-aromatic condensed heteropolycyclic group, the substituted C₁-C₂₀ alkylene group, the substituted C₂-C₂₀ alkenylene group, and the substituted C₂-C₂₀ alkynylene group is selected from:
   deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group;
   a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, - Cl, -Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), and -P(=O)(Q₁₁)(Q₁₂);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, a biphenyl group, and a terphenyl group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), and -P(=O)(Q₂₁)(Q₂₂); and
   -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂), and
   Q₁₁ to Q₁₃, Q₂₁ to Q₂₃, Q₃₁ to Q₃₃, and Q₄₁ to Q₄₂ are each independently selected from hydrogen, deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, a biphenyl group, and a terphenyl group.

One or more embodiments of the present disclosure provide an arylamine-based polymer including a repeating unit represented by Formula 11 or formed by cross-linking a cross-linkable arylamine-based compound represented by Formula 1b:

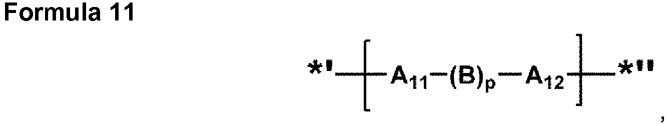

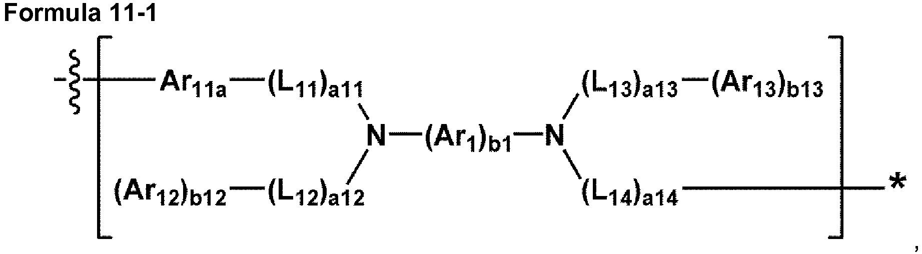

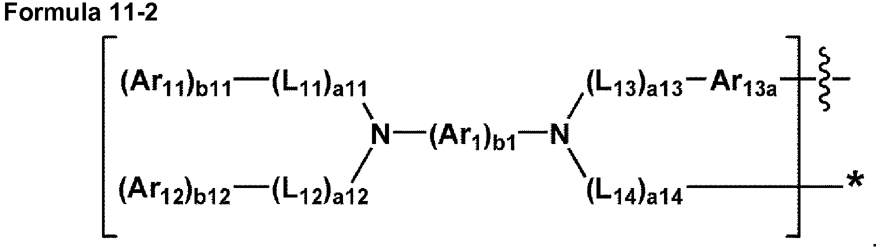

In Formula 11, A₁₁ and A₁₂ are each independently selected from groups represented by Formulae 11-1 and 11-2, wherein A₁₁ and A₁₂ are identical to or different from each other,
in Formula 11-1, * indicates a binding site to (B)ₚ in Formula 11,
in Formulae 11, 11-1, and 11-2, *', *", and 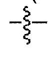 each indicate a binding site to a neighboring repeating unit,
in Formulae 11-1 and 11-2, Ar₁₁ₐ and Ar₁₃ₐ are each a residue formed after cross-linking, and the repeating units are linked by a divalent cyclobutane group,
in Formula 11, B and p are each independently the same as described above,
in Formulae 11-1 and 11-2, L₁₁ to L₁₄, a11 to a14, b11 to b13, Ar₁, and b1 are each independently the same as described above, and
in Formulae 11-1 and 11-2, Ar₁₁ to Ar₁₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group,.

One or more embodiments of the present disclosure provide an organic light-emitting device including: a first electrode; a second electrode facing the first electrode; and an organic layer between the first electrode and the second electrode and including an emission layer, wherein the organic layer further includes at least one of the arylamine-based polymer.

One or more embodiments of the present disclosure provide an electronic apparatus including a thin film transistor and the organic light-emitting device, wherein the thin film transistor includes a source electrode, a drain electrode, an active layer, and a gate electrode, and the first electrode of the organic light-emitting device is electrically connected to one of the source electrode and the drain electrode of the thin film transistor.

At least some of the above and other features of the present invention are set out in the claims.

### BRIEF DESCRIPTION OF THE DRAWINGS

These and/or other aspects will become apparent and more readily appreciated from the following description of the embodiments, taken in conjunction with the accompanying drawings, in which:

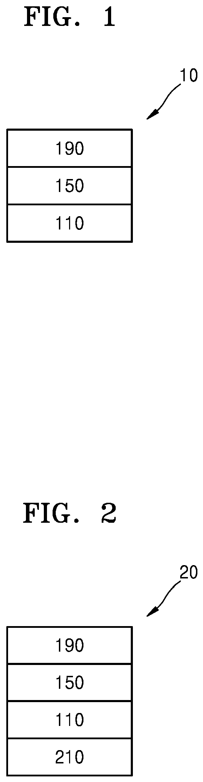

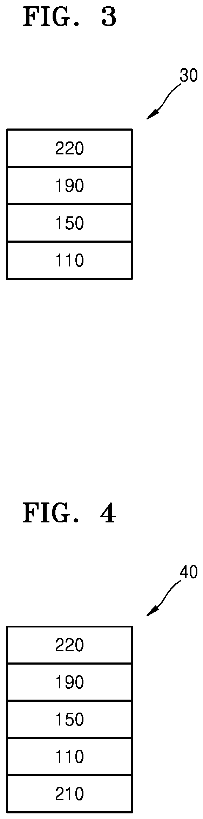

FIGS. 1-4 are schematic views of an organic light-emitting device according to embodiments of the present disclosure.

### DETAILED DESCRIPTION

Reference will now be made in more detail to embodiments, examples of which are illustrated in the accompanying drawings, wherein like reference numerals refer to like elements throughout, and duplicative descriptions may not be provided. In this regard, the present embodiments may have different forms and should not be construed as being limited to the descriptions set forth herein. Accordingly, the embodiments are merely described below, by referring to the drawings, to explain aspects of the present description. As used herein, the term "and/or" includes any and all combinations of one or more of the associated listed items. Expressions such "at least one of", "one of", "selected from", "at least one selected from", and "one selected from", when preceding a list of elements, modify the entire list of elements and do not modify the individual elements of the list.

The thicknesses of layers, films, panels, regions, etc., may be exaggerated in the drawings for clarity. It will be understood that when an element such as a layer, film, region, or substrate is referred to as being "on" another element, it can be directly on the other element or intervening element(s) may also be present. In contrast, when an element is referred to as being "directly on" another element, no intervening elements are present.

The use of "may" when describing embodiments of the present disclosure refers to "one or more embodiments of the present disclosure."

According to one or more embodiments of the present disclosure, a cross-linkable arylamine-based compound is represented by Formula 1a or 1b:

**Formula 1a** A₁-(B)ₚ-A₂,

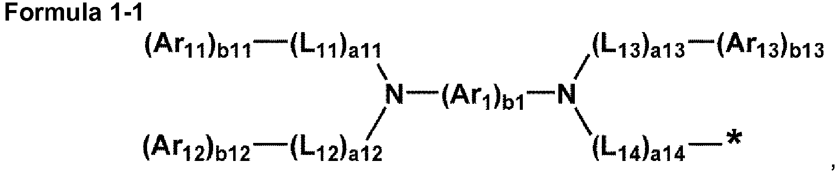

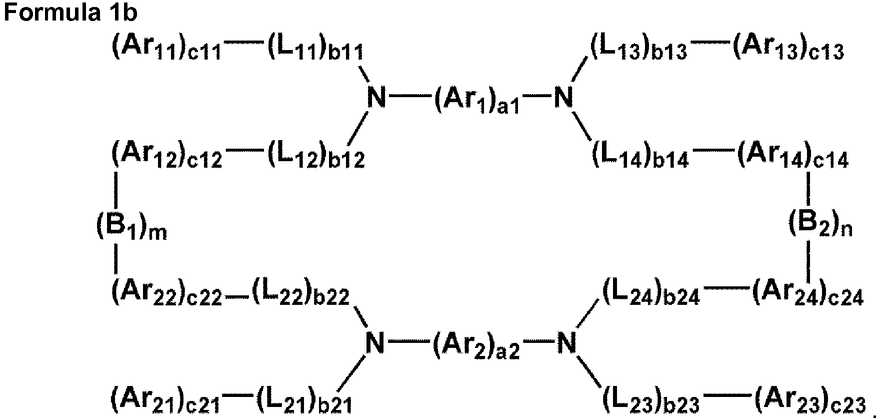

Formulae 1a, 1-1, and 1b will be described below in more detail.

In Formula 1a, A₁ and A₂ are each a group represented by Formula 1-1, and A₁ and A₂ are identical to or different from each other.

In some embodiments, for example, in Formula 1a, A₁ and A₂ may be identical to each other.

In Formula 1a, B is selected from a substituted or unsubstituted C₅-C₆₀ carbocyclic group, a substituted or unsubstituted C₁-C₆₀ heterocyclic group, and *'-Si(Q₄₁)(Q₄₂)-*". For example, in Formula 1a, B may be selected from a substituted or unsubstituted C₅-C₃₀ carbocyclic group, a substituted or unsubstituted C₁-C₃₀ heterocyclic group, and *'-Si(Q₄₁)(Q₄₂)-*". For example, in Formula 1a, B may be selected from a substituted or unsubstituted C₅-C₃₀ carbocyclic group, a substituted or unsubstituted C₁-C₂₀ heterocyclic group, and *'-Si(Q₄₁)(Q₄₂)-*".

In some embodiments, in Formula 1a, *-(B)ₚ-*' may be selected from groups represented by Formulae 3-1 to 3-19, but embodiments of the present disclosure are not limited thereto:

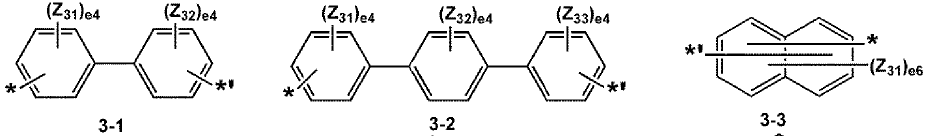

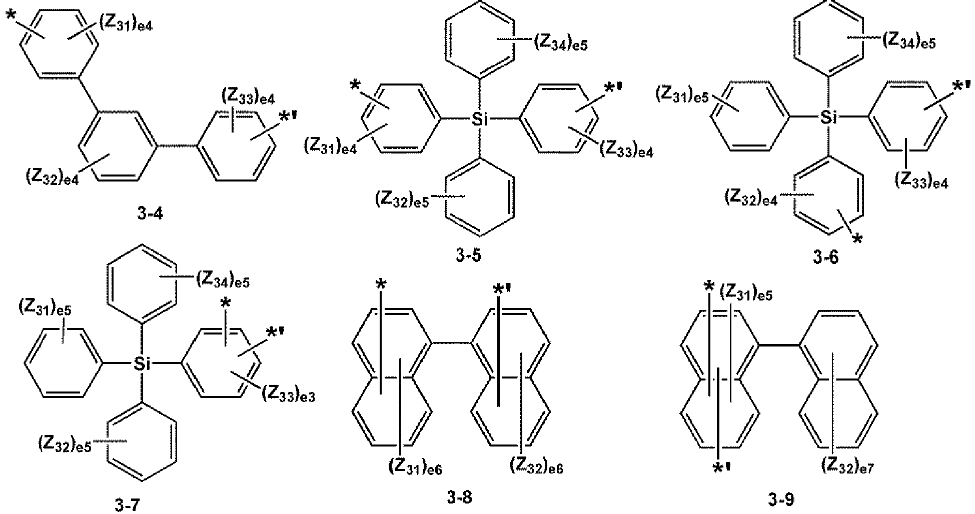

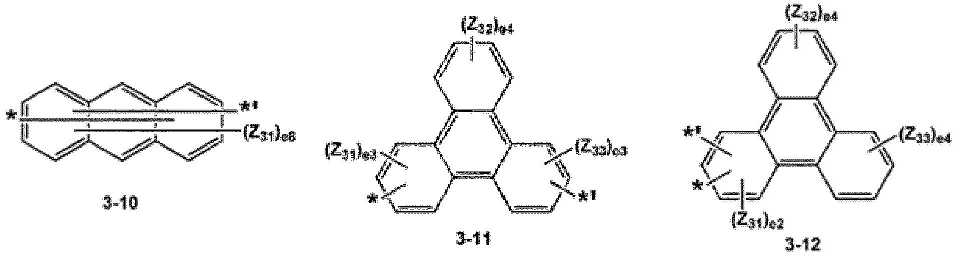

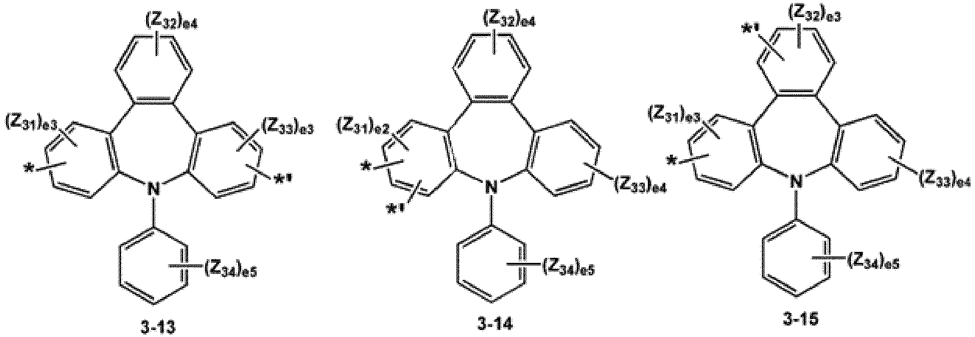

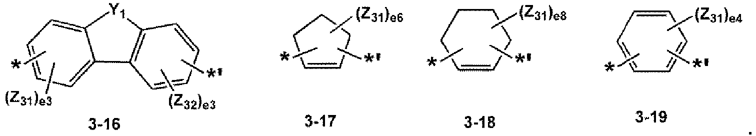

In Formulae 3-1 to 3-19,
Y₁ is O, S, C(Z₃₅)(Z₃₆), N(Z₃₅), or Si(Z₃₅)(Z₃₆),
Z₃₁ to Z₃₆ are each independently selected from hydrogen, deuterium, -F, - Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopropenyl group, a cyclobutenyl group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthyl group, a fluorenyl group, a spiro-bifluorenyl group, a spiro-benzofluorene-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, an isoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, an isobenzothiazolyl group, a benzoxazolyl group, an isobenzoxazolyl group, a triazolyl group, a tetrazolyl group, an oxadiazolyl group, a triazinyl group, a carbazolyl group, a dibenzofuranyl group, a dibenzothiophenyl group, a dibenzosilolyl group, a benzocarbazolyl group, a naphtho benzofuranyl group, a naphtho benzothiophenyl group, a naphthobenzosilolyl group, a dibenzocarbazolyl group, a dinaphthofuranyl group, a dinaphtho thiophenyl group, a dinaphtho silolyl group, a thiadiazolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indeno pyrrolyl group, an indolopyrrolyl group, an indeno carbazolyl group, an indolocarbazolyl group, -Si(Q₃₁)(Q₃₂)(Q₃₃), and -N(Q₃₁)(Q₃₂), and
Q₃₁ to Q₃₃ are each independently selected from:
   a C₁-C₁₀ alkyl group, a C₁-C₁₀ alkoxy group, a phenyl group, a biphenyl group, a terphenyl group, and a naphthyl group; and
   a phenyl group, a biphenyl group, a terphenyl group, and a naphthyl group, each substituted with at least one selected from a C₁-C₁₀ alkyl group, a C₁-C₁₀ alkoxy group, and a phenyl group.
   e2 is an integer of 0 to 2,
   e3 is an integer of 0 to 3,
   e4 is an integer of 0 to 4,
   e5 is an integer of 0 to 5,
   e6 is an integer of 0 to 6,
   e7 is an integer of 0 to 7,
   e8 is an integer of 0 to 8, and
   * and *' each indicate a binding site to a neighboring atom.

In Formula 1a, p is an integer of 1 to 10. For example, p may be 1, 2, 3, 4, 5, 6, 7, 8, 9 or 10.

In Formula 1-1, * indicates a binding site to (B)ₚ in Formula 1a.

The cross-linkable arylamine-based compound represented by Formula 1a may be symmetrical with respect to *'-(B)ₚ-*", but embodiments of the present disclosure are not limited thereto.

The cross-linkable arylamine-based compound represented by Formula 1b may be symmetrical with respect to an axis passing through Ar₁ and Ar₂ in Formula 1b.

Ar₁ and Ar₂ in Formula 1b are each independently selected from a substituted or unsubstituted C₅-C₆₀ carbocyclic group and a substituted or unsubstituted C₁-C₆₀ heterocyclic group. For example, Ar₁ in Formula 1-1 and Ar₁ and Ar₂ in Formula 1b may each independently be selected from a substituted or unsubstituted C₅-C₃₀ carbocyclic group and a substituted or unsubstituted C₁-C₃₀ heterocyclic group. For example, Ar₁ and Ar₂ in Formula 1b may each independently be selected from a substituted or unsubstituted C₅-C₂₀ carbocyclic group and a substituted or unsubstituted C₁-C₂₀ heterocyclic group. For example, Ar₁ and Ar₂ in Formula 1b may each independently be selected from a substituted or unsubstituted C₆-C₁₀ carbocyclic group and a substituted or unsubstituted C₁-C₁₀ heterocyclic group.

In some embodiments not forming part of the present invention, in Formula 1-1, Ar₁ may be selected from a substituted or unsubstituted C₅-C₃₀ carbocyclic group and a substituted or unsubstituted C₁-C₃₀ heterocyclic group. For example, in Formula 1-1, Ar₁ may be selected from a substituted or unsubstituted C₅-C₂₀ carbocyclic group and a substituted or unsubstituted C₁-C₂₀ heterocyclic group. For example, in Formula 1-1, Ar₁ may be selected from a substituted or unsubstituted C₆-C₁₀ carbocyclic group and a substituted or unsubstituted C₁-C₁₀ heterocyclic group.

In the embodiment of the present invention, in Formula 1-1, Ar₁ is selected from a benzene group, a pyridine group, a pyrimidine group, a pyrazine group, a triazine group, a furan group, a thiophene group, an imidazole group, a thiazole group, an isoxazole group, and an oxazole group, and
in Formula 1b, Ar₁ and Ar₂ may each independently be selected from:
a C₆-C₆₀ (*e.g.* C₆-C₃₀) arylene group and a C₁-C₆₀ (*e.g.* C₁-C₂₀) heteroarylene group; and
a C₆-C₆₀ (*e.g.* C₆-C₃₀) arylene group and a C₁-C₆₀ (*e.g.* C₁-C₂₀) heteroarylene group, each substituted with at least one selected from deuterium, -F, -Cl, -Br, -I, a hydroxyl group, a cyano group, a nitro group, an amidino group, a hydrazino group, a hydrazono group, a C₁-C₆₀ (*e.g*. C₁-C₂₀) alkyl group, a C₁-C₆₀ (*e.g.* C₁-C₂₀) alkoxy group, -N(Q₃₁)(Q₃₂), and a cross-linkable group,
but embodiments of the present disclosure are not limited thereto.

For example, in Formula 1-1, Ar₁ may be selected from a benzene group, a pyridine group, a furan group, and a thiophene group.

For example, Ar₁ in Formula 1-1 may be a benzene group, and Ar₁ and Ar₂ in Formula 1b may each independently be selected from:
a phenylene group and a naphthylene group; and
a phenylene group and a naphthylene group, each substituted with at least one selected from deuterium, -F, -Cl, -Br, -I, a hydroxyl group, a cyano group, a nitro group, an amidino group, a hydrazino group, a hydrazono group, a C₁-C₆₀ (*e.g.* C₁-C₂₀) alkyl group, a C₁-C₆₀ (*e.g.* C₁-C₂₀) alkoxy group, -N(Q₃₁)(Q₃₂), and a cross-linkable group.

In Formula 1-1, b1 is an integer of four or more, and four or more Ar₁(s) are identical to or different from each other In Formula 1b, a1 and a2 are each independently an integer of 1 to 5, wherein, when a1 is two or more, two or more Ar₁(s) are identical to or different from each other; and when a2 is two or more, two or more Ar₂(s) are identical to or different from each other.

In some embodiments, in Formula 1-1, four or more Ar₁(s) may be identical to each other. In some embodiments, in Formula 1-1, four or more Ar₁(s) may be different from each other.

In some embodiments, in Formula 1b, Ar₁ and Ar₂ may be identical to or different from each other. In some embodiments, in Formula 1b, Ar₁ and Ar₂ may be identical to each other.

In some embodiments, in Formula 1-1, four or more Ar₁(s) may be identical to each other, and in Formula 1b, when a1 is two or more, two or more Ar₁(s) may be identical to each other; and when a2 is two or more, two or more Ar₂(s) may be identical to each other.

In some embodiments, in Formula 1b, *-(Ar₁)ₐ₁-*' and *-(Ar₂)ₐ₂-*' may be identical to each other.

In Formula 1-1, b1 may be an integer of 4 to 6. For example, in Formula 1-1, b1 may be 4 or 5. For example, in Formula 1-1, b1 may be 4. For example, in Formula 1-1, b1 may be 5.

In some embodiments, in Formula 1-1, Ar₁ may be a benzene group, and b1 may be 4 or 5. For example, in Formula 1-1, Ar₁ may be a benzene group, and b1 may be 4. For example, in Formula 1-1, Ar₁ may be a benzene group, and b1 may be 5.

L₁₁ to L₁₄ in Formula 1-1 and L₁₁ to L₁₄ and L₂₁ to L₂₄ in Formula 1b are each independently a substituted or unsubstituted C₅-C₆₀ carbocyclic group or a substituted or unsubstituted C₁-C₆₀ heterocyclic group. For example, L₁₁ to L₁₄ in Formula 1-1 and L₁₁ to L₁₄ and L₂₁ to L₂₄ in Formula 1b may each independently be a substituted or unsubstituted C₅-C₃₀ carbocyclic group or a substituted or unsubstituted C₁-C₃₀ heterocyclic group. For example, L₁₁ to L₁₄ in Formula 1-1 and L₁₁ to L₁₄ and L₂₁ to L₂₄ in Formula 1b may each independently be a substituted or unsubstituted C₅-C₂₀ carbocyclic group or a substituted or unsubstituted C₁-C₂₀ heterocyclic group. For example, L₁₁ to L₁₄ in Formula 1-1 and L₁₁ to L₁₄ and L₂₁ to L₂₄ in Formula 1b may each independently be a substituted or unsubstituted C₆-C₁₄ carbocyclic group or a substituted or unsubstituted C₁-C₁₀ heterocyclic group.

In some embodiments, L₁₁ to L₁₄ in Formula 1-1 and L₁₁ to L₁₄ and L₂₁ to L₂₄ in Formula 1b may each independently be selected from groups represented by Formulae 2-1 to 2-37, but embodiments of the present disclosure are not limited thereto:

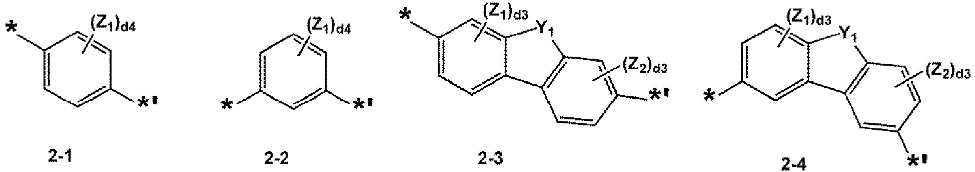

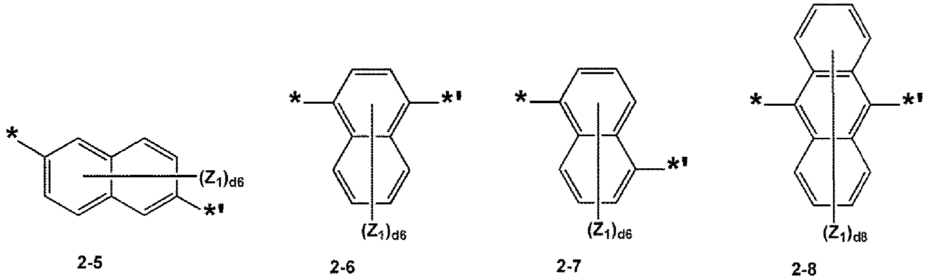

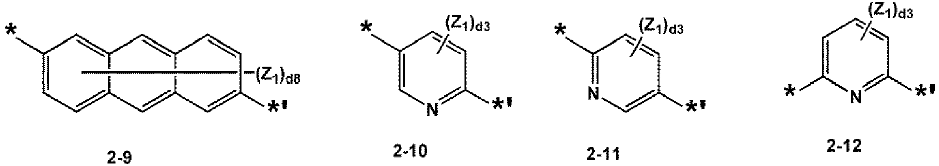

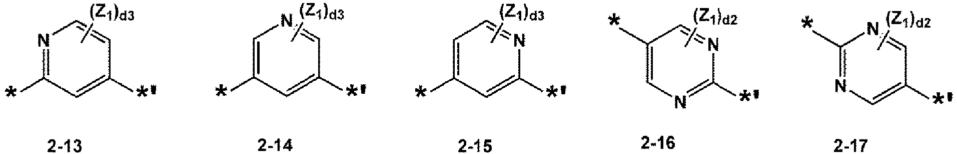

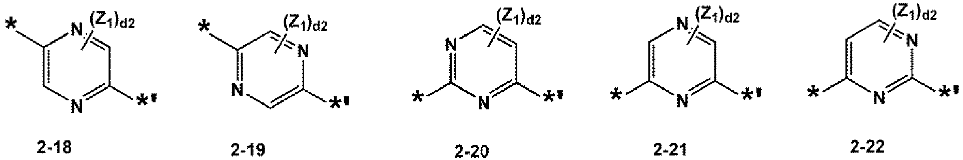

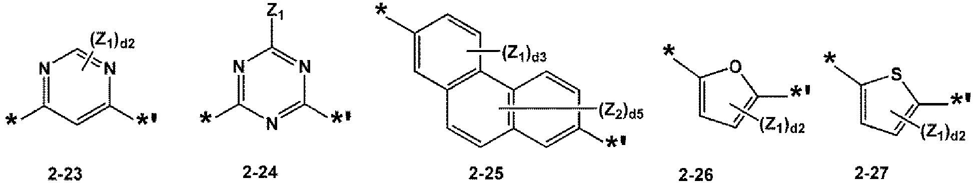

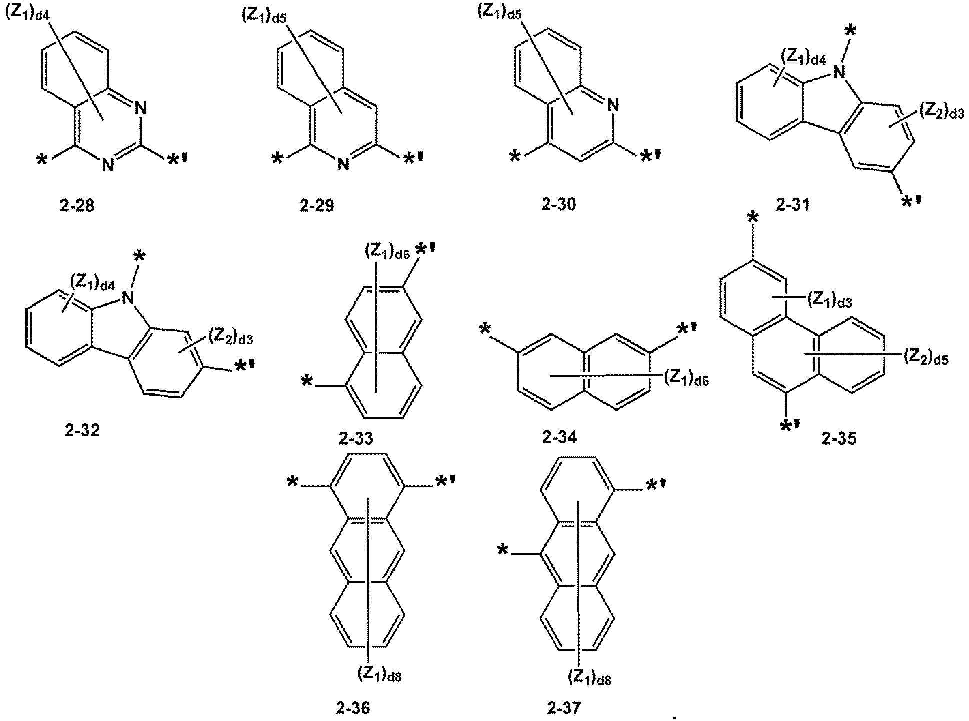

In Formulae 2-1 to 3-37,
Y₁ is O, S, C(Z₃)(Z₄), N(Z₅), or Si(Z₆)(Z₇),
Z₁ to Z₇ are each independently selected from hydrogen, deuterium, -F, -Cl, - Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, and an imidazopyridinyl group,
d2 is an integer of 0 to 2,
d3 is an integer of 0 to 3,
d4 is an integer of 0 to 4,
d5 is an integer of 0 to 5,
d6 is an integer of 0 to 6,
d8 is an integer of 0 to 8, and
* and *' each indicate a binding site to a neighboring atom.

In Formula 1-1, a11 to a14 are each independently 0, 1, 2, 3, or 4, wherein, when a11 is 0, *'-(L₁₁)ₐ₁₁-*" is equivalent to a single bond; when a12 is 0, *'-(L₁₂)ₐ₁₂-*" is equivalent to a single bond; when a13 is 0, *'-(L₁₃)ₐ₁₃-*" is equivalent to a single bond; and when a14 is 0, *'-(L₁₄)ₐ₁₄-*" is equivalent to a single bond.

In Formula 1-1, when a11 is two or more, two or more L₁₁(s) are identical to or different from each other; when a12 is two or more, two or more L₁₂(s) are identical to or different from each other; when a13 is two or more, two or more L₁₃(s) are identical to or different from each other; and when a14 is two or more, two or more L₁₄(s) are identical to or different from each other.

In Formula 1b, b11 to b14 and b21 to b24 are each independently an integer of 0 to 3, wherein, when b11 is 0, *-(L₁₁)_{b11}-*' is equivalent to a single bond; when b12 is 0, *-(L₁₂)_{b12}-*' is equivalent to a single bond; when b13 is 0, *-(L₁₃)_{b13}-*' is equivalent to a single bond; when b14 is 0, *-(L₁₄)_{b14}-*' is equivalent to a single bond; when b21 is 0, *-(L₂₁)_{b21}-*' is equivalent to a single bond; when b22 is 0, *-(L₂₂)_{b22}-*' is equivalent to a single bond; when b23 is 0, *-(L₂₃)_{b23}-*' is equivalent to a single bond; and when b24 is 0, *-(L₂₄)_{b24}-*' is equivalent to a single bond.

In Formula 1b, when b11 is two or more, two or more L₁₁(s) are identical to or different from each other; when b12 is two or more, two or more L₁₂(s) are identical to or different from each other; when b13 is two or more, two or more L₁₃(s) are identical to or different from each other; when b14 is two or more, two or more L₁₄(s) are identical to or different from each other; when b21 is two or more, two or more L₂₁(s) are identical to or different from each other; when b22 is two or more, two or more L₂₂(s) are identical to or different from each other; when b23 is two or more, two or more L₂₃(s) are identical to or different from each other; and when b24 is two or more, two or more L₂₄(s) are identical to or different from each other.

In Formula 1b, b11 to b14 and b21 to b24 may each independently be an integer of 0 to 2.

In Formula 1b, B₁ and B₂ are each independently selected from a single bond, a substituted or unsubstituted C₁-C₂₀ alkylene group, a substituted or unsubstituted C₂-C₂₀ alkenylene group, and a substituted or unsubstituted C₂-C₂₀ alkynylene group. For example, in Formula 1b, B₁ and B₂ may each independently be selected from a single bond, a substituted or unsubstituted C₁-C₁₀ alkylene group, a substituted or unsubstituted C₂-C₁₀ alkenylene group, and a substituted or unsubstituted C₂-C₁₀ alkynylene group. For example, in Formula 1b, B₁ and B₂ may each independently be selected from a single bond, a substituted or unsubstituted C₁-C₅ alkylene group, a substituted or unsubstituted C₂-C₅ alkenylene group, and a substituted or unsubstituted C₂-C₅ alkynylene group.

In some embodiments, in Formula 1b, B₁ and B₂ may each independently be selected from:
a single bond;
a methylene group, an ethylene group, a propylene group, an isopropylene group, a butylene group, an isobutylene group, an ethenylene group, a propenylene group, a butenylene group, an ethynylene group, a propynylene group, and a butynylene group; and
a methylene group, an ethylene group, a propylene group, an isopropylene group, a butylene group, an isobutylene group, an ethenylene group, a propenylene group, a butenylene group, an ethynylene group, a propynylene group, and a butynylene group, each substituted with at least one selected from deuterium, -F, -Cl, - Br, -I, a hydroxyl group, a cyano group, a nitro group, an amidino group, a hydrazino group, a hydrazono group, a methyl group, an ethyl group, a propyl group, an isopropyl group, a butyl group, and an isobutyl group,
but embodiments of the present disclosure are not limited thereto.

For example, in Formula 1b, B₁ and B₂ may each independently be selected from:
a single bond;
a methylene group, ethylene group, an ethenylene group, and an ethynylene group; and
a methylene group, an ethylene group, an ethenylene group, and an ethynylene group, each substituted with at least one selected from deuterium, -F, -Cl, - Br, -I, a hydroxyl group, a cyano group, a nitro group, an amidino group, a hydrazino group, a hydrazono group, a methyl group, and an ethyl group.

In Formula 1b, m and n are each independently an integer of 1 to 3, wherein, when m is two or more, two or more B₁(s) are identical to or different from each other; and when n is two or more, two or more B₂(s) are identical to or different from each other.

In some embodiments, Ar₁₁ to Ar₁₃ in Formula 1-1 and Ar₁₁, Ar₁₃, Ar₂₁, and Ar₂₃ in Formula 1b are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g.* C₆-C₃₀) aryl group, a substituted or unsubstituted C₆-C₆₀ (*e.g.* C₆-C₃₀) aryloxy group, a substituted or unsubstituted C₆-C₆₀ (*e.g.* C₆-C₃₀) arylthio group, a substituted or unsubstituted C₁-C₆₀ (*e.g.* C₁-C₂₀) heteroaryl group, a substituted or unsubstituted monovalent non-aromatic condensed polycyclic group, and a substituted or unsubstituted monovalent non-aromatic condensed heteropolycyclic group.

In some embodiments, Ar₁₁ to Ar₁₃ in Formula 1-1 and Ar₁₁, Ar₁₃, Ar₂₁, and Ar₂₃ in Formula 1b may each independently be selected from a substituted or unsubstituted C₆-C₆₀ (*e.g.* C₆-C₃₀) aryl group and a substituted or unsubstituted C₁-C₆₀ (*e.g.* C₁-C₂₀) heteroaryl group, but embodiments of the present disclosure are not limited thereto.

In some embodiments, Ar₁₁ to Ar₁₃ in Formula 1-1 and Ar₁₁, Ar₁₃, Ar₂₁, and Ar₂₃ in Formula 1b may each independently be a substituted or unsubstituted C₆-C₆₀ (*e.g.* C₆-C₃₀) aryl group.

For example, in Formula 1-1, A₁₁ to Ar₁₃ may each independently be selected from:
a C₆-C₆₀ (*e.g.* C₆-C₃₀) aryl group; and
a C₆-C₆₀ (*e.g.* C₆-C₃₀) aryl group substituted with at least one selected from deuterium, -F, -Cl, -Br, -I, a hydroxyl group, a cyano group, a nitro group, an amidino group, a hydrazino group, a hydrazono group, a C₂-C₆₀ (*e.g.* C₂-C₂₀) alkenyl group, a C₂-C₆₀ (*e.g.* C₂-C₂₀) alkynyl group, and a C₁-C₆₀ (*e.g.* C₁-C₂₀) alkoxy group, and
in Formula 1b, Ar₁₁, Ar₁₃, Ar₂₁, and Ar₂₃ may each independently be selected from:
   a phenyl group, a biphenyl group, a terphenyl group, and a naphthyl group; and
   a phenyl group, a biphenyl group, a terphenyl group, and a naphthyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a cross-linkable group, and -N(Q₃₁)(Q₃₂),
   but embodiments of the present disclosure are not limited thereto.

For example, in Formula 1b, Ar₁₁, Ar₁₃, Ar₂₁, and Ar₂₃ may each independently be selected from:
a phenyl group and a naphthyl group; and
a phenyl group and a naphthyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a cross-linkable group, and -N(Q₃₁)(Q₃₂).

In Formula 1b, Ar₁₂, Ar₁₄, Ar₂₂, and Ar₂₄ are each independently selected from a single bond, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (*e.g.* C₆-C₃₀) arylene group, a substituted or unsubstituted C₁-C₆₀ (*e*.*g*. C₁-C₂₀) heteroarylene group, a substituted or unsubstituted divalent non-aromatic condensed polycyclic group, and a substituted or unsubstituted divalent non-aromatic condensed heteropolycyclic group.

In some embodiments, in Formula 1b, Ar₁₂, Ar₁₄, Ar₂₂, and Ar₂₄ may each independently be selected from:
a single bond;
a phenylene group and a naphthylene group; and
a phenylene group and a naphthylene group, each substituted with at least one selected from deuterium, -F, -Cl, -Br, -I, a hydroxyl group, a cyano group, a nitro group, an amidino group, a hydrazino group, a hydrazono group, a C₁-C₆₀ (*e.g.* C₁-C₂₀) alkyl group, a C₁-C₆₀ (*e.g.* C₁-C₂₀) alkoxy group, -N(Q₃₁)(Q₃₂), and a cross-linkable group,
but embodiments of the present disclosure are not limited thereto.

In Formula 1b, Ar₁₁, Ar₁₃, Ar₂₁, and Ar₂₃ may be identical to or different from each other, and Ar₁₂, Ar₁₄, Ar₂₂, and Ar₂₄ may be identical to or different from each other.

In some embodiments, in Formula 1b, Ar₁₁ and Ar₁₃ may be identical to each other, Ar₂₁ and Ar₂₃ may be identical to each other, Ar₁₂ and Ar₁₄ may be identical to each other, and Ar₂₂ and Ar₂₄ may be identical to each other.

In some embodiments, in Formula 1b, Ar₁₁ and Ar₂₁ may be identical to each other, Ar₁₃ and Ar₂₃ may be identical to each other, Ar₁₂ and Ar₂₂ may be identical to each other, and Ar₁₄ and Ar₂₄ may be identical to each other.

In some embodiments, in Formula 1b, Ar_{11,} Ar₁₃, Ar₂₁, and Ar₂₃ may be identical to each other, and Ar₁₂, Ar₁₄, Ar₂₂, and Ar₂₄ may be identical to each other.

In Formula 1-1, b11 to b13 are each independently 1, 2, 3, 4, or 5, wherein, when b11 is two or more, two or more Ar₁₁(s) are identical to or different from each other; when b12 is two or more, two or more Ar₁₂(s) are identical to or different from each other; and when b13 is two or more, two or more Ar₁₃(s) are identical to or different from each other.

In Formula 1b, c11 to c14 and c21 to c24 are each independently an integer of 1 to 5, wherein, when c11 is two or more, two or more Ar₁₁(s) are identical to or different from each other; when c12 is two or more, two or more Ar₁₂(s) are identical to or different from each other; when c13 is two or more, two or more Ar₁₃(s) are identical to or different from each other; when c14 is two or more, two or more Ar₁₄(s) are identical to or different from each other; when c21 is two or more, two or more Ar₂₁(s) are identical to or different from each other; when c22 is two or more, two or more Ar₂₂(s) are identical to or different from each other; when c23 is two or more, two or more Ar₂₃(s) are identical to or different from each other; and when c24 is two or more, two or more Ar₂₄(s) are identical to or different from each other.

At least one of Ar₁₁ to Ar₁₃ in Formula 1-1 and at least one of Ar₁, Ar₂, Ar₁₁ to Ar₁₄, and Ar₂₁ to Ar₂₄ in Formula 1b is substituted with a cross-linkable group.

In some embodiments, in Formula 1-1, one of Ar₁₁ and Ar₁₃ may be substituted with a cross-linkable group. For example, in Formula 1-1, Ar₁₁ may be substituted with a cross-linkable group. For example, in Formula 1-1, Ar₁₃ may be substituted with a cross-linkable group.

In some embodiments, in Formula 1b, substituents of Ar₁₁, Ar₁₃, Ar₂₁, and Ar₂₃ may include a cross-linkable group.

In some embodiments, in Formula 1b, at least two groups of Ar₁₁, Ar₁₃, Ar₂₁, and Ar₂₃ may be substituted with a cross-linkable group.

In some embodiments, in Formula 1b, at least two groups of Ar₁, Ar₂, Ar₁₂, Ar₁₄, Ar₂₂, and Ar₂₄ may be substituted with a cross-linkable group, and B₁ and B₂ may each be a single bond, but embodiments of the present disclosure are not limited thereto.

In some embodiments, the cross-linkable group may include at least one selected from a vinylene moiety, a styrene moiety, a cyclobutane moiety, and an epoxy moiety, but embodiments of the present disclosure are not limited thereto.

In some embodiments, for example, the cross-linkable group may be selected from groups represented by Formulae 4-1 to 4-14:

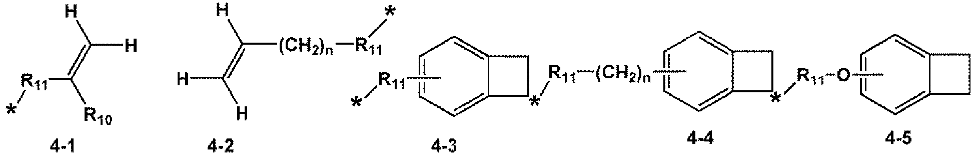

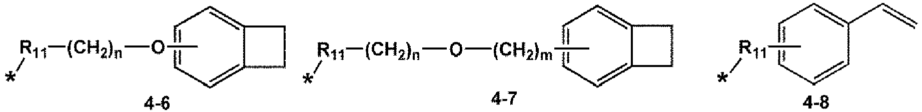

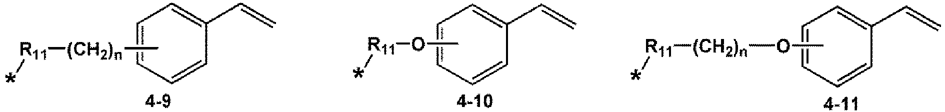

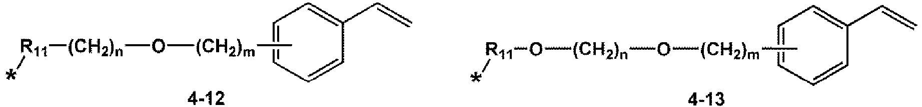

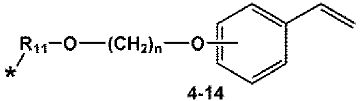

In Formulae 4-1 to 4-14,
R₁₀ is a hydrogen atom or a substituted or unsubstituted C₁-C₂₀ alkyl group,
R₁₁ is selected from a single bond, a substituted or unsubstituted C₁-C₂₀ alkylene group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (*e.g.* C₆-C₃₀) arylene group, a substituted or unsubstituted C₁-C₆₀ (*e*.*g*. C₁-C₂₀) heteroarylene group, a substituted or unsubstituted divalent non-aromatic condensed polycyclic group, and a substituted or unsubstituted divalent non-aromatic condensed heteropolycyclic group,
m and n are each independently an integer of 1 to 10, and
* indicates a binding site to a neighboring atom.

In Formulae 4-1 to 4-14, R₁₁ may be a single bond, a substituted or unsubstituted C₁-C₂₀ alkylene group, a substituted or unsubstituted C₆-C₆₀ (*e.g.* C₆-C₃₀) arylene group, or a substituted or unsubstituted C₁-C₆₀ (*e.g.* C₁-C₂₀) heteroarylene group, but embodiments of the present disclosure are not limited thereto.

For example, in Formulae 4-1 to 4-14, R₁₁ may be a single bond, a substituted or unsubstituted C₁-C₂₀ alkylene group, or a substituted or unsubstituted C₆-C₆₀ (*e.g.* C₆-C₃₀) arylene group.

For example, in Formulae 4-1 to 4-14, R₁₁ may be a single bond, a methylene group, or a phenylene group.

For example, in Formulae 4-1 to 4-14, R₁₁ may be a single bond or a methylene group.

For example, in Formulae 4-1 to 4-14, R₁₁ may be a methylene group.

In some embodiments, one or more termini (e.g., ends) of the cross-linkable group may be linked to form a cyclobutane group.

In some embodiments, the cross-linkable arylamine-based compound may be selected from Compounds 1 to 40:

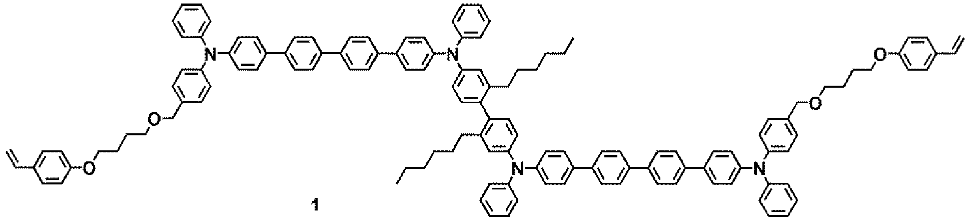

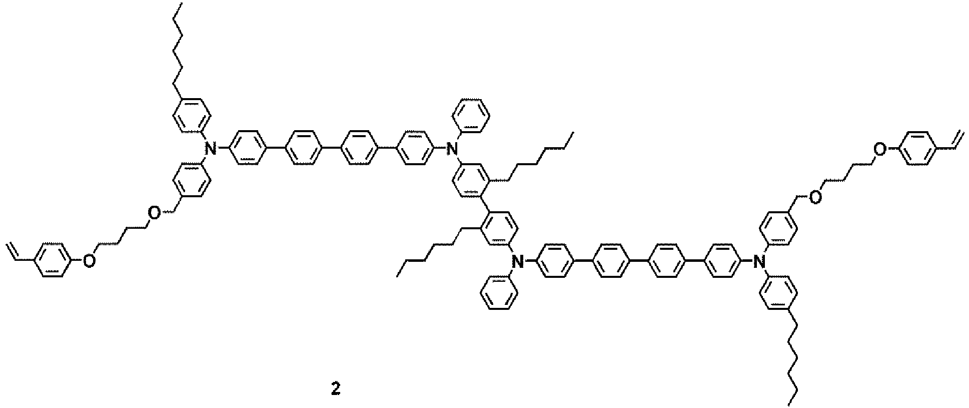

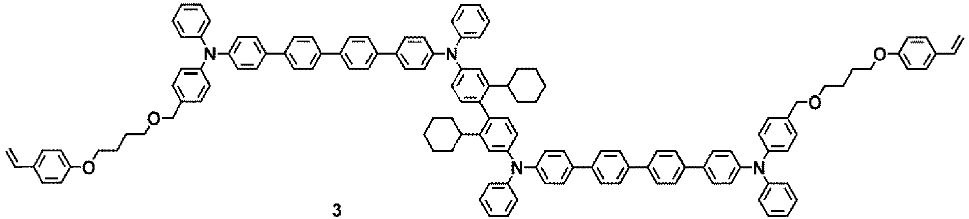

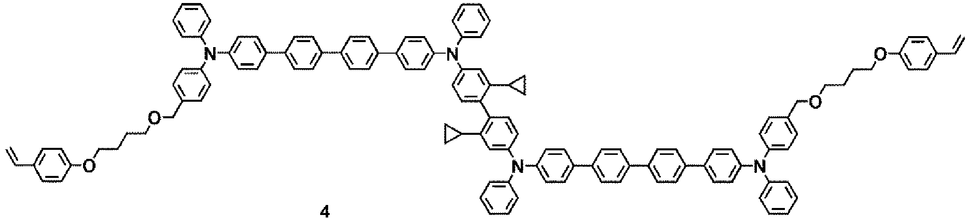

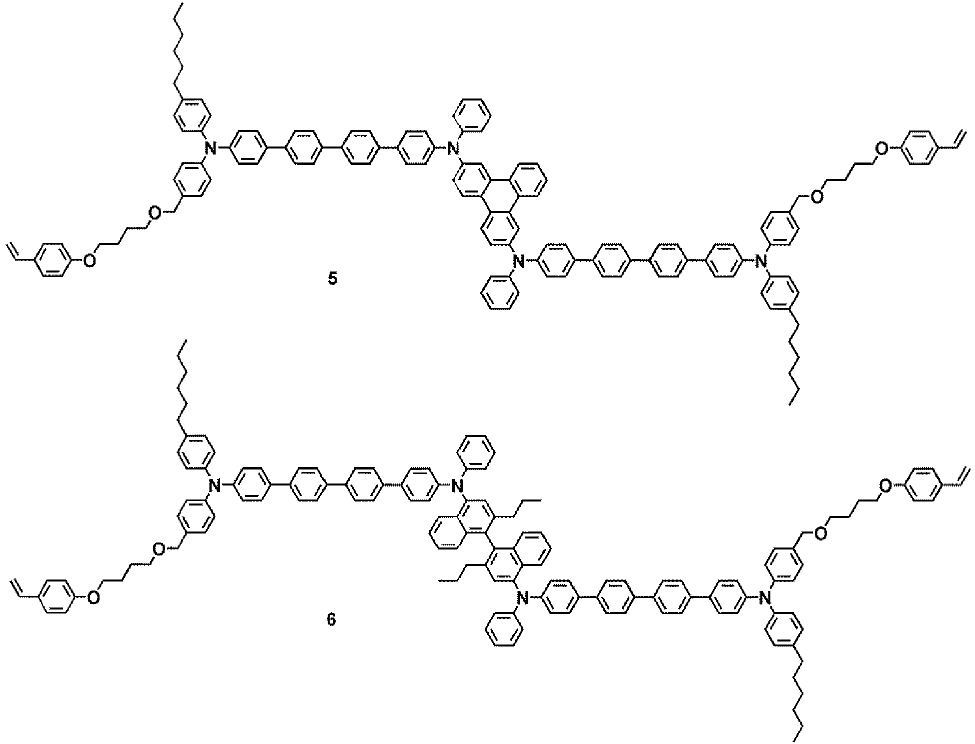

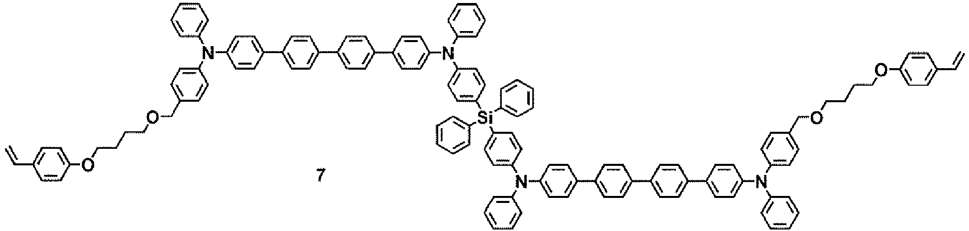

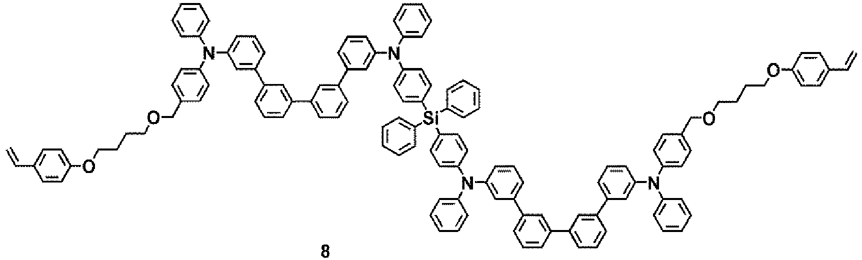

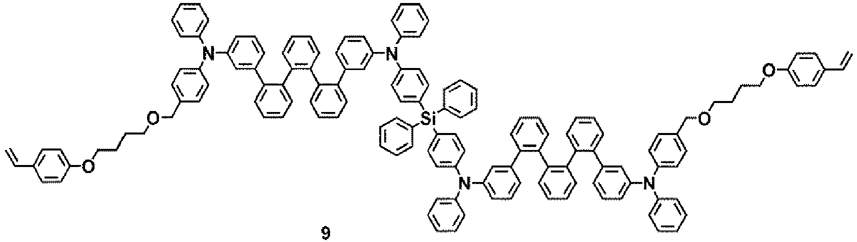

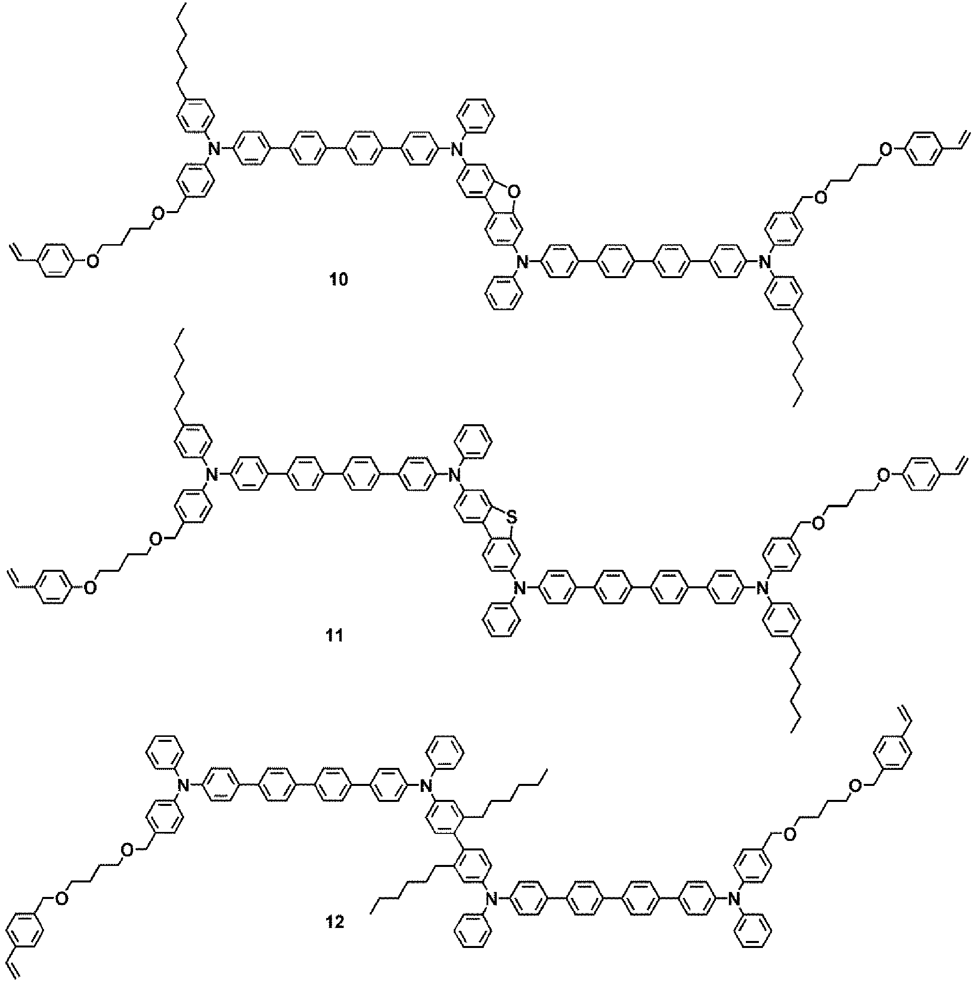

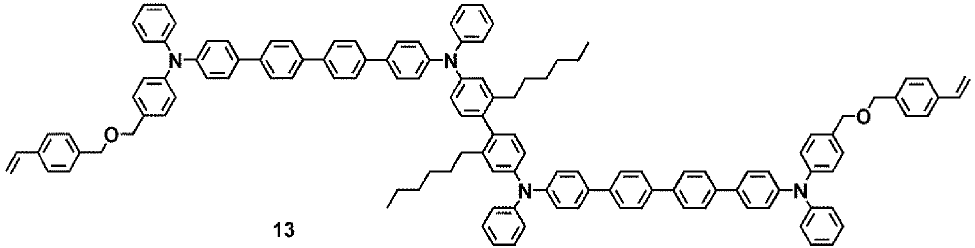

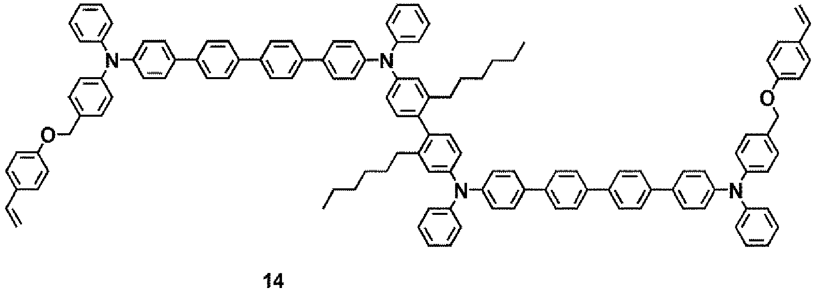

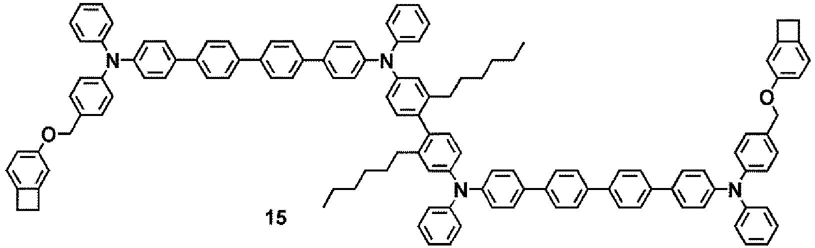

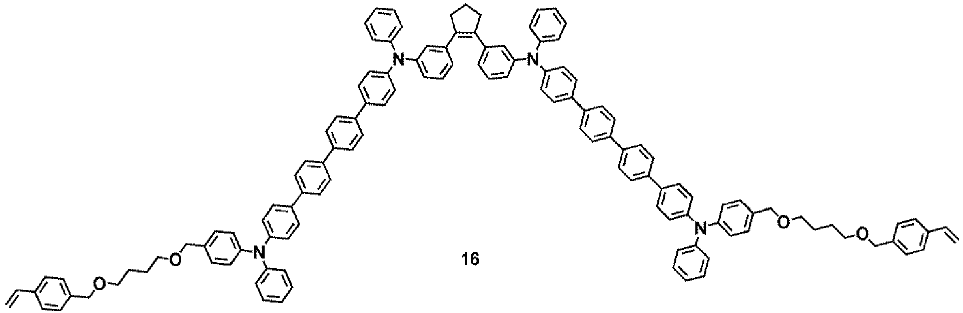

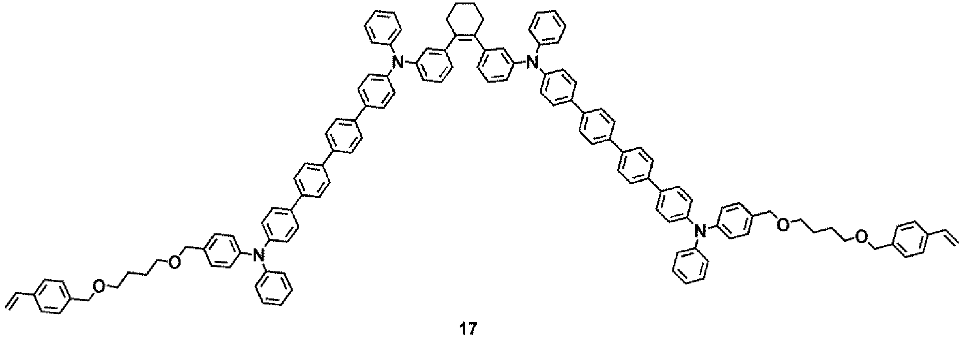

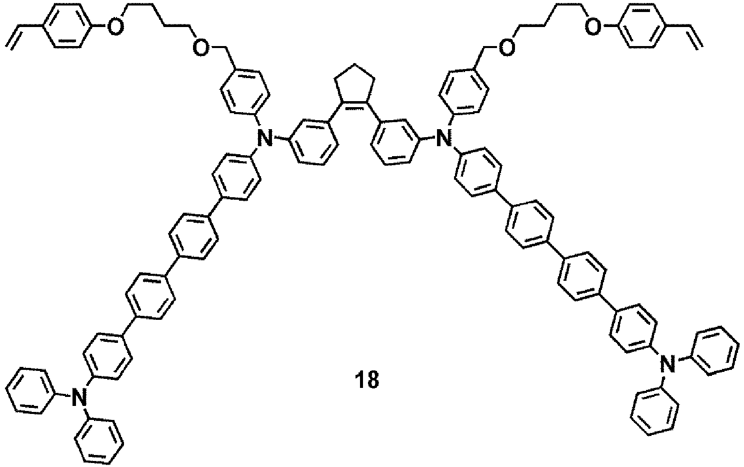

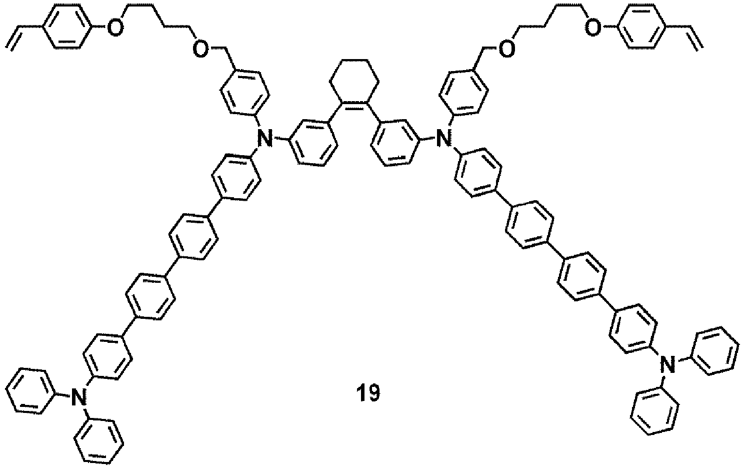

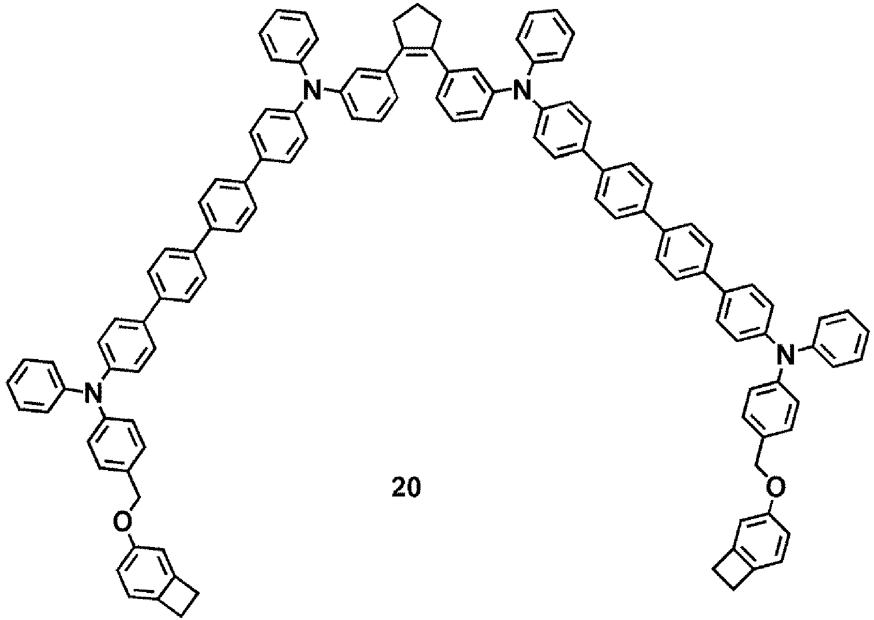

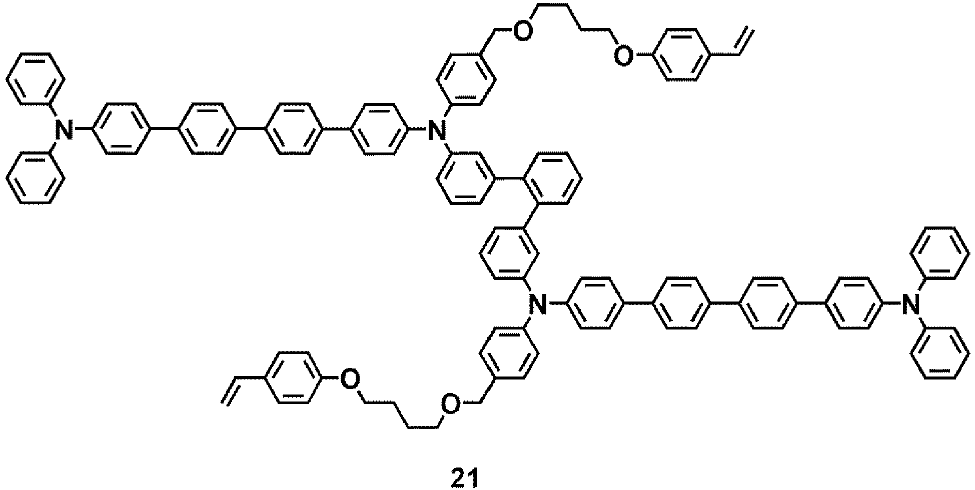

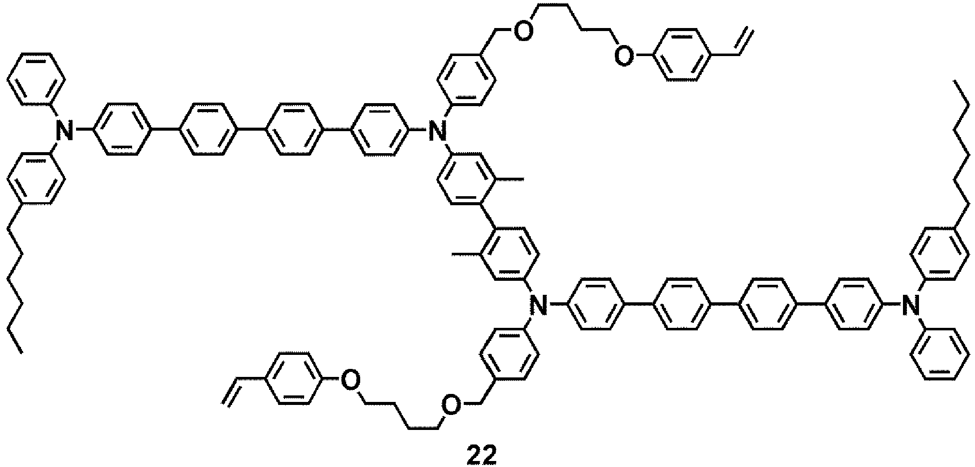

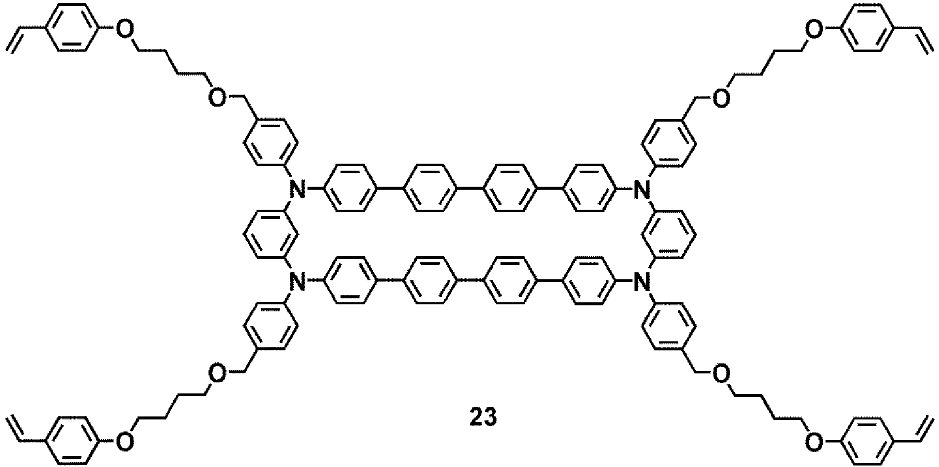

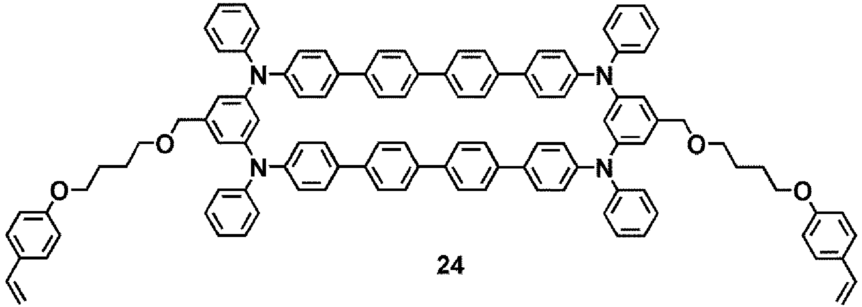

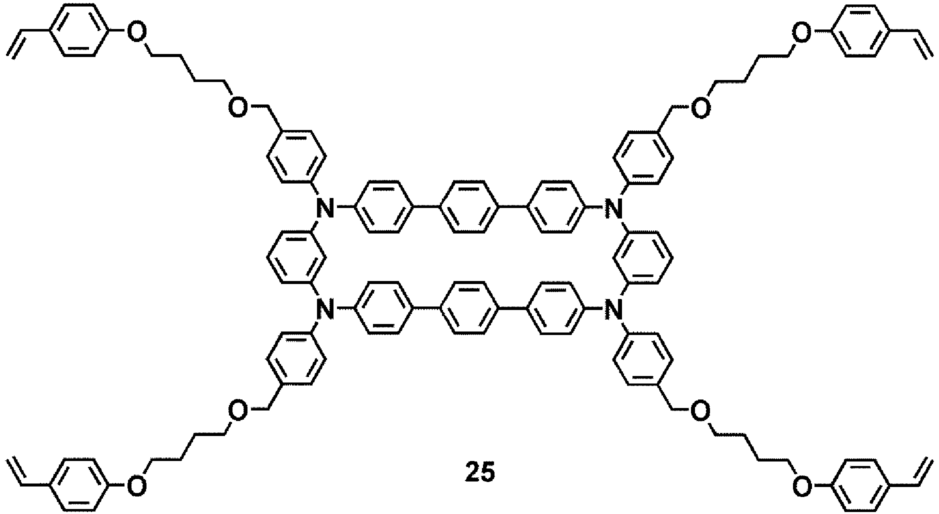

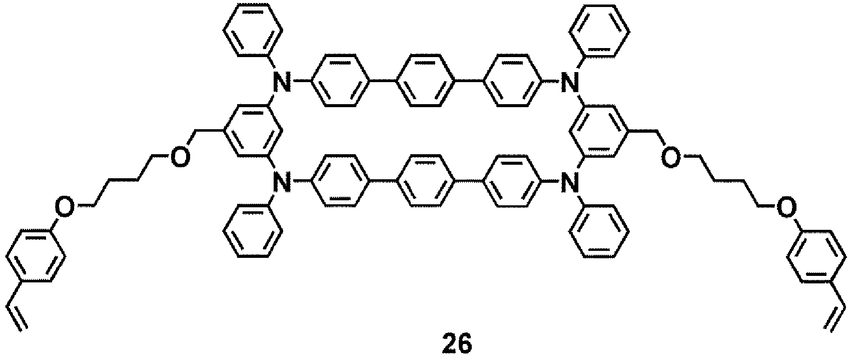

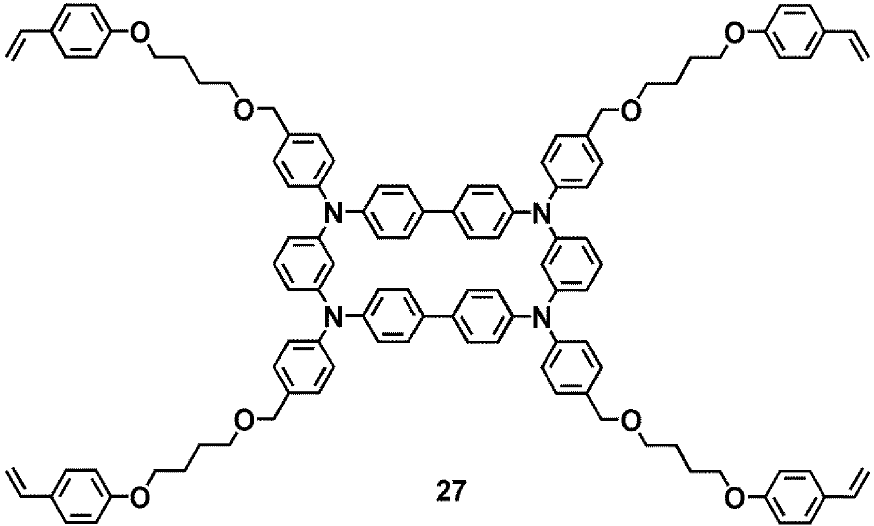

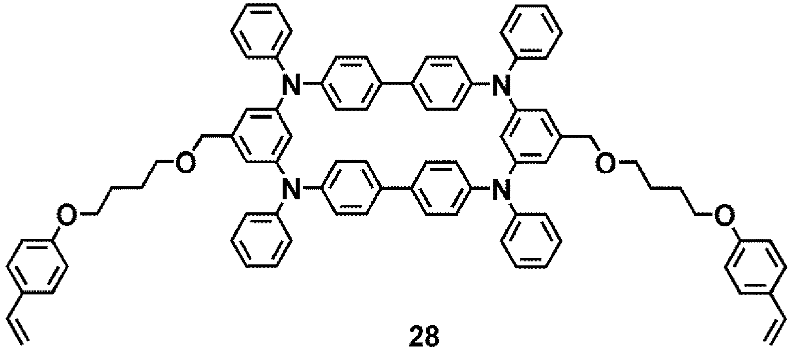

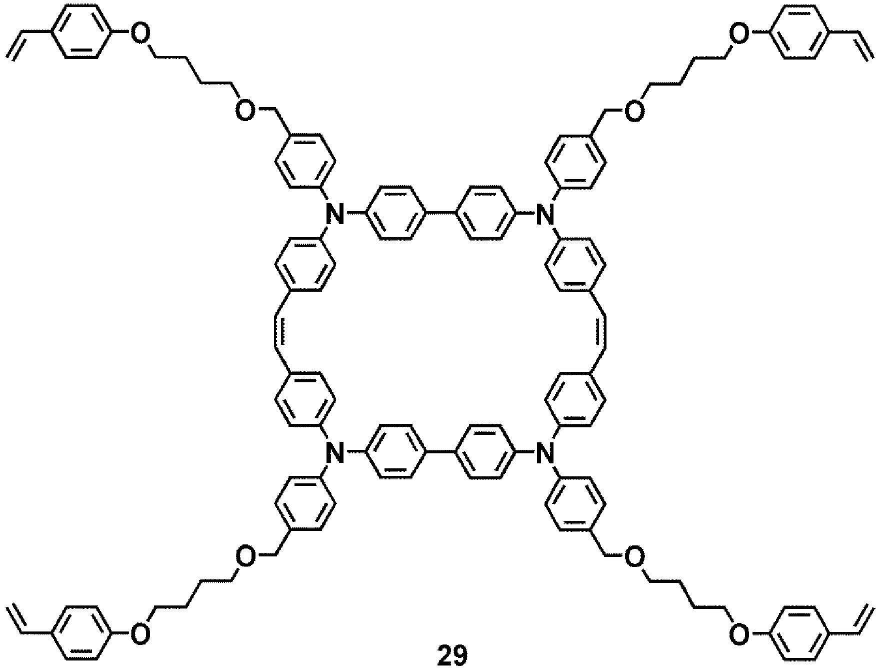

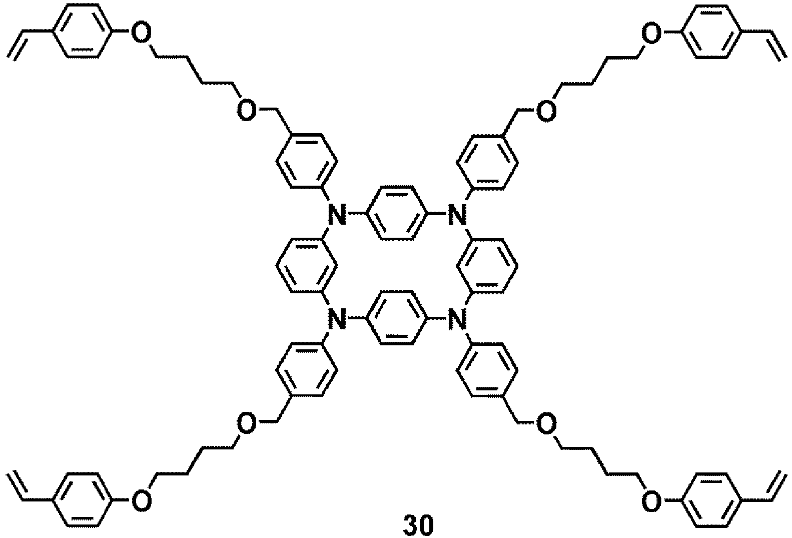

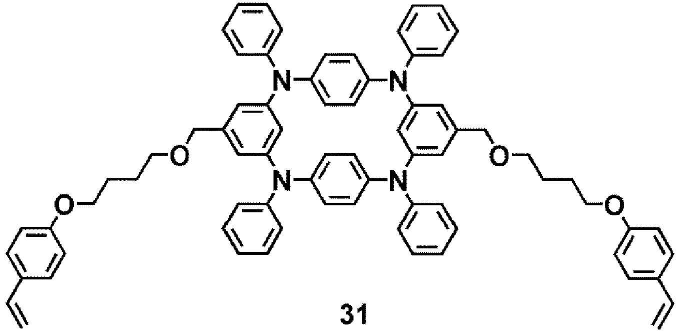

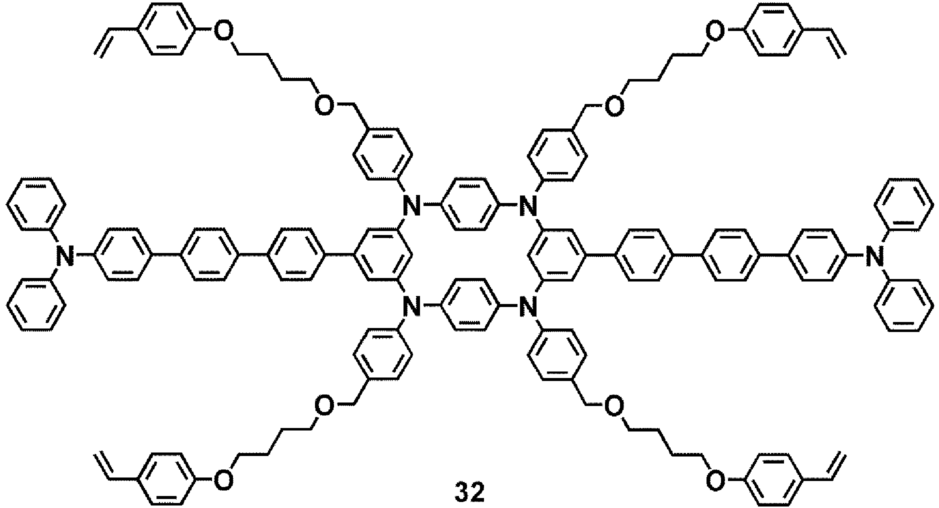

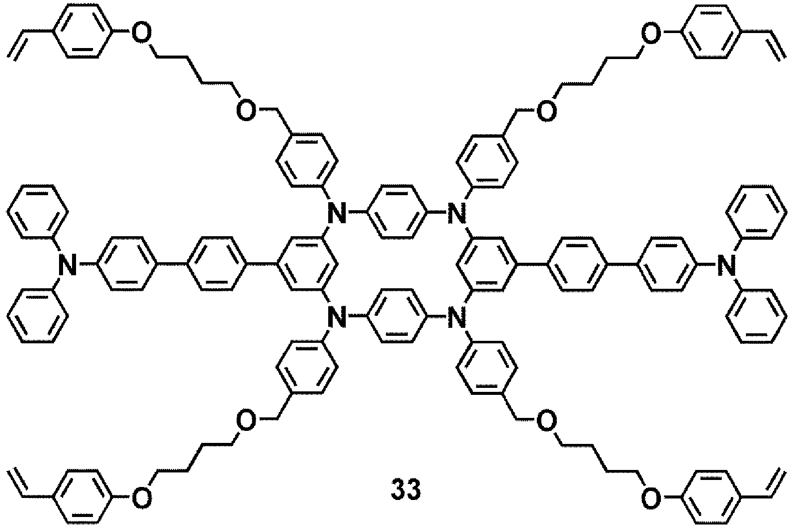

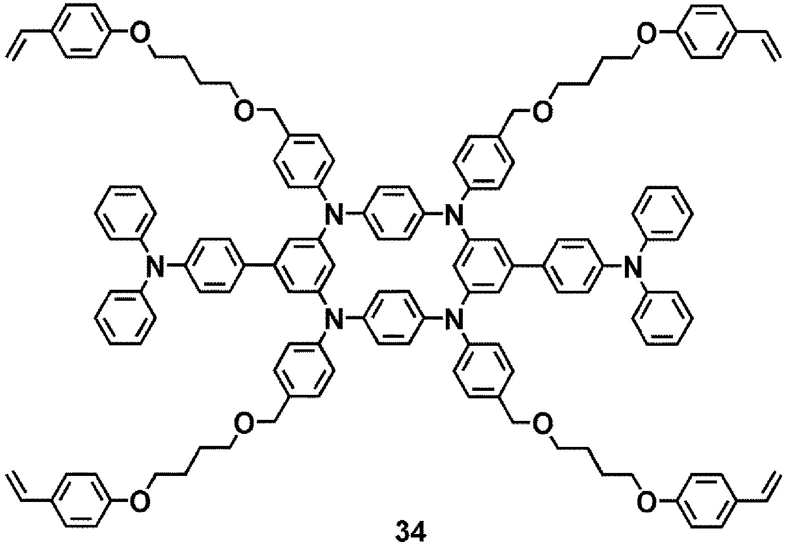

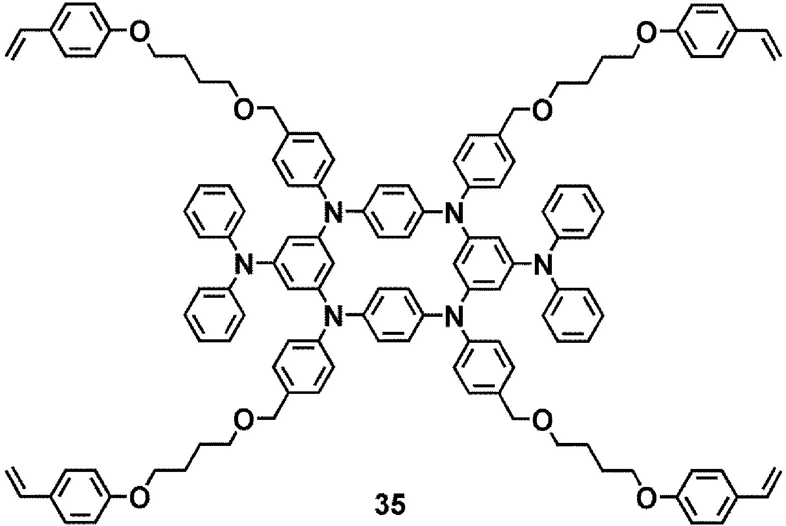

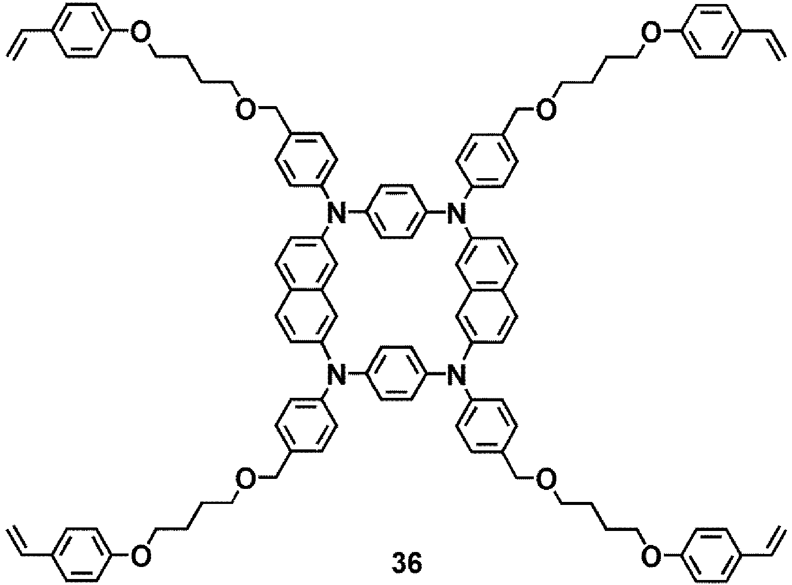

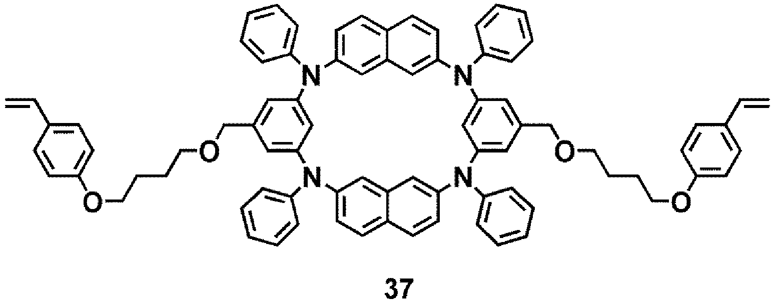

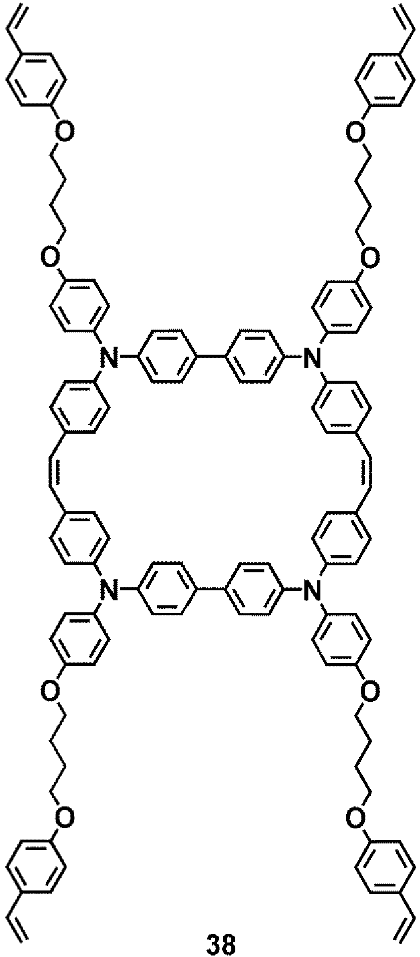

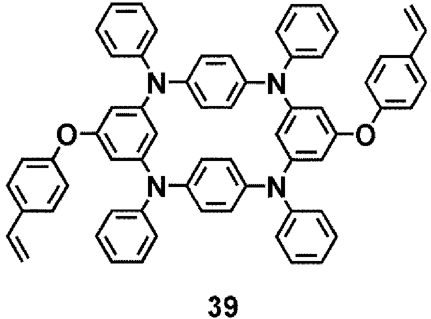

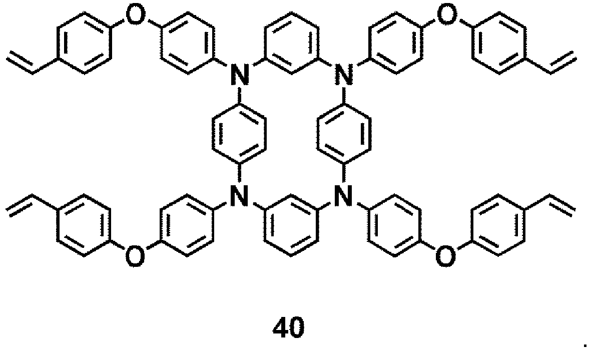

In the cross-linkable arylamine-based compound represented by Formula 1a, two diamine groups are linked via a linker "(B)ₚ" such as a carbocyclic group, a heterocyclic group, or a silane group. The compound structure is conducive to the formation of a structurally amorphous thin film.

In addition, when the cross-linkable arylamine-based compound represented by Formula 1a includes four or more aromatic rings between two arylamine groups, charge mobility may be improved and driving voltage may be reduced, thereby facilitating the manufacture of an OLED having a low driving voltage.

In addition, the cross-linkable arylamine-based compound represented by Formula 1b has a ring shape. When a hole transport layer is formed from a compound including this feature, charge mobility may be improved and driving voltage may be reduced, thereby facilitating the manufacture of an OLED having a low driving voltage.

In some embodiments, the cross-linkable arylamine-based compound may be cross-linked to form an arylamine-based polymer.

In some embodiments, the arylamine-based polymer may have a weight average molecular weight of about 3,000 g/mol or more. The polymer may include an oligomer condensed with two cross-linkable arylamine-based compounds or a polymer condensed with two or more cross-linkable arylamine-based compounds.

The arylamine-based polymer includes a repeating unit represented by Formula 11, and/or may be formed by cross-linking the cross-linkable arylamine-based compound represented by Formula 1b:

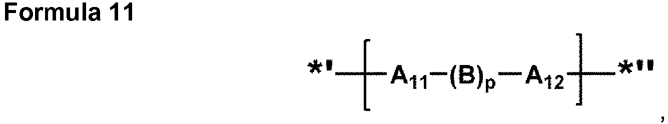

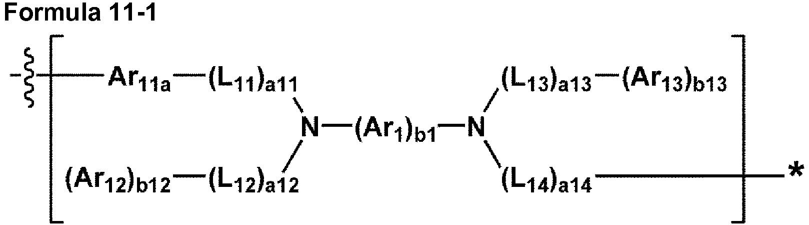

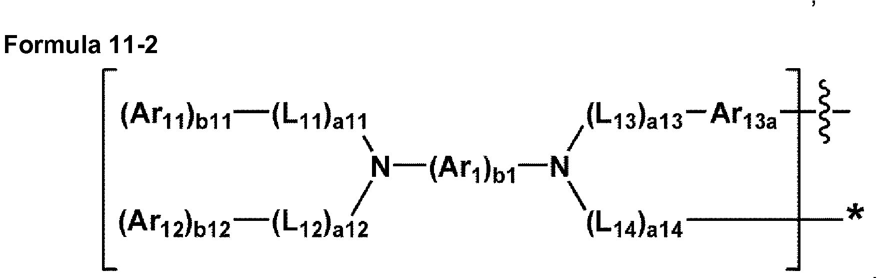

In Formula 11, A₁₁ and A₁₂ are each independently selected from groups represented by Formulae 11-1 and 11-2, and A₁₁ and A₁₂ are identical to or different from each other,
in Formula 11-1, * indicates a binding site to (B)ₚ,
in Formulae 11, 11-1, and 11-2, *', *", and - 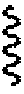 - each indicate a binding site to a neighboring repeating unit,
in Formulae 11-1 and 11-2, Ar₁₁ₐ and Ar₁₃ₐ are each a residue formed after cross-linking, and the repeating units are linked by a divalent cyclobutane group,
in Formula 11, B and p are each independently the same as described above,
in Formulae 11-1 and 11-2, L₁₁ to L₁₄, a11 to a14, b11 to b13, Ar₁, and b1 are each independently be the same as described above, and
in Formulae 11-1 and 11-2, Ar₁₁ to Ar₁₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group.

The term "residue formed after cross-linking", as used herein, refers to a residue formed after a cross-linkable group included in one repeating unit is linked to a cross-linkable group included in another repeating unit.

In some embodiments, the arylamine-based polymer may be formed by linking at least two types or kinds of repeating units, and cross-linking groups (for example, an ethylene group) at one or more termini (ends) of the repeating unit may react with each other to form a ring-type (ring structure) linker, for example, a cyclobutane group. For example, the repeating units may be linked by a divalent cyclobutane group.

In some embodiments, the arylamine-based polymer may be formed by cross-linking between monomers or oligomers of the cross-linkable arylamine-based compounds. The arylamine-based polymer may be a polymer in which the cross-linkable arylamine-based compound is radially or linearly cross-linked. In some embodiments, cross-linking groups (for example, an ethylene group) at one or more termini (ends) of the cross-linkable arylamine-based compound may react with each other to form a ring-type (ring structure) linker, for example, a cyclobutane group.

In Formulae 11-1 and 11-2, Ar₁₁ₐ and Ar₁₃ₐ may each independently be selected from groups represented by Formulae 5-1 to 5-8, but embodiments of the present disclosure are not limited thereto:

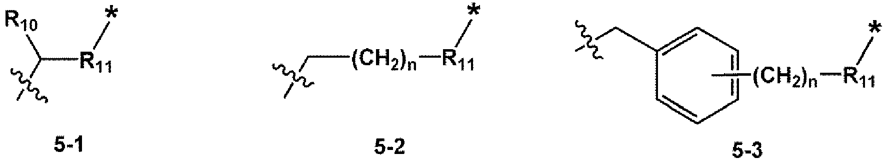

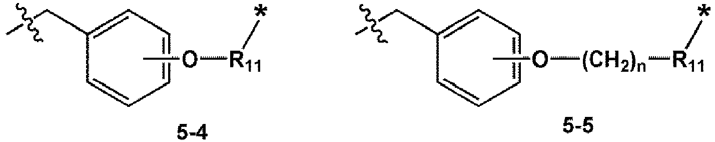

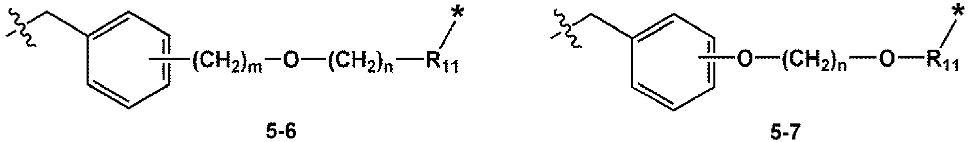

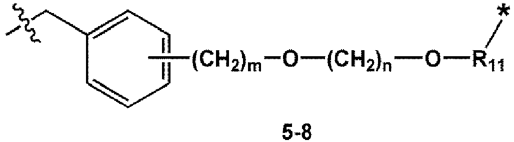

In Formulae 5-1 to 5-8,
R₁₀ is a hydrogen atom or a substituted or unsubstituted C₁-C₂₀ alkyl group,
R₁₁ is selected from a single bond, a substituted or unsubstituted C₁-C₂₀ alkyl group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (*e.g.* C₆-C₃₀) arylene group, a substituted or unsubstituted C₁-C₆₀ (*e.g.* C₁-C₂₀) heteroarylene group, a substituted or unsubstituted divalent non-aromatic condensed polycyclic group, and a substituted or unsubstituted divalent non-aromatic condensed heteropolycyclic group, and
m and n are each independently an integer of 1 to 10, * indicates a binding site to a neighboring atom, and - 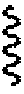 - indicates a binding site to a neighboring repeating unit.

In some embodiments, in Formulae 5-1 to 5-8, R₁₁ may be a single bond, a substituted or unsubstituted C₁-C₂₀ alkyl group, a substituted or unsubstituted C₆-C₆₀ (*e.g.* C₆-C₃₀) arylene group, or a substituted or unsubstituted C₁-C₆₀ (*e.g.* C₁-C₂₀) heteroarylene group, but embodiments of the present disclosure are not limited thereto.

For example, in Formulae 5-1 to 5-8, R₁₁ may be a single bond, a substituted or unsubstituted C₁-C₂₀ alkyl group, or a substituted or unsubstituted C₆-C₆₀ (*e.g.* C₆-C₃₀) arylene group.

For example, in Formulae 5-1 to 5-8, R₁₁ may be a single bond, a methylene group, or a phenylene group.

For example, in Formulae 5-1 to 5-8, R₁₁ may be a single bond or a methylene group.

For example, in Formulae 5-1 to 5-8, R₁₁ may be a methylene group.

In Formulae 11-1 and 11-2, Ar₁ may be a benzene group, and b1 may be 4 or 5.

Suitable synthesis methods for obtaining the arylamine-based polymer obtained from the cross-linkable arylamine-based compounds represented by Formulae 1a and 1b and/or the arylamine-based polymer represented by Formula 11 may be easily recognized by those of skilled in the art, for example, by referring to the Examples provided below.

According to one or more embodiments of the present disclosure, an organic light-emitting device includes: a first electrode; a second electrode facing the first electrode; and an organic layer between the first electrode and the second electrode and including an emission layer, wherein the organic layer includes at least one of the arylamine-based polymer.

As used herein, the expression "(an organic layer) includes at least one of the arylamine-based polymer" may include a case in which "(an organic layer) includes one identical (e.g., only one type or kind of) arylamine-based polymer represented by Formula 11", in addition to a case in which "(an organic layer) includes two or more different (types or kinds of) arylamine-based polymers represented by Formula 11".

For example, the organic layer may be formed by cross-linking Compound 1. In this case, Compound 1 may be cross-linked to the emission layer of the organic light-emitting device. In some embodiments, the organic layer may include Compound 1 and Compound 2 cross-linked to each other. In this case, Compound 1 and Compound 2 may be cross-linked to the same layer (for example, both Compound 1 and Compound 2 may be (simultaneously) cross-linked to the emission layer). Alternatively, Compound 1 and Compound 2 may each be cross-linked to different layers (for example, Compound 1 may be cross-linked to the emission layer and Compound 2 may be cross-linked to the electron transport region).

For example, in the organic layer, Compound 23 may be radially or linearly cross-linked. In this case, Compound 23 may be cross-linked to the emission layer of the organic light-emitting device. In some embodiments, the organic layer may include Compound 23 and Compound 24 cross-linked to each other, or may include Compound 23 and Compound 24 cross-linked at the same time (e.g., separately and simultaneously). In some embodiments, Compound 23 and Compound 24 may be cross-linked to the same layer (for example, both Compound 23 and Compound 24 may be (simultaneously) cross-linked to the emission layer). Alternatively, Compound 23 and Compound 24 may each be cross-linked to different layers (for example, Compound 23 may be cross-linked to the emission layer and Compound 24 may be cross-linked to the electron transport region).

In some embodiments,
the first electrode of the organic light-emitting device may be an anode,
the second electrode of the organic light-emitting device may be a cathode, and
the organic layer may include: i) a hole transport region between the first electrode and the emission layer and including a hole injection layer, a hole transport layer, a buffer layer, an electron blocking layer, or any combination thereof; and ii) an electron transport region between the emission layer and the second electrode and including a hole blocking layer, an electron transport layer, an electron injection layer, or any combination thereof.

In some embodiments, the hole transport region may include at least one of the arylamine-based polymer.

For example, the hole transport region may include a hole injection layer and a hole transport layer, and the hole transport layer may include at least one of the arylamine-based polymer.

The term "organic layer", as used herein, may refer to a single layer and/or a plurality of layers between the first electrode and the second electrode of the organic light-emitting device. The material included in the "organic layer" is not limited to being an organic material.

### Description of FIG. 1

FIG. 1 is a schematic view of an organic light-emitting device 10 according to an embodiment of the present disclosure. The organic light-emitting device 10 includes a first electrode 110, an organic layer 150, and a second electrode 190.

Hereinafter, the structure of the organic light-emitting device 10 according to an embodiment of the present disclosure and a method of manufacturing the organic light-emitting device 10 will be described in connection with FIG. 1.

### First electrode 110

In FIG. 1, a substrate may be additionally under the first electrode 110 or above the second electrode 190. The substrate may be a glass substrate and/or a plastic substrate, each having excellent mechanical strength, thermal stability, transparency, surface smoothness, ease of handling, and/or water resistance.

The first electrode 110 may be formed by depositing and/or sputtering a material for forming the first electrode 110 on the substrate. When the first electrode 110 is an anode, the material for a first electrode may be selected from materials with a high work function to facilitate hole injection.

The first electrode 110 may be a reflective electrode, a semi-transmissive electrode, or a transmissive electrode. When the first electrode 110 is a transmissive electrode, the material for forming the first electrode may be selected from indium tin oxide (ITO), indium zinc oxide (IZO), tin oxide (SnO₂), zinc oxide (ZnO), and combinations thereof, but embodiments of the present disclosure are not limited thereto. In some embodiments, when the first electrode 110 is a semi-transmissive electrode or a reflective electrode, the material for forming the first electrode may be selected from magnesium (Mg), silver (Ag), aluminium (Al), aluminium-lithium (Al-Li), calcium (Ca), magnesium-indium (Mg-In), magnesium-silver (Mg-Ag), and combinations thereof, but embodiments of the present disclosure are not limited thereto.

The first electrode 110 may have a single-layered structure, or a multi-layered structure including two or more layers. For example, the first electrode 110 may have a three-layered structure of ITO/Ag/ITO, but embodiments of the structure of the first electrode 110 are not limited thereto.

### Organic layer 150

The organic layer 150 is on the first electrode 110. The organic layer 150 may include an emission layer.

The organic layer 150 may further include a hole transport region between the first electrode 110 and the emission layer, and/or an electron transport region between the emission layer and the second electrode 190.

### Hole transport region in organic layer 150

The hole transport region may have: i) a single-layered structure including a single layer including a single material; ii) a single-layered structure including a single layer including a plurality of different materials; or iii) a multi-layered structure including a plurality of layers including a plurality of different materials.

The hole transport region may include at least one layer selected from a hole injection layer, a hole transport layer, an emission auxiliary layer, and an electron blocking layer.

In some embodiments, for example, the hole transport region may have a single-layered structure including a single layer including a plurality of different materials, or a multi-layered structure having a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron blocking layer structure, wherein the constituting layers of each structure are sequentially stacked from the first electrode 110 in the stated order, but embodiments of the structure of the hole transport region are not limited thereto.

The hole transport region may include the compound represented by Formula 1a or 1b, the arylamine-based polymer represented by Formula 11 (which is a cross-linked product of the compound represented by Formula 1a or 1b), or a cross-linked product of the compound represented by Formula 1b.

A thickness of the hole transport region may be about 100 Å to about 10,000 Å, for example, about 100 Å to about 7000 Å, about 100 Å to about 5000 Å, about 100 Å to about 3000 Å, or about 100 Å to about 1,000 Å. When the hole transport region includes at least one selected from a hole injection layer and a hole transport layer, the thickness of the hole injection layer may be about 100 Å to about 9,000 Å, for example, about 100 Å to about 7,000 Å, about 100 Å to about 5,000 Å, about 100 Å to about 3,000 Å, about 100 Å to about 2,000 Å or about 100 Å to about 1,000 Å, and the thickness of the hole transport layer may be about 50 Å to about 2,000 Å, for example, about 100 Å to about 1500 Å. When the thicknesses of the hole transport region, the hole injection layer, and the hole transport layer are within these ranges, satisfactory hole transporting characteristics may be obtained without a substantial increase in driving voltage.

The emission auxiliary layer may increase light-emission efficiency by compensating for an optical resonance distance according to the wavelength of light emitted by an emission layer (e.g., be used to adjust the optical resonance distance to match the wavelength of light emitted from the emission layer). Further, the electron blocking layer may block the flow of electrons from an electron transport region. The emission auxiliary layer and the electron blocking layer may include the same materials as described above.

### p-dopant

The hole transport region may further include, in addition to the above materials, a charge-generation material for the improvement of conductive properties. The charge-generation material may be homogeneously or non-homogeneously dispersed in the hole transport region.

In some embodiments, the charge-generation material may be a p-dopant.

In some embodiments, the p-dopant may have a lowest unoccupied molecular orbital (LUMO) energy level of less than -3.5 eV.

The p-dopant may include at least one selected from a quinone derivative, a metal oxide, and a cyano group-containing compound, but embodiments of the present disclosure are not limited thereto.

For example, the p-dopant may include at least one selected from:
a quinone derivative (such as tetracyanoquinodimethane (TCNQ) and/or 2,3,5,6-tetrafluoro-7,7,8,8-tetracyanoquinodimethane (F4-TCNQ));
a metal oxide (such as tungsten oxide and/or molybdenum oxide);
1,4,5,8,9,12-hexaazatriphenylene-hexacarbonitrile (HAT-CN); and
a compound represented by Formula 221,
but embodiments of the present disclosure are not limited thereto:

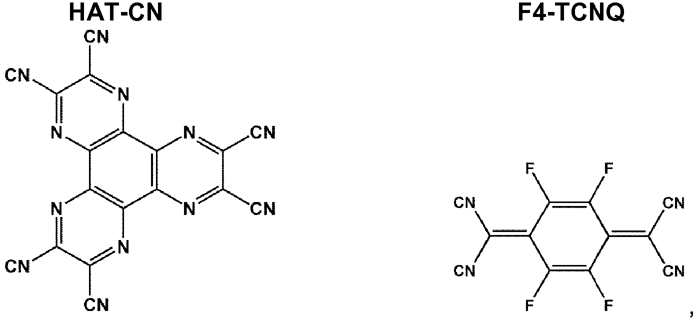

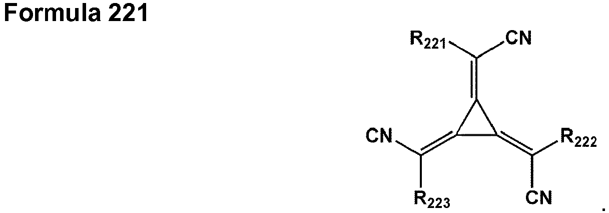

In Formula 221,
R₂₂₁ to R₂₂₃ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g.* C₆-C₃₂) aryl group, a substituted or unsubstituted C₁-C₆₀ (*e.g.* C₁-C₂₀) heteroaryl group, a substituted or unsubstituted monovalent non-aromatic condensed polycyclic group, and a substituted or unsubstituted monovalent non-aromatic condensed heteropolycyclic group, provided that at least one selected from R₂₂₁ to R₂₂₃ has at least one substituent selected from a cyano group, -F, -Cl, -Br, -I, a C₁-C₂₀ alkyl group substituted with -F, a C₁-C₂₀ alkyl group substituted with -Cl, a C₁-C₂₀ alkyl group substituted with -Br, and a C₁-C₂₀ alkyl group substituted with -I.

### Emission layer in organic layer 150

When the organic light-emitting device 10 is a full-color organic light-emitting device, the emission layer may be patterned into a red emission layer, a green emission layer, or a blue emission layer, according to a sub-pixel. In some embodiments, the emission layer may have a stacked structure of two or more layers selected from a red emission layer, a green emission layer, and a blue emission layer, in which the two or more layers may contact each other or may be separated from each other. In some embodiments, the emission layer may include two or more materials selected from a red light-emitting material, a green light-emitting material, and a blue light-emitting material, in which the two or more materials are mixed with each other in a single layer to emit white light.

The emission layer may include a host and a dopant. The dopant may include at least one selected from a phosphorescent dopant and a fluorescent dopant.

In the emission layer, an amount of the dopant material may be about 0.01 parts by weight to about 15 parts by weight based on 100 parts by weight of the host material, but embodiments of the present disclosure are not limited thereto.

A thickness of the emission layer may be about 100 Å to about 1,000 Å, for example, about 100 Å to about 800 Å, about 150 Å to about 800 Å, about 150 Å to about 700 Å, about 150 Å to about 650 Å, about 200 Å to about 600 Å or about 300 Å to about 400 Å. When the thickness of the emission layer is within this range, excellent light-emission characteristics may be obtained without a substantial increase in driving voltage.

### Host in emission layer

In some embodiments, the host may include a compound represented by Formula 301:

**Formula 301** [Ar₃₀₁]_{xb11}-[(L₃₀₁)_{xb1}-R₃₀₁]_{xb21}.

In Formula 301,
Ar₃₀₁ may be a substituted or unsubstituted C₅-C₆₀ carbocyclic group or a substituted or unsubstituted C₁-C₆₀ heterocyclic group,
xb11 may be 1, 2, or 3,
L₃₀₁ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group;
xb1 may be an integer from 0 to 5,
R₃₀₁ may be selected from deuterium, -F, -Cl, -Br, -I, a hydroxyl group, a cyano group, a nitro group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), and - P(=O)(Q₃₀₁)(Q₃₀₂),
xb21 may be an integer from 1 to 5, and
Q₃₀₁ to Q₃₀₃ may each independently be selected from a C₁-C₁₀ alkyl group, a C₁-C₁₀ alkoxy group, a phenyl group, a biphenyl group, a terphenyl group, and a naphthyl group, but embodiments of the present disclosure are not limited thereto.

For example, Ar₃₀₁ may be a substituted or unsubstituted C₆-C₃₂ carbocyclic group or a substituted or unsubstituted C₁-C₁₂ heterocyclic group, but embodiments are not limited thereto.

In some embodiments, Ar₃₀₁ in Formula 301 may be selected from:
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group; and
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group, each substituted with at least one selected from deuterium, - F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, - Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and - P(=O)(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group, but embodiments of the present disclosure are not limited thereto.

In Formula 301, when xb11 is two or more, two or more of Ar₃₀₁(s) may be linked via a single bond.

In some embodiments, the compound represented by Formula 301 may be represented by Formula 301-1 or 301-2:

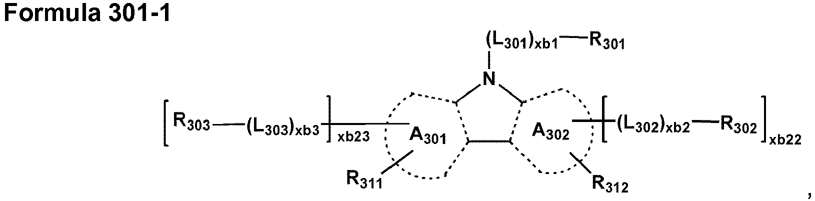

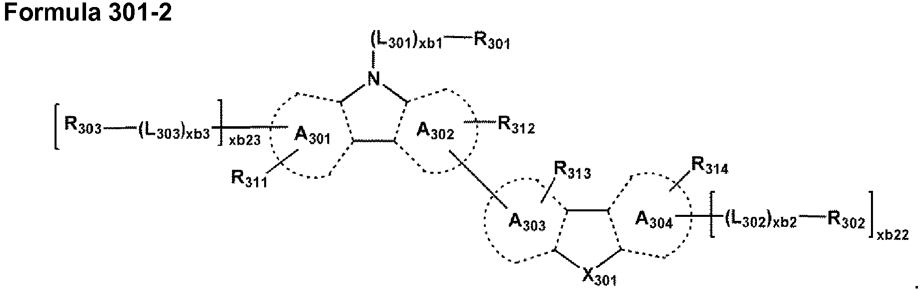

In Formulae 301-1 and 301-2,
A₃₀₁ to A₃₀₄ may each independently be selected from a benzene, a naphthalene, a phenanthrene, a fluoranthene, a triphenylene, a pyrene, a chrysene, a pyridine, a pyrimidine, an indene, a fluorene, a spiro-bifluorene, a benzofluorene, a dibenzofluorene, an indole, a carbazole, a benzocarbazole, a dibenzocarbazole, a furan, a benzofuran, a dibenzofuran, a naphthofuran, a benzonaphthofuran, a dinaphthofuran, a thiophene, a benzothiophene, a dibenzothiophene, a naphthothiophene, a benzonaphthothiophene, and a dinaphthothiophene,
X₃₀₁ may be O, S, or N-[(L₃₀₄)_{xb4}-R₃₀₄],
R₃₁₁ to R₃₁₄ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
xb22 and xb23 may each independently be 0, 1, or 2,
L₃₀₁, xb1, R₃₀₁, and Q₃₁ to Q₃₃ may each independently be the same as described above,
L₃₀₂ to L₃₀₄ may each independently be the same as described in connection with L₃₀₁,
xb2 to xb4 may each independently be the same as described in connection with xb1, and
R₃₀₂ to R₃₀₄ may each independently be the same as described in connection with R₃₀₁.

For example, L₃₀₁ to L₃₀₄ in Formulae 301, 301-1, and 301-2 may each independently be selected from: a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₃₀ arylene group, a substituted or unsubstituted C₁-C₂₀ heteroarylene group, a substituted or unsubstituted divalent non-aromatic condensed polycyclic group, and a substituted or unsubstituted divalent non-aromatic condensed heteropolycyclic group, but embodiments are not limited thereto.

For example, in Formulae 301, 301-1, and 301-2, L₃₀₁ to L₃₀₄ may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), - B(Q₃₁)(Q₃₂), -C(=O)(Q₃₁), -S(=O)₂(Q₃₁), and -P(=O)(Q₃₁)(Q₃₂), and
Q₃₁ and Q₃₃ may each independently be the same as described above.

As another example, R₃₀₁ to R₃₀₄ in Formulae 301, 301-1, and 301-2 may each independently be selected from: deuterium, -F, -Cl, -Br, -I, a hydroxyl group, a cyano group, a nitro group, an amidino group, a hydrazino group, a hydrazono group, a substituted or unsubstituted C₁-C₆₀ (*e.g.* C₁-C₁₀) alkyl group, a substituted or unsubstituted C₂-C₆₀ (*e.g.* C₂-C₁₀) alkenyl group, a substituted or unsubstituted C₂-C₆₀ (*e.g.* C₂-C₁₀) alkynyl group, a substituted or unsubstituted C₁-C₆₀ (*e*.*g*. C₁-C₁₀) alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₃₀ aryl group, a substituted or unsubstituted C₆-C₃₀ aryloxy group, a substituted or unsubstituted C₆-C₃₀ arylthio group, a substituted or unsubstituted C₁-C₂₀ heteroaryl group, a substituted or unsubstituted monovalent non-aromatic condensed polycyclic group, a substituted or unsubstituted monovalent non-aromatic condensed heteropolycyclic group, -Si(Q₃₀₁)(Q₃₀₂)(Q₃₀₃), -N(Q₃₀₁)(Q₃₀₂), - B(Q₃₀₁)(Q₃₀₂), -C(=O)(Q₃₀₁), -S(=O)₂(Q₃₀₁), and -P(=O)(Q₃₀₁)(Q₃₀₂), but embodiments are not limited thereto.

In some embodiments, in Formulae 301, 301-1, and 301-2, R₃₀₁ to R₃₀₄ may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), - B(Q₃₁)(Q₃₂), -C(=O)(Q₃₁), -S(=O)₂(Q₃₁), and -P(=O)(Q₃₁)(Q₃₂), and
Q₃₁ and Q₃₃ may each independently be the same as described above.

In some embodiments, the host may include an alkaline earth metal complex. For example, the host may include a complex selected from a Be complex (for example, Compound H55), a Mg complex, and a Zn complex. For example, the host may be selected from a Be complex (for example, Compound H55) and a Mg complex. In some embodiments, the host may include a zinc (Zn) complex.

The host may include at least one selected from 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), and Compounds H1 to H55, but embodiments of the present disclosure are not limited thereto:

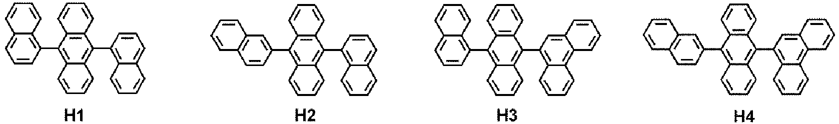

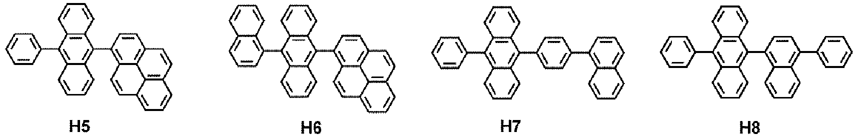

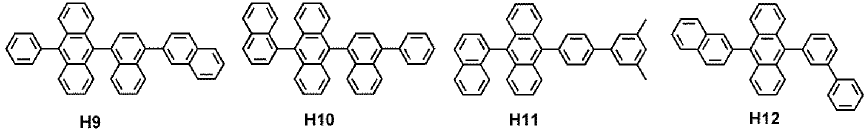

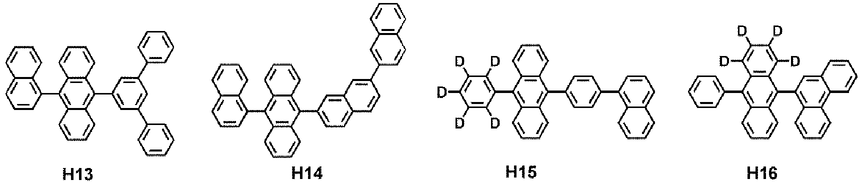

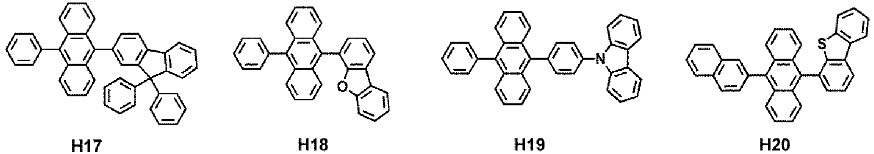

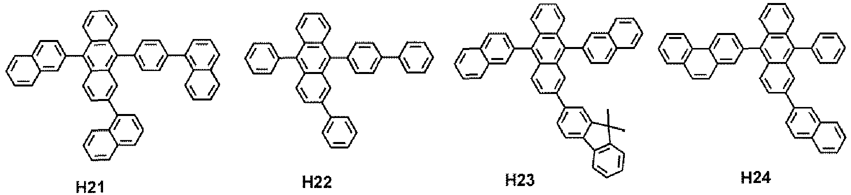

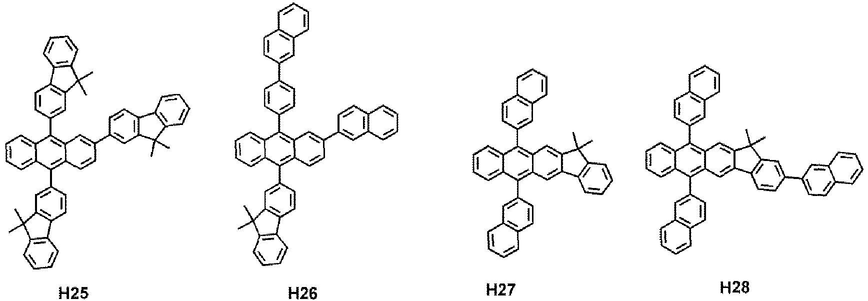

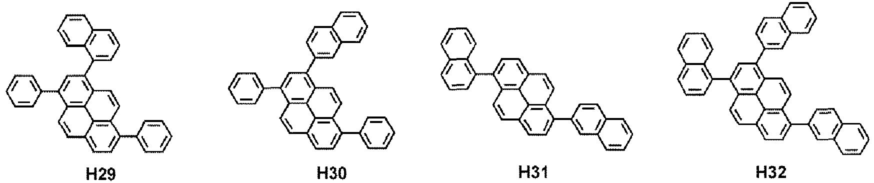

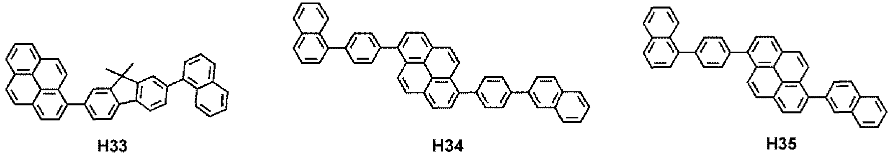

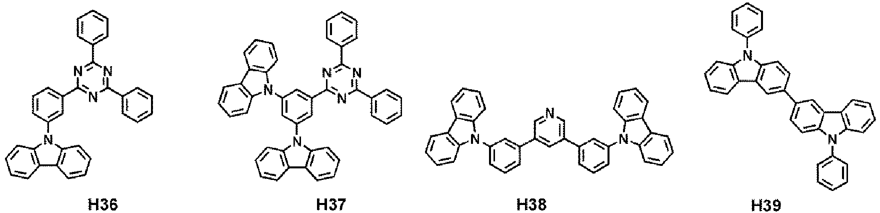

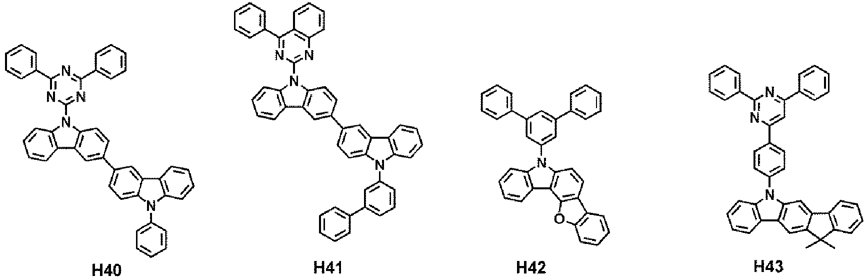

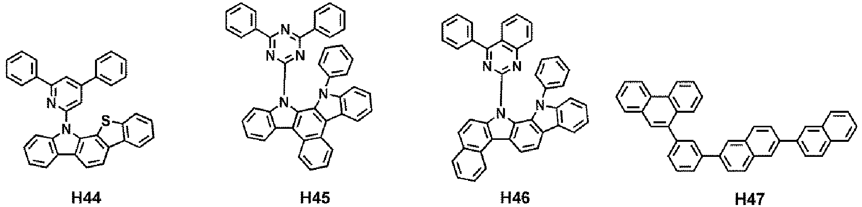

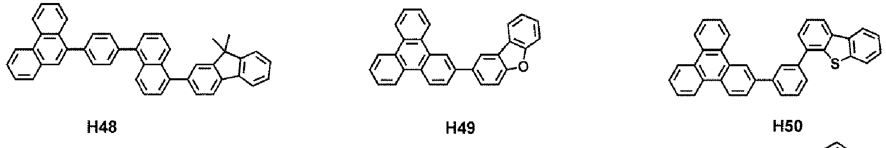

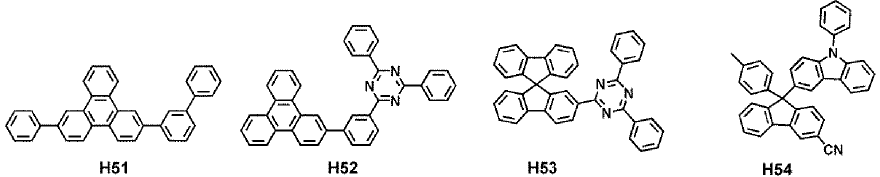

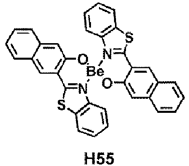

In some embodiments, the host may include at least one selected from a silicon-containing compound (for example, BCPDS, and/or the like) and a phosphine oxide-containing compound (for example, POPCPA, and/or the like).

However, embodiments of the present disclosure are not limited thereto. In some embodiments, the host may include only one compound, or two or more different compounds (for example BCPDS and POPCPA).

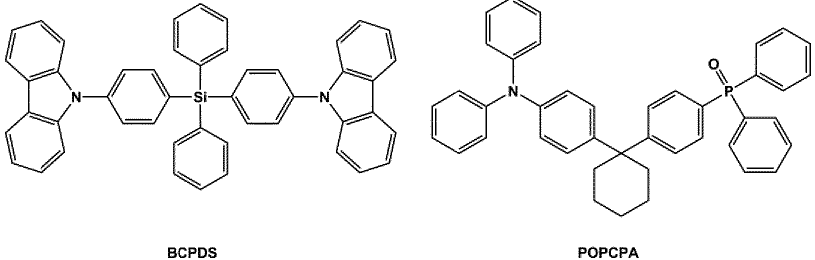

### Phosphorescent dopant included in emission layer in organic layer 150

In some embodiments, the phosphorescent dopant may include an organometallic complex represented by Formula 401:

**Formula 401** M(L₄₀₁)_{xc1}(L₄₀₂)_{xc2},

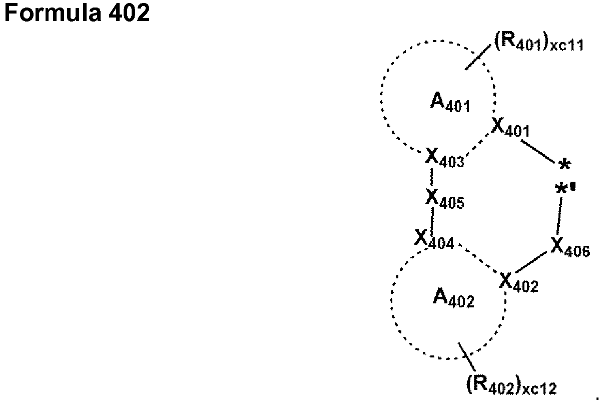

In Formulae 401 and 402,
M may be selected from iridium (Ir), platinum (Pt), palladium (Pd), osmium (Os), titanium (Ti), zirconium (Zr), hafnium (Hf), europium (Eu), terbium (Tb), rhodium (Rh), and thulium (Tm),
L₄₀₁ may be selected from ligands represented by Formula 402, and xc1 may be 1, 2, or 3, wherein, when xc1 is two or more, two or more L₄₀₁(s) may be identical to or different from each other,
L₄₀₂ may be an organic ligand, and xc2 may be an integer from 0 to 4, wherein, when xc2 is two or more, two or more L₄₀₂(s) may be identical to or different from each other,
X₄₀₁ to X₄₀₄ may each independently be nitrogen or carbon,
X₄₀₁ and X₄₀₃ may be linked via a single bond or a double bond, and X₄₀₂ and X₄₀₄ may be linked via a single bond or a double bond,
A₄₀₁ and A₄₀₂ may each independently be selected from a C₅-C₆₀ (*e.g.* C₅-C₃₀) carbocyclic group or a C₁-C₆₀ (*e.g.* C₁-C₂₀) heterocyclic group,
X₄₀₅ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C=*', wherein Q₄₁₁ and Q₄₁₂ may each independently be hydrogen, deuterium, a C₁-C₂₀ alkyl group, a C₁-C₂₀ alkoxy group, a phenyl group, a biphenyl group, a terphenyl group, or a naphthyl group,
X₄₀₆ may be a single bond, O, or S,
R₄₀₁ and R₄₀₂ may each independently be selected from hydrogen, deuterium, -F, -Cl, -Br, -I, a hydroxyl group, a cyano group, a nitro group, an amidino group, a hydrazino group, a hydrazono group, a substituted or unsubstituted C₁-C₂₀ alkyl group, a substituted or unsubstituted C₁-C₂₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g.* C₆-C₃₀) aryl group, a substituted or unsubstituted C₆-C₆₀ (*e.g.* C₆-C₃₀) aryloxy group, a substituted or unsubstituted C₆-C₆₀ (*e.g*. C₆-C₃₀) arylthio group, a substituted or unsubstituted C₁-C₆₀ (*e.g.* C₁-C₂₀) heteroaryl group, a substituted or unsubstituted monovalent non-aromatic condensed polycyclic group, and a substituted or unsubstituted monovalent non-aromatic condensed heteropolycyclic group, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁), and -P(=O)(Q₄₀₁)(Q₄₀₂), wherein Q₄₀₁ to Q₄₀₃ may each independently be selected from a C₁-C₁₀ alkyl group, a C₁-C₁₀ alkoxy group, a C₆-C₂₀ aryl group, and a C₁-C₂₀ heteroaryl group,
xc11 and xc12 may each independently be an integer from 0 to 10, and
* and *' in Formula 402 each indicate a binding site to M in Formula 401.

In some embodiments, A₄₀₁ and A₄₀₂ in Formula 402 may each independently be selected from a benzene group, a naphthalene group, a fluorene group, a spiro-bifluorene group, an indene group, a pyrrole group, a thiophene group, a furan group, an imidazole group, a pyrazole group, a thiazole group, an isothiazole group, an oxazole group, an isoxazole group, a pyridine group, a pyrazine group, a pyrimidine group, a pyridazine group, a quinoline group, an isoquinoline group, a benzoquinoline group, a quinoxaline group, a quinazoline group, a carbazole group, a benzimidazole group, a benzofuran group, a benzothiophene group, an isobenzothiophene group, a benzoxazole group, an isobenzoxazole group, a triazole group, a tetrazole group, an oxadiazole group, a triazine group, a dibenzofuran group, and a dibenzothiophene group.

In some embodiments, in Formula 402, i) X₄₀₁ may be nitrogen, and X₄₀₂ may be carbon, or ii) X₄₀₁ and X₄₀₂ may each be nitrogen at the same time.

In some embodiments, R₄₀₂ and R₄₀₂ in Formula 401 may each independently be selected from:
hydrogen, deuterium, -F, -Cl, -Br, -I, a hydroxyl group, a cyano group, a nitro group, an amidino group, a hydrazino group, a hydrazono group, a C₁-C₂₀ alkyl group, and a C₁-C₂₀ alkoxy group;
a C₁-C₂₀ alkyl group, and a C₁-C₂₀ alkoxy group, each substituted with at least one selected from deuterium, -F, -Cl, -Br, -I, a hydroxyl group, a cyano group, a nitro group, an amidino group, a hydrazino group, a hydrazono group, a phenyl group, a naphthyl group, a cyclopentyl group, a cyclohexyl group, an adamantanyl group, a norbornanyl group, and a norbornenyl group;
a cyclopentyl group, a cyclohexyl group, an adamantanyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group;
a cyclopentyl group, a cyclohexyl group, an adamantanyl group, a norbornanyl group, a norbornenyl group a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, an adamantanyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group; and
-Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), and -P(=O)(Q₄₀₁)(Q₄₀₂), and
Q₄₀₁ to Q₄₀₃ may each independently be selected from a C₁-C₁₀ alkyl group, a C₁-C₁₀ alkoxy group, a phenyl group, a biphenyl group, and a naphthyl group, but embodiments of the present disclosure are not limited thereto.

In some embodiments, when xc1 in Formula 401 is two or more, two A₄₀₁(s) in two or more L₄₀₁(s) may optionally be linked via X₄₀₇, which is a linking group, or two A₄₀₂(s) in two or more L₄₀₁(s) may optionally be linked via X₄₀₈, which is a linking group (see Compounds PD1 to PD4 and PD7). X₄₀₇ and X₄₀₈ may each independently be a single bond, *-O-*', *-S-*', *-C(=O)-*', *-N(Q₄₁₃)-*', *-C(Q₄₁₃)(Q₄₁₄)-*', or *-C(Q₄₁₃)=C(Q₄₁₄)-*' (wherein Q₄₁₃ and Q₄₁₄ may each independently be hydrogen, deuterium, a C₁-C₂₀ alkyl group, a C₁-C₂₀ alkoxy group, a phenyl group, a biphenyl group, a terphenyl group, or a naphthyl group), but embodiments of the present disclosure are not limited thereto.

L₄₀₂ in Formula 401 may be a monovalent, divalent, or trivalent organic ligand. For example, L₄₀₂ may be selected from halogen, diketone (for example, acetylacetonate), carboxylic acid (for example, picolinate), -C(=O), isonitrile, -CN, and a phosphorus-containing material (for example, phosphine or phosphite), but embodiments of the present disclosure are not limited thereto.

In some embodiments, the phosphorescent dopant may be selected from, for example, Compounds PD1 to PD25, but embodiments of the present disclosure are not limited thereto:

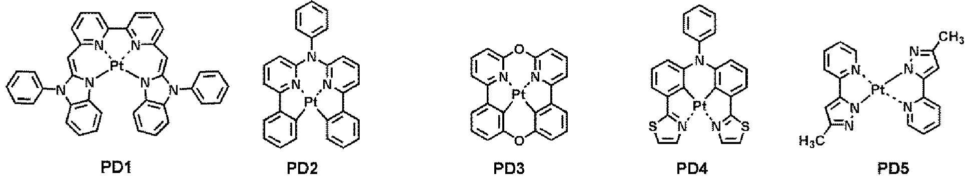

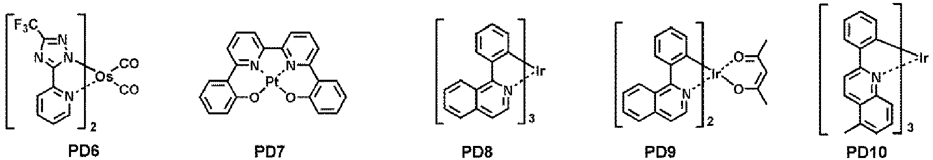

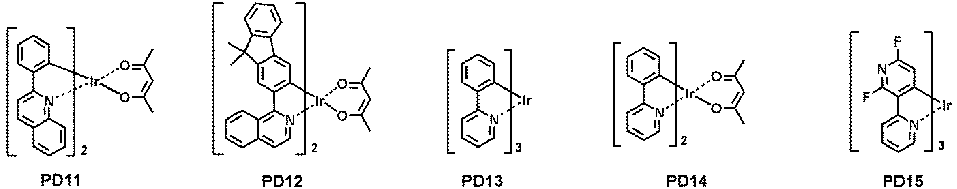

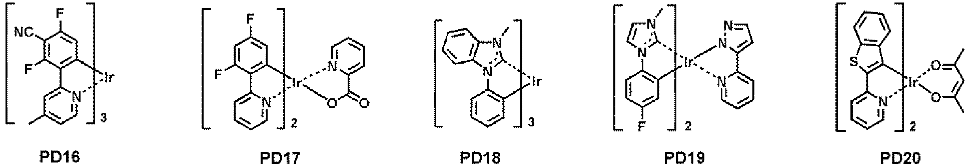

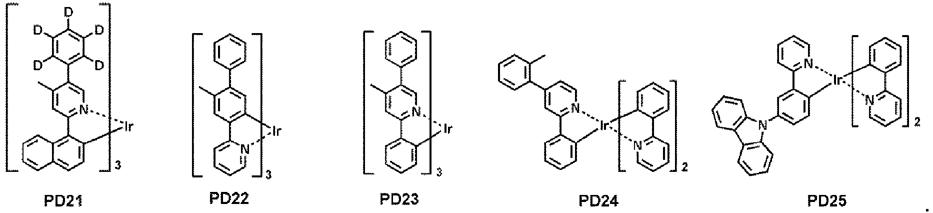

### Fluorescent dopant in emission layer

The fluorescent dopant may include an arylamine compound or a styrylamine compound.

The fluorescent dopant may include a compound represented by Formula 501:

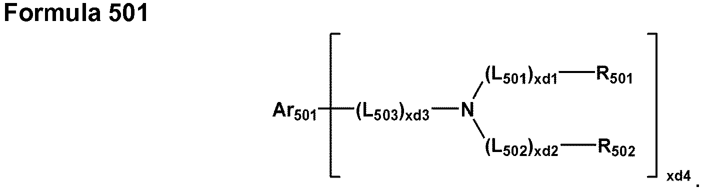

In Formula 501,
Ar₅₀₁ may be a substituted or unsubstituted C₅-C₆₀ carbocyclic group or a substituted or unsubstituted C₁-C₆₀ heterocyclic group,
L₅₀₁ to L₅₀₃ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (*e.g.* C₆-C₃₀) arylene group, a substituted or unsubstituted C₁-C₆₀ (*e.g.* C₁-C₂₀) heteroarylene group, a substituted or unsubstituted divalent non-aromatic condensed polycyclic group, and a substituted or unsubstituted divalent non-aromatic condensed heteropolycyclic group,
xd1 to xd3 may each independently be an integer of 0 to 3,
R₅₀₁ and R₅₀₂ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g.* C₆-C₃₀) aryl group, a substituted or unsubstituted C₆-C₆₀ (*e.g.* C₆-C₃₀) aryloxy group, a substituted or unsubstituted C₆-C₆₀ (*e.g*. C₆-C₃₀) arylthio group, a substituted or unsubstituted C₁-C₆₀ (*e.g.* C₁-C₂₀) heteroaryl group, a substituted or unsubstituted monovalent non-aromatic condensed polycyclic group, and a substituted or unsubstituted monovalent non-aromatic condensed heteropolycyclic group, and
xd4 may be an integer of 1 to 6.

For example, Ar₅₀₁ may be a substituted or unsubstituted C₅-C₃₀ carbocyclic group or a substituted or unsubstituted C₁-C₂₀ heterocyclic group, but embodiments are not limited thereto.

In some embodiments, Ar₅₀₁ in Formula 501 may be selected from:
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group; and
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

In some embodiments, L₅₀₁ to L₅₀₃ in Formula 501 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

In some embodiments, R₅₀₁ and R₅₀₂ in Formula 501 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, and - Si(Q₃₁)(Q₃₂)(Q₃₃), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In some embodiments, xd4 in Formula 501 may be 2, but embodiments of the present disclosure are not limited thereto.

For example, the fluorescent dopant may be selected from Compounds FD1 to FD22:

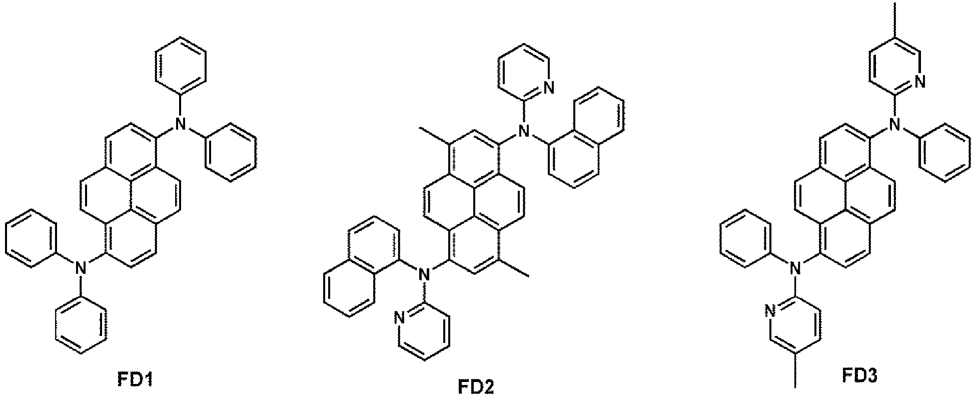

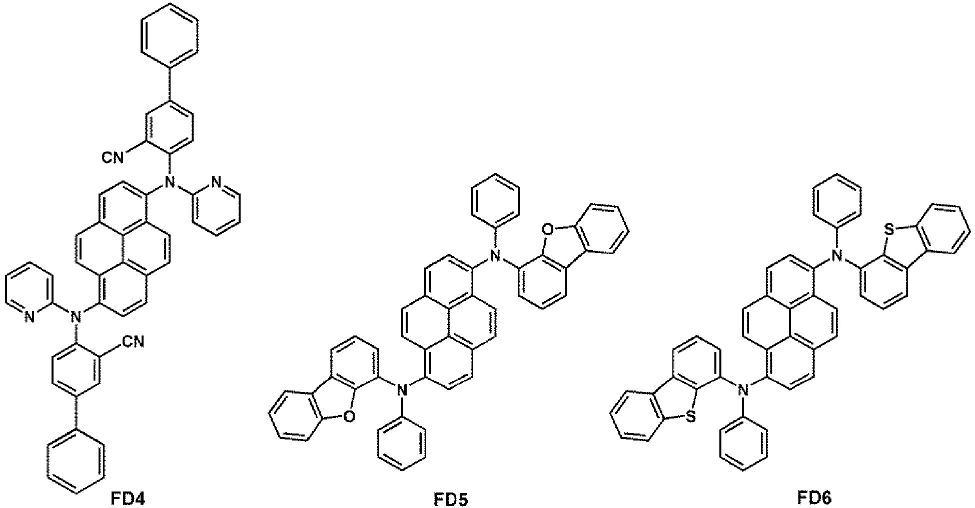

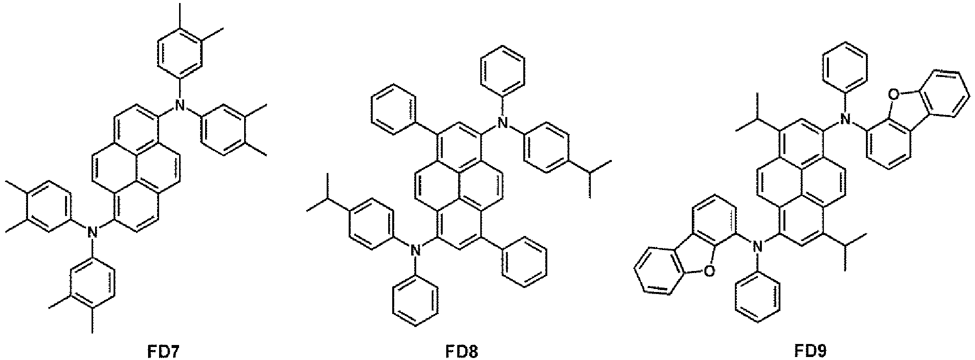

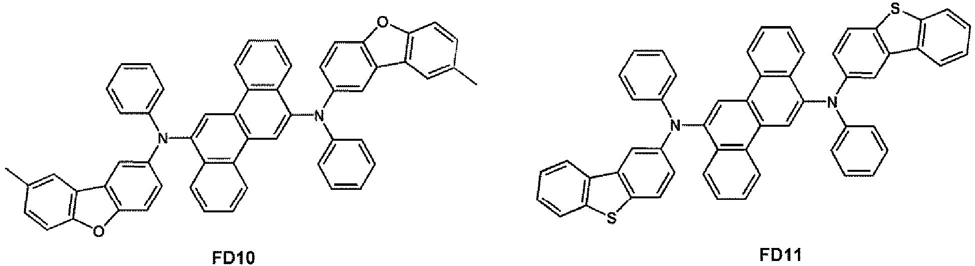

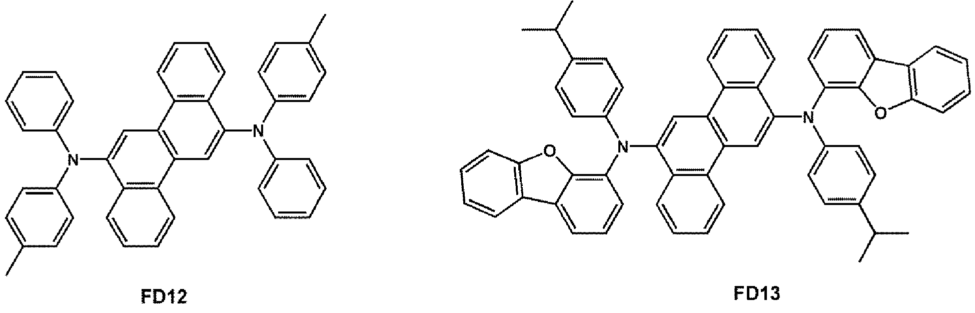

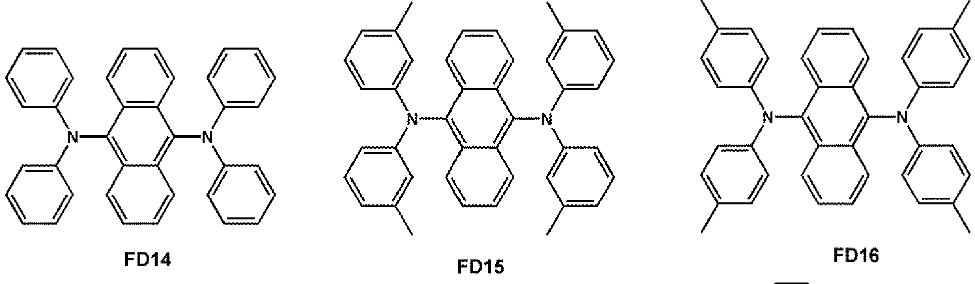

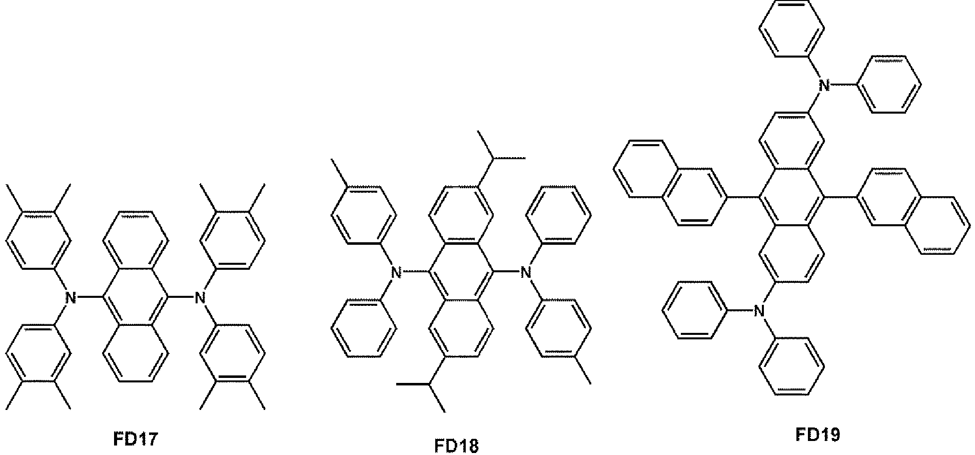

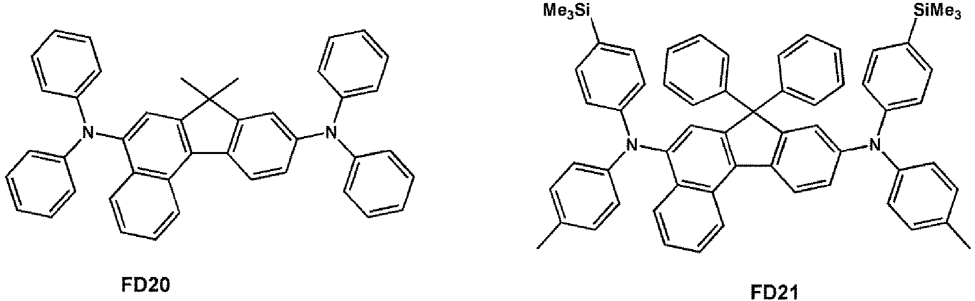

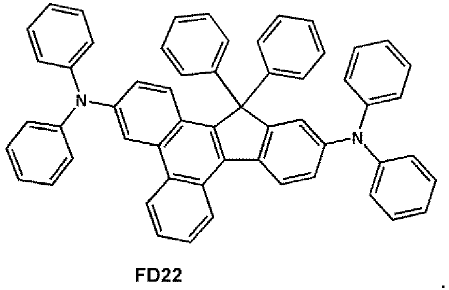

In some embodiments, the fluorescent dopant may be selected from the following compounds, but embodiments of the present disclosure are not limited thereto:

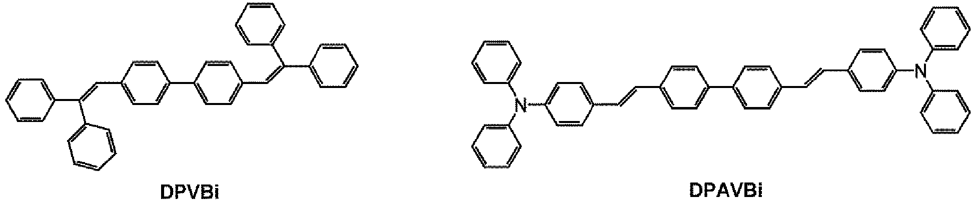

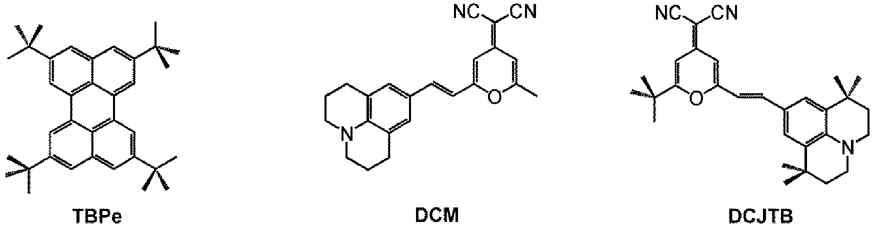

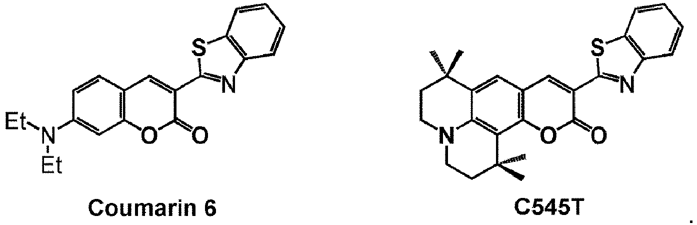

### Electron transport region in organic layer 150

The electron transport region may have: i) a single-layered structure including a single layer including a single material; ii) a single-layered structure including a single layer including a plurality of different materials; or iii) a multi-layered structure having a plurality of layers including a plurality of different materials.

The electron transport region may include at least one selected from a buffer layer, a hole blocking layer, an electron control layer, an electron transport layer, and an electron injection layer, but embodiments of the present disclosure are not limited thereto.

For example, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein the constituting layers of each structure are sequentially stacked from the emission layer. However, embodiments of the structure of the electron transport region are not limited thereto.

The electron transport region (for example, a buffer layer, a hole blocking layer, an electron control layer, or an electron transport layer in the electron transport region) may include a metal-free compound containing at least one π electron-depleted nitrogen-containing ring.

The term "π electron-depleted nitrogen-containing ring", as used herein, indicates a C₁-C₆₀ (*e.g.* a C₁-C₃₀) heterocyclic group having at least one *-N=*' moiety as a ring-forming moiety.

For example, the "π electron-depleted nitrogen-containing ring" may be: i) a 5-membered to 7-membered heteromonocyclic group having at least one *-N=*' moiety; ii) a heteropolycyclic group in which two or more 5-membered to 7-membered heteromonocyclic groups each having at least one *-N=*' moiety are condensed with each other; or iii) a heteropolycyclic group in which at least one of 5-membered to 7-membered heteromonocyclic groups, each having at least one *-N=*' moiety, is condensed with at least one C₅-C₆₀ (*e.g.* a C₅-C₃₀) carbocyclic group.

Non-limiting examples of the π electron-depleted nitrogen-containing ring include an imidazole, a pyrazole, a thiazole, an isothiazole, an oxazole, an isoxazole, a pyridine, a pyrazine, a pyrimidine, a pyridazine, an indazole, a purine, a quinoline, an isoquinoline, a benzoquinoline, a phthalazine, a naphthyridine, a quinoxaline, a quinazoline, a cinnoline, a phenanthridine, an acridine, a phenanthroline, a phenazine, a benzimidazole, an isobenzothiazole, a benzoxazole, an isobenzoxazole, a triazole, a tetrazole, an oxadiazole, a triazine, a thiadiazole, an imidazopyridine, an imidazopyrimidine, and an azacarbazole, but are not limited thereto.

For example, the electron transport region may include a compound represented by Formula 601:

**Formula 601** [Ar₆₀₁]ₓₑ₁₁-[(L₆₀₁)ₓₑ₁-R₆₀₁]ₓₑ₂₁.

In Formula 601,
Ar₆₀₁ may be a substituted or unsubstituted C₅-C₆₀ carbocyclic group or a substituted or unsubstituted C₁-C₆₀ heterocyclic group,
xe11 may be 1, 2, or 3,
L₆₀₁ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xe1 may be an integer from 0 to 5,
R₆₀₁ may be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₆₀₁)(Q₆₀₂)(Q₆₀₃), - -C(=O)(Q₆₀₁), -S(=O)₂(Q₆₀₁), and -P(=O)(Q₆₀₁)(Q₆₀₂),
Q₆₀₁ to Q₆₀₃ may each independently be a C₁-C₁₀ alkyl group, a C₁-C₁₀ alkoxy group, a phenyl group, a biphenyl group, a terphenyl group, or a naphthyl group, and
xe21 may be an integer from 1 to 5.

In some embodiments, at least one of the xe11 Ar₆₀₁(s) and at least one of the xe21 R₆₀₁(s) may include the π electron-depleted nitrogen-containing ring.

For example, Ar₆₀₁ may be a substituted or unsubstituted C₅-C₃₀ carbocyclic group or a substituted or unsubstituted C₁-C₂₀ heterocyclic group, but embodiments are not limited thereto.

In some embodiments, ring Ar₆₀₁ in Formula 601 may be selected from:
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group; and
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -S(=O)₂(Q₃₁), and -P(=O)(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

When xe11 in Formula 601 is two or more, two or more Ar₆₀₁(s) may be linked via a single bond.

In some embodiments, Ar₆₀₁ in Formula 601 may be an anthracene group.

In some embodiments, a compound represented by Formula 601 may be represented by Formula 601-1:

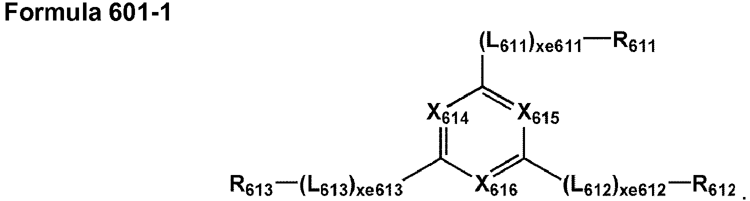

In Formula 601-1,
X₆₁₄ may be N or C(R₆₁₄), X₆₁₅ may be N or C(R₆₁₅), X₆₁₆ may be N or C(R₆₁₆), and at least one selected from X₆₁₄ to X₆₁₆ may be N,
L₆₁₁ to L₆₁₃ may each independently be the same as described in connection with L₆₀₁,
xe611 to xe613 may each independently be the same as described in connection with xe1,
R₆₁₁ to R₆₁₃ may each independently be the same as described in connection with R₆₀₁, and
R₆₁₄ to R₆₁₆ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

For example, L₆₀₁ and L₆₁₁ to L₆₁₃ in Formulae 601 and 601-1 may each independently be selected from: a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₃₀ arylene group, a substituted or unsubstituted C₁-C₂₀ heteroarylene group, a substituted or unsubstituted divalent non-aromatic condensed polycyclic group, and a substituted or unsubstituted divalent non-aromatic condensed heteropolycyclic group, but embodiments are not limited thereto.

In some embodiments, L₆₀₁ and L₆₁₁ to L₆₁₃ in Formulae 601 and 601-1 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group,
but embodiments of the present disclosure are not limited thereto.

In some embodiments, xe1 and xe611 to xe613 in Formulae 601 and 601-1 may each independently be 0, 1, or 2.

For example, R₆₀₁ and R₆₁₁ to R₆₁₃ in Formulae 601 and 601-1 may each independently be selected from: a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₃₀ aryl group, a substituted or unsubstituted C₆-C₃₀ aryloxy group, a substituted or unsubstituted C₆-C₃₀ arylthio group, a substituted or unsubstituted C₁-C₂₀ heteroaryl group, a substituted or unsubstituted monovalent non-aromatic condensed polycyclic group, a substituted or unsubstituted monovalent non-aromatic condensed heteropolycyclic group, - Si(Q₆₀₁)(Q₆₀₂)(Q₆₀₃), -C(=O)(Q₆₀₁), -S(=O)₂(Q₆₀₁), and -P(=O)(Q₆₀₁)(Q₆₀₂).

In some embodiments, R₆₀₁ and R₆₁₁ to R₆₁₃ in Formulae 601 and 601-1 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group;
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
-S(=O)₂(Q₆₀₁) and -P(=O)(Q₆₀₁)(Q₆₀₂), and
Q₆₀₁ and Q₆₀₂ may each independently be the same as described above.

The electron transport region may include at least one compound selected from Compounds ET1 to ET36, but embodiments of the present disclosure are not limited thereto:

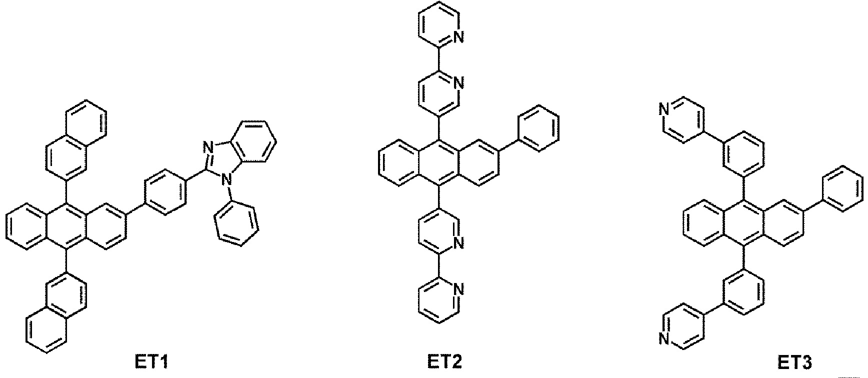

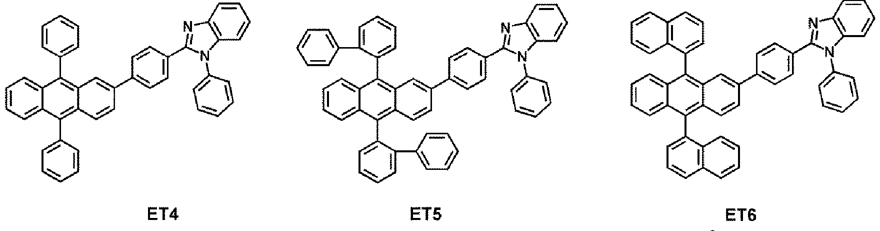

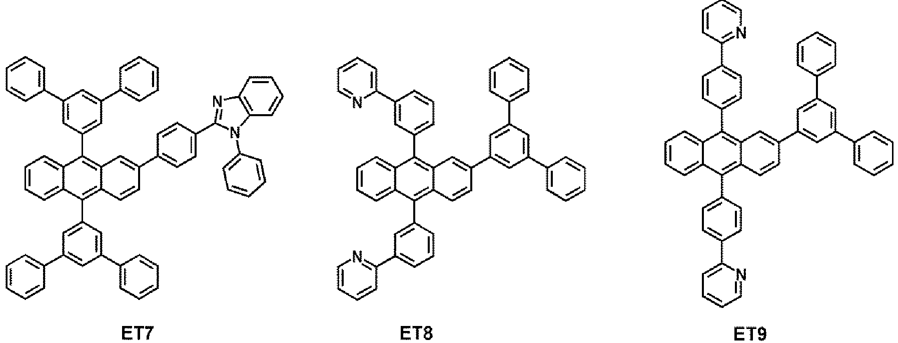

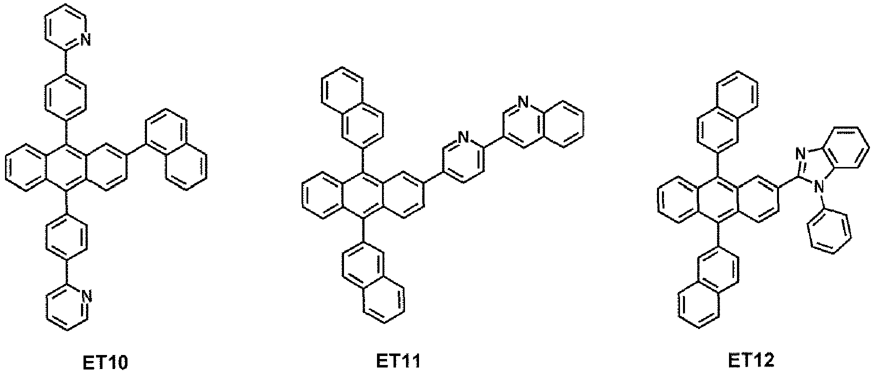

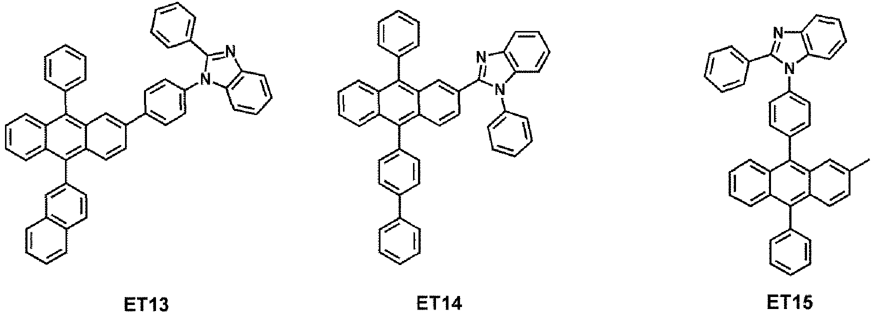

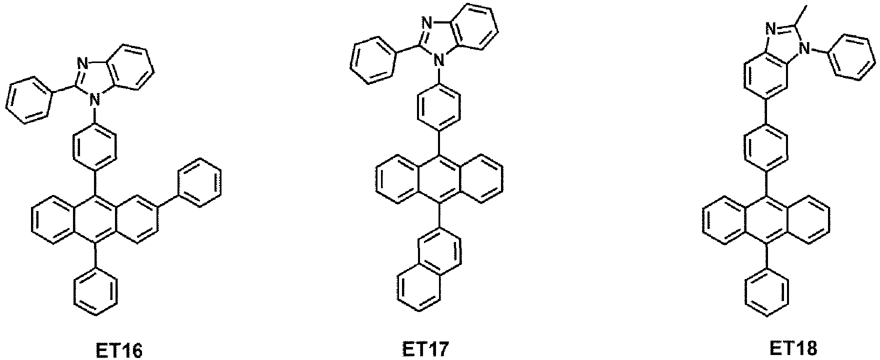

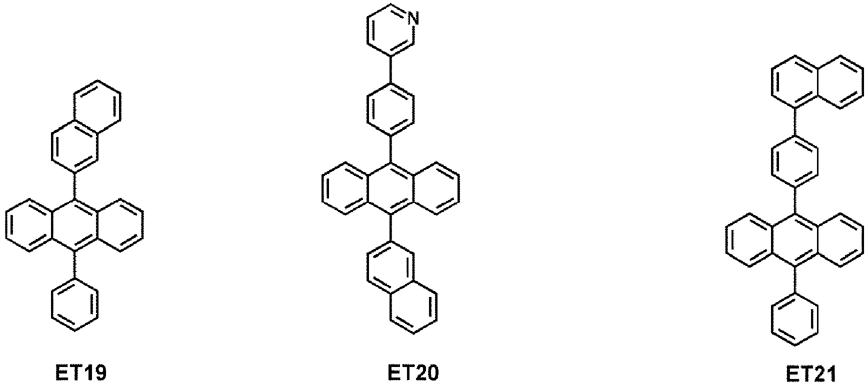

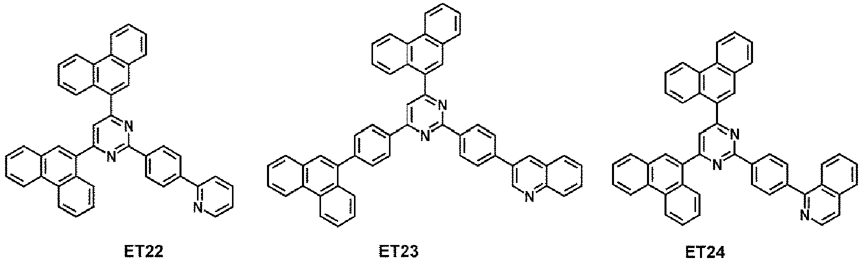

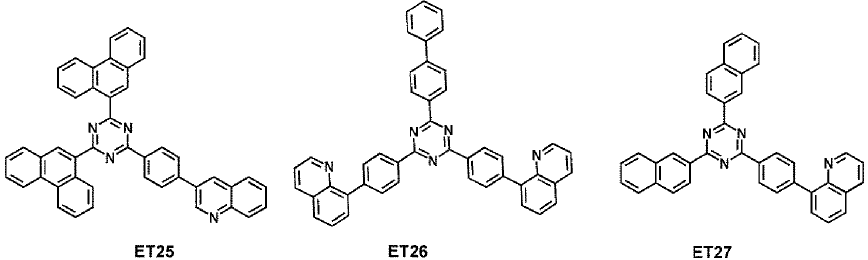

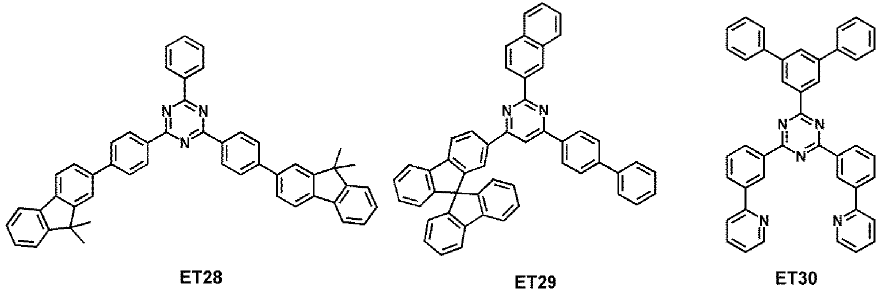

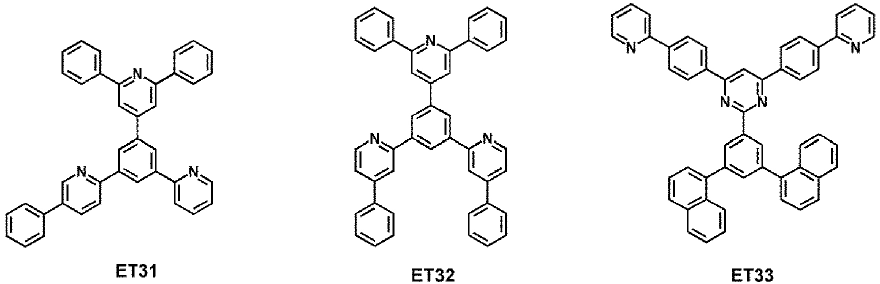

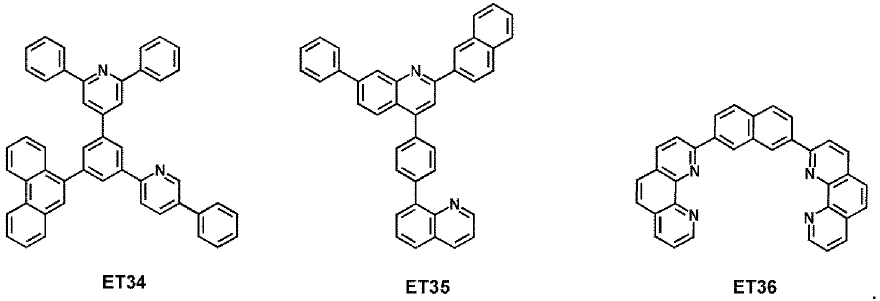

In some embodiments, the electron transport region may include at least one selected from 2,9-dimethyl-4,7-diphenyl-1,10-phenanthroline (BCP), 4,7-diphenyl-1,10-phenanthroline (Bphen), Alq₃, BAlq, 3-(biphenyl-4-yl)-5-(4-tert-butylphenyl)-4-phenyl-4H-1,2,4-triazole (TAZ), and NTAZ:

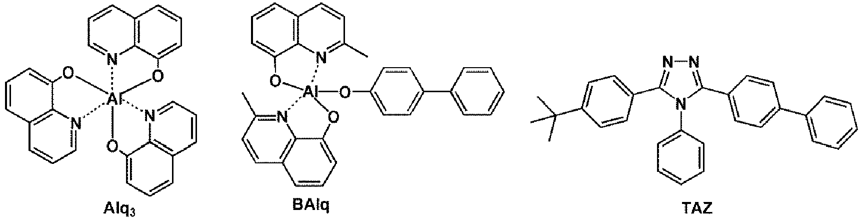

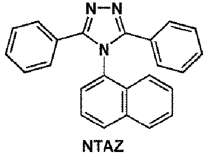

In some embodiments, the electron transport region may include a phosphine oxide-containing compound (for example, TSPO1 and/or the like), but embodiments of the present disclosure are not limited thereto. In some embodiments, the phosphine oxide-containing compound may be used in a hole blocking layer in the electron transport region, but embodiments of the present disclosure are not limited thereto.

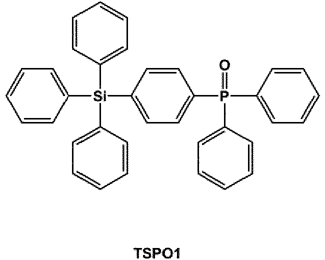

The thicknesses of the buffer layer, the hole blocking layer, and the electron control layer may each be about 20 Å to about 1,000 Å, for example, about 20 Å to about 700 Å, about 20 Å to about 500 Å, about 20 Å to about 400 Å, about 30 Å to about 400 Å or about 30 Å to about 300 Å. When the thicknesses of the buffer layer, the hole blocking layer, and the electron control layer are within these ranges, the electron blocking layer may have excellent electron blocking characteristics or electron control characteristics without a substantial increase in driving voltage.

A thickness of the electron transport layer may be about 100 Å to about 1,000 Å, for example, about 100 Å to about 700 Å, about 100 Å to about 600 Å, about 150 Å to about 600 Å, about 150 Å to about 500 Å or about 150 Å to about 250 Å. When the thickness of the electron transport layer is within the range described above, the electron transport layer may have satisfactory electron transport characteristics without a substantial increase in driving voltage.

The electron transport region (for example, the electron transport layer in the electron transport region) may further include, in addition to the materials described above, a metal-containing material.

The metal-containing material may include at least one selected from an alkali metal complex and an alkaline earth-metal complex. The alkali metal complex may include a metal ion selected from a lithium (Li) ion, a sodium (Na) ion, a potassium (K) ion, a rubidium (Rb) ion, and a cesium (Cs) ion, and the alkaline earth-metal complex may include a metal ion selected from a beryllium (Be) ion, a magnesium (Mg) ion, a calcium (Ca) ion, a strontium (Sr) ion, and a barium (Ba) ion. A ligand coordinated with the metal ion of the alkali metal complex or the alkaline earth-metal complex may be selected from a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy diphenyloxadiazole, a hydroxy diphenylthiadiazole, a hydroxy phenylpyridine, a hydroxy phenylbenzimidazole, a hydroxy phenylbenzothiazole, a bipyridine, a phenanthroline, and a cyclopentadiene, but embodiments of the present disclosure are not limited thereto.

For example, the metal-containing material may include a Li complex. The Li complex may include, for example, Compound ET-D1 (lithium quinolate, LiQ) or ET-D2.

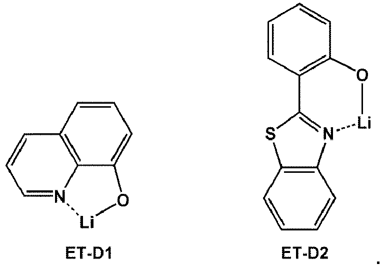

The electron transport region may include an electron injection layer that facilitates injection of electrons from the second electrode 190. The electron injection layer may directly contact the second electrode 190.

The electron injection layer may have: i) a single-layered structure including a single layer including a single material; ii) a single-layered structure including a single layer including a plurality of different materials; or iii) a multi-layered structure having a plurality of layers including a plurality of different materials.

The electron injection layer may include an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combination thereof.

The alkali metal may be selected from Li, Na, K, Rb, and Cs. In some embodiments, the alkali metal may be Li, Na, or Cs. In some embodiments, the alkali metal may be Li or Cs, but embodiments of the present disclosure are not limited thereto.

The alkaline earth metal may be selected from Mg, Ca, Sr, and Ba.

The rare earth metal may be selected from scandium (Sc), yttrium (Y), cerium (Ce), terbium (Tb), ytterbium (Yb), and gadolinium (Gd).

The alkali metal compound, the alkaline earth-metal compound, and the rare earth metal compound may be selected from oxides and halides (for example, fluorides, chlorides, bromides, and/or iodides) of the alkali metal, the alkaline earth-metal, and the rare earth metal.

The alkali metal compound may be selected from alkali metal oxides (such as Li₂O, Cs₂O, and/or K₂O), and alkali metal halides (such as LiF, NaF, CsF, KF, Lil, Nal, Csl, KI, and/or Rbl). In some embodiments, the alkali metal compound may be selected from LiF, Li₂O, NaF, Lil, Nal, Csl, and KI, but embodiments of the present disclosure are not limited thereto.

The alkaline earth-metal compound may be selected from alkaline earth-metal oxides (such as BaO, SrO, CaO, BaₓSr₁₋ₓO (0<x<1), and/or BaₓCa₁₋ₓO (0<x<1)). In some embodiments, the alkaline earth-metal compound may be selected from BaO, SrO, and CaO, but embodiments of the present disclosure are not limited thereto.

The rare earth metal compound may be selected from YbF₃, ScF₃, ScO₃, Y₂O₃, Ce₂O₃, GdF₃, and TbF₃. In some embodiments, the rare earth metal compound may be selected from YbF₃, ScF₃, TbF₃, YbI₃, ScI₃, and TbI₃, but embodiments of the present disclosure are not limited thereto.

The alkali metal complex, the alkaline earth-metal complex, and the rare earth metal complex may respectively include an alkali metal ion, alkaline earth-metal ion, or rare earth metal ion as described above, and the ligand coordinated with the metal ion of the alkali metal complex, the alkaline earth-metal complex, or the rare earth metal complex may be selected from a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy diphenyloxadiazole, a hydroxy diphenylthiadiazole, a hydroxy phenylpyridine, a hydroxy phenylbenzimidazole, a hydroxy phenylbenzothiazole, a bipyridine, a phenanthroline, and a cyclopentadiene, but embodiments of the present disclosure are not limited thereto.

The electron injection layer may include (e.g., consist of) an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combination thereof, as described above. In some embodiments, the electron injection layer may further include an organic material. When the electron injection layer further includes an organic material, the alkali metal, alkaline earth metal, rare earth metal, alkali metal compound, alkaline earth-metal compound, rare earth metal compound, alkali metal complex, alkaline earth-metal complex, rare earth metal complex, or combination thereof may be homogeneously or non-homogeneously dispersed in a matrix including the organic material.

A thickness of the electron injection layer may be about 1 Å to about 100 Å, for example, about 3 Å to about 90 Å or about 3 Å to about 20 Å. When the thickness of the electron injection layer is within the range described above, the electron injection layer may have satisfactory electron injection characteristics without a substantial increase in driving voltage.

### Second electrode 190

The second electrode 190 may be on the organic layer 150 having such a structure. The second electrode 190 may be a cathode that is an electron injection electrode, and in this regard, a material for forming the second electrode 190 may be selected from metal, an alloy, an electrically conductive compound, and a combination thereof, each having a relatively low work function.

The second electrode 190 may include at least one selected from lithium (Li), silver (Ag), magnesium (Mg), aluminium (Al), aluminium-lithium (Al-Li), calcium (Ca), magnesium-indium (Mg-In), magnesium-silver (Mg-Ag), ITO, and IZO, but embodiments of the present disclosure are not limited thereto. The second electrode 190 may be a transmissive electrode, a semi-transmissive electrode, or a reflective electrode.

The second electrode 190 may have a single-layered structure, or a multi-layered structure including two or more layers.

Layers constituting the hole transport region, an emission layer, and layers constituting the electron transport region may be formed in a certain (set or predetermined) region using one or more suitable methods selected from vacuum deposition, spin coating, casting, Langmuir-Blodgett (LB) deposition, ink-jet printing, laser-printing, and laser-induced thermal imaging.

When the layers constituting the hole transport region, the emission layer, and/or the layers constituting the electron transport region are formed by vacuum deposition, the deposition may be performed at a deposition temperature of about 100°C to about 500°C, a vacuum degree of about 10⁻⁸ torr to about 10⁻³ torr, and a deposition speed of about 0.01 Å/sec to about 100 Å/sec, depending on the material to be included in each layer, and the structure of each layer to be formed.

When the layers constituting the hole transport region, the emission layer, and the layers constituting the electron transport region are formed by spin coating, the spin coating may be performed at a coating speed of about 2,000 rpm to about 5,000 rpm and at a heat treatment temperature of about 80°C to 200°C, depending on the material to be included in each layer, and the structure of each layer to be formed.

An organic light-emitting device 20 as illustrated in FIG. 2 includes a first capping layer 210, a first electrode 110, an organic layer 150, and a second electrode 190 sequentially stacked in this stated order. An organic light-emitting device 30 as illustrated in FIG. 3 includes a first electrode 110, an organic layer 150, a second electrode 190, and a second capping layer 220 sequentially stacked in this stated order. An organic light-emitting device 40 as illustrated in FIG. 4 includes a first capping layer 210, a first electrode 110, an organic layer 150, a second electrode 190, and a second capping layer 220 sequentially stacked in this stated order.

In FIGS. 2 to 4, the first electrode 110, the organic layer 150, and the second electrode 190 may be understood by referring to the description presented in connection with FIG. 1.

In the organic layer 150 of each of the organic light-emitting devices 20 and 40, light generated in the emission layer may pass through the first electrode 110 (which is a semi-transmissive or transmissive electrode) and the first capping layer 210 toward the outside. In the organic layer 150 of each of the organic light-emitting devices 30 and 40, light generated in the emission layer may pass through the second electrode 190 (which is a semi-transmissive or transmissive electrode) and the second capping layer 220 toward the outside.

The first capping layer 210 and the second capping layer 220 may increase the external luminescent efficiency of the device according to the principle of constructive interference.

The first capping layer 210 and the second capping layer 220 may each independently be an organic capping layer including an organic material, an inorganic capping layer including an inorganic material, or a composite capping layer including an organic material and an inorganic material.

At least one selected from the first capping layer 210 and the second capping layer 220 may include at least one material selected from carbocyclic compounds, heterocyclic compounds, amine-based compounds, porphyrin derivatives, phthalocyanine derivatives, a naphthalocyanine derivatives, alkali metal complexes, and alkaline earth-based complexes. The carbocyclic compounds, the heterocyclic compounds, and the amine-based compounds may each be optionally substituted with a substituent containing at least one element selected from O, N, S, selenium (Se), silicon (Si), fluorine (F), chlorine (Cl), bromine (Br), and iodine (I). In some embodiments, at least one selected from the first capping layer 210 and the second capping layer 220 may include an amine-based compound. In some embodiments, at least one selected from the first capping layer 210 and the second capping layer 220 may each independently include the compound represented by Formula 201 or the compound represented by Formula 202.

In some embodiments, at least one selected from the first capping layer 210 and the second capping layer 220 may each independently include a compound selected from Compounds HT28 to HT33 and Compounds CP1 to CP5, but embodiments of the present disclosure are not limited thereto.

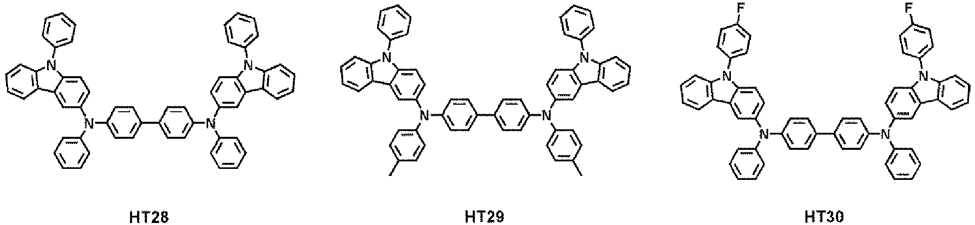

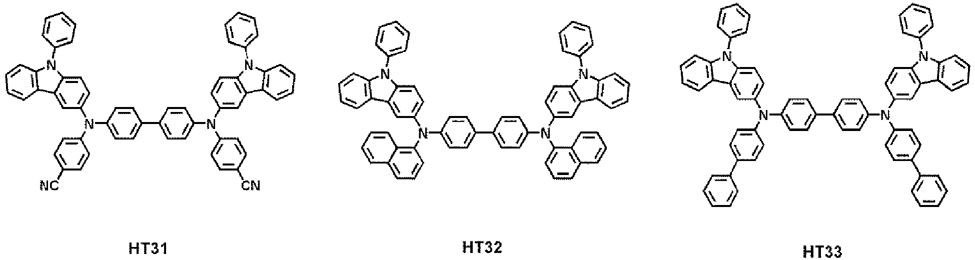

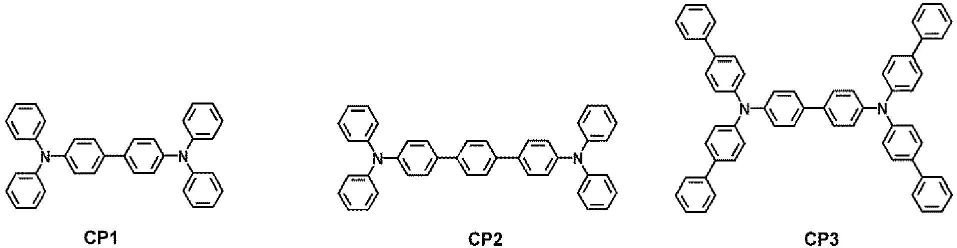

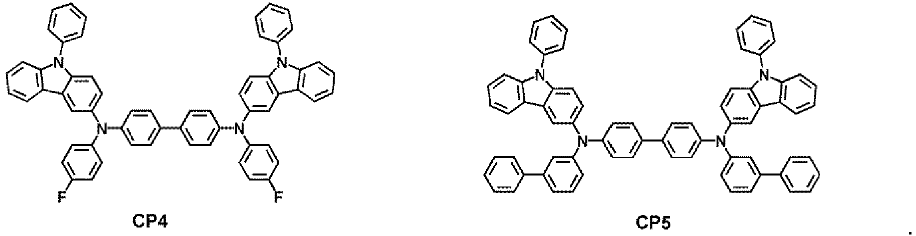

Hereinbefore, the organic light-emitting device according to an embodiment of the present disclosure has been described in connection with FIGS. 1-4. However, embodiments of the present disclosure are not limited thereto.

### Apparatus

The organic light-emitting device may be included in one or more suitable apparatuses. For example, a light-emitting apparatus, an authentication apparatus, or an electronic apparatus, which includes the organic light-emitting device, may be provided.

The light-emitting apparatus may further include, in addition to the organic light-emitting device, a thin film transistor including a source electrode and a drain electrode. One of the source electrode and the drain electrode of the thin film transistor may be electrically connected to one of the first electrode and the second electrode of the organic light-emitting device. The light-emitting apparatus may be used in one or more suitable displays, light sources, and the like.

The authentication apparatus may be, for example, a biometric authentication apparatus for authenticating an individual using biometric information of a biometric body (for example, a fingertip (fingerprint), a pupil, or the like).

The authentication apparatus may further include, in addition to the organic light-emitting device, a biometric information collector.

The electronic apparatus may be applied to personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram (ECG) displays, ultrasonic diagnostic devices, or endoscope displays), fish finders, various measuring instruments, meters (for example, meters for a vehicle, an aircraft, and a vessel), projectors, and/or the like, but embodiments of the present disclosure are not limited thereto.

### General definition of substituents

The term "C₁-C₆₀ alkyl group" as used herein refers to a linear or branched aliphatic saturated hydrocarbon monovalent group including 1 to 60 carbon atoms, and non-limiting examples thereof include a methyl group, an ethyl group, a propyl group, an isobutyl group, a sec-butyl group, a tert-butyl group, a pentyl group, an isoamyl group, and a hexyl group. The term "C₁-C₆₀ alkylene group" as used herein refers to a divalent group having substantially the same structure as the C₁-C₆₀ alkyl group. Corresponding definitions apply to other ranges given for the number of carbon atoms in an alkyl/alkylene group.

The term "C₂-C₆₀ alkenyl group" as used herein refers to a hydrocarbon group including at least one double bond in the middle or at the terminus of the C₂-C₆₀ alkyl group, and non-limiting examples thereof include an ethenyl group, a propenyl group, and a butenyl group. The term "C₂-C₆₀ alkenylene group" as used herein refers to a divalent group having substantially the same structure as the C₂-C₆₀ alkenyl group. Corresponding definitions apply to other ranges given for the number of carbon atoms in an alkenyl/alkenylene group.

The term "C₂-C₆₀ alkynyl group" as used herein refers to a hydrocarbon group including at least one triple bond in the middle or at the terminus of the C₂-C₆₀ alkyl group, and non-limiting examples thereof include an ethynyl group and a propynyl group. The term "C₂-C₆₀ alkynylene group" as used herein refers to a divalent group having substantially the same structure as the C₂-C₆₀ alkynyl group. Corresponding definitions apply to other ranges given for the number of carbon atoms in an alkynyl/alkynylene group.

The term "C₁-C₆₀ alkoxy group" as used herein refers to a monovalent group represented by -OA₁₀₁ (wherein A₁₀₁ is a C₁-C₆₀ alkyl group), and non-limiting examples thereof include a methoxy group, an ethoxy group, and an isopropyloxy group. Corresponding definitions apply to other ranges given for the number of carbon atoms in an alkoxy group.

The term "C₃-C₁₀ cycloalkyl group" as used herein refers to a monovalent saturated hydrocarbon monocyclic group including 3 to 10 carbon atoms, and non-limiting examples thereof include a cyclopropyl group, a cyclobutyl group, a cyclopentyl group, a cyclohexyl group, and a cycloheptyl group. The term "C₃-C₁₀ cycloalkylene group" as used herein refers to a divalent group having substantially the same structure as the C₃-C₁₀ cycloalkyl group.

The term "C₁-C₁₀ heterocycloalkyl group" as used herein refers to a monovalent monocyclic group including at least one heteroatom selected from N, O, Si, P, and S as a ring-forming atom and 1 to 10 carbon atoms, and non-limiting examples thereof include a 1,2,3,4-oxatriazolidinyl group, a tetrahydrofuranyl group, and a tetrahydrothiophenyl group. The term "C₁-C₁₀ heterocycloalkylene group" as used herein refers to a divalent group having substantially the same structure as the C₁-C₁₀ heterocycloalkyl group.

The term "C₃-C₁₀ cycloalkenyl group" as used herein refers to a monovalent monocyclic group including 3 to 10 carbon atoms, at least one double bond in the ring thereof, and no aromaticity, and non-limiting examples thereof include a cyclopentenyl group, a cyclohexenyl group, and a cycloheptenyl group. The term "C₃-C₁₀ cycloalkenylene group" as used herein refers to a divalent group having substantially the same structure as the C₃-C₁₀ cycloalkenyl group.

The term "C₁-C₁₀ heterocycloalkenyl group" as used herein refers to a monovalent monocyclic group including at least one heteroatom selected from N, O, Si, P, and S as a ring-forming atom, 1 to 10 carbon atoms, and at least one double bond in its ring. Non-limiting examples of the C₁-C₁₀ heterocycloalkenyl group include a 4,5-dihydro-1,2,3,4-oxatriazolyl group, a 2,3-dihydrofuranyl group, and a 2,3-dihydrothiophenyl group. The term "C₁-C₁₀ heterocycloalkenylene group" as used herein refers to a divalent group having substantially the same structure as the C₁-C₁₀ heterocycloalkenyl group.

The term "C₆-C₆₀ aryl group" as used herein refers to a monovalent group including a carbocyclic aromatic system having 6 to 60 carbon atoms, and the term "C₆-C₆₀ arylene group" as used herein refers to a divalent group having a carbocyclic aromatic system including 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused (e.g., condensed) to each other. Corresponding definitions apply to other ranges given for the number of carbon atoms in an aryl/arylene group.

The term "C₁-C₆₀ heteroaryl group" as used herein refers to a monovalent group including a carbocyclic aromatic system that has at least one heteroatom selected from N, O, Si, P, and S as a ring-forming atom, in addition to 1 to 60 carbon atoms. The term "C₁-C₆₀ heteroarylene group" as used herein refers to a divalent group having a carbocyclic aromatic system that has at least one heteroatom selected from N, O, Si, P, and S as a ring-forming atom, in addition to 1 to 60 carbon atoms. Non-limiting examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be condensed with each other. Corresponding definitions apply to other ranges given for the number of carbon atoms in an heteroaryl/heteroarylene group.

The term "C₆-C₆₀ aryloxy group" as used herein refers to -OA₁₀₂ (wherein A₁₀₂ is a C₆-C₆₀ aryl group), and the term "C₆-C₆₀ arylthio group" as used herein indicates -SA₁₀₃ (wherein A₁₀₃ is a C₆-C₆₀ aryl group). Corresponding definitions apply to other ranges given for the number of carbon atoms in an aryloxy group and an arylthio group.

The term "monovalent non-aromatic condensed polycyclic group" as used herein refers to a monovalent group (for example, including 8 to 60 carbon atoms) including two or more rings condensed with each other, only carbon atoms as ring-forming atoms, and no aromaticity in its entire molecular structure. A non-limiting example of the monovalent non-aromatic condensed polycyclic group is a fluorenyl group. The term "divalent non-aromatic condensed polycyclic group" as used herein refers to a divalent group having substantially the same structure as the monovalent non-aromatic condensed polycyclic group.

The term "monovalent non-aromatic condensed heteropolycyclic group" as used herein refers to a monovalent group (for example, including 1 to 60 carbon atoms) including two or more rings condensed to each other, at least one heteroatom selected from N, O, Si, P, and S, other than carbon atoms, as a ring-forming atom, and no aromaticity in its entire molecular structure. A non-limiting example of the monovalent non-aromatic condensed heteropolycyclic group is a carbazolyl group. The term "divalent non-aromatic condensed heteropolycyclic group" as used herein refers to a divalent group having substantially the same structure as the monovalent non-aromatic condensed heteropolycyclic group.

The term "C₅-C₆₀ carbocyclic group" as used herein refers to a monocyclic or polycyclic group including 5 to 60 carbon atoms, and only carbon atoms as ring-forming atoms. The C₅-C₆₀ carbocyclic group may be an aromatic carbocyclic group or a non-aromatic carbocyclic group. The C₅-C₆₀ carbocyclic group may be a ring (such as benzene), a monovalent group (such as a phenyl group), or a divalent group (such as a phenylene group). In some embodiments, depending on the number of substituents connected to the C₅-C₆₀ carbocyclic group, the C₅-C₆₀ carbocyclic group may be a trivalent group or a quadrivalent group. Corresponding definitions apply to other ranges given for the number of carbon atoms in a carbocyclic group.

The term "C₁-C₆₀ heterocyclic group" as used herein refers to a group having substantially the same structure as the C₄-C₆₀ carbocyclic group, except that at least one heteroatom selected from N, O, Si, P, and S is used as a ring-forming atom in addition to carbon (the number of carbon atoms may be from 1 to 60). Corresponding definitions apply to other ranges given for the number of carbon atoms in a heterocyclic group.

In the present application, at least one substituent of the substituted C₅-C₆₀ (*e.g.* C₅-C₃₀) carbocyclic group, the substituted C₁-C₆₀ (*e.g.* C₁-C₂₀) heterocyclic group, the substituted or unsubstituted C₃-C₁₀ cycloalkylene group, the substituted or unsubstituted C₁-C₁₀ heterocycloalkylene group, the substituted or unsubstituted C₃-C₁₀ cycloalkenylene group, a substituted or unsubstituted C₁-C₁₀ heterocycloalkenylene group, the substituted or unsubstituted C₆-C₆₀ (*e.g.* C₆-C₃₀) arylene group, the substituted or unsubstituted C₁-C₆₀ (*e.g.* C₁-C₂₀) heteroarylene group, the substituted or unsubstituted divalent non-aromatic condensed polycyclic group, the substituted or unsubstituted divalent non-aromatic condensed heteropolycyclic, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ (*e.g*. C₆-C₃₀) aryl group, the substituted C₆-C₆₀ (*e.g.* C₆-C₃₀) aryloxy group, the substituted C₆-C₆₀ (*e.g.* C₆-C₃₀) arylthio group, the substituted C₁-C₆₀ (*e.g.* C₁-C₂₀) heteroaryl group, the substituted monovalent non-aromatic condensed polycyclic group, the substituted monovalent non-aromatic condensed heteropolycyclic group, the substituted C₁-C₂₀ alkylene group, the substituted C₂-C₂₀ alkenylene group, and the substituted C₂-C₂₀ alkynylene group may be selected from:
deuterium, -F, -Cl, -Br, -I, a hydroxyl group, a cyano group, a nitro group, an amidino group, a hydrazino group, a hydrazono group, a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, and a C₁-C₆₀ (*e.g.* C₁-C₂₀) alkoxy group;
a C₁-C₆₀ (*e.g.* C₁-C₂₀) alkyl group, a C₂-C₆₀ *(e.g.* C₂-C₂₀) alkenyl group, a C₂-C₆₀ (*e.g.* C₂-C₂₀) alkynyl group, and a C₁-C₆₀ (*e.g.* C₁-C₂₀) alkoxy group, each substituted with at least one selected from deuterium, -F, -Cl, -Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, a monovalent non-aromatic condensed heteropolycyclic group, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), and -P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g*. C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, a monovalent non-aromatic condensed heteropolycyclic group, a biphenyl group, and a terphenyl group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g*. C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ *(e.g.* C₆-C₃₀) aryl group, a C₆-C₆₀ *(e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, a monovalent non-aromatic condensed heteropolycyclic group, -Si(Q₂₁)(Q₂₂)(Q₂₃), -N(Q₂₁)(Q₂₂), - B(Q₂₁)(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂), and
Q₁₁ to Q₁₃, Q₂₁ to Q₂₃, Q₃₁ to Q₃₃, and Q₄₁ to Q₄₂ may each independently be selected from hydrogen, deuterium, -F, -Cl, -Br, -I, a hydroxyl group, a cyano group, a nitro group, an amidino group, a hydrazino group, a hydrazono group, a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₁-C₆₀ (*e.g.* C₁-C₂₀) heteroaryl group, a monovalent non-aromatic condensed polycyclic group, a monovalent non-aromatic condensed heteropolycyclic group, a biphenyl group, and a terphenyl group.

The term "Ph" as used herein refers to a phenyl group, the term "Me" as used herein refers to a methyl group, the term "Et" as used herein refers to an ethyl group, the term "ter-Bu" or "Bu^{t}" as used herein refers to a tert-butyl group, and the term "OMe" as used herein refers to a methoxy group.

The term "biphenyl group" as used herein refers to "a phenyl group substituted with a phenyl group." In other words, a "biphenyl group" is a substituted phenyl group having a C₆-C₆₀ aryl group (phenyl group) as a substituent.

The term "terphenyl group" as used herein refers to "a phenyl group substituted with a biphenyl group." In other words, the "terphenyl group" is a phenyl group having a C₆-C₆₀ aryl group (phenyl group) substituted with a C₆-C₆₀ aryl group (phenyl group) as a substituent.

* and *' used herein, unless defined otherwise, each refer to a binding site to a neighboring atom in a corresponding formula.

Hereinafter, a compound according to embodiments of the present disclosure and an organic light-emitting device according to embodiments of the present disclosure will be described in more detail with reference to Synthesis Examples and Examples. The wording "B was used instead of A" used in describing Synthesis Examples indicates that an identical molar equivalent of B was used in place of A.

### Examples

### Synthesis Example 1: Synthesis of Compound 1

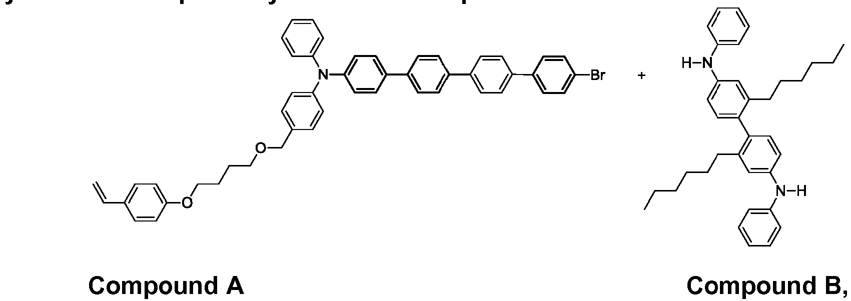

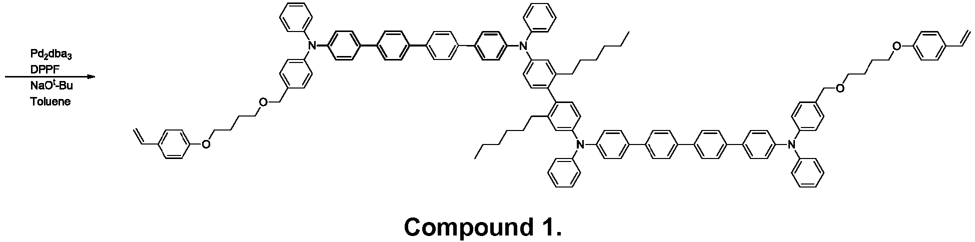

6.0 g (8 mmol) of Compound A, 2.0 g (4 mmol) of Compound B, 183 mg (0.05 eq.) of tris(dibenzylideneacetone) dipalladium(0), 55.4 mg (0.1 eq.) of 1,1'-bis (diphenylphosphino) ferrocene, and 1.2 g (12 mmol) of sodium t-butoxide were added to 80 mL of toluene in a nitrogen atmosphere and refluxed for 24 hours. After the reaction was completed, the mixture was passed through a silica gel pad and washed with toluene. The solvent was removed by evaporation, and the resulting product was recrystallized and separated by silica gel chromatography to obtain 1.8 g (yield of 25 %) of Compound 1.

MS (MALDI-TOF) m/z: 1855 [M]+.

### Synthesis Example 2: Synthesis of Compound 4

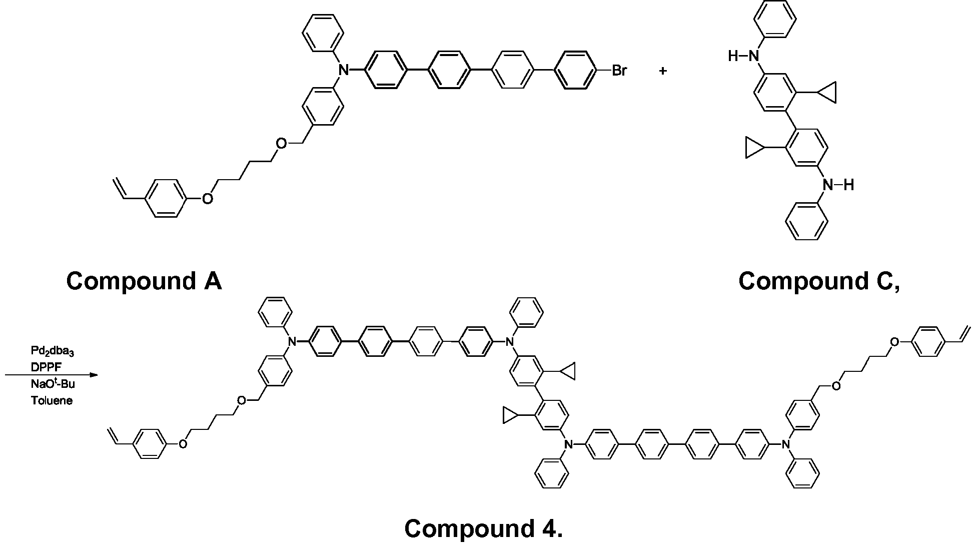

1.4 g (yield of 20 %) of Compound 4 was synthesized in substantially the same manner as in the Synthesis of Compound 1, except that Compound C was instead of Compound B.

MS (MALDI-TOF) m/z: 1766 [M]+.

### Synthesis Example 3: Synthesis of Compound 7

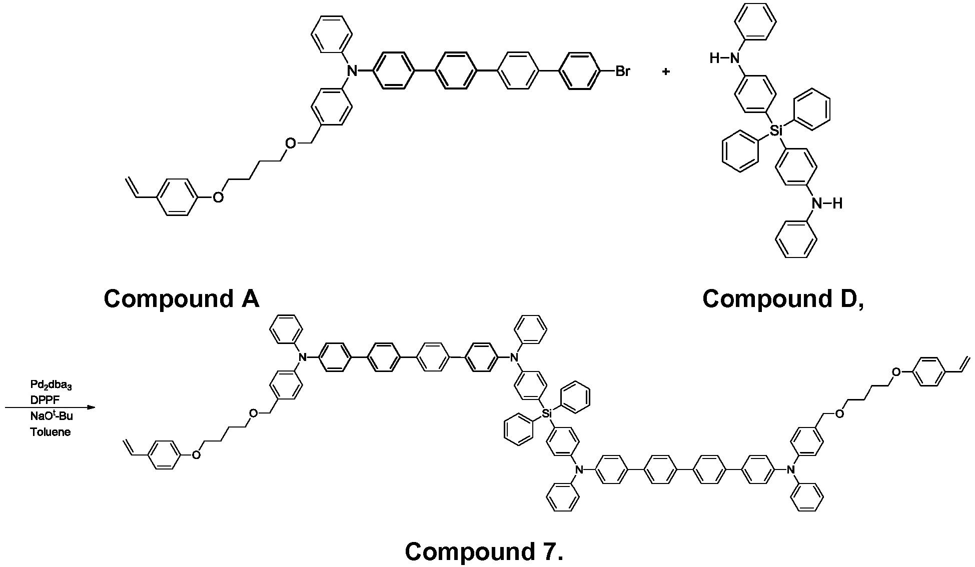

2.0 g (yield of 27 %) of Compound 7 was synthesized in substantially the same manner as in the Synthesis of Compound 1, except that Compound D was instead of Compound B.

MS (MALDI-TOF) m/z: 1868 [M]+.

### Synthesis Example 4: Synthesis of Compound 16

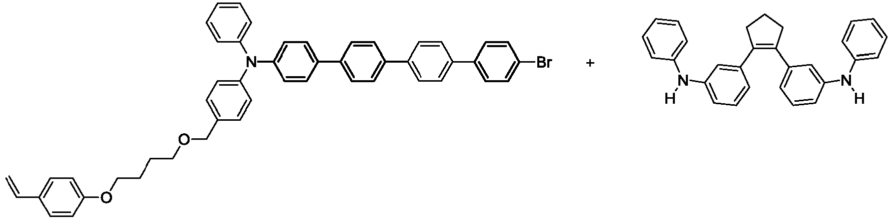

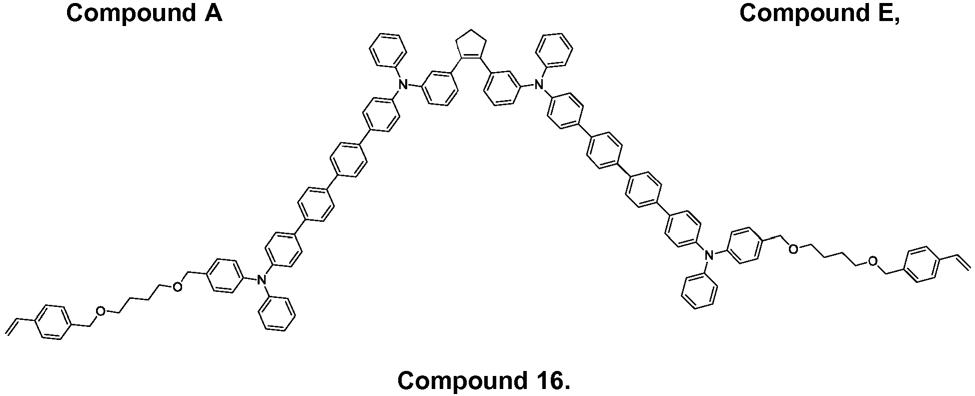

1.5 g (yield of 21 %) of Compound 16 was synthesized in substantially the same manner as in the Synthesis of Compound 1, except that Compound E was instead of Compound B.

MS (MALDI-TOF) m/z: 1780 [M]+.

### Synthesis Example 5: Synthesis of Compound 30

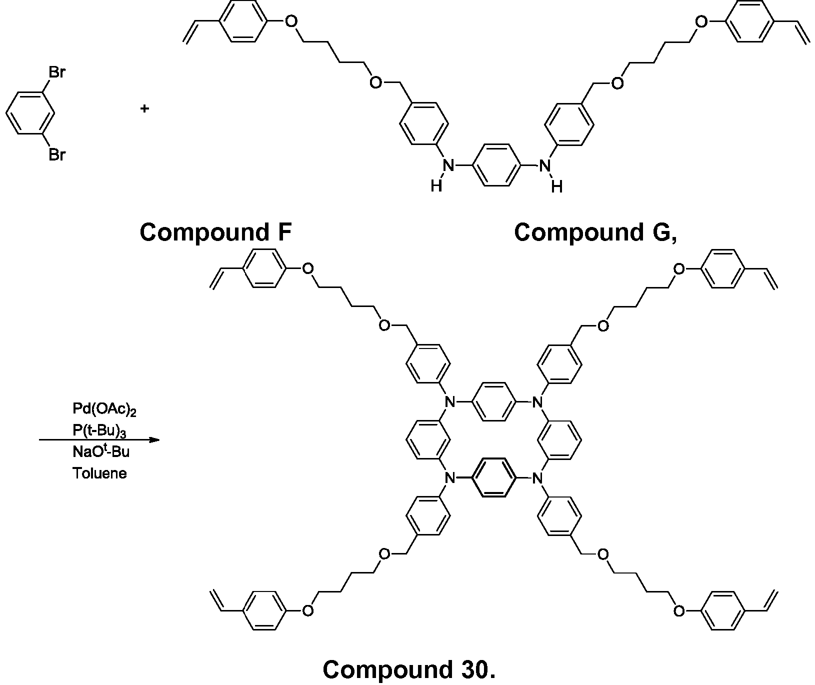

0.93 g (4 mmol) of Compound F, 2.6 g (4 mmol) of Compound G, 90 mg (0.10 eq.) of palladium acetate, 240 mg (0.30 eq.) of tri-t-butyl phosphine, and 1.2 g (12 mmol) of sodium t-butoxide were added to 40 mL of toluene in a nitrogen atmosphere and refluxed at a temperature of 120 °C for 24 hours. After the reaction was completed, the mixture was passed through a silica gel pad and washed with toluene. The solvent was removed by evaporation, and the resulting product was recrystallized and separated by silica gel chromatography to obtain 0.29 g (yield of 5 %) of Compound 30.

MS (MALDI-TOF) m/z: 1484 [M]+.

### Synthesis Example 6: Synthesis of Compound 36

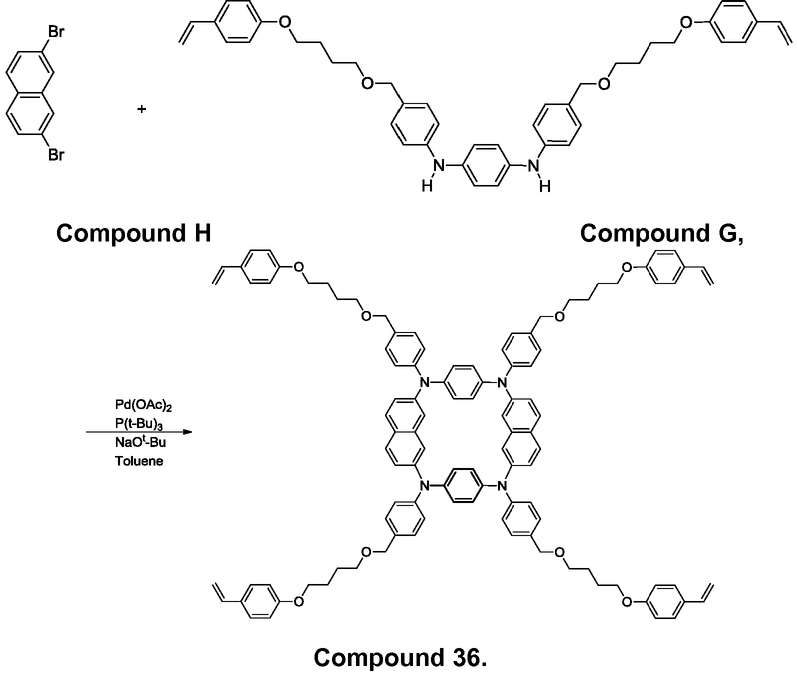

0.310 g (yield of 10 %) of Compound 36 was synthesized in substantially the same manner as in the Synthesis of Compound 30, except that Compound H was used instead of Compound F.

MS (MALDI-TOF) m/z: 1584 [M]+.

### Synthesis Example 7: Synthesis of Compound 40

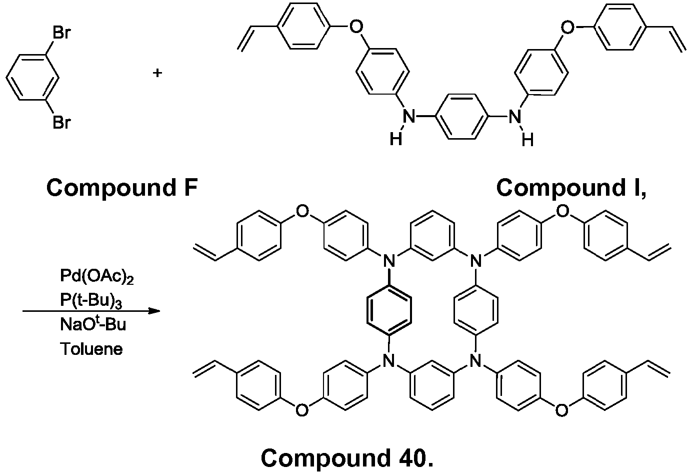

0.205 g (yield of 9 %) of Compound 40 was synthesized in substantially the same manner as in the Synthesis of Compound 30, except that Compound I was used instead of Compound G.

MS (MALDI-TOF) m/z: 1140 [M]+.

Synthesis methods of compounds other than the above Compounds may also be easily recognized by those of ordinary skill in the art, for example, by referring to the synthesis mechanisms and source materials described above.

### Example 1

As an anode, a Corning 15 Ω/cm² (120 nm) ITO glass substrate was cut to a size of 50 mm x 50 mm x 0.7 mm, sonicated with isopropyl alcohol and pure water each for 5 minutes, and then cleaned by exposure to ultraviolet (UV) rays and ozone for 30 minutes.

PEDOT-PSS (Al4083) manufactured by Bayer was coated on the glass substrate and thermally treated at a temperature of 150 °C for 30 minutes to form a hole injection layer having a thickness of 1,000 Å. A mixture including 2 g of xylene solution and 0.1 g of Compound 1 was spin-coated on the hole injection layer, dried at a temperature of 100 °C for 10 minutes, and thermally cross-linked at a temperature of 200 °C for 30 minutes to form a hole transport layer having a thickness of 200 Å.

A solution in which Compound H-1 (host) and Compound D-1 (dopant) (a host ratio of 5%) were dissolved in xylene solution was spin-coated on the hole transport layer and dried at a temperature of 100 °C for 10 minutes to form an emission layer having a thickness of 350 Å.

Compound E-1 and 8-hydroxyquinolinolato-lithium (LiQ) were vacuum-deposited on the emission layer at a ratio of 5:5 (1:1) to form an electron transport layer having a thickness of 200 Å. LiQ (electron injection layer) having a thickness of 10 Å and Al (cathode) having a thickness of 2,000 Å were sequentially vacuum-deposited on the electron transport layer, thereby completing the manufacture of an organic light-emitting device.

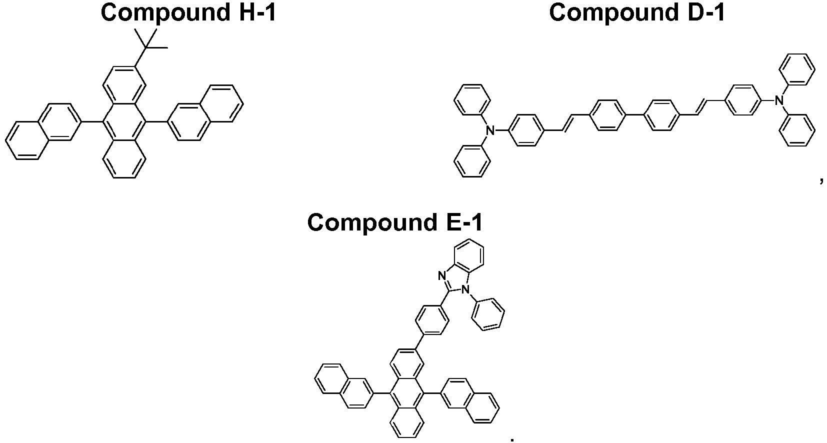

### Examples 2 to 7 and Comparative Examples 1 to 5

Organic light-emitting devices were manufactured in substantially the same manner as in Example 1, except that each of the Compounds shown in Table 1 were used instead of Compound 1 in forming a hole transport layer.

**Table 1**

| | Hole transport layer compound | Driving voltage (@700nit) | Current efficiency (cd/A) | Lifespan (T90) |
|---|---|---|---|---|
| Example 1 | 1 | 7.1 | 5.1 | 50 |
| Example 2 | 4 | 7.3 | 5.2 | 35 |
| Example 3 | 7 | 7.2 | 5.4 | 40 |
| Example 4 | 16 | 6.8 | 5.9 | 51 |
| Example 5 | 30 | 6.9 | 5.8 | 54 |
| Example 6 | 36 | 7.0 | 6.1 | 70 |
| Example 7 | 40 | 6.8 | 5.8 | 60 |
| Comparative Example 1 | A-1 | 9.5 | 4.0 | 5 |
| Comparative Example 2 | A-2 | 9.7 | 4.1 | 10 |
| Comparative Example 3 | A-3 | 10.1 | 3.1 | 1 |
| Comparative Example 4 | A-4 | 9.2 | 4.2 | 10 |
| Comparative Example 5 | A-5 | 9.8 | 3.2 | 2 |

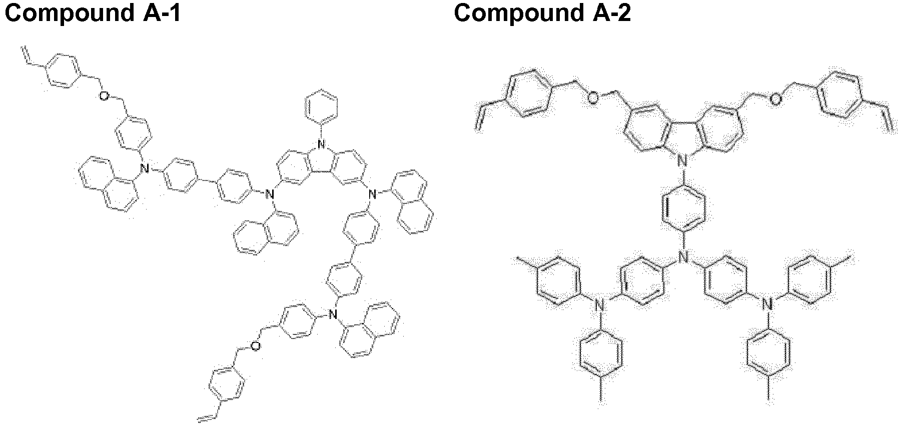

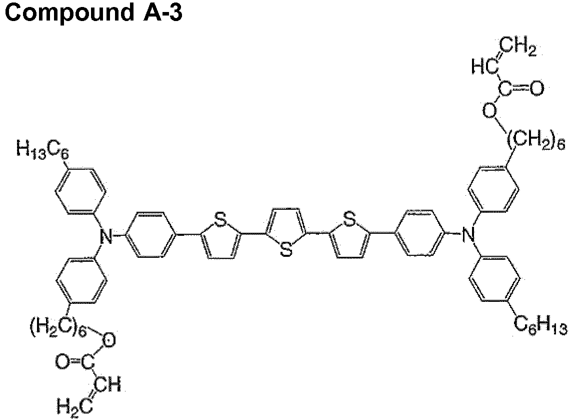

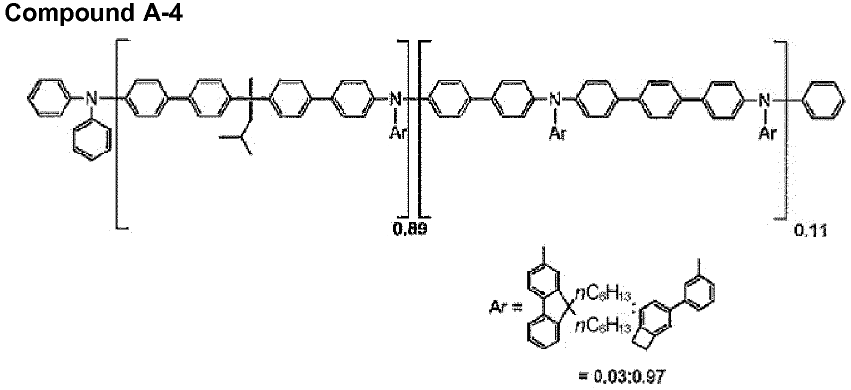

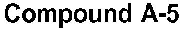

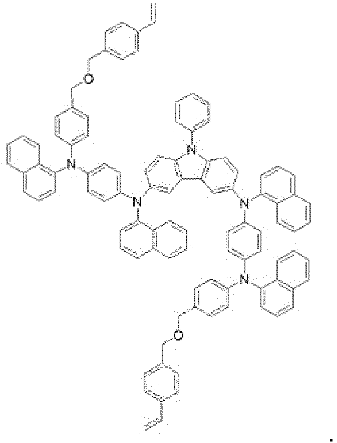

Referring to Table 1, it is confirmed that the organic light-emitting devices of Examples 1 to 7 have a low driving voltage, high efficiency, and excellent lifespan characteristics, compared with the organic light-emitting devices of Comparative Examples 1 to 5.

The organic light-emitting device according to embodiments of the present disclosure may have improved device characteristics, such as a low driving voltage, high efficiency, and a long lifespan.

It should be understood that embodiments described herein should be considered in a descriptive sense only and not for purposes of limitation. Descriptions of features or aspects within each embodiment should typically be considered as available for other similar features or aspects in other embodiments.

As used herein, the terms "use", "using", and "used" may be considered synonymous with the terms "utilize", "utilizing", and "utilized", respectively. Further, the terms "substantially", "about", and similar terms are used as terms of approximation and not as terms of degree, and are intended to account for the inherent deviations in measured or calculated values that would be recognized by those of ordinary skill in the art.

Also, any numerical range recited herein is intended to include all subranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein.

While one or more embodiments have been described with reference to the drawings, it will be understood by those of ordinary skill in the art that various changes in form and details may be made therein without departing from the scope as defined by the following claims.

## Claims

1. An organic light-emitting device (10, 20, 30, 40) comprising:
a first electrode (110);
a second electrode (190) facing the first electrode (110); and
an organic layer (150) between the first electrode (110) and the second electrode (190) and comprising an emission layer,
**characterized in that**
the organic layer (150) further comprises at least one of an arylamine-based polymer formed by cross-linking a cross-linkable arylamine-based compound represented by Formula 1a or 1b:
**Formula 1a** A₁-(B)ₚ-A₂, 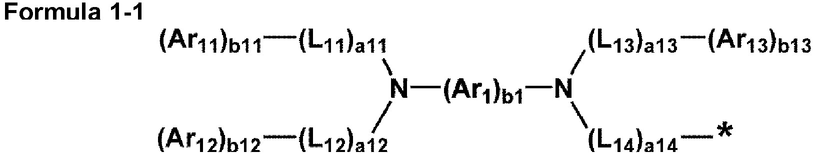 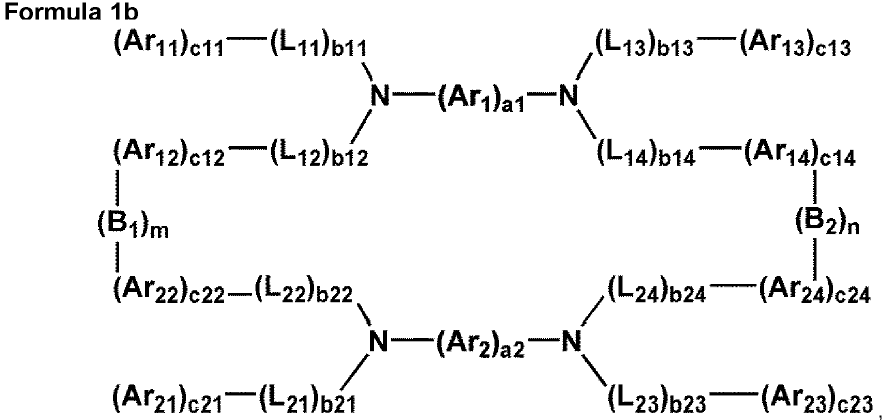
wherein, in Formula 1a, A₁ and A₂ are each a group represented by Formula 1-1, and A₁ and A₂ are identical to or different from each other,
in Formula 1a, B is selected from a substituted or unsubstituted C₅-C₆₀ carbocyclic group, a substituted or unsubstituted C₁-C₆₀ heterocyclic group, and *'-Si(Q₄₁)(Q₄₂)-*"; and p is an integer of 1 to 10,
in Formula 1-1, * indicates a binding site to (B)ₚ in Formula 1a,
in Formula 1-1, Ar₁ is selected from a benzene group, a pyridine group, a pyrimidine group, a pyrazine group, a triazine group, a furan group, a thiophene group, an imidazole group, a thiazole group, an isoxazole group, and an oxazole group,
in Formula 1-1, b1 is an integer of four or more, and four or more Ar₁(s) are identical to or different from each other,
in Formula 1-1, L₁₁ to L₁₄ are each independently a substituted or unsubstituted C₅-C₆₀ carbocyclic group or a substituted or unsubstituted C₁-C₆₀ heterocyclic group,
in Formula 1-1, a11 to a14 are each independently 0, 1, 2, 3, or 4,
in Formula 1-1, when a11 is 0, *'-(L₁₁)ₐ₁₁-*" is a single bond; when a12 is 0, *'-(L₁₂)ₐ₁₂-*" is a single bond; when a13 is 0, *'-(L₁₃)ₐ₁₃-*" is a single bond; when a14 is 0, *'-(L₁₄)ₐ₁₄-*" is a single bond; when a11 is two or more, two or more L₁₁(s) are identical to or different from each other; when a12 is two or more, two or more L₁₂(s) are identical to or different from each other; when a13 is two or more, two or more L₁₃(s) are identical to or different from each other; and when a14 is two or more, two or more L₁₄(s) are identical to or different from each other,
in Formula 1-1, Ar₁₁ to Ar₁₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein at least one of Ar₁₁ to Ar₁₃ is substituted with a cross-linkable group,
in Formula 1-1, b11 to b13 are each independently 1, 2, 3, 4, or 5, wherein, when b11 is two or more, two or more Ar₁₁(s) are identical to or different from each other; when b12 is two or more, two or more Ar₁₂(s) are identical to or different from each other; and when b13 is two or more, two or more Ar₁₃(s) are identical to or different from each other,
in Formula 1b, Ar₁ and Ar₂ are each independently a substituted or unsubstituted C₅-C₆₀ carbocyclic group or a substituted or unsubstituted C₁-C₆₀ heterocyclic group, wherein Ar₁ and Ar₂ are identical to or different from each other,
in Formula 1b, a1 and a2 are each independently an integer of 1 to 5, wherein, when a1 is two or more, two or more Ar₁(s) are identical to or different from each other; and when a2 is two or more, two or more Ar₂(s) are identical to or different from each other,
in Formula 1b, L₁₁ to L₁₄ and L₂₁ to L₂₄ are each independently a substituted or unsubstituted C₅-C₆₀ carbocyclic group or a substituted or unsubstituted C₁-C₆₀ heterocyclic group,
in Formula 1b, b11 to b14 and b21 to b24 are each independently an integer of 0 to 3, wherein, when b11 is 0, *-(L₁₁)_{b11}-*' is a single bond; when b12 is 0, *-(L₁₂)_{b12}-*' is a single bond; when b13 is 0, *-(L₁₃)_{b13}-*' is a single bond; when b14 is 0, *-(L₁₄)_{b14}-*' is a single bond; when b21 is 0, *-(L₂₁)_{b21}-*' is a single bond; when b22 is 0, *-(L₂₂)_{b22}-*' is a single bond; when b23 is 0, *-(L₂₃)_{b23}-*' is a single bond; when b24 is 0, *-(L₂₄)_{b24}-*' is a single bond; when b11 is two or more, two or more L₁₁(s) are identical to or different from each other; when b12 is two or more, two or more L₁₂(s) are identical to or different from each other; when b13 is two or more, two or more L₁₃(s) are identical to or different from each other; when b14 is two or more, two or more L₁₄(s) are identical to or different from each other; when b21 is two or more, two or more L₂₁(s) are identical to or different from each other; when b22 is two or more, two or more L₂₂(s) are identical to or different from each other; when b23 is two or more, two or more L₂₃(s) are identical to or different from each other; and when b24 is two or more, two or more L₂₄(s) are identical to or different from each other,
in Formula 1b, B₁ and B₂ are each independently selected from a single bond, a substituted or unsubstituted C₁-C₂₀ alkylene group, a substituted or unsubstituted C₂-C₂₀ alkenylene group, and a substituted or unsubstituted C₂-C₂₀ alkynylene group,
in Formula 1b, m and n are each independently an integer of 1 to 3, wherein, when m is two or more, two or more B₁(s) are identical to or different from each other; and when n is two or more, two or more B₂(s) are identical to or different from each other,
in Formula 1b, Ar₁₁, Ar₁₃, Ar₂₁, and Ar₂₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group,
in Formula 1b, Ar₁₂, Ar₁₄, Ar₂₂, and Ar₂₄ are each independently selected from a single bond, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
in Formula 1b, c11 to c14 and c21 to c24 are each independently an integer of 1 to 5, wherein, when c11 is two or more, two or more Ar₁₁(s) are identical to or different from each other; when c12 is two or more, two or more Ar₁₂(s) are identical to or different from each other; when c13 is two or more, two or more Ar₁₃(s) may be identical to or different from each other; when c14 is two or more, two or more Ar₁₄(s) are identical to or different from each other; when c21 is two or more, two or more Ar₂₁(s) are identical to or different from each other; when c22 is two or more, two or more Ar₂₂(s) are identical to or different from each other; when c23 is two or more, two or more Ar₂₃(s) are identical to or different from each other; and when c24 is two or more, two or more Ar₂₄(s) are identical to or different from each other,
in Formula 1b, at least one group selected from Ar₁, Ar₂, Ar₁₁ to Ar₁₄, and Ar₂₁ to Ar₂₄ is substituted with a cross-linkable group,
*'and *" each indicate a binding site to a neighboring atom,
at least one substituent of the substituted C₅-C₆₀ carbocyclic group, the substituted C₁-C₆₀ heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, the substituted monovalent non-aromatic condensed heteropolycyclic group, the substituted C₁-C₂₀ alkylene group, the substituted C₂-C₂₀ alkenylene group, and the substituted C₂-C₂₀ alkynylene group is selected from:
deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, - Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₁₁)(Q₁₂)(Q₁₃), -N(Q₁₁)(Q₁₂), -B(Q₁₁)(Q₁₂), -C(=O)(Q₁₁), - S(=O)₂(Q₁₁), and -P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, a biphenyl group, and a terphenyl group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and - P(=O)(Q₃₁)(Q₃₂), and
Q₁₁ to Q₁₃, Q₂₁ to Q₂₃, Q₃₁ to Q₃₃, and Q₄₁ to Q₄₂ are each independently selected from hydrogen, deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, a biphenyl group, and a terphenyl group.

2. An organic light-emitting device (10, 20, 30, 40) according to claim 1, wherein:
the first electrode (110) is an anode,
the second electrode (190) is a cathode, and
the organic layer (150) further comprises i) a hole transport region between the first electrode and the emission layer and comprising a hole injection layer, a hole transport layer, a buffer layer, an electron blocking layer, or any combination thereof; and ii) an electron transport region between the emission layer and the second electrode and comprising a hole blocking layer, an electron transport layer, an electron injection layer, or any combination thereof.

3. An organic light-emitting device (10, 20, 30, 40) according to claim 2, wherein the hole transport region comprises at least one of the arylamine-based polymer.

4. An organic light-emitting device (10, 20, 30, 40) according to claim 2, wherein:
the hole transport region comprises a hole injection layer and a hole transport layer, and
the hole transport layer comprises at least one of the arylamine-based polymer.

5. An electronic apparatus comprising:
a thin film transistor; and
the organic light-emitting device (10, 20, 30, 40) according to any one of claims 1 to 4,
wherein the thin film transistor comprises a source electrode, a drain electrode, an active layer, and a gate electrode, and
the first electrode of the organic light-emitting device is electrically connected to one of the source electrode and the drain electrode of the thin film transistor.

6. A cross-linkable arylamine-based compound represented by Formula 1a or 1b:
**Formula 1a** A₁-(B)ₚ-A₂, 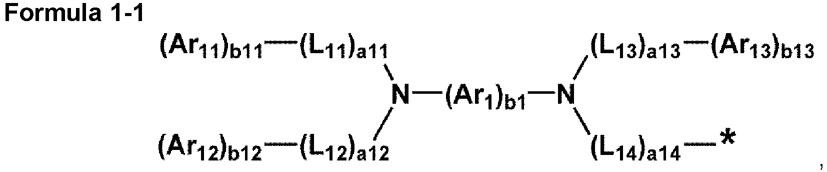 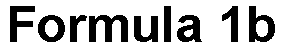 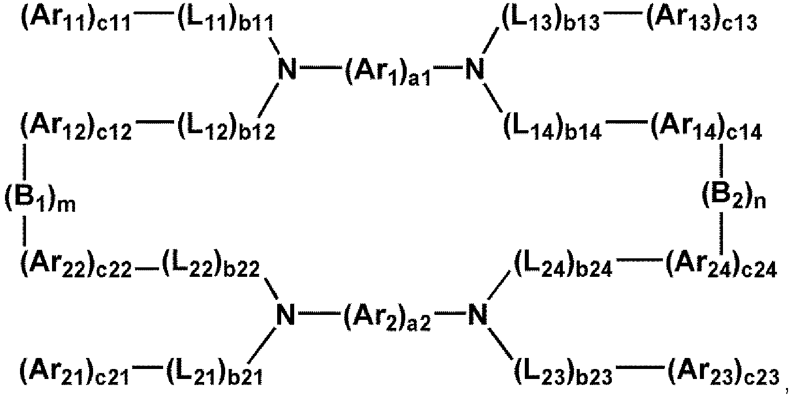
wherein, in Formula 1a, A₁ and A₂ are each a group represented by Formula 1-1, and A₁ and A₂ are identical to or different from each other,
in Formula 1a, B is selected from a substituted or unsubstituted C₅-C₆₀ carbocyclic group, a substituted or unsubstituted C₁-C₆₀ heterocyclic group, and *'-Si(Q₄₁)(Q₄₂)-*"; and p is an integer of 1 to 10,
in Formula 1-1, * indicates a binding site to (B)ₚ in Formula 1a,
in Formula 1-1, Ar₁ is selected from a benzene group, a pyridine group, a pyrimidine group, a pyrazine group, a triazine group, a furan group, a thiophene group, an imidazole group, a thiazole group, an isoxazole group, and an oxazole group,in Formula 1-1, b1 is an integer of four or more, and four or more Ar₁(s) are identical to or different from each other,
in Formula 1-1, L₁₁ to L₁₄ are each independently a substituted or unsubstituted C₅-C₆₀ carbocyclic group or a substituted or unsubstituted C₁-C₆₀ heterocyclic group,
in Formula 1-1, a11 to a14 are each independently 0, 1, 2, 3, or 4,
in Formula 1-1, when a11 is 0, *'-(L₁₁)ₐ₁₁-*" is a single bond; when a12 is 0, *'-(L₁₂)ₐ₁₂-*" is a single bond; when a13 is 0, *'-(L₁₃)ₐ₁₃-*" is a single bond; when a14 is 0, *'-(L₁₄)ₐ₁₄-*" is a single bond; when a11 is two or more, two or more L₁₁(s) are identical to or different from each other; when a12 is two or more, two or more L₁₂(s) are identical to or different from each other; when a13 is two or more, two or more L₁₃(s) are identical to or different from each other; and when a14 is two or more, two or more L₁₄(s) are identical to or different from each other,
in Formula 1-1, Ar₁₁ to Ar₁₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein at least one of Ar₁₁ to Ar₁₃ is substituted with a cross-linkable group,
in Formula 1-1, b11 to b13 are each independently 1, 2, 3, 4, or 5, wherein, when b11 is two or more, two or more Ar₁₁(s) are identical to or different from each other; when b12 is two or more, two or more Ar₁₂(s) are identical to or different from each other; and when b13 is two or more, two or more Ar₁₃(s) are identical to or different from each other,
in Formula 1b, Ar₁ and Ar₂ are each independently a substituted or unsubstituted C₅-C₆₀ carbocyclic group or a substituted or unsubstituted C₁-C₆₀ heterocyclic group, wherein Ar₁ and Ar₂ are identical to or different from each other,
in Formula 1b, a1 and a2 are each independently an integer of 1 to 5, wherein, when a1 is two or more, two or more Ar₁(s) are identical to or different from each other; and when a2 is two or more, two or more Ar₂(s) are identical to or different from each other,
in Formula 1b, L₁₁ to L₁₄ and L₂₁ to L₂₄ are each independently a substituted or unsubstituted C₅-C₆₀ carbocyclic group or a substituted or unsubstituted C₁-C₆₀ heterocyclic group,
in Formula 1b, b11 to b14 and b21 to b24 are each independently an integer of 0 to 3, wherein, when b11 is 0, *-(L₁₁)_{b11}-*' is a single bond; when b12 is 0, *-(L₁₂)_{b12}-*' is a single bond; when b13 is 0, *-(L₁₃)_{b13}-*' is a single bond; when b14 is 0, *-(L₁₄)_{b14}-*' is a single bond; when b21 is 0, *-(L₂₁)_{b21}-*' is a single bond; when b22 is 0, *-(L₂₂)_{b22}-*' is a single bond; when b23 is 0, *-(L₂₃)_{b23}-*' is a single bond; when b24 is 0, *-(L₂₄)_{b24}-*' is a single bond; when b11 is two or more, two or more L₁₁(s) are identical to or different from each other; when b12 is two or more, two or more L₁₂(s) are identical to or different from each other; when b13 is two or more, two or more L₁₃(s) are identical to or different from each other; when b14 is two or more, two or more L₁₄(s) are identical to or different from each other; when b21 is two or more, two or more L₂₁(s) are identical to or different from each other; when b22 is two or more, two or more L₂₂(s) are identical to or different from each other; when b23 is two or more, two or more L₂₃(s) are identical to or different from each other; and when b24 is two or more, two or more L₂₄(s) are identical to or different from each other,
in Formula 1b, B₁ and B₂ are each independently selected from a single bond, a substituted or unsubstituted C₁-C₂₀ alkylene group, a substituted or unsubstituted C₂-C₂₀ alkenylene group, and a substituted or unsubstituted C₂-C₂₀ alkynylene group,
in Formula 1b, m and n are each independently an integer of 1 to 3, wherein, when m is two or more, two or more B₁(s) are identical to or different from each other; and when n is two or more, two or more B₂(s) are identical to or different from each other,
in Formula 1b, Ar₁₁, Ar₁₃, Ar₂₁, and Ar₂₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group,
in Formula 1b, Ar₁₂, Ar₁₄, Ar₂₂, and Ar₂₄ are each independently selected from a single bond, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
in Formula 1b, c11 to c14 and c21 to c24 are each independently an integer of 1 to 5, wherein, when c11 is two or more, two or more Ar₁₁(s) are identical to or different from each other; when c12 is two or more, two or more Ar₁₂(s) are identical to or different from each other; when c13 is two or more, two or more Ar₁₃(s) may be identical to or different from each other; when c14 is two or more, two or more Ar₁₄(s) are identical to or different from each other; when c21 is two or more, two or more Ar₂₁(s) are identical to or different from each other; when c22 is two or more, two or more Ar₂₂(s) are identical to or different from each other; when c23 is two or more, two or more Ar₂₃(s) are identical to or different from each other; and when c24 is two or more, two or more Ar₂₄(s) are identical to or different from each other,
in Formula 1b, at least one group of Ar₁, Ar₂, Ar₁₁ to Ar₁₄, and Ar₂₁ to Ar₂₄ is substituted with a cross-linkable group,
*'and *" each indicate a binding site to a neighboring atom,
at least one substituent of the substituted C₅-C₆₀ carbocyclic group, the substituted C₁-C₆₀ heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, the substituted monovalent non-aromatic condensed heteropolycyclic group, the substituted C₁-C₂₀ alkylene group, the substituted C₂-C₂₀ alkenylene group, and the substituted C₂-C₂₀ alkynylene group is selected from:
deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, - Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₁₁)(Q₁₂)(Q₁₃), -N(Q₁₁)(Q₁₂), -B(Q₁₁)(Q₁₂), -C(=O)(Q₁₁), - S(=O)₂(Q₁₁), and -P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, a biphenyl group, and a terphenyl group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), and -P(=O)(Q₂₁)(Q₂₂), and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and - P(=O)(Q₃₁)(Q₃₂), and
Q₁₁ to Q₁₃, Q₂₁ to Q₂₃, Q₃₁ to Q₃₃, and Q₄₁ to Q₄₂ are each independently selected from hydrogen, deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, a biphenyl group, and a terphenyl group.

7. A cross-linkable arylamine-based compound according to claim 6, wherein in Formula 1a, A₁ and A₂ are identical to each other.

8. A cross-linkable arylamine-based compound according to claim 6 or claim 7, wherein the arylamine-based compound represented by Formula 1a is symmetrical with respect to *'-(B)ₚ-*".

9. A cross-linkable arylamine-based compound according to any one of claims 6 to 8, wherein:
in Formula 1b, Ar₁ and Ar₂ are each independently selected from:
a C₆-C₆₀ arylene group and a C₁-C₆₀ heteroarylene group; and
a C₆-C₆₀ arylene group and a C₁-C₆₀ heteroarylene group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₁-C₆₀ alkoxy group, -N(Q₃₁)(Q₃₂), and a cross-linkable group,
wherein Q₃₁ and Q₃₂ are the same as defined in any one of claims 6 to 8.

10. A cross-linkable arylamine-based compound according to any one of claims 6 to 9, wherein:
in Formula 1-1, Ar₁ is a benzene group, and b1 is 4 or 5, and
in Formula 1b, Ar₁ and Ar₂ are each independently selected from:
a phenylene group and a naphthylene group; and
a phenylene group and a naphthylene group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₁-C₆₀ alkoxy group, -N(Q₃₁)(Q₃₂), and a cross-linkable group,
wherein Q₃₁ and Q₃₂ are the same as defined in any one of claims 6 to 8.

11. A cross-linkable arylamine-based compound according to any one of claims 6 to 10, wherein:
in Formula 1-1, the four or more Ar₁(s) are identical to each other, and
in Formula 1b, when a1 is two or more, two or more Ar₁(s) are identical to each other, and when a2 is two or more, two or more Ar₂(s) are identical to each other.

12. A cross-linkable arylamine-based compound according to any one of claims 6 to 11, wherein, in Formula 1b, *-(Ar₁)ₐ₁-*' and *-(Ar₂)ₐ₂-*' are identical to each other.

13. A cross-linkable arylamine-based compound according to any one of claims 6 to 12, wherein:
L₁₁ to L₁₄ in Formula 1-1 and L₁₁ to L₁₄ and L₂₁ to L₂₄ in Formula 1b are each independently selected from groups represented by Formulae 2-1 to 2-37: 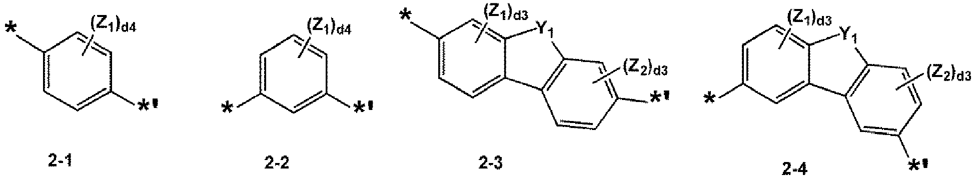 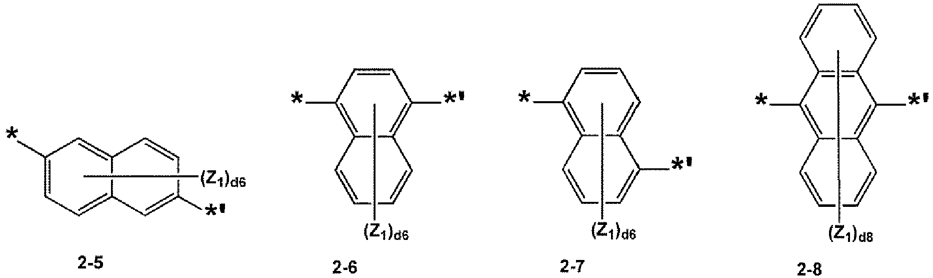 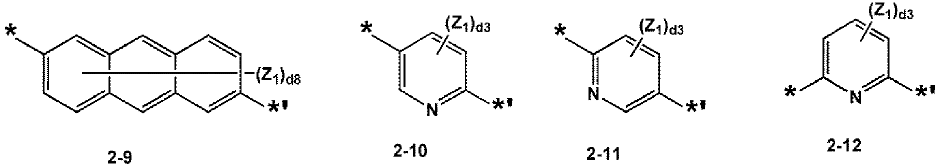 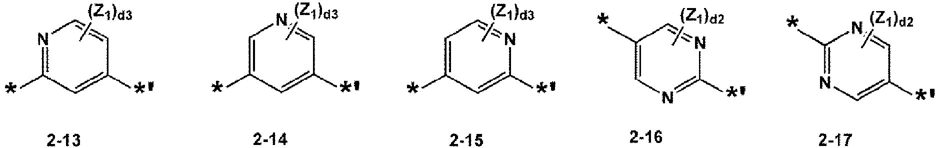 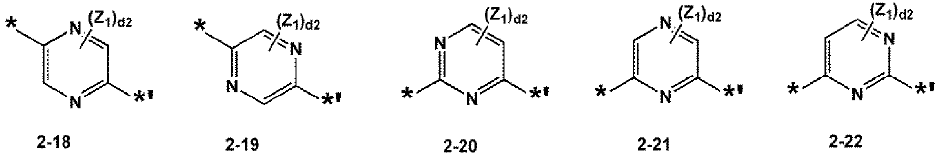 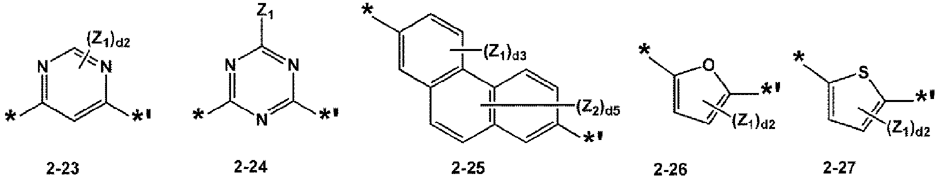 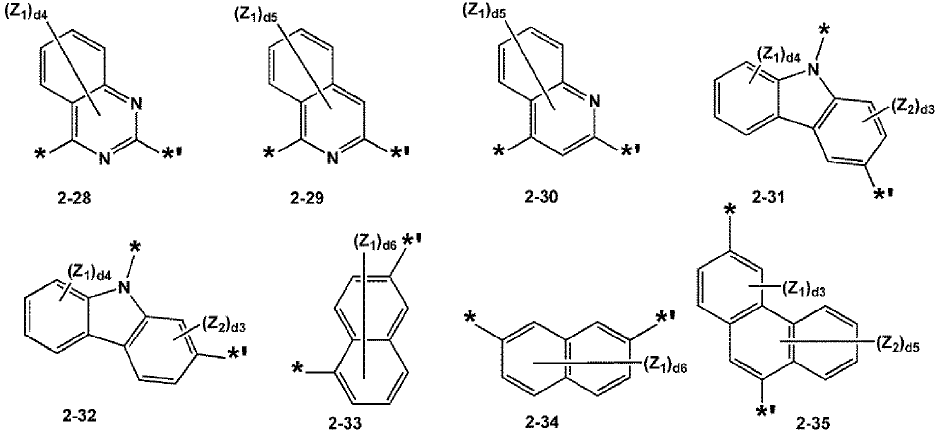 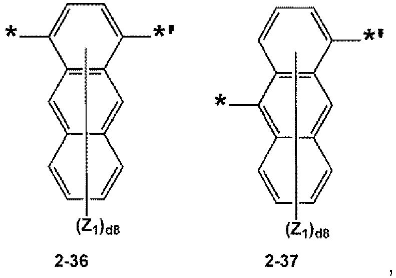 wherein, in Formulae 2-1 to 3-37,
Y₁ is O, S, C(Z₃)(Z₄), N(Z₅), or Si(Z₆)(Z₇),
Z₁ to Z₇ are each independently selected from:
hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, and an imidazopyridinyl group,
d2 is an integer of 0 to 2,
d3 is an integer of 0 to 3,
d4 is an integer of 0 to 4,
d5 is an integer of 0 to 5,
d6 is an integer of 0 to 6,
d8 is an integer of 0 to 8, and
* and *' each indicate a binding site to a neighboring atom.

14. A cross-linkable arylamine-based compound according to any one of claims 6 to 13, wherein:
in Formula 1a, *-(B)ₚ-*' is selected from groups represented by Formulae 3-1 to 3-19: 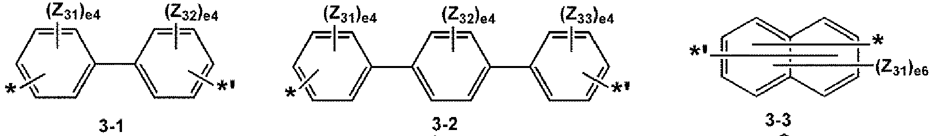 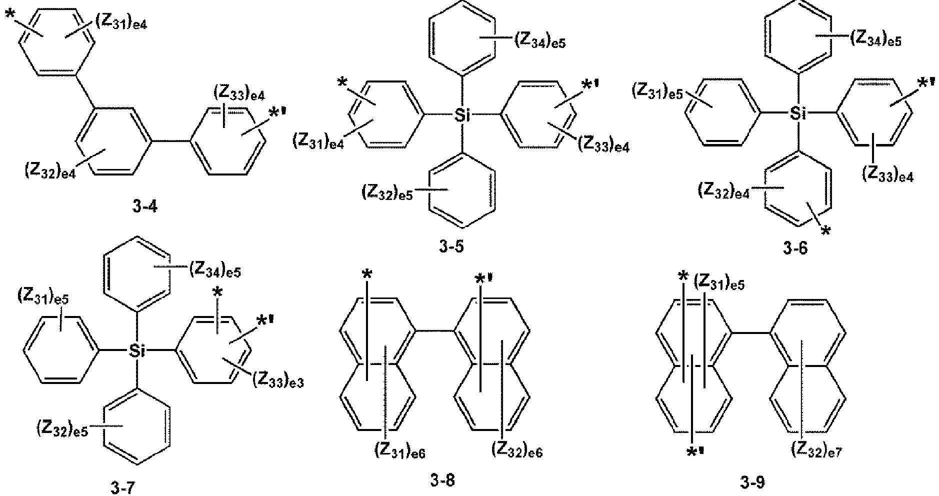 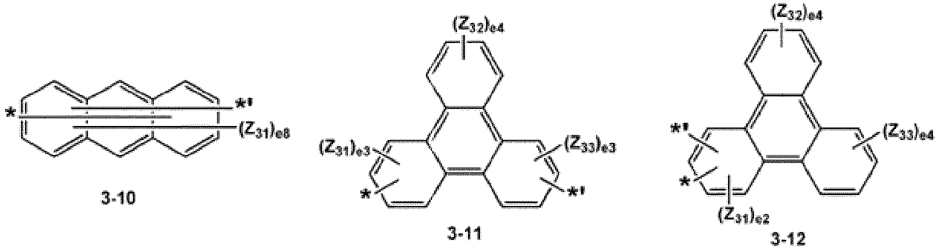 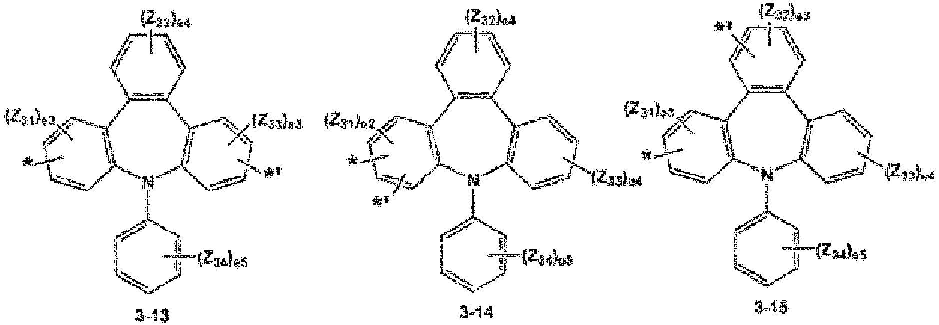 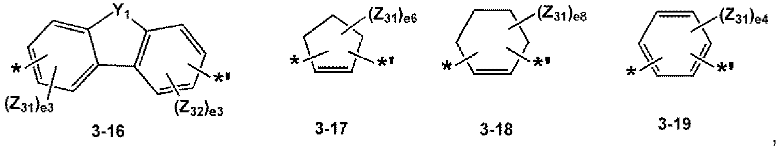 wherein, in Formulae 3-1 to 3-19,
Y₁ is O, S, C(Z₃₅)(Z₃₆), N(Z₃₅), or Si(Z₃₅)(Z₃₆),
Z₃₁ to Z₃₆ are each independently selected from hydrogen, deuterium, -F, -Cl, - Br, -I, a hydroxyl group, a cyano group, a nitro group, an amidino group, a hydrazino group, a hydrazono group, a C₁-C₂₀ alkyl group, a C₁-C₂₀ alkoxy group, a cyclopropenyl group, a cyclobutenyl group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthyl group, a fluorenyl group, a spiro-bifluorenyl group, a spiro-benzofluorene-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, an isoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, an isobenzothiazolyl group, a benzoxazolyl group, an isobenzoxazolyl group, a triazolyl group, a tetrazolyl group, an oxadiazolyl group, a triazinyl group, a carbazolyl group, a dibenzofuranyl group, a dibenzothiophenyl group, a dibenzosilolyl group, a benzocarbazolyl group, a naphtho benzofuranyl group, a naphtho benzothiophenyl group, a naphthobenzosilolyl group, a dibenzocarbazolyl group, a dinaphthofuranyl group, a dinaphtho thiophenyl group, a dinaphtho silolyl group, a thiadiazolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indeno pyrrolyl group, an indolopyrrolyl group, an indeno carbazolyl group, an indolocarbazolyl group, - Si(Q₃₁)(Q₃₂)(Q₃₃), and -N(Q₃₁)(Q₃₂),
Q₃₁ to Q₃₃ are each independently selected from:
a C₁-C₁₀ alkyl group, a C₁-C₁₀ alkoxy group, a phenyl group, a biphenyl group, a terphenyl group, and a naphthyl group; and
a phenyl group, a biphenyl group, a terphenyl group, and a naphthyl group, each substituted with at least one selected from a C₁-C₁₀ alkyl group, a C₁-C₁₀ alkoxy group, and a phenyl group,
e2 is an integer of 0 to 2,
e3 is an integer of 0 to 3,
e4 is an integer of 0 to 4,
e5 is an integer of 0 to 5,
e6 is an integer of 0 to 6,
e7 is an integer of 0 to 7,
e8 is an integer of 0 to 8, and
* and *' each indicate a binding site to a neighboring atom.

15. A cross-linkable arylamine-based compound according to any one of claims 6 to 14, wherein:
in Formula 1-1, Ar₁₁ to Ar₁₃ are each independently selected from:
a C₆-C₆₀ aryl group; and
a C₆-C₆₀ aryl group substituted with at least one selected from deuterium, -F, - Cl, -Br, -I, a hydroxyl group, a cyano group, a nitro group, an amidino group, a hydrazino group, a hydrazono group, a C₂-C₆₀ alkenyl group, a C₂-C₆₀ alkynyl group, and a C₁-C₆₀ alkoxy group, and
in Formula 1b, Ar₁₁, Ar₁₃, Ar₂₁, and Ar₂₃ are each independently selected from:
a phenyl group, a biphenyl group, a terphenyl group, and a naphthyl group; and
a phenyl group, a biphenyl group, a terphenyl group, and a naphthyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a cross-linkable group, and -N(Q₃₁)(Q₃₂),
wherein Q₃₁ and Q₃₂ are the same as defined in any one of claims 6 to 14.

16. A cross-linkable arylamine-based compound according to any one of claims 6 to 15, wherein in Formula 1b, Ar₁₂, Ar₁₄, Ar₂₂, and Ar₂₄ are each independently selected from:
a single bond;
a phenylene group and a naphthylene group; and
a phenylene group and a naphthylene group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₁-C₆₀ alkoxy group, -N(Q₃₁)(Q₃₂), and a cross-linkable group,
wherein Q₃₁ and Q₃₂ are the same as defined in any one of claims 6 to 14.

17. A cross-linkable arylamine-based compound according to any one of claims 6 to 16, wherein in Formula 1b, Ar₁₁ and Ar₁₃ are identical to each other, Ar₂₁ and Ar₂₃ are identical to each other, Ar₁₂ and Ar₁₄ are identical to each other, and Ar₂₂ and Ar₂₄ are identical to each other.

18. A cross-linkable arylamine-based compound according to any one of claims 6 to 17, wherein in Formula 1b, substituents of Ar₁₁, Ar₁₃, Ar₂₁, and Ar₂₃ comprise a cross-linkable group.

19. A cross-linkable arylamine-based compound according to any one of claims 6 to 18, wherein in Formula 1b,
substituents of at least two groups selected from Ar₁, Ar₂, Ar₁₂, Ar₁₄, Ar₂₂, and Ar₂₄ comprise a cross-linkable group, and B₁ and B₂ are each a single bond.

20. A cross-linkable arylamine-based compound according to any one of claims 6 to 19, wherein the cross-linkable group comprises at least one selected from a vinylene moiety, a styrene moiety, a cyclobutane moiety, and an epoxy moiety.

21. A cross-linkable arylamine-based compound according to any one of claims 6 to 20, wherein the cross-linkable group is selected from groups represented by Formulae 4-1 to 4-14: 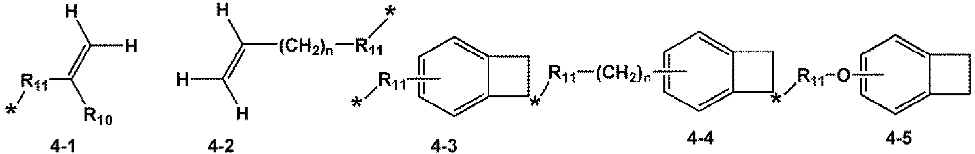 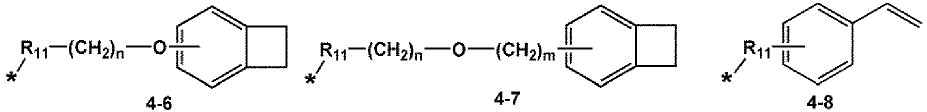 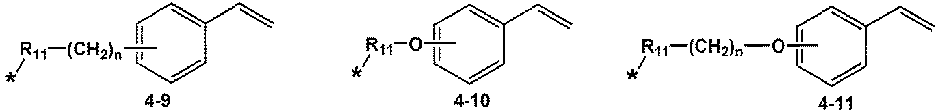 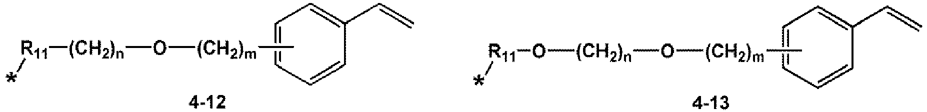 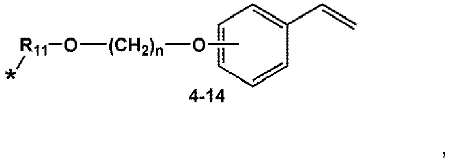 wherein, in Formulae 4-1 to 4-14,
R₁₀ is a hydrogen atom or a substituted or unsubstituted C₁-C₂₀ alkyl group,
R₁₁ is selected from a single bond, a substituted or unsubstituted C₁-C₂₀ alkylene group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
m and n are each independently an integer of 1 to 10, and
* indicates a binding site to a neighboring atom.

22. A cross-linkable arylamine-based compound according to claim 6, wherein the cross-linkable arylamine-based compound is selected from Compounds 1 to 40:

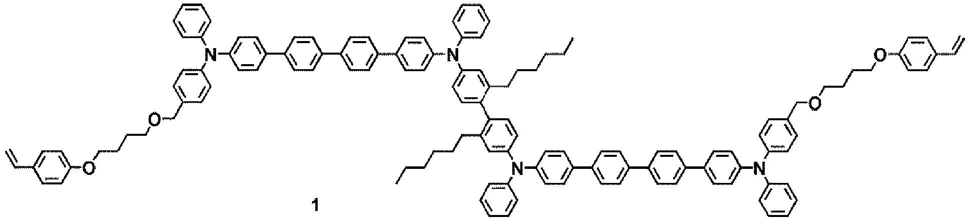

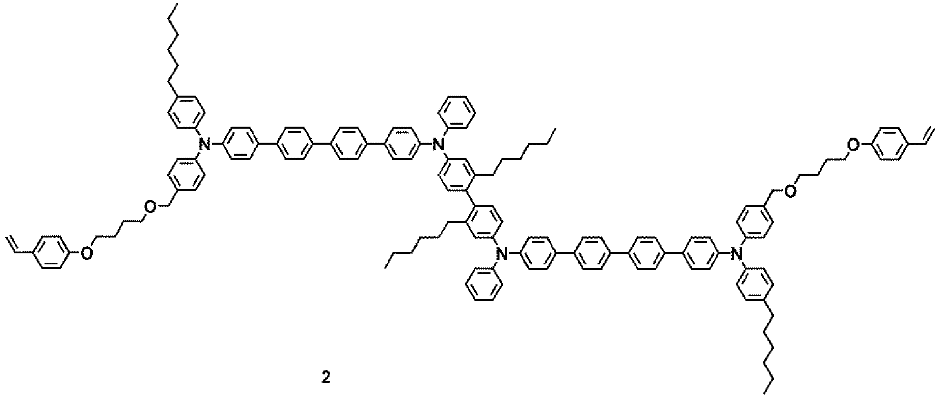

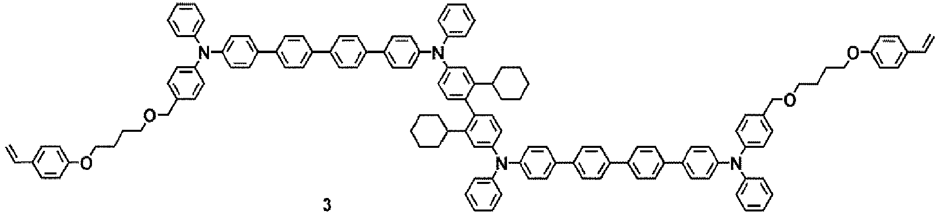

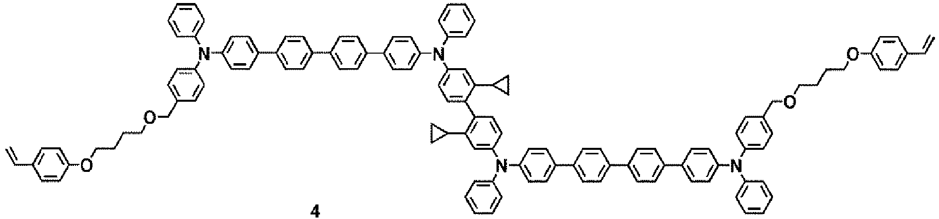

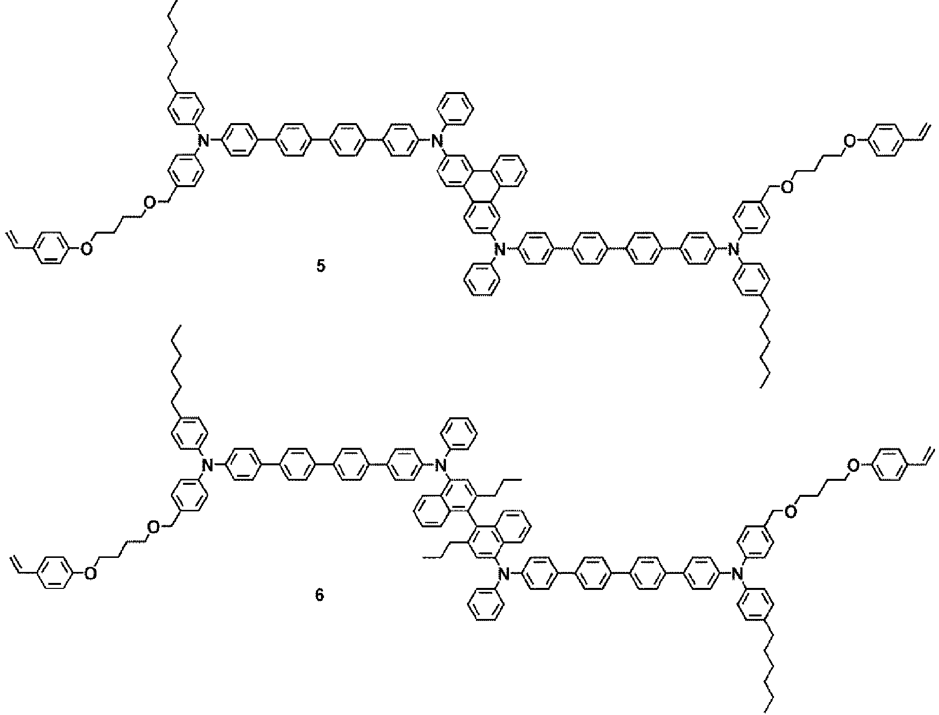

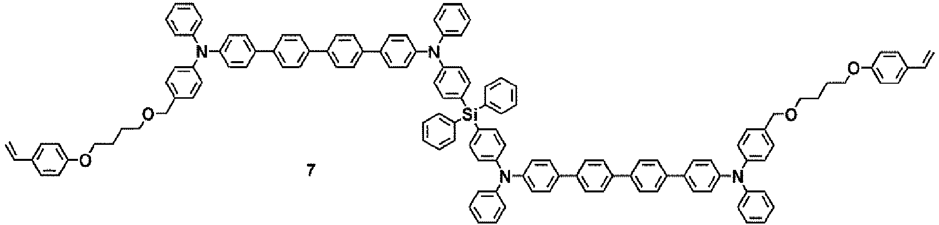

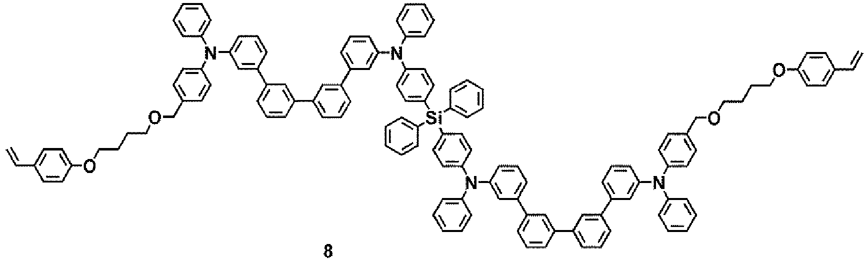

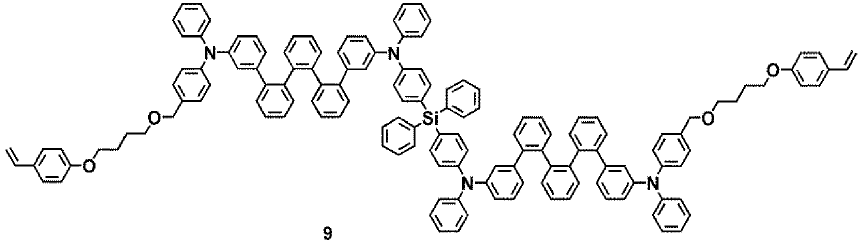

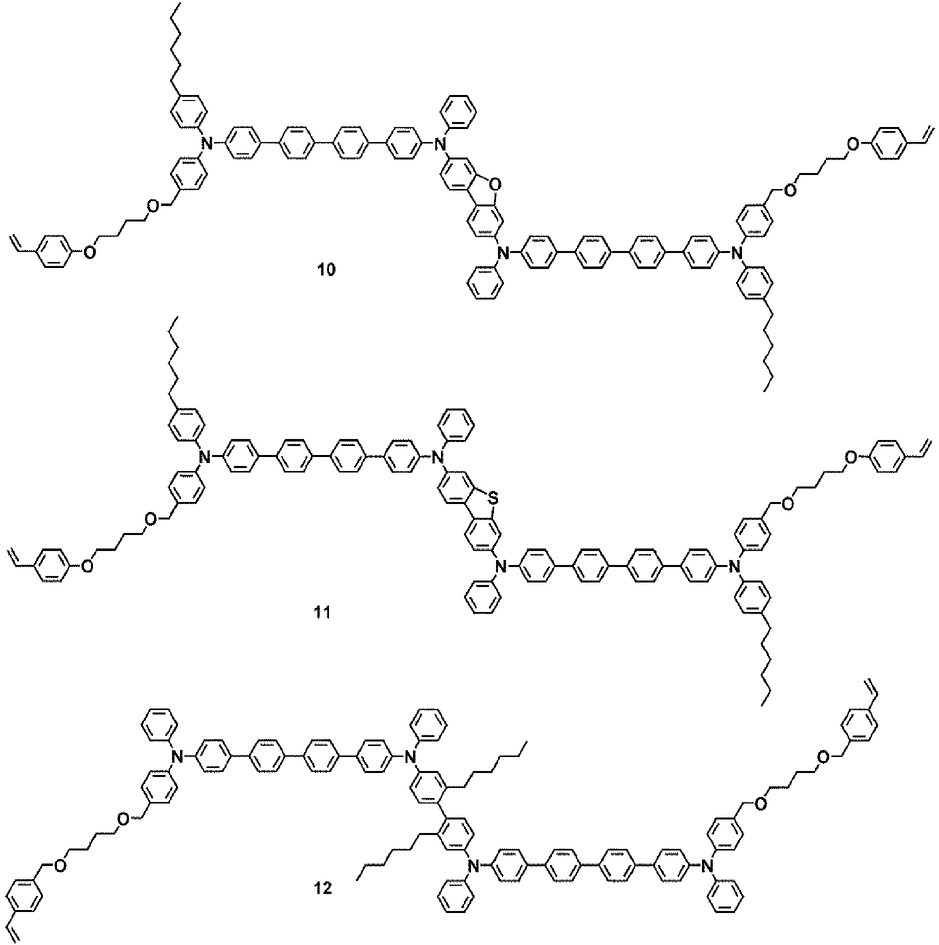

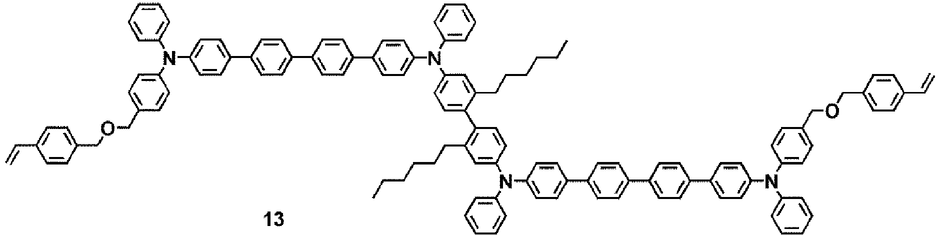

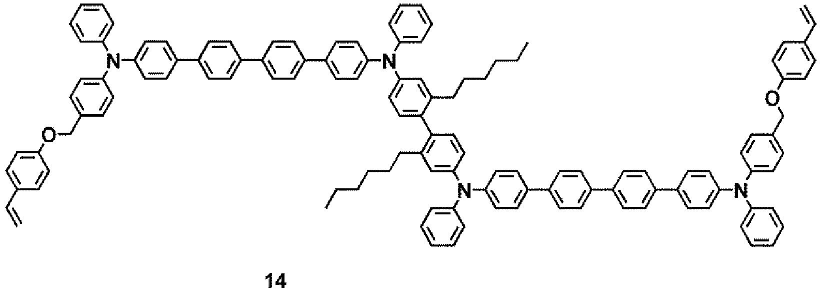

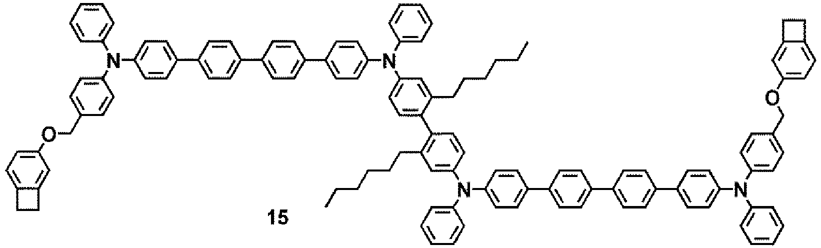

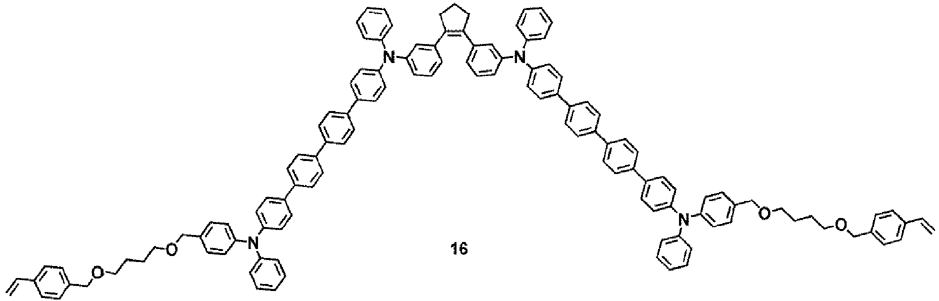

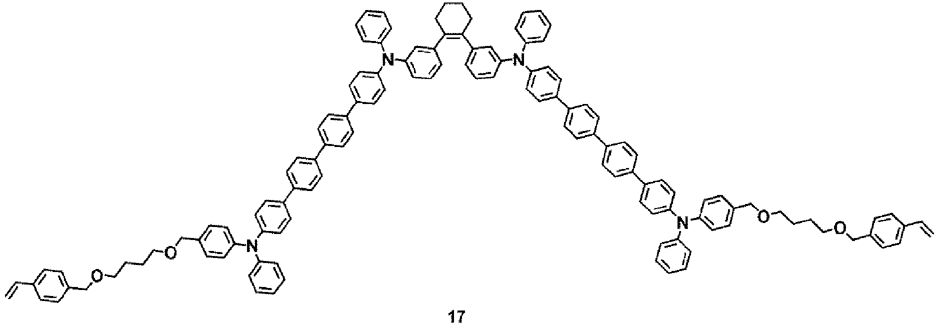

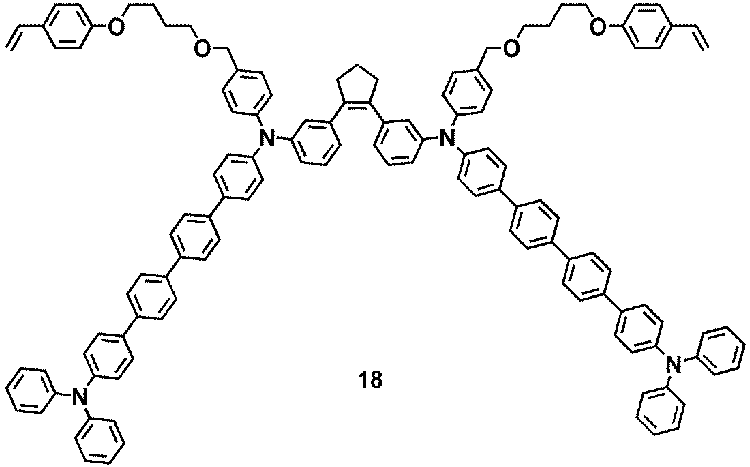

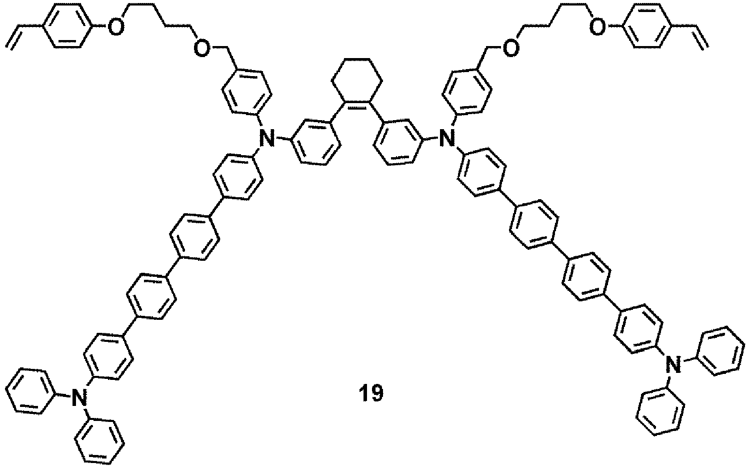

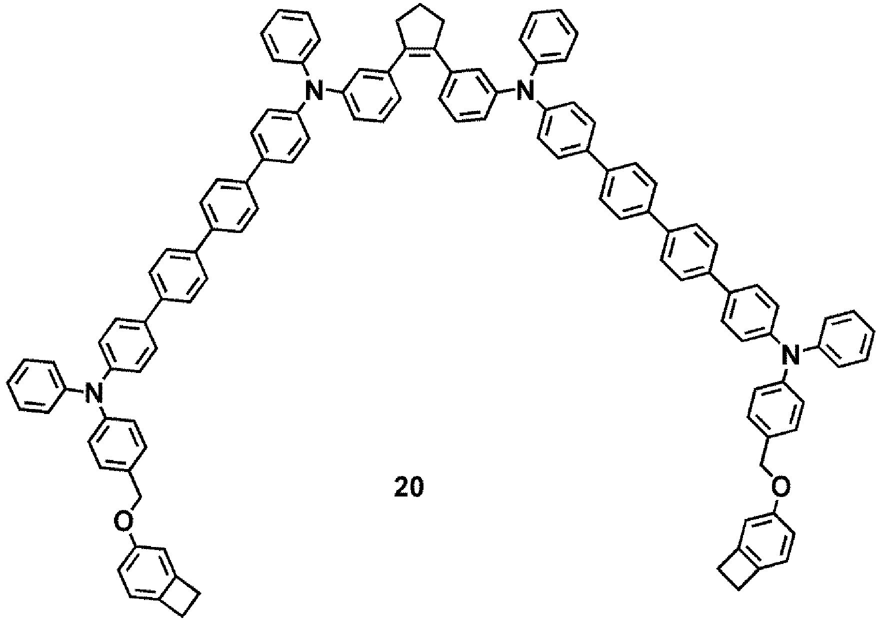

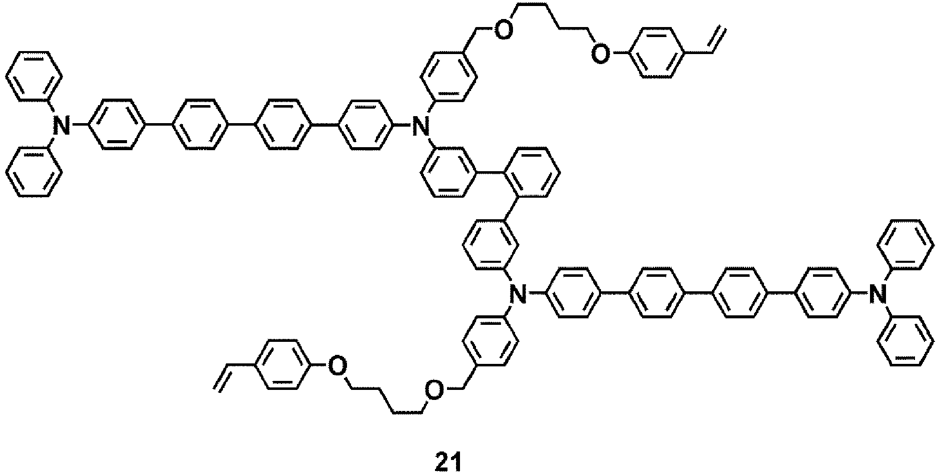

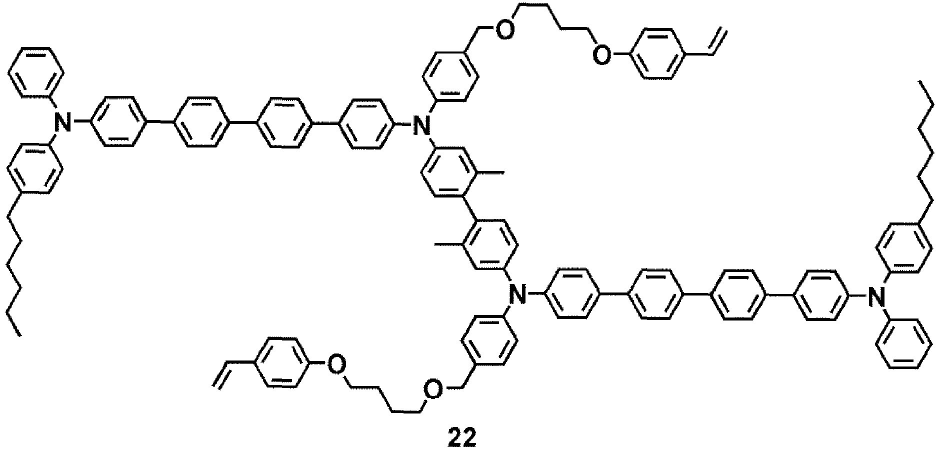

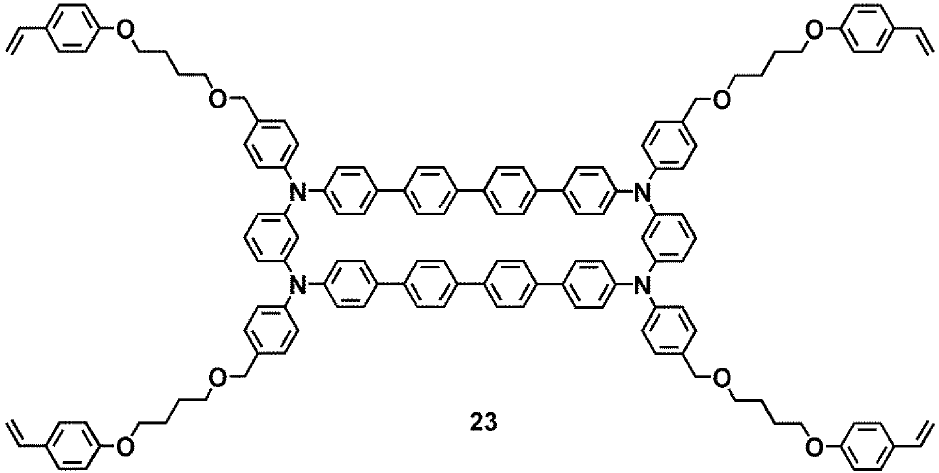

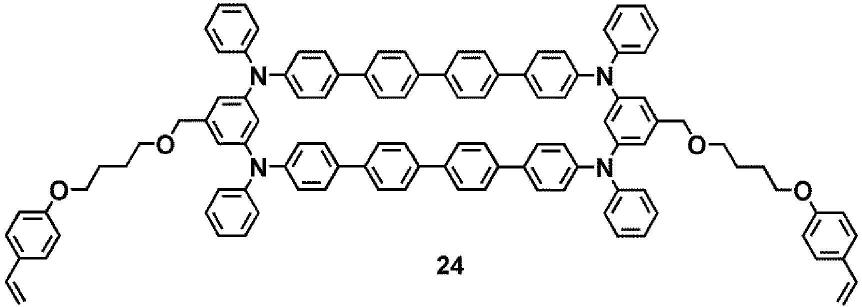

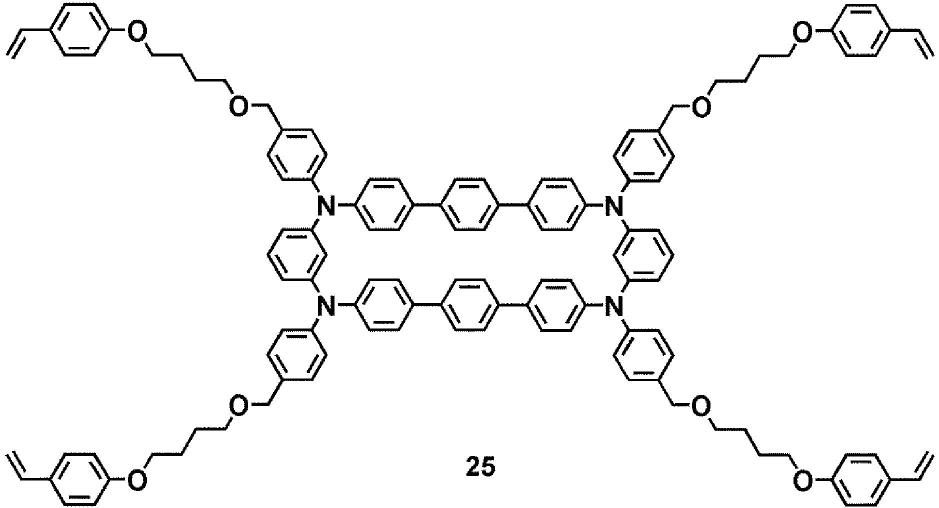

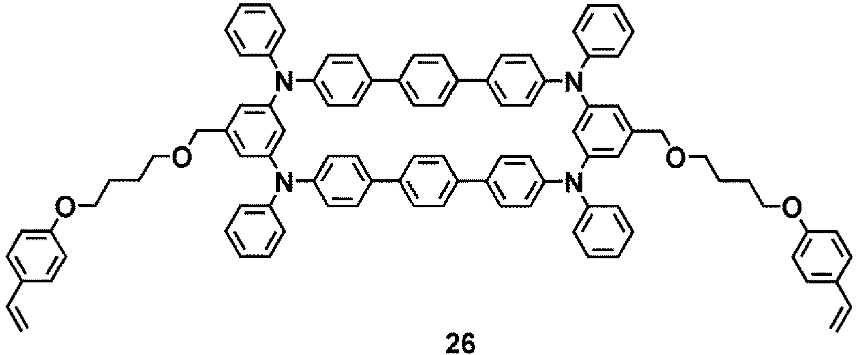

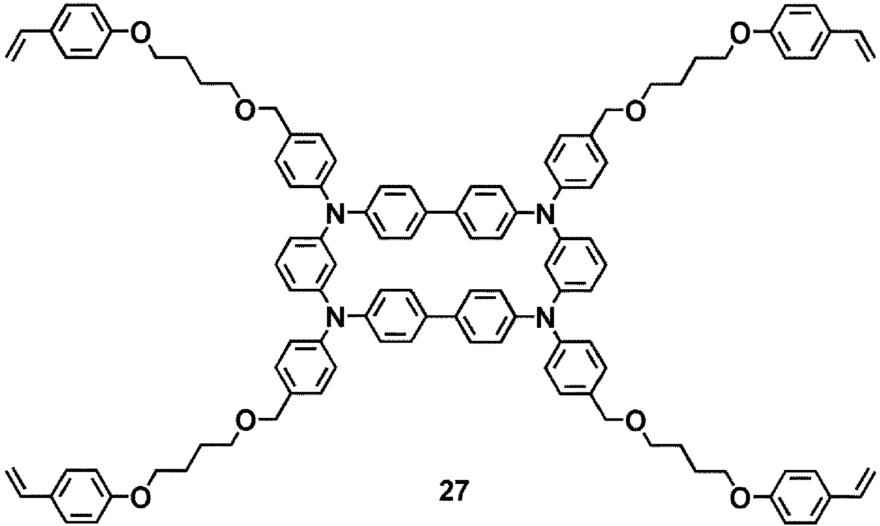

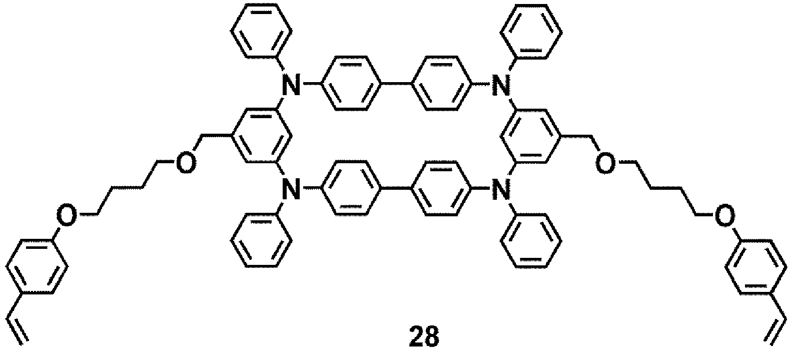

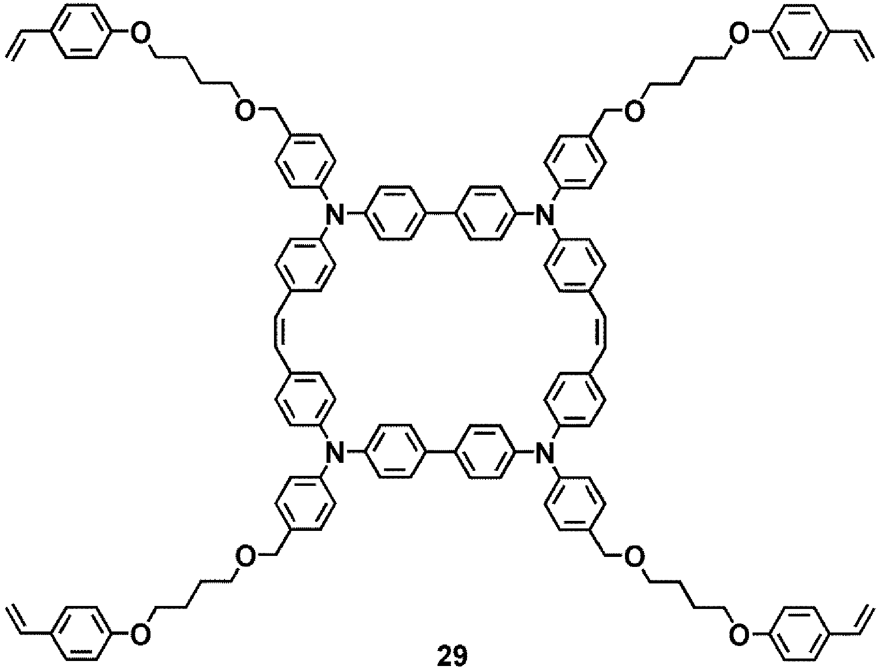

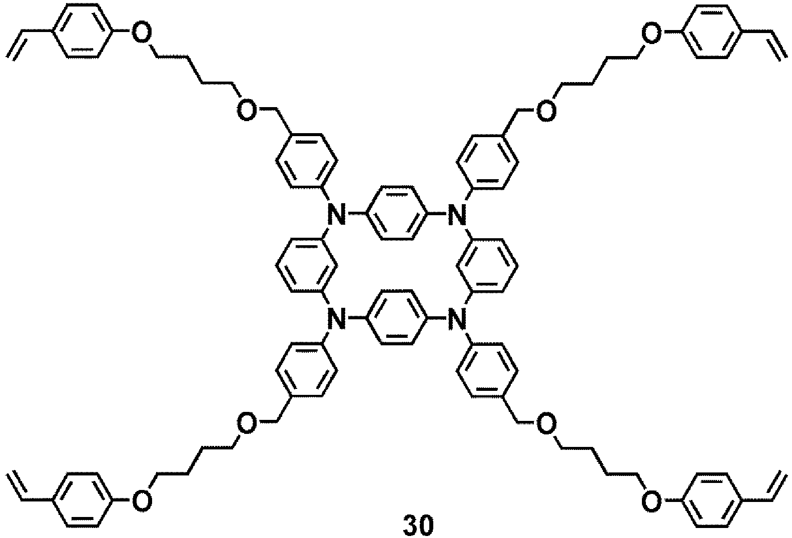

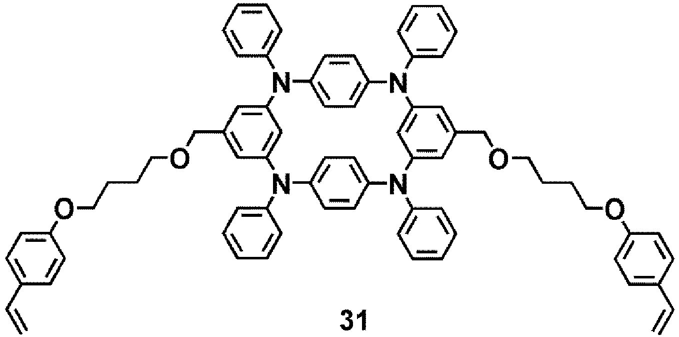

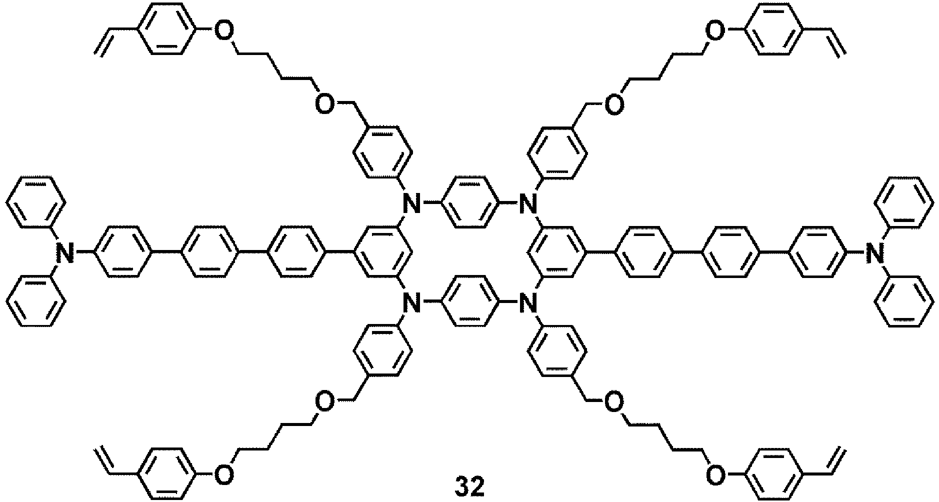

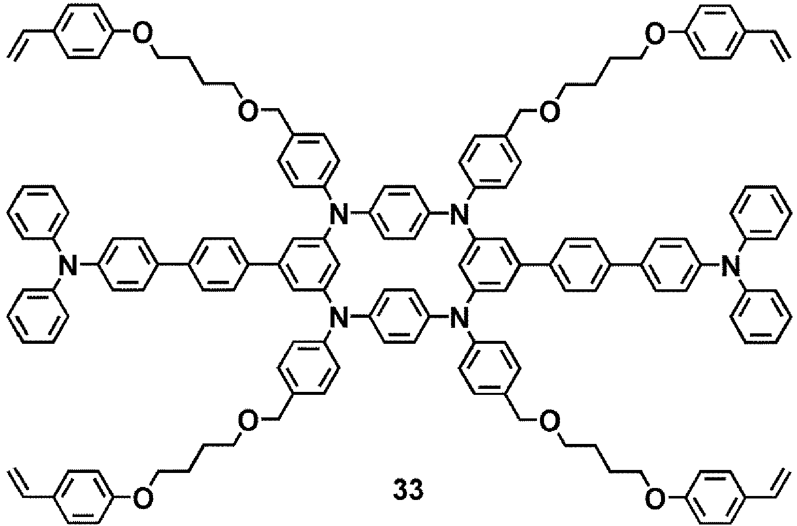

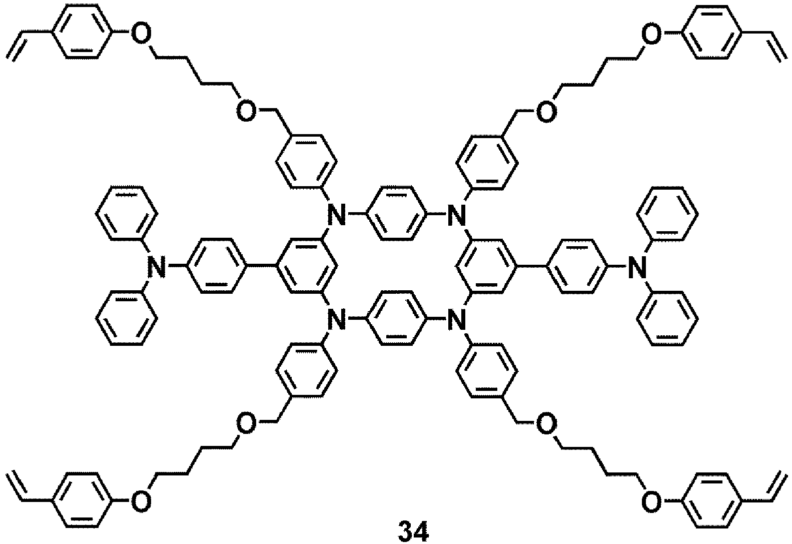

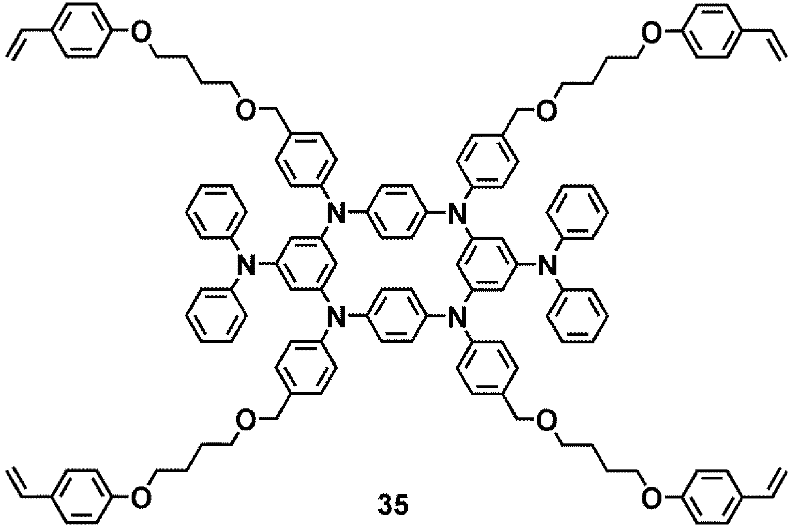

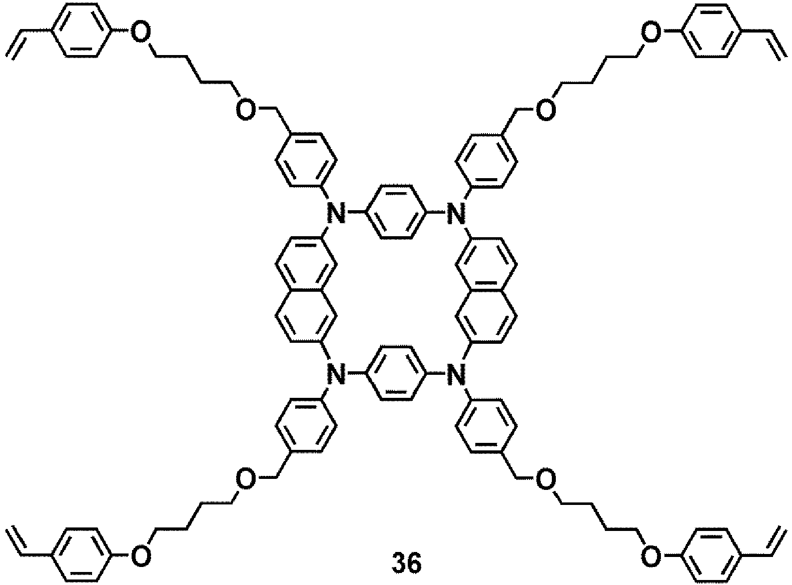

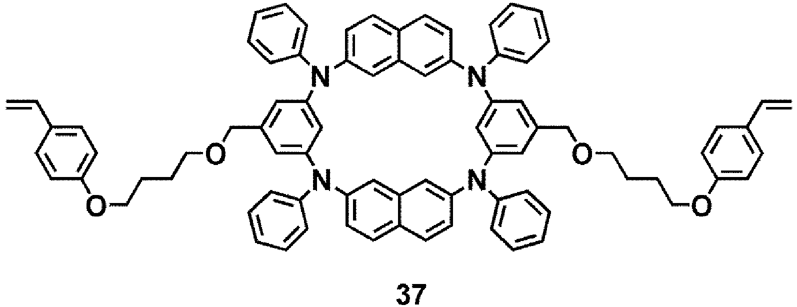

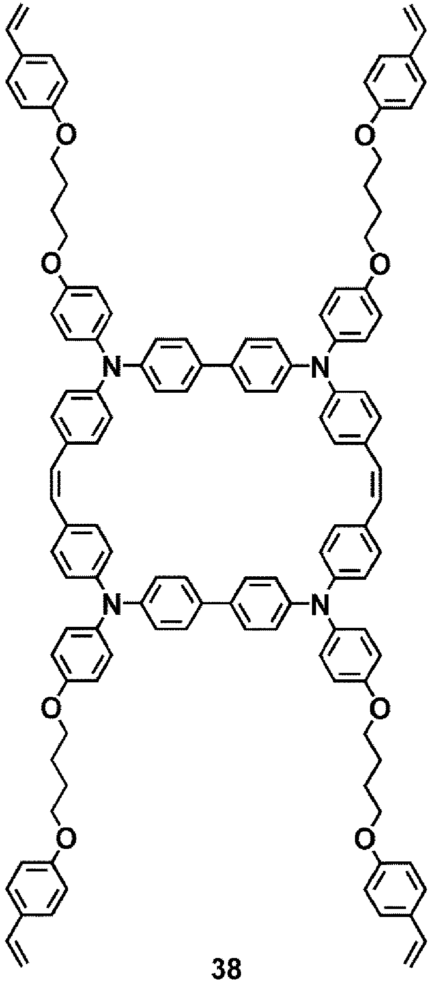

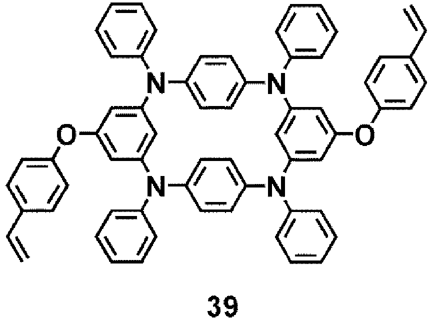

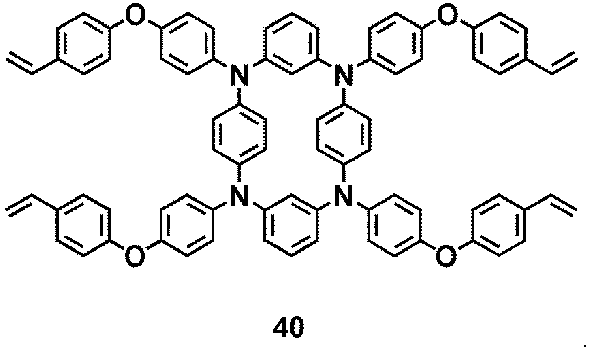

23. An arylamine-based polymer comprising a repeating unit represented by Formula 11 or formed by cross-linking a cross-linkable arylamine-based compound represented by Formula 1b: 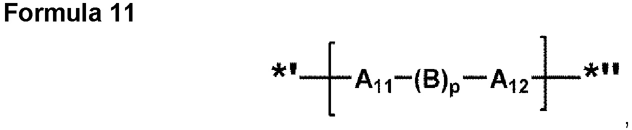 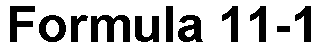 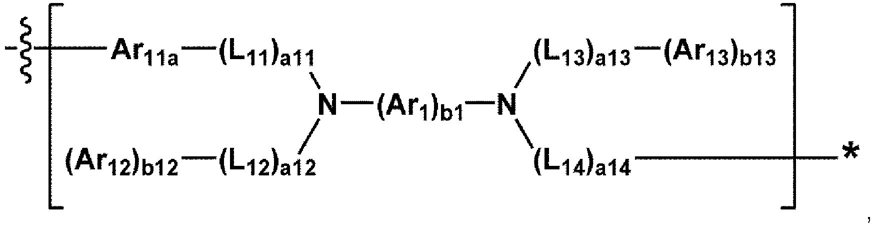 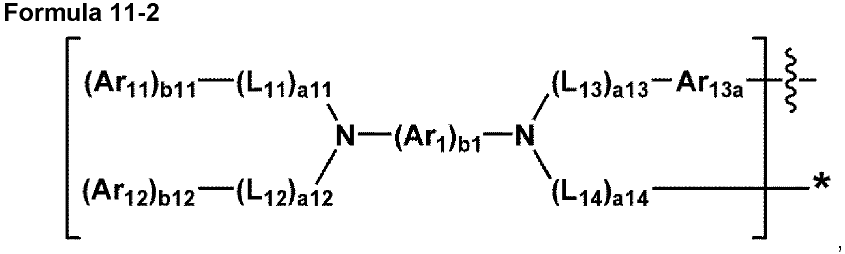 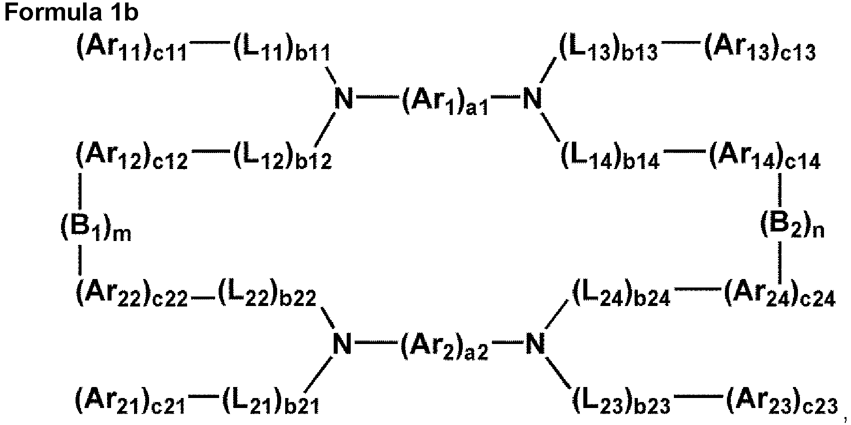
wherein, in Formula 11, A₁₁ and A₁₂ are each independently selected from groups represented by Formulae 11-1 and 11-2, and A₁₁ and A₁₂ are identical to or different from each other,
in Formula 11-1, * indicates a binding site to (B)ₚ in Formula 11,
in Formulae 11, 11-1, and 11-2, *', *", and - 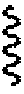 - each indicate a binding site to a neighboring repeating unit,
in Formulae 11-1 and 11-2, Ar₁₁ₐ and Ar₁₃ₐ are each a residue formed after cross-linking, and the repeating units are linked by a divalent cyclobutane group,
in Formula 11, B is selected from a substituted or unsubstituted C₆-C₆₀ carbocyclic group, a substituted or unsubstituted C₁-C₆₀ heterocyclic group, and *'-Si(Q₄₁)(Q₄₂)-*", p is an integer of 1 to 10,
in Formulae 11-1 and 11-2, Ar₁ is selected from a benzene group, a pyridine group, a pyrimidine group, a pyrazine group, a triazine group, a furan group, a thiophene group, an imidazole group, a thiazole group, an isoxazole group, and an oxazole group,
b1 is an integer of four or more, and four or more Ar₁(s) are identical to or different from each other,
in Formulae 11-1 and 11-2, L₁₁ to L₁₄ are each independently a substituted or unsubstituted C₆-C₆₀ carbocyclic group or a substituted or unsubstituted C₁-C₆₀ heterocyclic group,
in Formulae 11-1 and 11-2, a11 to a14 are each independently 0, 1, 2, 3, or 4, wherein, when a11 is 0, *'-(L₁₁)ₐ₁₁-*" is a single bond; when a12 is 0, *'-(L₁₂)ₐ₁₂-*" is a single bond; when a13 is 0, *'-(L₁₃)ₐ₁₃-*" is a single bond; when a14 is 0, *'-(L₁₄)ₐ₁₄-*" is a single bond; when a11 is two or more, two or more L₁₁(s) are identical to or different from each other; when a12 is two or more, two or more L₁₂(s) are identical to or different from each other; when a13 is two or more, two or more L₁₃(s) are identical to or different from each other; and when a14 is two or more, two or more L₁₄(s) are identical to or different from each other,
in Formulae 11-1 and 11-2, Ar₁₁ to Ar₁₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein at least one of Ar₁₁ to Ar₁₃ is substituted with a cross-linkable group,
in Formulae 11-1 and 11-2, b11 to b13 are each independently 1, 2, 3, 4, or 5, wherein, when b11 is two or more, two or more Ar₁₁(s) are identical to or different from each other; when b12 is two or more, two or more Ar₁₂(s) are identical to or different from each other; and when b13 is two or more, two or more Ar₁₃(s) are identical to or different from each other,
in Formulae 11-1 and 11-2, Ar₁ and Ar₂ are each independently a substituted or unsubstituted C₆-C₆₀ carbocyclic group or a substituted or unsubstituted C₁-C₆₀ heterocyclic group, wherein Ar₁ and Ar₂ are identical to or different from each other,
in Formula 1b, Ar₁ and Ar₂ are each independently a substituted or unsubstituted C₆-C₆₀ carbocyclic group or a substituted or unsubstituted C₁-C₆₀ heterocyclic group, wherein Ar₁ and Ar₂ are identical to or different from each other,
in Formula 1b, a1 and a2 are each independently an integer of 1 to 5, wherein, when a1 is two or more, two or more Ar₁(s) are identical to or different from each other; and when a2 is two or more, two or more Ar₂(s) are identical to or different from each other,
in Formula 1b, L₁₁ to L₁₄ and L₂₁ to L₂₄ are each independently a substituted or unsubstituted C₆-C₆₀ carbocyclic group or a substituted or unsubstituted C₁-C₆₀ heterocyclic group,
in Formula 1b, b11 to b14 and b21 to b24 are each independently an integer of 0 to 3, wherein, when b11 is 0, *-(L₁₁)_{b11}-*' is a single bond; when b12 is 0, *-(L₁₂)_{b12}-*' is a single bond; when b13 is 0, *-(L₁₃)_{b13}-*' is a single bond; when b14 is 0, *-(L₁₄)_{b14}-*' is a single bond; when b21 is 0, *-(L₂₁)_{b21}-*' is a single bond; when b22 is 0, *-(L₂₂)_{b22}-*' is a single bond; when b23 is 0, *-(L₂₃)_{b23}-*' is a single bond; when b24 is 0, *-(L₂₄)_{b24}-*' is a single bond; when b11 is two or more, two or more L₁₁(s) are identical to or different from each other; when b12 is two or more, two or more L₁₂(s) are identical to or different from each other; when b13 is two or more, two or more L₁₃(s) are identical to or different from each other; when b14 is two or more, two or more L₁₄(s) are identical to or different from each other; when b21 is two or more, two or more L₂₁(s) are identical to or different from each other; when b22 is two or more, two or more L₂₂(s) are identical to or different from each other; when b23 is two or more, two or more L₂₃(s) are identical to or different from each other; and when b24 is two or more, two or more L₂₄(s) are identical to or different from each other,
in Formula 1b, B₁ and B₂ are each independently selected from a single bond, a substituted or unsubstituted C₁-C₂₀ alkylene group, a substituted or unsubstituted C₂-C₂₀ alkenylene group, and a substituted or unsubstituted C₂-C₂₀ alkynylene group,
in Formula 1b, m and n are each independently an integer of 1 to 3, wherein, when m is two or more, two or more B₁(s) are identical to or different from each other; and when n is two or more, two or more B₂(s) are identical to or different from each other,
in Formula 1b, Ar₁₁, Ar₁₃, Ar₂₁, and Ar₂₃ are each independently selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group,
in Formula 1b, Ar₁₂, Ar₁₄, Ar₂₂, and Ar₂₄ are each independently selected from a single bond, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
in Formula 1b, c11 to c14 and c21 to c24 are each independently an integer of 1 to 5, wherein, when c11 is two or more, two or more Ar₁₁(s) are identical to or different from each other; when c12 is two or more, two or more Ar₁₂(s) are identical to or different from each other; when c13 is two or more, two or more Ar₁₃(s) may be identical to or different from each other; when c14 is two or more, two or more Ar₁₄(s) are identical to or different from each other; when c21 is two or more, two or more Ar₂₁(s) are identical to or different from each other; when c22 is two or more, two or more Ar₂₂(s) are identical to or different from each other; when c23 is two or more, two or more Ar₂₃(s) are identical to or different from each other; and when c24 is two or more, two or more Ar₂₄(s) are identical to or different from each other,
in Formula 1b, at least one group of Ar₁, Ar₂, Ar₁₁ to Ar₁₄, and Ar₂₁ to Ar₂₄ is substituted with a cross-linkable group.

24. An arylamine-based polymer according to claim 23, wherein in Formulae 11-1 and 11-2, Ar₁₁ₐ and Ar₁₃ₐ are each independently selected from groups represented by Formulae 5-1 to 5-8: 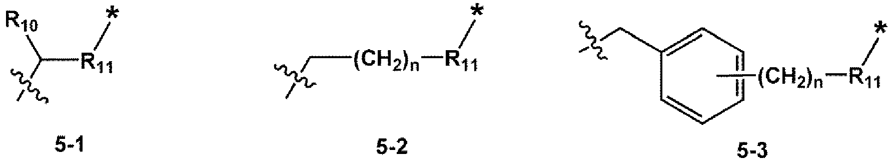 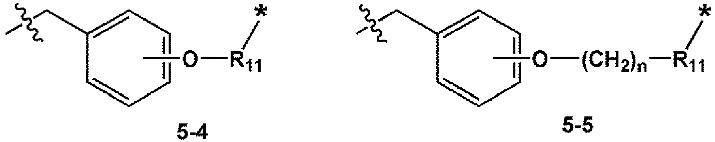 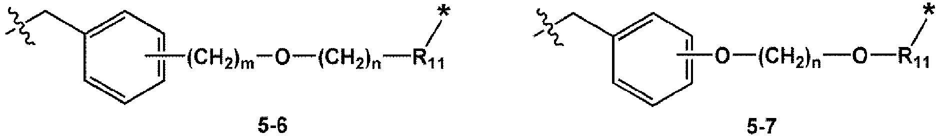 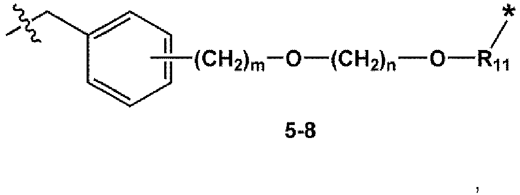 wherein, in Formulae 5-1 to 5-8,
R₁₀ is a hydrogen atom or a substituted or unsubstituted C₁-C₂₀ alkyl group,
R₁₁ is selected from a single bond, a substituted or unsubstituted C₁-C₂₀ alkylene group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
m and n are each independently an integer of 1 to 10,
* indicates a binding site to a neighboring atom, and 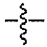
indicates a binding site to a neighboring repeating unit.

25. An arylamine-based polymer according to claim 23 or claim 24, wherein in Formulae 11-1 and 11-2, Ar₁ is a benzene group, and b1 is 4 or 5.

## Patentansprüche

1. Organische lichtemittierende Vorrichtung (10, 20, 30, 40), umfassend:
eine erste Elektrode (110);
eine zweite Elektrode (190), die der ersten Elektrode (110) zugewandt ist; und
eine organische Schicht (150) zwischen der ersten Elektrode (110) und der zweiten Elektrode (190) und eine Emissionsschicht umfassend,
**dadurch gekennzeichnet, dass** die organische Schicht (150) weiter mindestens eines von einem auf Arylamin basierenden Polymer, das durch Vernetzung einer vernetzbaren Verbindung auf Arylaminbasis gebildet wird, die durch die Formel 1a oder 1b dargestellt wird:
**Formel 1a** A₁-(B)ₚ-A₂, 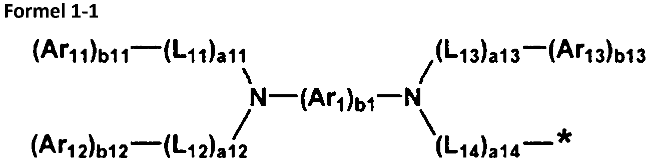 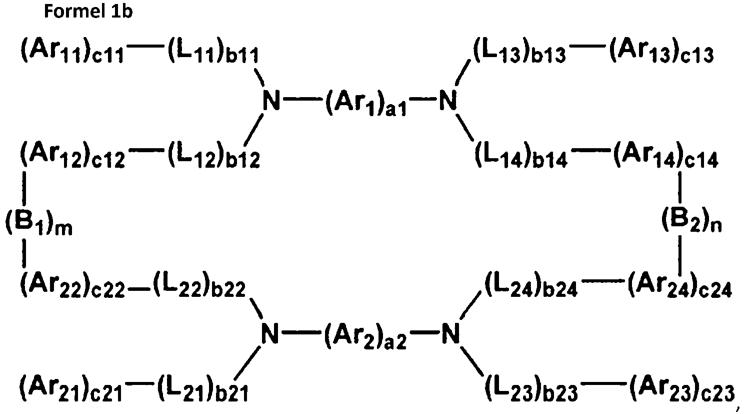
wobei in Formel 1a A₁ und A₂ jeweils eine durch die Formel 1-1 dargestellte Gruppe sind, und A₁ und A₂ identisch oder voneinander verschieden sind,
in Formel 1a B aus einer substituierten oder unsubstituierten carbocyclischen C₅-C₆₀-Gruppe, einer substituierten oder unsubstituierten heterocyclischen C₁-C₆₀-Gruppe, und *'- Si(Q₄₁)(Q₄₂)-*" ausgewählt ist; und p eine ganze Zahl von 1 bis 10 ist,
in Formel 1-1 * eine Bindestelle für (B)ₚ in Formel 1a angibt,
in Formel 1-1 Ar₁ aus einer Benzolgruppe, einer Pyridingruppe, einer Pyrimidingruppe, einer Pyrazingruppe, einer Triazingruppe, einer Furangruppe, einer Thiophengruppe, einer Imidazolgruppe, einer Thiazolgruppe, einer Isoxazolgruppe und einer Oxazolgruppe ausgewählt wird,
in Formel 1-1 b1 eine ganze Zahl von vier oder mehr ist, und vier oder mehr Ar₁(s) identisch oder voneinander verschieden sind.
in Formel 1-1 sind L₁₁ bis L₁₄ jeweils unabhängig eine substituierte oder unsubstituierte carbocyclische C₅-C₆₀-Gruppe, oder eine substituierte oder unsubstituierte heterocyclische C₁-C₆₀ -Gruppe,
in Formel 1-1 a11 bis a14 jeweils unabhängig voneinander 0, 1,2, 3, oder 4 sind,
in Formel 1-1, wenn a11 gleich 0 ist, *'-(L₁₁)ₐ₁₁-*" eine Einfachbindung ist; wenn a12 gleich 0 ist, *'- (L₁₂)ₐ₁₂-*" eine Einfachbindung ist; wenn a13 gleich 0 ist, *'-(L₁₃)ₐ₁₃-*" eine Einfachbindung ist; wenn a14 gleich 0 ist, *'-(L₁₄)ₐ₁₄-*" eine Einfachbindung ist; wenn a11 zwei oder mehr ist, zwei oder mehr L₁₁(s) identisch oder verschieden sind; wenn a12 zwei oder mehr ist, zwei oder mehr L₁₂(s) identisch oder verschieden sind; wenn a13 zwei oder mehr ist, zwei oder mehr L₁₃(s) identisch oder verschieden sind; und wenn a14 zwei oder mehr ist, zwei oder mehr L₁₄(s) identisch oder verschieden sind,
in Formel 1-1 Ar₁₁ bis Ar₁₁ jeweils unabhängig voneinander ausgewählt sind aus einer substituierten oder unsubstituierten C₃-C₁₀-Cycloalkylgruppe einer substituierten oder unsubstituierten C₁-C₁₀-Heterocycloalkylgruppe, einer substituierten oder unsubstituierten -C₃-C₁₀-Cycloalkenylgruppe, einer substituierten oder unsubstituierten C₁-C₁-Heterocycloalkenylgruppe, einer substituierten oder unsubstituierten -C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe und einer substituierten oder unsubstituierten monovalenten nicht-aromatischen kondensierten heteropolycyclischen Gruppe, wobei mindestens eines von Arn bis Arn mit einer vernetzbaren Gruppe substituiert ist,
in Formel 1-1 b11 bis b13 jeweils unabhängig voneinander 1,2,3, 4, oder 5 sind, wobei, wenn b11 zwei oder mehr ist, zwei oder mehr Ar₁₁(s) identisch oder voneinander verschieden sind; wenn b12 zwei oder mehr ist, zwei oder mehr Ar₁₂(s) identisch oder voneinander verschieden sind; und wenn b13 zwei oder mehr ist, zwei oder mehr Ar₁₃(S) identisch oder voneinander verschieden sind,
in Formel 1-1 Ar₁ und Ar₂ jeweils unabhängig eine substituierte oder unsubstituierte carbocyclische C₅-C₆₀-Gruppe sind, oder eine substituierte oder unsubstituierte heterocyclische C₁-C₆₀-Gruppe sind, wobei Ar₁ und Ar₂ identisch oder voneinander verschieden sind,
in Formel 1b, a1 und a2 jeweils unabhängig voneinander eine ganze Zahl von 1 bis 5 sind, wobei, wenn a1 zwei oder mehr ist, zwei oder mehr Ar₁(S) identisch oder voneinander verschieden sind; und wenn a2 zwei oder mehr ist, zwei oder mehr Ar₂(s) identisch oder verschieden sind,
in Formel 1b L₁₁ bis L₁₄ und L₂₁ bis L₂₄ jeweils unabhängig eine substituierte oder unsubstituierte carbocyclische C₅-C₆₀-Gruppe, oder eine substituierte oder unsubstituierte heterocyclische C₁-C₆₀ -Gruppe sind,
in Formel 1b b11 bis b14 und b21 bis b24 jeweils unabhängig voneinander eine ganze Zahl von 0 bis 3 sind, wobei, wenn b11 gleich 0 ist, *-(L₁₁)_{b11}-*' eine Einfachbindung ist; wenn b12 gleich 0 ist, *-(L₁₂)_{b12}-*' eine Einfachbindung ist; wenn b13 gleich 0 ist, *-(L₁₃)_{b13}-*' eine Einfachbindung ist; wenn b14 gleich 0 ist, *-(L₁₄)_{b14}-*' eine Einfachbindung ist; wenn b21 gleich 0 ist, *-(L₂₁)_{b21}-*' eine Einfachbindung ist; wenn b22 gleich 0 ist, *-(L₂₂)_{b22}-*' eine Einfachbindung ist; wenn b23 gleich 0 ist, *-(L₂₃)_{b23}-*' eine Einfachbindung ist; wenn b24 gleich 0 ist, *-(L₂₄)_{b24}-*' eine Einfachbindung ist; wenn b11 zwei oder mehr ist, zwei oder mehr L₁₁(s) identisch oder voneinander verschieden sind; wenn b12 zwei oder mehr ist, zwei oder mehr L₁₂(s) identisch oder voneinander verschieden sind; wenn b13 zwei oder mehr ist, zwei oder mehr L₁₃(s) identisch oder voneinander verschieden sind; wenn b14 zwei oder mehr ist, zwei oder mehr L₁₄(s) identisch oder voneinander verschieden sind; wenn b21 zwei oder mehr ist, zwei oder mehr L₂₁(s) identisch oder voneinander verschieden sind; wenn b22 zwei oder mehr ist, zwei oder mehr L₂₂(s) identisch oder voneinander verschieden sind; wenn b23 zwei oder mehr ist, zwei oder mehr L₂₃(s) identisch oder voneinander verschieden sind; und wenn b24 zwei oder mehr ist, zwei oder mehr L₂₄(s) identisch oder voneinander verschieden sind,
in Formel 1b B₁ und B₂ jeweils unabhängig aus einer Einfachbindung, einer substituierten oder unsubstituierten C₁-C₂₀-Alkylengruppe, einer substituierten oder unsubstituierten C₂-C₂₀-Alkenylengruppe und einer substituierten oder unsubstituierten C₂-C₂₀-Alkynylengruppe ausgewählt sind,
in Formel 1b, m und n jeweils unabhängig voneinander eine ganze Zahl von 1 bis 3 sind, wobei, wenn m zwei oder mehr ist, zwei oder mehr B₁(s) identisch oder voneinander verschieden sind; und wenn n zwei oder mehr ist, zwei oder mehr B₂(s) identisch oder voneinander verschieden sind,
in Formel 1b Ar₁₁, Ar₁₃, Ar₂₁ und Ar₂₃ jeweils unabhängig voneinander ausgewählt sind aus einer substituierten oder unsubstituierten C₃-C₁₀-Cycloalkylgruppe einer substituierten oder unsubstituierten C₁-C₁₀ -Heterocycloalkylgruppe, einer substituierten oder unsubstituierten C₃-C₁₀ -Cycloalkenylgruppe, einer substituierten oder unsubstituierten C₁-C₁₀ -Heterocycloalkenylgruppe, einer substituierten oder unsubstituierten C₆-C₆₀ -Arylgruppe, einer substituierten oder unsubstituierten C₆-C₆₀ - Aryloxygruppe, einer substituierten oder unsubstituierten C₆-C₆₀ -Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe und einer substituierten oder unsubstituierten monovalenten nicht-aromatischen kondensierten heteropolycyclischen Guppe,
in Formel 1b Ar₁₂, Ar₁₄, Ar₂₂, und Ar₂₄ jeweils unabhängig ausgewählt sind aus einer substituierten oder unsubstituierten C₃-C₁₀Cycloalkylengruppe, einer substituierten oder unsubstituierten C₁C₁₀-Heterocycloalkylengruppe, einer substituierten oder unsubstituierten C₃-C₁₀-Cycloalkenylengruppe, einer substituierten oder unsubstituierten C₁-C₁₀-Heterocycloalkenylengruppe, einer substituierten oder unsubstituierten -C₆-C₆₀-Arylengruppe, einer substituierten oder unsubstituierten C₁C₆₀-Heteroarylengruppe, einer substituierten oder unsubstituierten zweiwertigen nicht-aromatischen kondensierten polycyclischen Gruppe und einer substituierten oder unsubstituierten zweiwertigen nicht-aromatischen kondensierten heteropolycyclischen Gruppe,
in Formel 1b, c11 bis c14 und c21 bis c24 jeweils unabhängig voneinander eine ganze Zahl von 1 bis 5 sind, wobei, wenn c11 zwei oder mehr ist, zwei oder mehr Ar₁₁(s) identisch oder voneinander verschieden sind; wenn c12 zwei oder mehr ist, zwei oder mehr Ar₁₂(s) identisch oder voneinander verschieden sind; wenn c13 zwei oder mehr ist, zwei oder mehr Ar₁₃(s) identisch oder voneinander verschieden sein können; wenn c14 zwei oder mehr ist, zwei oder mehr Ar₁₄(s) identisch oder voneinander verschieden sind; wenn c21 zwei oder mehr ist, zwei oder mehr Ar₂₁(s) identisch oder voneinander verschieden sind; wenn c22 zwei oder mehr ist, zwei oder mehr Ar₂₂(s) identisch oder voneinander verschieden sind; wenn c23 zwei oder mehr ist, zwei oder mehr Ar₂₃(s) identisch oder voneinander verschieden sind; und wenn c24 zwei oder mehr ist, zwei oder mehr Ar₂₄(s) identisch oder voneinander verschieden sind,
in Formel 1b mindestens eine Gruppe, ausgewählt aus Ar₁, Ar₂, Ar₁₁ bis Ar₁₄ und Ar₂₁ bis Ar₂₄, durch eine vernetzbare Gruppe substituiert ist,
*'und *" jeweils eine Bindungsstelle zu einem benachbarten Atom angeben,
mindestens ein Substituent der substituierten carbocyclischen C₅-C₆₀-Gruppe, der substituierten heterocyclischen C₁-C₆₀-Gruppe, der substituierten C₃-C₁₀-Cycloalkylengruppe, der substituierten C₁-C₁₀-Heterocycloalkylengruppe, der substituierten C₃-C₁₀-Cycloalkenylengruppe, der substituierten C₁-C₁₀-Heterocycloalkenylengruppe, der substituierten C₆-C₆₀-Arylengruppe, der substituierten C₁-C₆₀-Heteroarylengruppe, der substituierten zweiwertigen nicht-aromatischen kondensierten polycyclischen Gruppe, der substituierten zweiwertigen nicht-aromatischen kondensierten heteropolycyclischen Gruppe, der substituierten C₃-C₁₀-Cycloalkylgruppe, der substituierten C₁-C₁₀-Heterocycloalkylgruppe, der substituierten C₃-C₁₀ Cycloalkenylgruppe, der substituierten C₁-C₁₀-Heterocycloalkenylgruppe, der substituierten C₆-C₆₀-Arylgruppe, der substituierten C₆-C₆₀-Aryloxygruppe, der substituierten C₆-C₆₀-Arylthiogruppe, der substituierten C₁-C₆₀-Heteroarylgruppe, der substituierten einwertigen nicht-aromatischen kondensierten polycyclischen Gruppe, der substituierten einwertigen nicht-aromatischen kondensierten heterocyclischen Gruppe, der substituierten C₁-C₂₀-Alkylengruppe, der substituierten C₂-C₂₀-Alkenylengruppe, und der substituierten C₂-C₂₀-Alkynylengruppe ausgewählt ist aus:
Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₁-C₆₀-Alkylgruppe, einer C₂-C₆₀-Alkenylgruppe, einer C₂-C₆₀-Alkinylgruppe und einer C₁-C₆₀-Alkoxygruppe;
einer C₁-C₆₀-Alkylgruppe, einer C₂-C₆₀-Alkenylgruppe, einer C₂-C₆₀-Alkinylgruppe und einer C₁-C₆₀-Alkoxygruppe, jeweils substituiert mit mindestens einem, ausgewählt aus Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₃-C₁₀-Cycloalkylgruppe, einer C₁₋C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nicht-aromatischen kondensierten polycyclischen Gruppe, einer einwertigen nicht-aromatischen kondensierten heteropolycyclischen Gruppe, - Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁) und -P(=O)(Q₁₁)(Q₁₂);
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nicht-aromatischen kondensierten polycyclischen Gruppe, einer einwertigen nicht-aromatischen kondensierten heteropolycyclischen Gruppe, einer Biphenylgruppe und einer Terphenylgruppe;
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nicht-aromatischen kondensierten polycyclischen Gruppe, einer einwertigen nicht-aromatischen kondensierten heteropolycyclischen Gruppe, einer Biphenylgruppe und einer Terphenylgruppe, jeweils substituiert mit mindestens einem, ausgewählt aus Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₁-C₆₀-Alkylgruppe, einer C₂-C₆₀-Alkenylgruppe, einer C₂-C₆₀-Alkynylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nicht-aromatischen kondensierten polycyclischen Gruppe, einer einwertigen nicht-aromatischen kondensierten heteropolycyclischen Gruppe, einer einwertigen nicht-aromatischen kondensierten heteropolycyclischen Gruppe,-Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), und - P(=O)(Q₂₁)(Q₂₂); und
-Si(Q₃₁)(Q₃₂)(Q₃₃), -N(Q₃₁)(Q₃₂), -B(Q₃₁)(Q₃₂), -C(=O)(Q₃₁), -S(=O)2(Q₃₁) und - P(=O)(Q₃₁)(Q₃₂) und
Q₁₁ bis Q₁₃, Q₂₁ bis Q₂₃, Q₃₁ bis Q₃₃, und Q₄₁ bis Q₄₂ jeweils unabhängig ausgewählt sind aus Wasserstoff, Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₁-C₆₀-Alkylgruppe, einer C₂-C₆₀-Alkenylgruppe, einer C₂-C₆₀-Alkynylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer einwertigen nicht-aromatischen kondensierten polycyclischen Gruppe, einer einwertigen nicht-aromatischen kondensierten heteropolycyclischen Gruppe, einer Biphenylgruppe und einer Terphenylgruppe.

2. Organische lichtemittierende Vorrichtung (10, 20, 30, 40) nach Anspruch 1, wobei:
die erste Elektrode (110) eine Anode ist,
die zweite Elektrode (190) eine Kathode ist, und
die organische Schicht (150) weiter i) eine Lochtransportzone zwischen der ersten Elektrode und der Emissionsschicht, die eine Lochinjektionsschicht, eine Lochtransportschicht, eine Pufferschicht, eine Elektronenblockierschicht oder eine beliebige Kombination davon umfasst; und ii) eine Elektronentransportzone zwischen der Emissionsschicht und der zweiten Elektrode umfasst, und eine Lochblockierschicht, eine Elektronentransportschicht, eine Elektroneninjektionsschicht oder eine beliebige Kombination davon umfasst.

3. Organische lichtemittierende Vorrichtung (10, 20, 30, 40) nach Anspruch 2, wobei die Lochtransportzone mindestens eines von dem auf Arylamin basierenden Polymer umfasst.

4. Organische lichtemittierende Vorrichtung (10, 20, 30, 40) nach Anspruch 2, wobei:
die Lochtransportzone eine Lochinjektionsschicht und eine Lochtransportschicht umfasst, und
die Lochtransportschicht mindestens eines von dem Arylamin basierenden Polymer umfasst.

5. Elektronische Einrichtung, umfassend:
ein Dünnschichttransistor; und
die organische lichtemittierende Vorrichtung (10, 20, 30, 40) nach einem der Ansprüche 1 bis 4,
wobei der Dünnschichttransistor eine Source-Elektrode und eine Drain-Elektrode, eine aktive Schicht und eine Gate-Elektrode umfasst, und
die erste Elektrode der organischen lichtemittierenden Vorrichtung elektrisch mit einer von der Source-Elektrode und der Drain-Elektrode des Dünnschichttransistors verbunden ist.

6. Vernetzbare auf Arylamin basierende Verbindung, die durch die Formel 1a oder 1b dargestellt ist:
Formel 1a A₁-(B)ₚ-A₂ 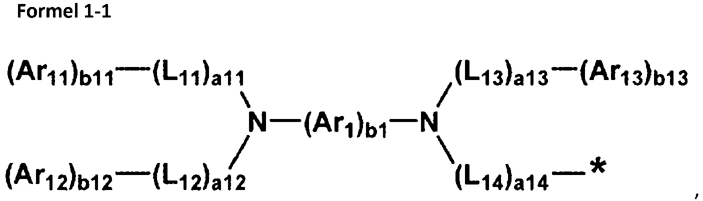 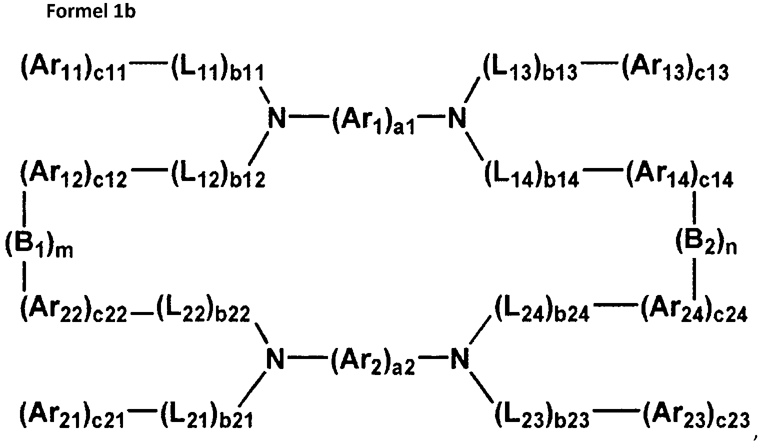
wobei in Formel 1a A₁ und A₂ jeweils eine durch die Formel 1-1 dargestellte Gruppe sind, und A₁ und A₂ identisch oder voneinander verschieden sind,
n Formel 1a B aus einer substituierten oder unsubstituierten carbocyclischen Cs-C₆₀-Gruppe, einer substituierten oder unsubstituierten heterocyclischen C₁-C₆₀-Gruppe, und *'- Si(Q₄₁)(Q₄₂)-*" ausgewählt ist; und p eine ganze Zahl von 1 bis 10 ist,
in Formel 1 -1 * eine Bindestelle für (B)ₚ in Formel 1 a angibt,
in Formel 1-1 Ar₁ aus einer Benzolgruppe, einer Pyridingruppe, einer Pyrimidingruppe, einer Pyrazingruppe, einer Triazingruppe, einer Furangruppe, einer Thiophengruppe, einer Imidazolgruppe, einer Thiazolgruppe, einer Isoxazolgruppe und einer Oxazolgruppe ausgewählt wird,in Formel 1-1 b1 eine ganze Zahl von vier oder mehr ist, und vier oder mehr Ar₁(s) sind identisch oder voneinander verschieden sind.
in Formel 1-1 sind L₁₁ bis L₁₄ jeweils unabhängig eine substituierte oder unsubstituierte carbocyclische C₁-C₆₀-Gruppe, oder eine substituierte oder unsubstituierte heterocyclische C₁-C₆₀ -Gruppe,
in Formel 1-1 a11 bis a14 jeweils unabhängig voneinander 0,1,2, 3, oder 4 sind,
in Formel 1-1, wenn a11 gleich 0 ist, *'-(L₁₁)ₐ₁₁-*" eine Einfachbindung ist; wenn a12 gleich 0 ist, *'-(L₁₂)ₐ₁₂-*" eine Einfachbindung ist; wenn a13 gleich 0 ist, *'-(L₁₃)ₐ₁₃-*" eine Einfachbindung ist; wenn a14 gleich 0 ist, *'-(L₁₄)ₐ₁₄-*" eine Einfachbindung ist; wenn a11 zwei oder mehr ist, zwei oder mehr L₁₁(s) identisch oder verschieden sind; wenn a12 zwei oder mehr ist, zwei oder mehr L₁₂(s) identisch oder verschieden sind; wenn a13 zwei oder mehr ist, zwei oder mehr L₁₃(s) identisch oder verschieden sind; und wenn a14 zwei oder mehr ist, zwei oder mehr L₁₄(s) identisch oder verschieden sind,
in Formel 1-1 Ar₁₁ bis Ar₁₃ jeweils unabhängig voneinander ausgewählt sind aus einer substituierten oder unsubstituierten C₃-C₁₀-Cycloalkylgruppe einer substituierten oder unsubstituierten C₁-C₁₀-Heterocycloalkylgruppe, einer substituierten oder unsubstituierten -C₃-C₁₀-Cycloalkenylgruppe, einer substituierten oder unsubstituierten C₁-C₁₀-Heterocycloalkenylgruppe, einer substituierten oder unsubstituierten -C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe und einer substituierten oder unsubstituierten monovalenten nicht-aromatischen kondensierten heteropolycyclischen Gruppe, wobei mindestens eines von Ar₁₁ bis Ar₁₃ mit einer vernetzbaren Gruppe substituiert ist,
in Formel 1-1 b11 bis b13 jeweils unabhängig voneinander 1,2,3, 4, oder 5 sind, wobei, wenn b11 zwei oder mehr ist, zwei oder mehr Ar₁₁(s) identisch oder voneinander verschieden sind; wenn b12 zwei oder mehr ist, zwei oder mehr Ar₁₂(s) identisch oder voneinander verschieden sind; und wenn b13 zwei oder mehr ist, zwei oder mehr Ar₁₃(S) identisch oder voneinander verschieden sind,
in Formel 1b Ar₁ und Ar₂ jeweils unabhängig eine substituierte oder unsubstituierte carbocyclische C₅-C₆₀-Gruppe sind, oder eine substituierte oder unsubstituierte heterocyclische C₁-C₆₀-Gruppe sind, wobei Ar₁ und Ar₂ identisch oder voneinander verschieden sind,
in Formel 1b, a1 und a2 jeweils unabhängig voneinander eine ganze Zahl von 1 bis 5 sind, wobei, wenn a1 zwei oder mehr ist, zwei oder mehr Ar₁(S) identisch oder voneinander verschieden sind; und wenn a2 zwei oder mehr ist, zwei oder mehr Ar₂(s) identisch oder verschieden sind,
in Formel 1b L₁₁ bis L₁₄ und L₂₁ bis L₂₄ jeweils unabhängig eine substituierte oder unsubstituierte carbocyclische C₅-C₆₀-Gruppe, oder eine substituierte oder unsubstituierte heterocyclische C₁-C₆₀ -Gruppe sind,
in Formel 1b b11 bis b14 und b21 bis b24 jeweils unabhängig voneinander eine ganze Zahl von 0 bis 3 sind, wobei, wenn b11 gleich 0 ist, *-(L₁₁)s₁₁-*' eine Einfachbindung ist; wenn b12 gleich 0 ist, *-(L₁₂)_{b12}-*' eine Einfachbindung ist; wenn b13 gleich 0 ist, *-(L₁₃)_{b13}-*' eine Einfachbindung ist; wenn b14 gleich 0 ist, *-(L₁₄)_{b14}-*' eine Einfachbindung ist; wenn b21 gleich 0 ist, *-(L₂₁)_{b21}-*' eine Einfachbindung ist; wenn b22 gleich 0 ist, *-(L₂₂)_{b22}-*' eine Einfachbindung ist; wenn b23 gleich 0 ist, *-(L₂₃)_{b23}-*' eine Einfachbindung ist; wenn b24 gleich 0 ist, *-(L₂₄)_{b24}-*' eine Einfachbindung ist; wenn b11 zwei oder mehr ist, zwei oder mehr L₁₁(s) identisch oder voneinander verschieden sind; wenn b12 zwei oder mehr ist, zwei oder mehr L₁₂(s) identisch oder voneinander verschieden sind; wenn b13 zwei oder mehr ist, zwei oder mehr L₁₃(s) identisch oder voneinander verschieden sind; wenn b14 zwei oder mehr ist, zwei oder mehr L₁₄(s) identisch oder voneinander verschieden sind; wenn b21 zwei oder mehr ist, zwei oder mehr L₂₁(s) identisch oder voneinander verschieden sind; wenn b22 zwei oder mehr ist, zwei oder mehr L₂₂(s) identisch oder voneinander verschieden sind; wenn b23 zwei oder mehr ist, zwei oder mehr L₂₃(s) identisch oder voneinander verschieden sind; und wenn b24 zwei oder mehr ist, zwei oder mehr L₂₄(s) identisch oder voneinander verschieden sind,
in Formel 1b B₁ und B₂ jeweils unabhängig aus einer Einfachbindung, einer substituierten oder unsubstituierten C₁-C₂₀-Alkylengruppe, einer substituierten oder unsubstituierten C₂-C₂₀-Alkenylengruppe und einer substituierten oder unsubstituierten C₂-C₂₀-Alkynylengruppe ausgewählt sind,
in Formel 1b, m und n jeweils unabhängig voneinander eine ganze Zahl von 1 bis 3 sind, wobei, wenn m zwei oder mehr ist, zwei oder mehr B₁(s) identisch oder voneinander verschieden sind; und wenn n zwei oder mehr ist, zwei oder mehr B₂(s) identisch oder voneinander verschieden sind,
in Formel 1b Ar₁₁, Ar₁₃, Ar₂₁und Ar₂₃ jeweils unabhängig voneinander ausgewählt sind aus einer substituierten oder unsubstituierten C₃-C₁₀-Cycloalkylgruppe einer substituierten oder unsubstituierten C₁-C₁₀ -Heterocycloalkylgruppe, einer substituierten oder unsubstituierten C₃-C₁₀ -Cycloalkenylgruppe, einer substituierten oder unsubstituierten C₁-C₁₀ -Heterocycloalkenylgruppe, einer substituierten oder unsubstituierten C₆-C₆₀ -Arylgruppe, einer substituierten oder unsubstituierten C₆-C₆₀ - Aryloxygruppe, einer substituierten oder unsubstituierten C₆-C₆₀ -Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe und einer substituierten oder unsubstituierten monovalenten nicht-aromatischen kondensierten heteropolycyclischen Guppe,
in Formel 1b Ar₁₂, Ar₁₄, Ar₂₂, und Ar₂₄ jeweils unabhängig ausgewählt sind aus einer substituierten oder unsubstituierten C₃-C₁₀Cycloalkylengruppe, einer substituierten oder unsubstituierten C₁C₁₀-Heterocycloalkylengruppe, einer substituierten oder unsubstituierten C₃-C₁₀-Cycloalkenylengruppe, einer substituierten oder unsubstituierten C₁-C₁₀-Heterocycloalkenylengruppe, einer substituierten oder unsubstituierten -C₆-C₆₀-Arylengruppe, einer substituierten oder unsubstituierten C₁C₆₀-Heteroarylengruppe, einer substituierten oder unsubstituierten zweiwertigen nicht-aromatischen kondensierten polycyclischen Gruppe und einer substituierten oder unsubstituierten zweiwertigen nicht-aromatischen kondensierten heteropolycyclischen Gruppe,
in Formel 1b, c11 bis c14 und c21 bis c24 jeweils unabhängig voneinander eine ganze Zahl von 1 bis 5 sind, wobei, wenn c11 zwei oder mehr ist, zwei oder mehr Ar₁₁(s) identisch oder voneinander verschieden sind; wenn c12 zwei oder mehr ist, zwei oder mehr Ar₁₂(s) identisch oder voneinander verschieden sind; wenn c13 zwei oder mehr ist, zwei oder mehr Ar₁₃(S) identisch oder voneinander verschieden sein können; wenn c14 zwei oder mehr ist, zwei oder mehr Ar₁₄(S) identisch oder voneinander verschieden sind; wenn c21 zwei oder mehr ist, zwei oder mehr Ar₂₁(s) identisch oder voneinander verschieden sind; wenn c22 zwei oder mehr ist, zwei oder mehr Ar₂₂(s) identisch oder voneinander verschieden sind; wenn c23 zwei oder mehr ist, zwei oder mehr Ar₂₃(s) identisch oder voneinander verschieden sind; und wenn c24 zwei oder mehr ist, zwei oder mehr Ar₂₄(s) identisch oder voneinander verschieden sind,
in Formel 1b mindestens eine Gruppe von Ar₁, Ar₂, Ar₁, bis Ar₁₄und Ar₂, bis Ar₂₄, durch eine vernetzbare Gruppe substituiert ist,
*'und *" jeweils eine Bindungsstelle zu einem benachbarten Atom angeben,
mindestens ein Substituent der substituierten carbocyclischen C₅-C₆₀-Gruppe, der substituierten heterocyclischen C₁-C₆₀-Gruppe, der substituierten C₃-C₁₀-Cycloalkylengruppe, der substituierten C₁-C₁₀-Heterocycloalkylengruppe, der substituierten C₃-C₁₀-Cycloalkenylengruppe, der substituierten C₁-C₁₀-Heterocycloalkenylengruppe, der substituierten C₆-C₆₀-Arylengruppe, der substituierten C₁-C₆₀-Heteroarylengruppe, der substituierten zweiwertigen nicht-aromatischen kondensierten polycyclischen Gruppe, der substituierten zweiwertigen nicht-aromatischen kondensierten heteropolycyclischen Gruppe, der substituierten C₃-C₁₀-Cycloalkylgruppe, der substituierten C₁-C₁₀-Heterocycloalkylgruppe, der substituierten C₃-C₁₀ Cycloalkenylgruppe, der substituierten C₁-C₁₀-Heterocycloalkenylgruppe, der substituierten C₆-C₆₀-Arylgruppe, der substituierten C₆-C₆₀-Aryloxygruppe, der substituierten C₆-C₆₀-Arylthiogruppe, der substituierten C₁-C₆₀-Heteroarylgruppe, der substituierten einwertigen nicht-aromatischen kondensierten polycyclischen Gruppe, der substituierten einwertigen nicht-aromatischen kondensierten heterocyclischen Gruppe, der substituierten C₁-C₂₀-Alkylengruppe, der substituierten C₂-C₂₀-Alkenylengruppe, und der substituierten C₂-C₂₀-Alkynylengruppe ausgewählt ist aus:
Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₁-C₆₀-Alkylgruppe, einer C₂-C₆₀-Alkenylgruppe, einer C₂-C₆₀-Alkinylgruppe und einer C₁-C₆₀-Alkoxygruppe;
einer C₁-C₆₀-Alkylgruppe, einer C₂-C₆₀-Alkenylgruppe, einer C₂-C₆₀-Alkinylgruppe und einer C₁-C₆₀-Alkoxygruppe, jeweils substituiert mit mindestens einem, ausgewählt aus Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nicht-aromatischen kondensierten polycyclischen Gruppe, einer einwertigen nicht-aromatischen kondensierten heteropolycyclischen Gruppe, - Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁) und -P(=O)(Q₁₁)(Q₁₂);
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nicht-aromatischen kondensierten polycyclischen Gruppe, einer einwertigen nicht-aromatischen kondensierten heteropolycyclischen Gruppe, einer Biphenylgruppe und einer Terphenylgruppe;
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nicht-aromatischen kondensierten polycyclischen Gruppe, und einer einwertigen nicht-aromatischen kondensierten heteropolycyclischen Gruppe, jeweils substituiert mit mindestens einem, ausgewählt aus Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₁-C₆₀-Alkylgruppe, einer C₂-C₆₀-Alkenylgruppe, einer C₂-C₆₀-Alkinylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nicht-aromatischen kondensierten polycyclischen Gruppe, einer einwertigen nicht-aromatischen kondensierten heteropolycyclischen Gruppe, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁) und -P(=O)(Q₂₁)(Q₂₂); und
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁) und - P(=O)(Q₃₁)(Q₃₂) und
Q₁₁ bis Q₁₃, Q₂₁ bis Q₂₃, Q₃₁ bis Q₃₃, und Q₄₁ bis Q₄₂ jeweils unabhängig ausgewählt sind aus Wasserstoff, Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₁-C₆₀-Alkylgruppe, einer C₂-C₆₀-Alkenylgruppe, einer C₂-C₆₀-Alkynylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer einwertigen nicht-aromatischen kondensierten polycyclischen Gruppe, einer einwertigen nicht-aromatischen kondensierten heteropolycyclischen Gruppe, einer Biphenylgruppe und einer Terphenylgruppe.

7. Vernetzbare auf Arylamin basierende Verbindung nach Anspruch 6, wobei in Formel 1a A₁ und A₂ zueinander identisch sind.

8. Vernetzbare auf Arylamin basierende Verbindung nach Anspruch 6 oder Anspruch 7, wobei die durch Formel 1a dargestellte auf Arylamin basierende Verbindung symmetrisch in Bezug auf *'-(B)ₚ-*" ist.

9. Vernetzbare auf Arylamin basierende Verbindung nach einem der Ansprüche 6 bis 8, wobei:
in Formel 1b Ar₁ und Ar₂ jeweils unabhängig voneinander ausgewählt werden aus:
einer C₆-C₆₀-Arylengruppe und einer C₁-C₆₀-Heteroarylengruppe; und
einer C₆-C₆₀-Arylengruppe und einer C₁-C₆₀-Heteroarylengruppe, jeweils substituiert durch mindestens eines, ausgewählt aus Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₁-C₆₀-Alkylgruppe, einer C₁-C₆₀-Alkoxygruppe, -N(Q₃₁)(Q₃₂), und einer vernetzbaren Gruppe,
wobei Q₃₁ und Q₃₂ dieselben wie in einem der Ansprüche 6 bis 8 definiert sind.

10. Vernetzbare auf Arylamin basierende Verbindung nach einem der Ansprüche 6 bis 9, wobei:
in Formel 1-1 Ar₁ eine Benzolgruppe ist, und b1 4 oder 5 ist, und
in Formel 1b Ar₁ und Ar₂ jeweils unabhängig voneinander ausgewählt werden aus:
einer Phenylengruppe und einer Naphthylengruppe; und
einer Phenylengruppe und einer Naphthylengruppe, jeweils substituiert durch eines, ausgewählt aus Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₁-C₆₀-Alkylgruppe, einer C₁-C₆₀-Alkoxygruppe, -N(Q₃₁)(Q₃₂), und einer vernetzbaren Gruppe,
wobei Q₃₁ und Q₃₂dieselben wie in einem der Ansprüche 6 bis 8 definiert sind.

11. Vernetzbare auf Arylamin basierende Verbindung nach einem der Ansprüche 6 bis 10, wobei:
in der Formel 1-1 die vier oder mehr Ar₁(s) zueinander identisch sind, und
in Formel 1b, wenn a1 zwei oder mehr ist, zwei oder mehr Ar₁(s) zueinander identisch sind, und wenn a2 zwei oder mehr ist, zwei oder mehr Ar₂(s) zueinander identisch sind.

12. Vernetzbare auf Arylamin basierende Verbindung nach einem der Ansprüche 6 bis 11, wobei in Formel 1b *-(Ar₁)ₐ₁-*' and *-(Ar₂)ₐ₂-*' zueinander identisch sind.

13. Vernetzbare auf Arylamin basierende Verbindung nach einem der Ansprüche 6 bis 12, wobei:
L₁₁ bis L₁₄ in Formel 1-1 und die L₁₁ bis L₁₄ und L₂₁ bis L₂₄ in Formel 1b jeweils unabhängig voneinander aus den durch die Formeln 2-1 bis 2-37 repräsentierten Gruppen ausgewählt werden: 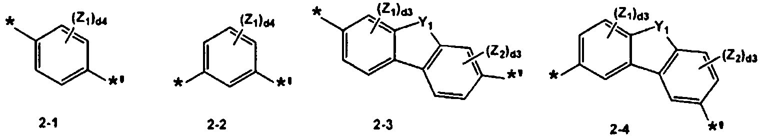 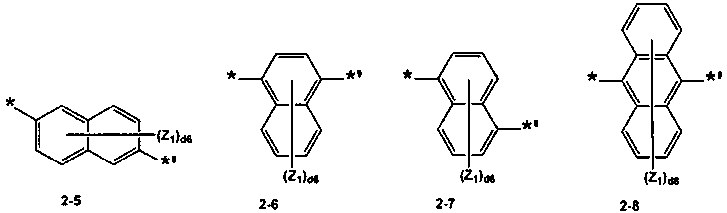 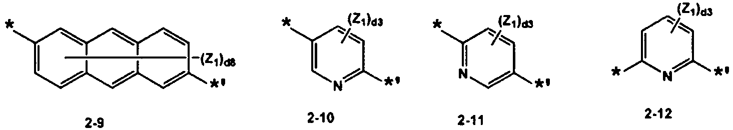 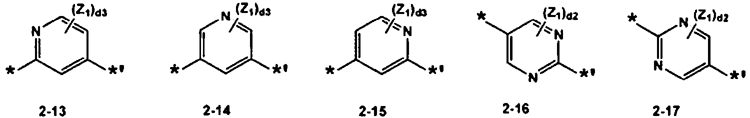 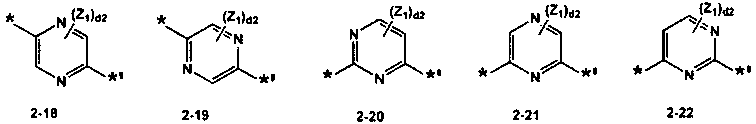 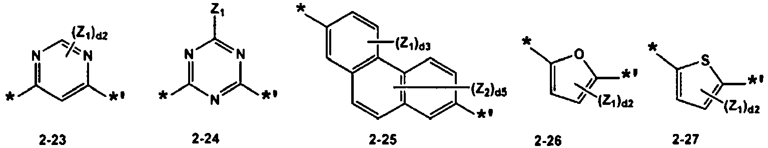 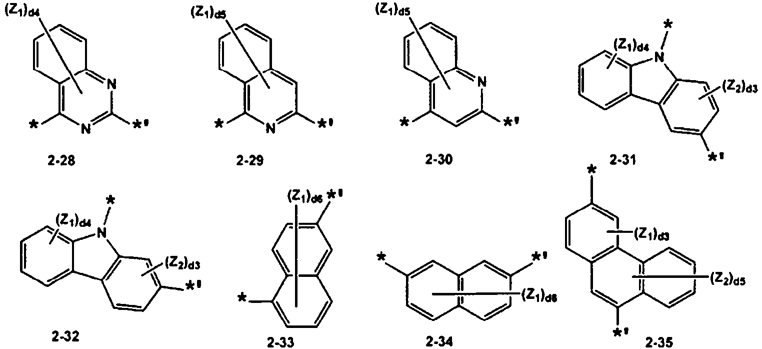 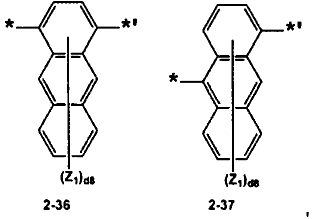 wobei in den Formeln 2-1 bis 3-37,
Y1 O, S, C(Z₃)(Z₄), N(Z₅), oder Si(Z₆)(Z₇) ist,
Z₁ bis Z₇ werden jeweils unabhängig voneinander ausgewählt sind aus:
Wasserstoff, Deuterium, -F, -Cl, -Br, -I, einer Hydroxylgruppe, einer Cyanogruppe, einer Nitrogruppe, einer Amidinogruppe, einer Hydrazinogruppe, einer Hydrazonogruppe, einer C₁-C₂₀-Alkylgruppe, einer C₁-C₂₀-Alkoxygruppe, einer Cyclopentylgruppe, einer Cyclohexylgruppe, einer Cycloheptylgruppe, einer Cyclopentenylgruppe, eine Cyclohexenylgruppe, einer Phenylgruppe, eine Biphenylgruppe, einer Terphenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spiro-Fluorenylgruppe, einer Benzofluorenylgruppe, einer Dibenzofluorenylgruppe, einer Phenalenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Naphthacenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Indolylgruppe, einer Isoindolylgruppe, einer Indazolylgruppe, einer Purinylgruppe, einer Chinolinylgruppe, einer Isochinolinylgruppe, einer Benzochinolinylgruppe, einer Phthalazinylgruppe, einer Naphthyridinylgruppe, einer Chinoxalinylgruppe, einer Chinazolinylgruppe, einer Cinnolinylgruppe, einer Carbazolylgruppe, einer Phenanthridinylgruppe, einer Acridinylgruppe, einer Phenanthrolinylgruppe, einer Phenazinyleingruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Dibenzocarbazolylgruppe, einer Benzocarbazolylgruppe, einer Thiadiazolylgruppe, und einer Imidazopyridinylgruppe;
d2 eine ganze Zahl von 0 bis 2 ist,
d3 eine ganze Zahl von 0 bis 3 ist,
d4 ist eine ganze Zahl von 0 bis 4 ist,
d5 eine ganze Zahl von 0 bis 5 ist,
d6 eine ganze Zahl von 0 bis 6 ist,
d8 eine ganze Zahl von 0 bis 8 ist, und
* und *' jeweils eine Bindestelle zu einem benachbarten Atom angibt.

14. Vernetzbare auf Arylamin basierende Verbindung nach einem der Ansprüche 6 bis 13, wobei:
in der Formel 1a *-(B)ₚ-*' aus den durch die Formeln 3-1 bis 3-19 dargestellten Gruppen ausgewählt wird: 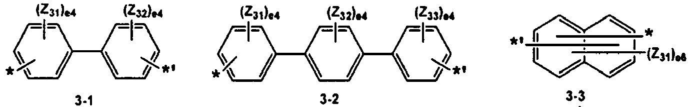 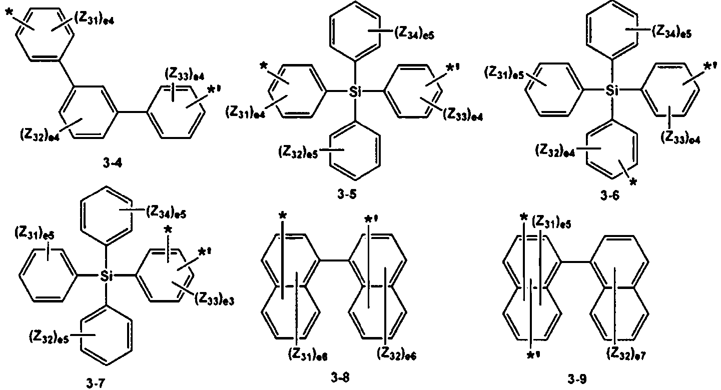 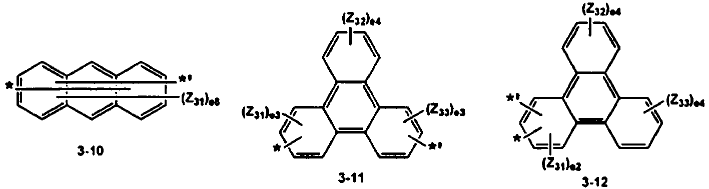 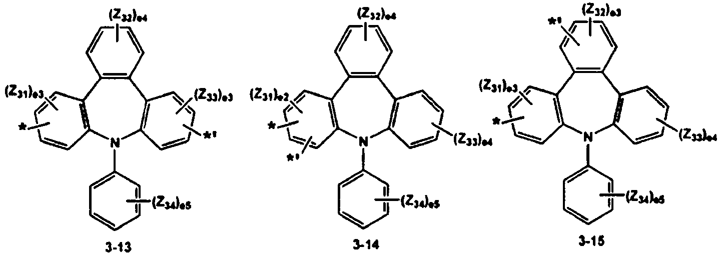 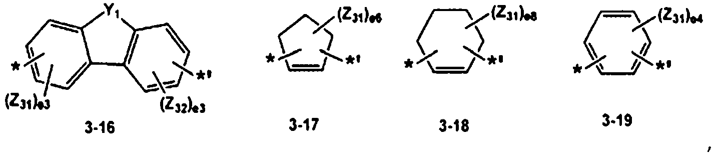 wobei in Formeln 3-1 bis 3-19
Y₁ O, S, C(Z₃₅)(Z₃₆), N(Z₃₅), oder Si(Z₃₅)(Z₃₆) ist,
Z₃₁ bis Z₃₆ jeweils unabhängig voneinander ausgewählt sind aus Wasserstoff, Deuterium, -F, -Cl, -Br, -I, einer Hydroxylgruppe, einer Cyanogruppe, einer Nitrogruppe, einer Amidinogruppe, einer Hydrazinogruppe, einer Hydrazonogruppe, einer C₁-C₂₀-Alkylgruppe, einer C₁-C₂₀-Alkoxygruppe, einer Cyclopropenylgruppe, einer Cyclobutenylgruppe, einer Cyclopentylgruppe, einer Cyclohexylgruppe, einer Cycloheptylgruppe, einer Cyclopentenylgruppe, eine Cyclohexenylgruppe, einer Phenylgruppe, eine Biphenylgruppe, einer Terphenylgruppe, einer Pentalenylgruppe, einer Indenylgruppe, einer Naphthylgruppe, einer Azulenylgruppe, einer Indacenylgruppe, einer Acenaphthylgruppe, einer Fluorenylgruppe, einer Spiro-Bifluorenylgruppe, einer Spiro-Benzofluoren-Fluorenyl gruppe, einer Benzofluorenylgruppe, einer Dibenzofluorenylgruppe, einer Phenalenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Perylenylgruppe, einer Pentacenylgruppe, einer Pyrrolylgruppe, einer Thiophenylgruppe, eine Furanylgruppe, einer Silolylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Indolylgruppe, einer Isoindolylgruppe, einer Indazolylgruppe, einer Purinylgruppe, einer Chinolinylgruppe, einer Isochinolinylgruppe, einer Benzochinolinylgruppe, einer Isochinolinylgruppe, Phthalazinylgruppe, einer Naphthyridinylgruppe, einer Chinoxalinylgruppe, einer Benzochinoxalinylgruppe, einer Chinazolinylgruppe, einer Benzochinazolinylgruppe, einer Cinnolinylgruppe, Phenanthridinylgruppe, einer Acridinylgruppe, einer Phenanthrolinylgruppe, einer Phenazinyleingruppe, einer Benzimidazolylgruppe, einer Benzofuranylgruppe, einer Benzothiophenylgruppe, einer Benzosilolylgruppe, einer Benzothiazolylgruppe, einer Isobenzothiazolyl gruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Carbazolylgruppe, einer Dibenzofuranylgruppe, einer Dibenzothiophenylgruppe, einer Dibenzosilolylgruppe, einer Benzocarbazolylgruppe, einer Naphthobenzofuranylgruppe, einer Naphthobenzothiophenylgruppe, einer Naphthobenzosilolylgruppe, einer Dibenzocarbazolylgruppe, einer Dinaphthofuranylgruppe, einer Dinaphthothiophenyl gruppe, einer Dinaphthosilolylgruppe, einer Thiadiazolylgruppe, einer Imidazopyridinylgruppe, einer Imidazopyrimidinylgruppe, einer Oxazolopyridinylgruppe, einer Thiazolopyridinylgruppe, einer Benzonaphthyridinylgruppe, einer Azafluorenylgruppe, einer Azaspiro-Bifluorenylgruppe, einer Azacarbazolylgruppe, einer Azadibenzofuranylgruppe, einer Azadibenzothiophenylgruppe, einer Azadibenzosilolylgruppe, einer Indenopyrrolylgruppe, einer Indolopyrrolylgruppe, einer Indenocarbazolylgruppe, einer Indolocarbazolylgruppe, - Si(Q₃₁)(Q₃₂)(Q₃₃), and -N(Q₃₁)(Q₃₂),
Q₃₁ und Q₃₃ jeweils unabhängig ausgewählt sind aus:
einer C₁-C₁₀-Alkylgruppe, einer C₁-C₁₀-Alkoxygruppe, einer Phenylgruppe, einer Biphenylgruppe, einer Terphenylgruppe, und einer Naphthylgruppe; und
einer Phenylgruppe, einer Biphenylgruppe, einer Terphenylgruppe und einer Naphthylgruppe, die jeweils mit mindestens einer, ausgewählt aus einer C₁-C₁₀-Alkylgruppe, einerC₁-C₁₀-Alkoxygruppe und einer Phenylgruppe substituiert sind,
e2 eine ganze Zahl von 0 bis 2 ist,
e3 eine ganze Zahl von 0 bis 3 ist,
e4 ist eine ganze Zahl von 0 bis 4 ist,
e5 eine ganze Zahl von 0 bis 5 ist,
e6 eine ganze Zahl von 0 bis 6 ist,
e7 eine ganze Zahl von 0 bis 7 ist;
e8 eine ganze Zahl von 0 bis 8 ist, und
* und *' jeweils eine Bindestelle zu einem benachbarten Atom angibt.

15. Vernetzbare auf Arylamin basierende Verbindung nach einem der Ansprüche 6 bis 14, wobei:
in Formel 1-1 Ar₁₁ bis Ar₁₃ jeweils unabhängig voneinander ausgewählt sind aus:
einer C₆-C₆₀-Arylgruppe; und
einer C₆-C₆₀-Arylgruppe, substituiert mit mindestens einem ausgewählt aus Deuterium, -F, - Cl, -Br, -I, einer Hydroxylgruppe, einer Cyanogruppe, einer Nitrogruppe, einer Amidinogruppe, einer Hydrazinogruppe, einer Hydrazonogruppe, einer C₂-C₆₀-Alkenylgruppe, einer C₂-C₆₀Alkynylgruppe, und einer C₁-C₆₀-Alkoxygruppe, und
in Formel 1b Ar₁₁, Ar₁₃, Ar₂₁, und Ar₂₃ jeweils unabhängig voneinander aus ausgewählt sind aus:
einer Phenylgruppe, einer Biphenylgruppe, einer Terphenylgruppe und einer Naphthylgruppe; und
einer Phenylgruppe, einer Biphenylgruppe, einer Terphenylgruppe und einer Naphthylgruppe, jeweils substituiert mit mindestens einem von Deuterium, -F, -Cl, -Br, -I, einer Hydroxylgruppe, einer Cyanogruppe, einer Nitrogruppe, einer Amidinogruppe, einer Hydrazinogruppe, einer Hydrazonogruppe, einer C₁-C₂₀-Alkylgruppe, einer C₁-C₂₀-Alkoxygruppe, einer Phenylgruppe, einer Biphenylgruppe, einer Terphenylgruppe, einer Naphthylgruppe, einer vernetzbaren Gruppe und -N(Q₃₁)(Q₃₂),
wobei Q₃₁ und Q₃₂ dieselben wie in einem der Ansprüche 6 bis 14 definiert sind.

16. Vernetzbare auf Arylamin basierende Verbindung nach einem der Ansprüche 6 bis 15, wobei in Formel 1b Ar₁₂, Ar₁₄, Ar₂₂, und Ar₂₄ jeweils unabhängig voneinander ausgewählt sind aus:
einer einzelnen Bindung;
einer Phenylengruppe und einer Naphthylengruppe; und
einer Phenylengruppe und einer Naphthylengruppe, jeweils substituiert durch eines, ausgewählt aus Deuterium, -F, -Cl, -Br, -I, einer Hydroxylgruppe, einer Cyanogruppe, einer Nitrogruppe, einer Amidinogruppe, einer Hydrazingruppe, einer Hydrazongruppe, einer C₁-C₆₀-Alkylgruppe, einer C₁-C₆₀-Alkoxygruppe, -N(Q₃₁)(Q₃₂), und einer vernetzbaren Gruppe,
wobei Q₃₁ und Q₃₂ dieselben wie in einem der Ansprüche 6 bis 14 definiert sind.

17. Vernetzbare auf Arylamin basierende Verbindung nach einem der Ansprüche 6 bis 16, wobei in Formel 1b Ar₁₁ und Ar₁₃ miteinander identisch sind, Ar₂₁ und Ar₂₃ miteinander identisch sind, Ar₁₂ und Ar₁₄ miteinander identisch sind und Ar₂₂ und Ar₂₄ miteinander identisch sind.

18. Vernetzbare auf Arylamin basierende Verbindung nach einem der Ansprüche 6 bis 17, wobei in Formel 1b Substituenten von Ar₁₁, Ar₁₃, Ar₂₁und Ar₂₃ eine vernetzbare Gruppe umfassen.

19. Vernetzbare auf Arylamin basierende Verbindung nach einem der Ansprüche 6 bis 18, wobei in Formel 1b,
Substituenten mindestens zweier Gruppen, ausgewählt aus Ar₁, Ar₂, Ar₁₂, Ar₁₄, Ar₂₂, und Ar₂₄ eine vernetzbare Gruppe umfassen, und B₁ und B₂ jeweils eine Einfachbindung sind.

20. Vernetzbare auf Arylamin basierende Verbindung nach einem der Ansprüche 6 bis 19, wobei die vernetzbare Gruppe mindestens eine ausgewählt aus einer Vinylen-Einheit, einer Styrol-Einheit, einer Cyclobutan-Einheit oder einer Epoxy-Einheit umfasst.

21. Vernetzbare auf Arylamin basierende Verbindung nach einem der Ansprüche 6 bis 20, wobei die vernetzbare Gruppe aus Gruppen ausgewählt ist, die durch die Formeln 4-1 bis 4-14 dargestellt sind: 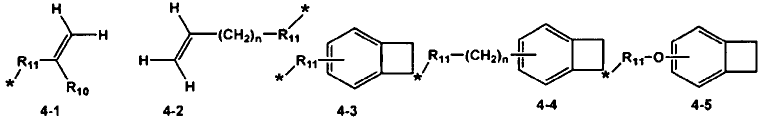 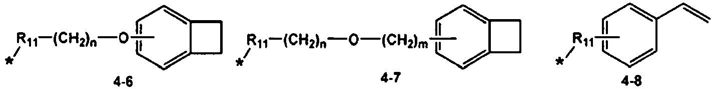 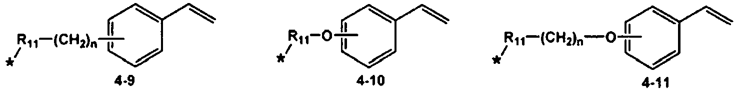 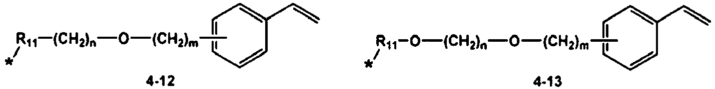 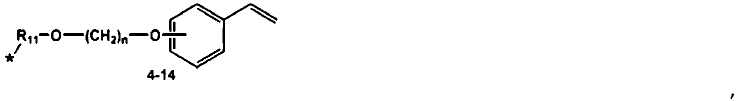 wobei in den Formeln 4-1 bis 4-14,
R₁₀ ein Wasserstoffatom oder eine substituierte oder unsubstituierte C₁-C₂₀-Alkylgruppe ist,
R₁₁ ausgewählt ist aus einer Einfachbindung, einer substituierten oder unsubstituierten C₁-C₂₀-Alkylengruppe, einer substituierten oder unsubstituierten C₃-C₁₀-Cycloalkylengruppe, einer substituierten oder unsubstituierten C₁-C₁₀-Heterocycloalkylengruppe, einer substituierten oder unsubstituierten C₃-C₁₀-Cycloalkenylengruppe, einer substituierten oder unsubstituierten C₁-C₁₀-Heterocycloalkenylengruppe, einer substituierten oder unsubstituierten C₆-C₆₀-Arylengruppe, einer substituierten oder unsubstituierten C₁-C₆₀-Heteroarylengruppe, einer substituierten oder unsubstituierten zweiwertigen nicht-aromatischen kondensierten polycyclischen Gruppe und einer substituierten oder unsubstituierten zweiwertigen nicht-aromatischen kondensierten heteropolycyclischen Gruppe,
m und n jeweils unabhängig eine ganze Zahl von 1 bis 10 sind, und
* eine Bindungsstelle an ein benachbartes Atom angibt.

22. Vernetzbare auf Arylamin basierende Verbindung nach Anspruch 6, wobei die vernetzbare auf Arylamin basierende Verbindung aus Verbindungen 1 bis 40 ausgewählt ist:

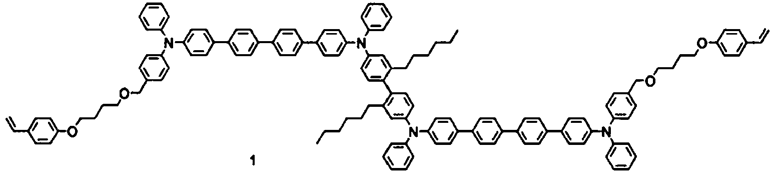

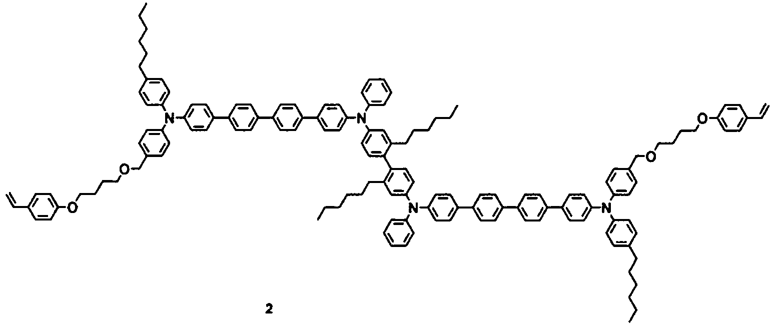

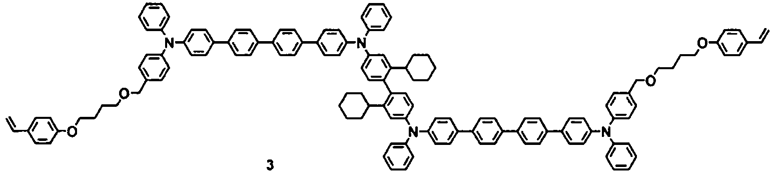

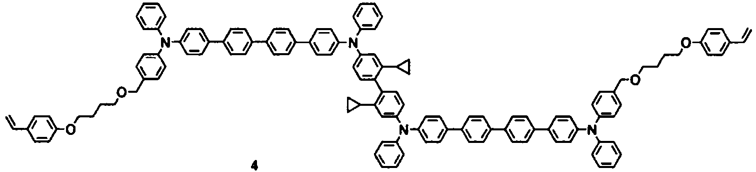

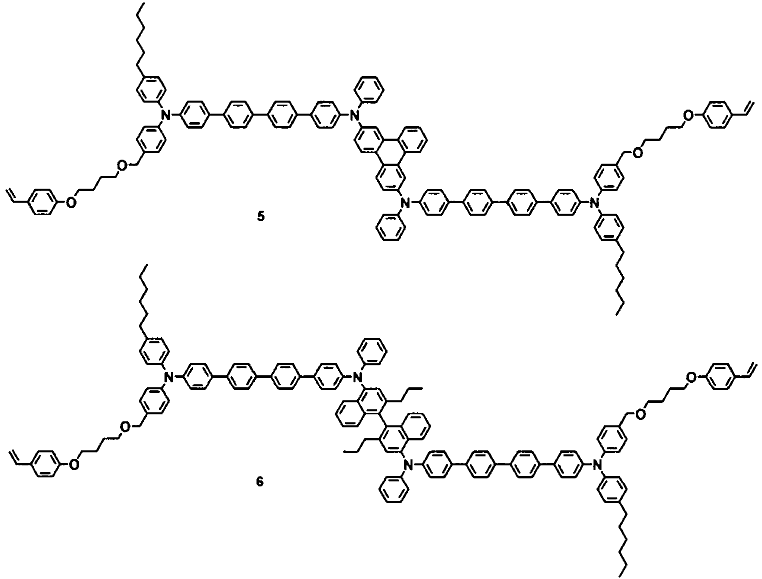

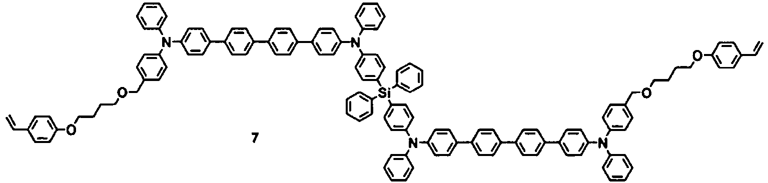

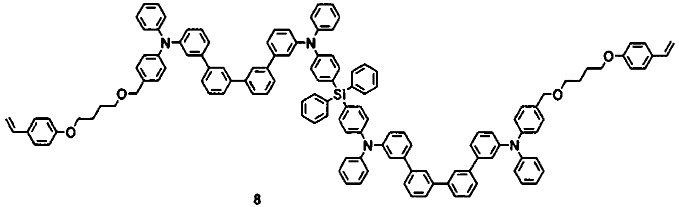

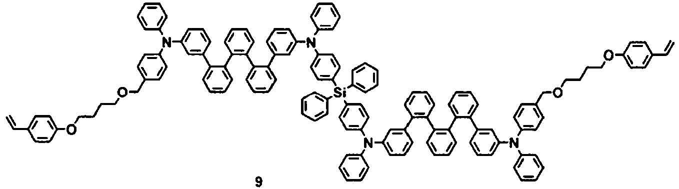

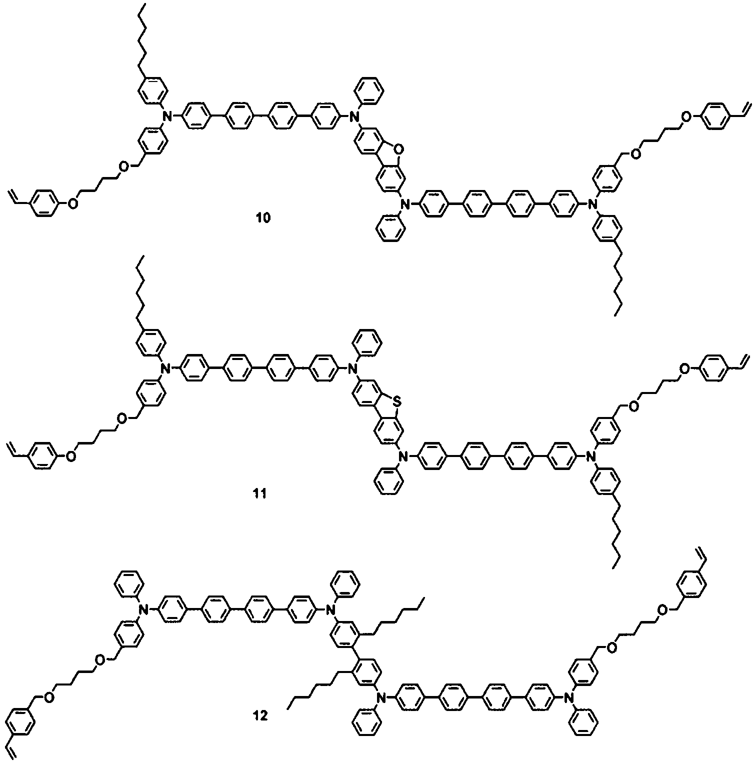

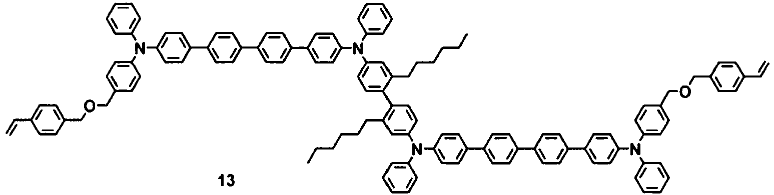

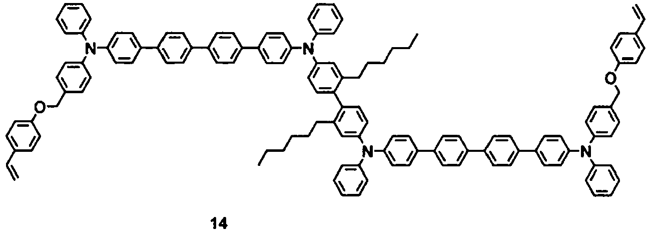

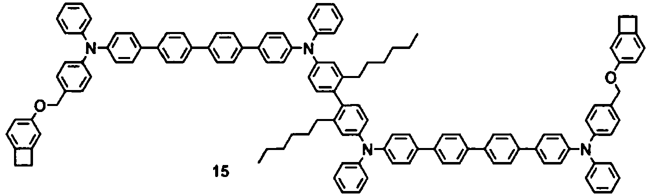

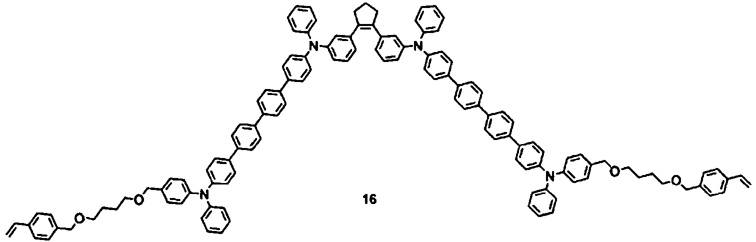

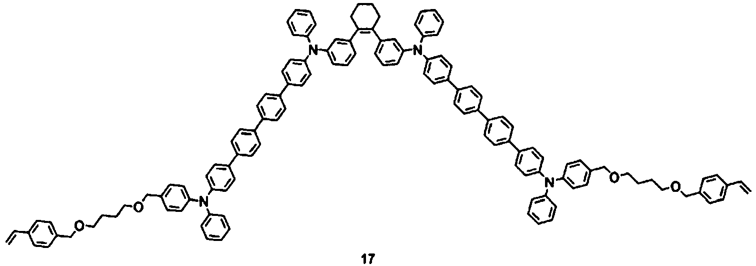

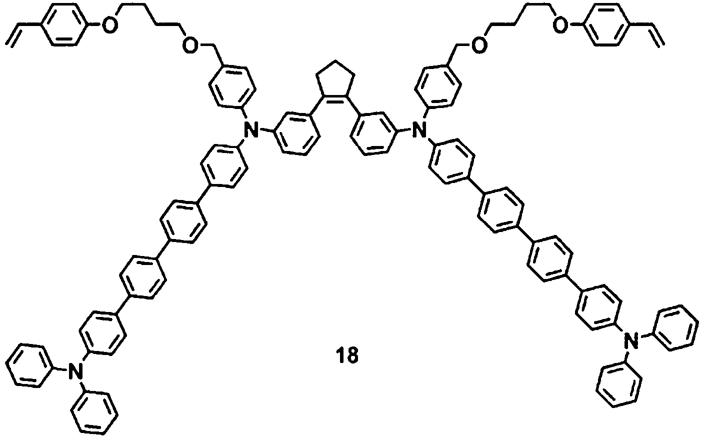

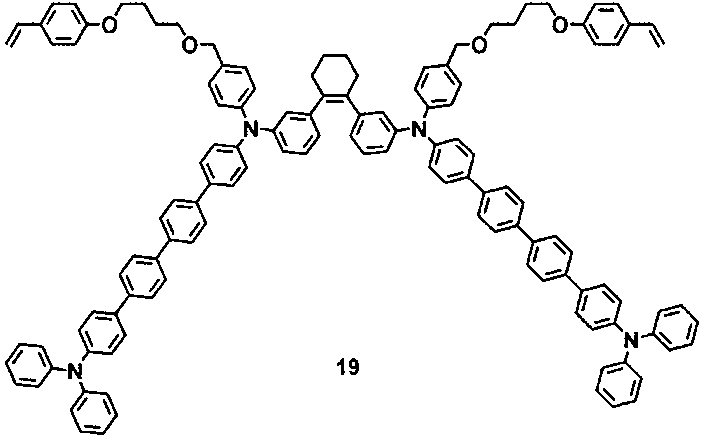

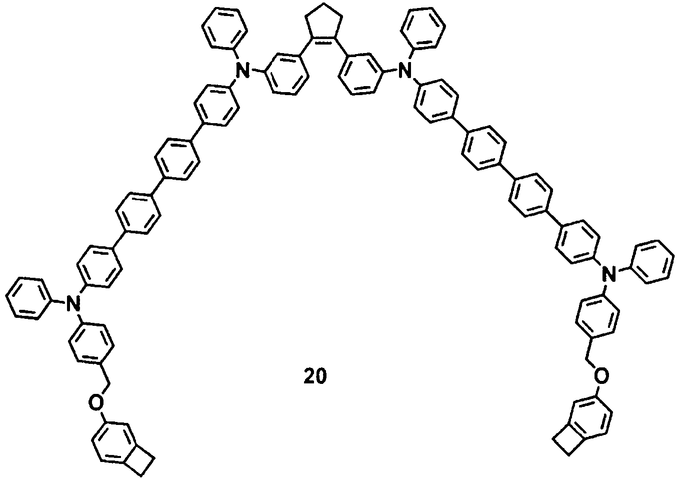

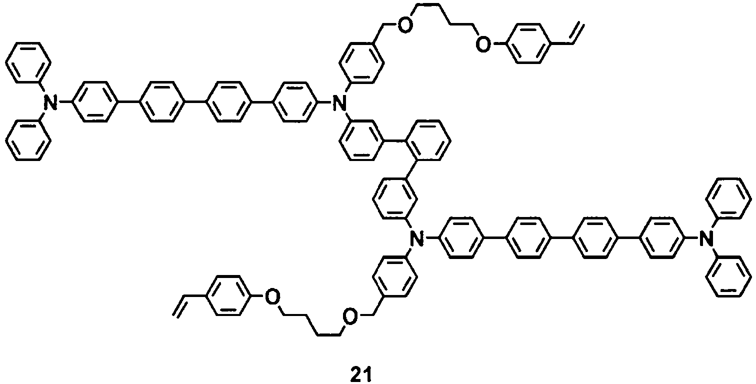

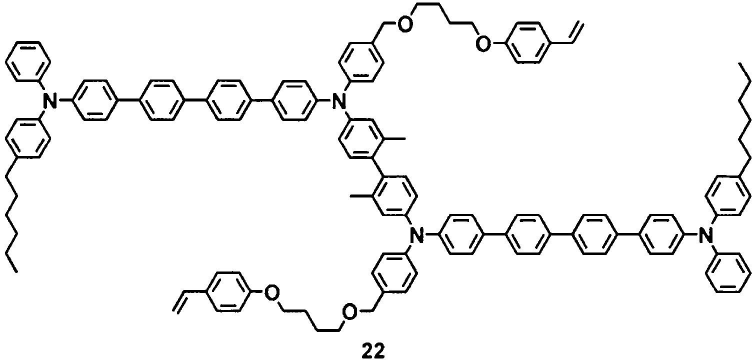

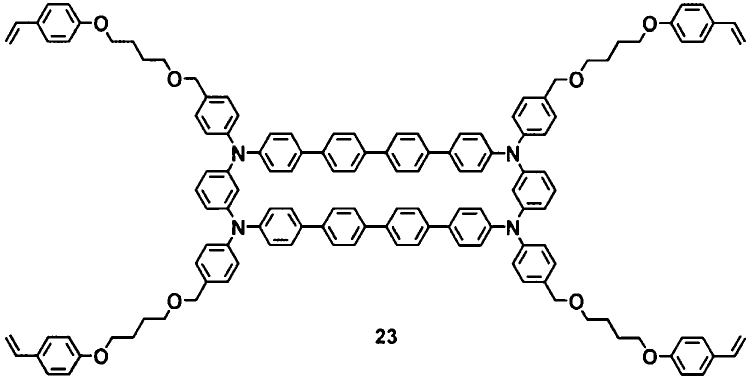

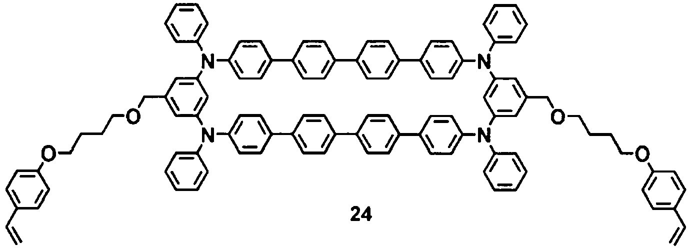

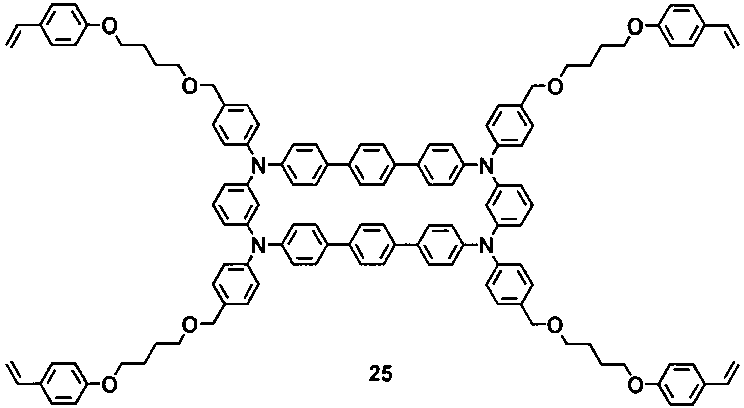

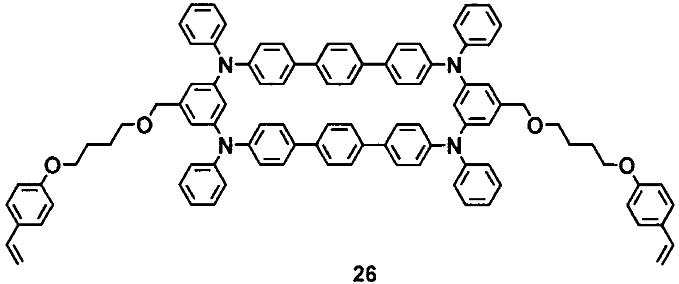

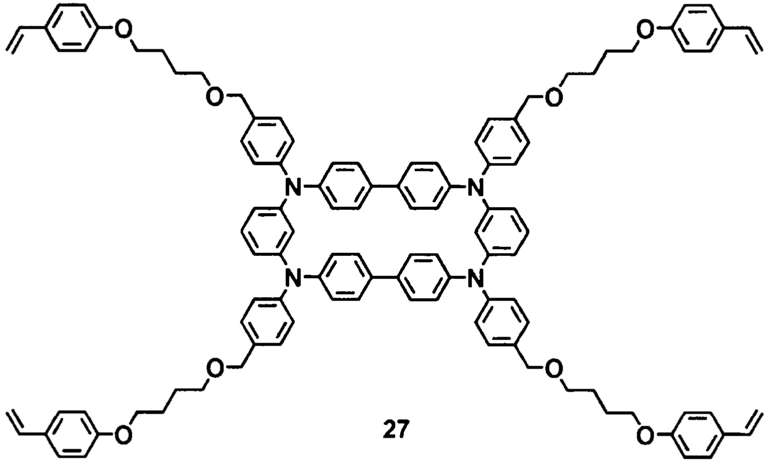

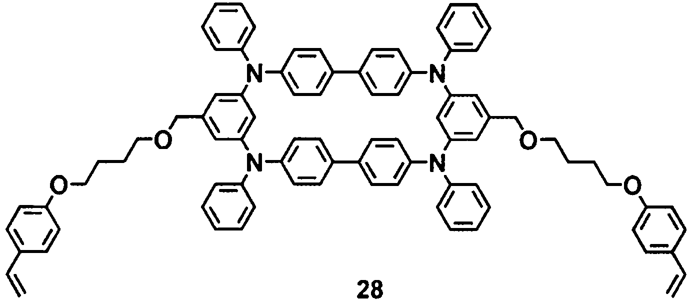

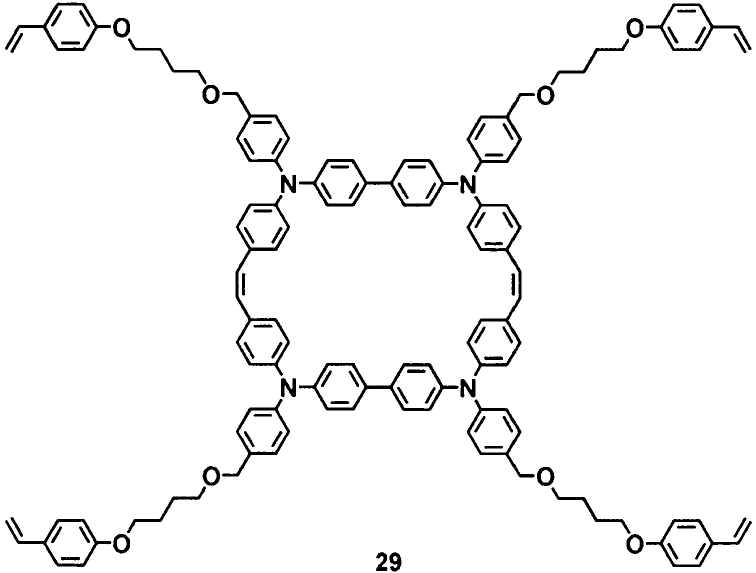

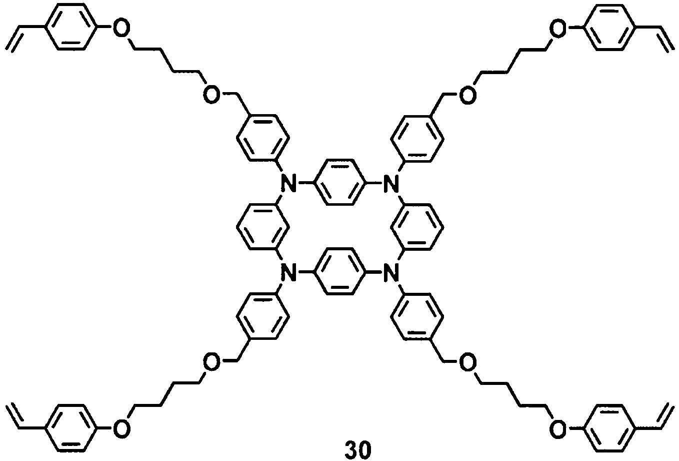

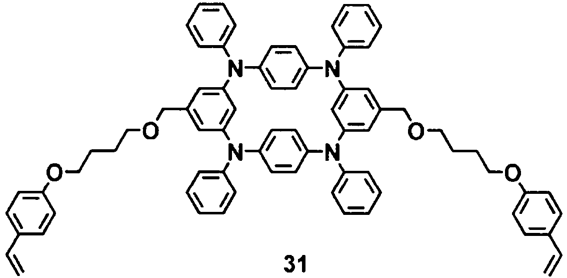

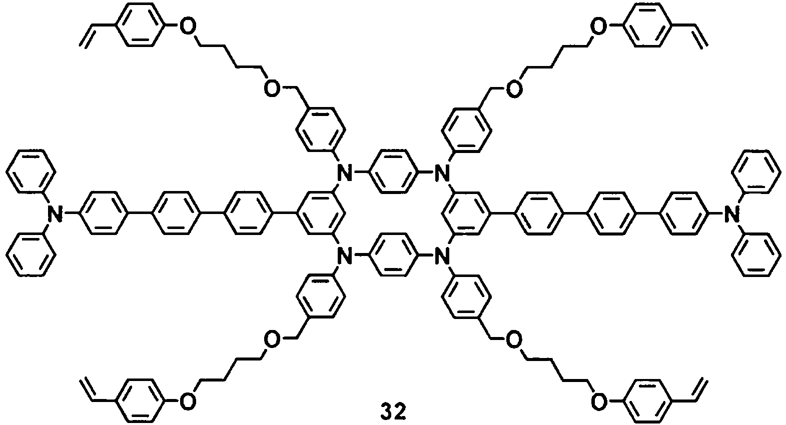

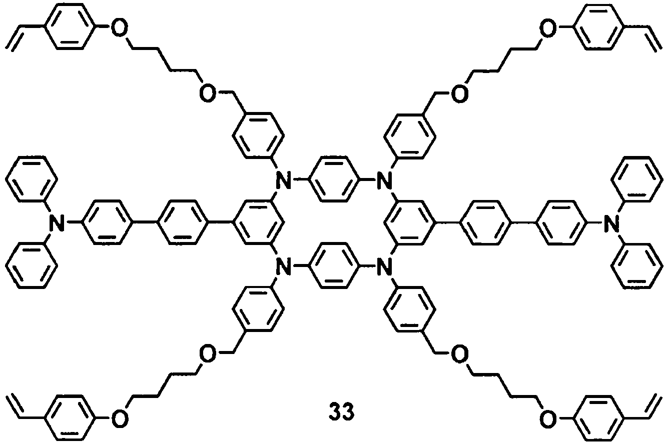

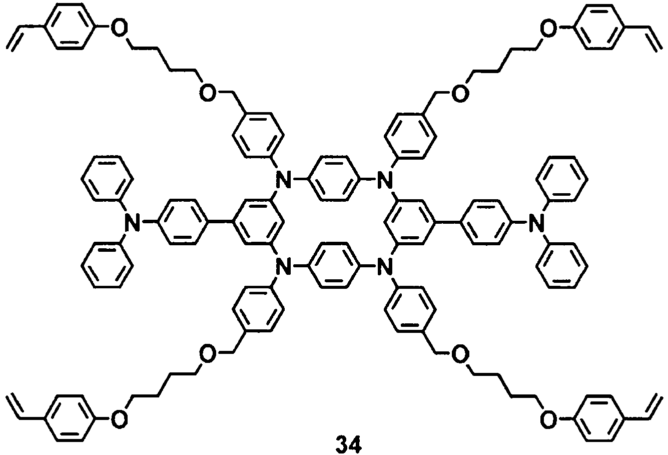

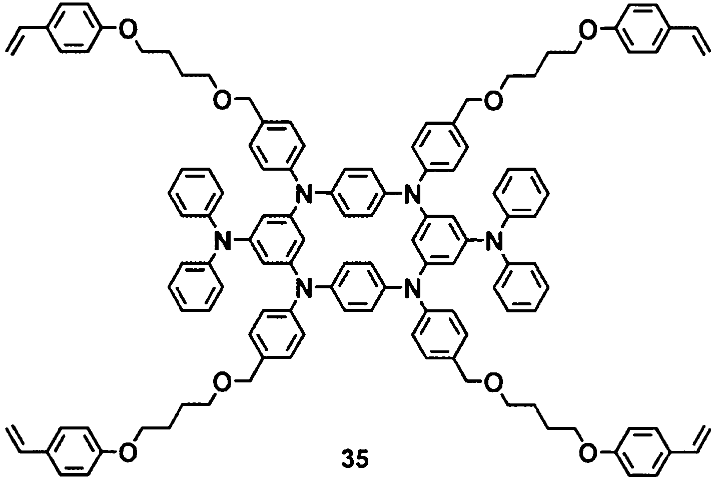

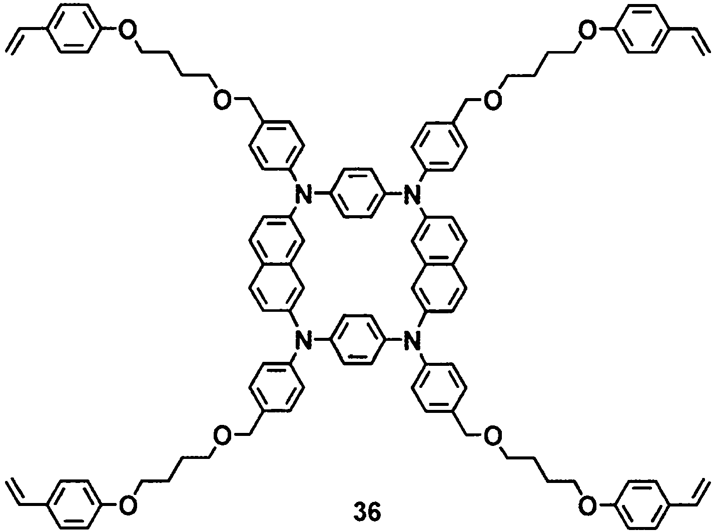

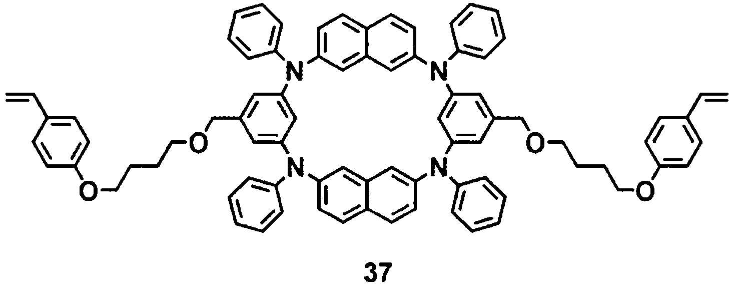

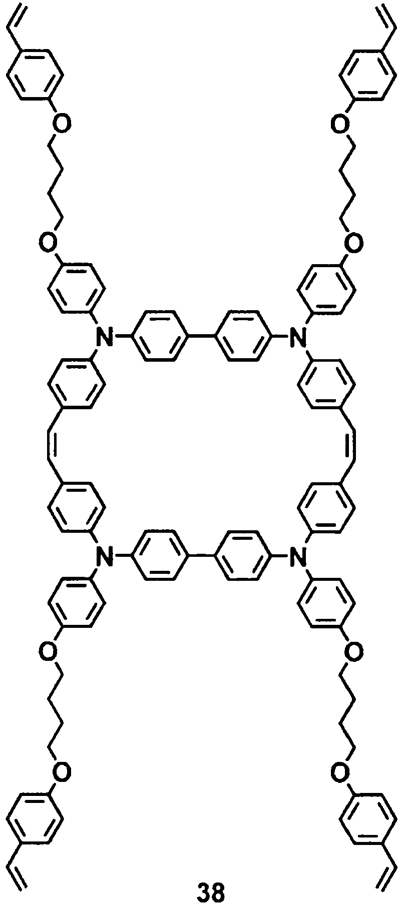

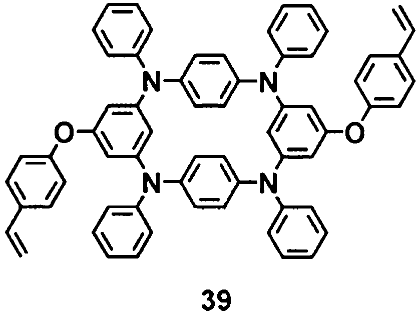

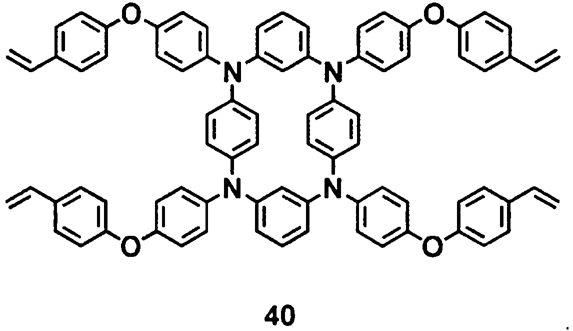

23. Auf Arylamin basierendes Polymer, das eine sich wiederholende Einheit umfasst, die durch Formel 11 dargestellt ist, oder durch Vernetzen einer vernetzbaren auf Arylamin basierenden Verbindung gebildet wird, die durch Formel 1b dargestellt ist: 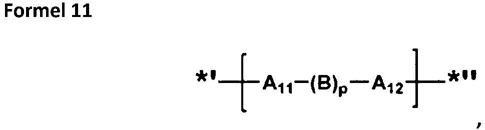 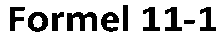 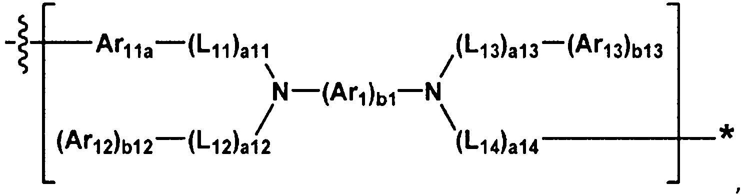 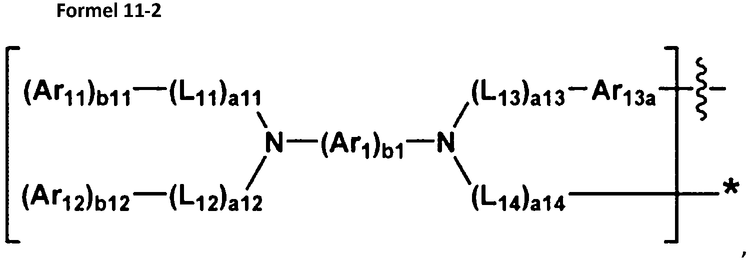 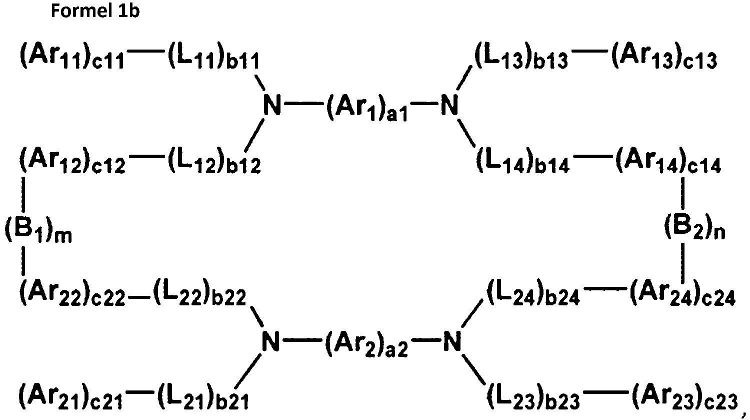
wobei in Formel 11 A₁₁ und A₁₂ jeweils unabhängig voneinander aus den durch die Formeln 11-1 und 11-2 dargestellten Gruppen ausgewählt werden und A₁₁ und A₁₂ identisch oder voneinander verschieden sind,
in Formel 11-1 * eine Bindestelle für (B)ₚ in Formel 11 angibt,
in den Formeln 11, 11-1 und 11-2, *', *", und 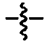 jeweils eine Bindestelle an eine benachbarte Wiederholungseinheit angeben,
In den Formeln 11-1 und 11-2 Ar₁₁ₐ und Ar₁₃, jeweils ein nach dem Vernetzen gebildeter Rest sind, und die sich wiederholenden Einheiten durch eine zweiwertige Cyclobutangruppe verlinkt sind,
In Formel 11 wird B aus einer substituierten oder unsubstituierten carbocyclischen C₅-C₆₀-Gruppe, einer substituierten oder unsubstituierten heterocyclischen C₁-C₆₀-Gruppe, und *'- Si(Q₄₁)(Q₄₂)-*" ausgewählt ist; p eine ganze Zahl von 1 bis 10 ist,
In den Formeln 11-1 und 11-2 AH aus einer Benzolgruppe, einer Pyridingruppe, einer Pyrimidingruppe, einer Pyrazingruppe, einer Triazingruppe, einer Furangruppe, einer Thiophengruppe, einer Imidazolgruppe, einer Thiazolgruppe, einer Isoxazolgruppe und einer Oxazolgruppe ausgewählt wird,
b1 eine ganze Zahl von vier oder mehr ist, und vier oder mehr Ar₁(s) identisch oder voneinander verschieden sind,
in Formeln 11-1 und 11-2 L₁₁ bis L₁₄ jeweils unabhängig eine substituierte oder unsubstituierte carbocyclische C₅-C₆₀-Gruppe, oder eine substituierte oder unsubstituierte heterocyclische C₁-C₆₀ -Gruppe sind,
in Formeln 11-1 und 11-2 sind a11 bis a14 jeweils unabhängig voneinander 0, 1, 2, 3, oder 4 sind, wobei, wenn a11 gleich 0 ist, *'-(L₁₁)ₐ₁₁-*" eine Einfachbindung ist; wenn a12 gleich 0 ist, *'- (L₁₂)ₐ₁₂-*" eine Einfachbindung ist; wenn a13 gleich 0 ist, *'-(L₁₃)ₐ₁₃-*" eine Einfachbindung ist; wenn a14 gleich 0 ist, *'-(L₁₄)ₐ₁₄-*" eine Einfachbindung ist; wenn a11 zwei oder mehr ist, zwei oder mehr L₁₁(s) identisch oder verschieden sind; wenn a12 zwei oder mehr ist, zwei oder mehr L₁₂(s) identisch oder verschieden sind; wenn a13 zwei oder mehr ist, zwei oder mehr L₁₃(s) identisch oder verschieden sind; und wenn a14 zwei oder mehr ist, zwei oder mehr L₁₄(s) identisch oder verschieden sind,
in Formeln 11-1 und 11-2 Ar₁₁ bis Ar₁₃ jeweils unabhängig voneinander ausgewählt sind aus einer substituierten oder unsubstituierten C₃-C₁₀-Cycloalkylgruppe einer substituierten oder unsubstituierten C₁-C₁₀-Heterocycloalkylgruppe, einer substituierten oder unsubstituierten -C₃-C₁₀-Cycloalkenylgruppe, einer substituierten oder unsubstituierten C₁-C₁₀-Heterocycloalkenylgruppe, einer substituierten oder unsubstituierten -C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe und einer substituierten oder unsubstituierten monovalenten nicht-aromatischen kondensierten heteropolycyclischen Guppe, wobei mindestens eines von Ar₁₁ bis Ar₁₃ mit einer vernetzbaren Gruppe substituiert ist,
in Formeln 11-1 und 11-2 b11 bis b13 jeweils unabhängig voneinander 1,2,3,4, oder 5 sind, wobei, wenn b11 zwei oder mehr ist, zwei oder mehr Ar₁₁(s) identisch oder voneinander verschieden sind; wenn b12 zwei oder mehr ist, zwei oder mehr Ar₁₂(s) identisch oder voneinander verschieden sind; und wenn b13 zwei oder mehr ist, zwei oder mehr Ar₁₃(s) identisch oder voneinander verschieden sind,
in Formeln 11-1 und 11-2 Ar₁ und Ar₂ jeweils unabhängig eine substituierte oder unsubstituierte carbocyclische C₅-C₆₀-Gruppe sind, oder eine substituierte oder unsubstituierte heterocyclische C₁-C₆₀-Gruppe sind, wobei Ar₁ und Ar₂ identisch oder voneinander verschieden sind,
in Formel 1b Ar₁ und Ar₂ jeweils unabhängig eine substituierte oder unsubstituierte carbocyclische C₅-C₆₀-Gruppe sind, oder eine substituierte oder unsubstituierte heterocyclische C₁-C₆₀-Gruppe sind, wobei Ar₁ und Ar₂ identisch oder voneinander verschieden sind,
in Formel 1b, a1 und a2 jeweils unabhängig voneinander eine ganze Zahl von 1 bis 5 sind, wobei, wenn a1 zwei oder mehr ist, zwei oder mehr Ar₁(s) identisch oder voneinander verschieden sind; und wenn a2 zwei oder mehr ist, zwei oder mehr Ar₂(s) identisch oder verschieden sind,
in Formel 1b, Ln bis Lu und L21 bis L24 jeweils unabhängig eine substituierte oder unsubstituierte carbocyclische C₅-C₆₀ Gruppe oder eine substituierte oder unsubstituierte heterocyclische C₁-C₆₀ Gruppe sind,
in Formel 1b b11 bis b14 und b21 bis b24 jeweils unabhängig voneinander eine ganze Zahl von 0 bis 3 sind, wobei, wenn b11 gleich 0 ist, *-(L₁₁)_{b11}-*' eine Einfachbindung ist; wenn b12 gleich 0 ist, *-(L₁₂)_{b12}-*' eine Einfachbindung ist; wenn b13 gleich 0 ist, *-(L₁₃)_{b13}-*' eine Einfachbindung ist; wenn b14 gleich 0 ist, *-(L₁₄)_{b14}-*' eine Einfachbindung ist; wenn b21 gleich 0 ist, *-(L₂₁)_{b21}-*' eine Einfachbindung ist; wenn b22 gleich 0 ist, *-(L₂₂)_{b22}-*' eine Einfachbindung ist; wenn b23 gleich 0 ist, *-(L₂₃)_{b23}-*' eine Einfachbindung ist; wenn b24 gleich 0 ist, *-(L₂₄)_{b24}-*' eine Einfachbindung ist; wenn b11 zwei oder mehr ist, zwei oder mehr L₁₁(s) identisch oder voneinander verschieden sind; wenn b12 zwei oder mehr ist, zwei oder mehr L₁₂(s) identisch oder voneinander verschieden sind; wenn b13 zwei oder mehr ist, zwei oder mehr L₁₃(s) identisch oder voneinander verschieden sind; wenn b14 zwei oder mehr ist, zwei oder mehr L₁₄(s) identisch oder voneinander verschieden sind; wenn b21 zwei oder mehr ist, zwei oder mehr L₂₁(s) identisch oder voneinander verschieden sind; wenn b22 zwei oder mehr ist, zwei oder mehr L₂₂(s) identisch oder voneinander verschieden sind; wenn b23 zwei oder mehr ist, zwei oder mehr L₂₃(s) identisch oder voneinander verschieden sind; und wenn b24 zwei oder mehr ist, zwei oder mehr L₂₄(s) identisch oder voneinander verschieden sind,
in Formel 1b B₁ und B₂ jeweils unabhängig aus einer Einfachbindung, einer substituierten oder unsubstituierten C₁-C₂₀-Alkylengruppe, einer substituierten oder unsubstituierten C₂-C₂₀-Alkenylengruppe und einer substituierten oder unsubstituierten C₂-C₂₀-Alkynylengruppe ausgewählt sind,
in Formel 1b, m und n jeweils unabhängig voneinander eine ganze Zahl von 1 bis 3 sind, wobei, wenn m zwei oder mehr ist, zwei oder mehr B₁(s) identisch oder voneinander verschieden sind; und wenn n zwei oder mehr ist, zwei oder mehr B₂(s) identisch oder voneinander verschieden sind,
in Formel 1b Ar₁₁, Ar₁₃, Ar₂₁und Ar₂₃ jeweils unabhängig voneinander ausgewählt sind aus einer substituierten oder unsubstituierten C₃-C₁₀-Cycloalkylgruppe einer substituierten oder unsubstituierten C₁-C₁₀ -Heterocycloalkylgruppe, einer substituierten oder unsubstituierten C₃-C₁₀ -Cycloalkenylgruppe, einer substituierten oder unsubstituierten C₁-C₁₀ -Heterocycloalkenylgruppe, einer substituierten oder unsubstituierten C₆-C₆₀ -Arylgruppe, einer substituierten oder unsubstituierten C₆-C₆₀ - Aryloxygruppe, einer substituierten oder unsubstituierten C₆-C₆₀ -Arylthiogruppe, einer substituierten oder unsubstituierten C₁-C₆₀-Heteroarylgruppe, einer substituierten oder unsubstituierten monovalenten nicht-aromatischen kondensierten polycyclischen Gruppe und einer substituierten oder unsubstituierten monovalenten nicht-aromatischen kondensierten heteropolycyclischen Guppe,
in Formel 1b Ar₁₂, Ar₁₄, Ar₂₂, und Ar₂₄ jeweils unabhängig ausgewählt sind aus einer substituierten oder unsubstituierten C₃-C₁₀Cycloalkylengruppe, einer substituierten oder unsubstituierten C₁C₁₀-Heterocycloalkylengruppe, einer substituierten oder unsubstituierten C₃-C₁₀-Cycloalkenylengruppe, einer substituierten oder unsubstituierten C₁-C₁₀-Heterocycloalkenylengruppe, einer substituierten oder unsubstituierten -C₆-C₆₀-Arylengruppe, einer substituierten oder unsubstituierten C₁C₆₀-Heteroarylengruppe, einer substituierten oder unsubstituierten zweiwertigen nicht-aromatischen kondensierten polycyclischen Gruppe und einer substituierten oder unsubstituierten zweiwertigen nicht-aromatischen kondensierten heteropolycyclischen Gruppe,
in Formel 1b, c11 bis c14 und c21 bis c24 jeweils unabhängig voneinander eine ganze Zahl von 1 bis 5 sind, wobei, wenn c11 zwei oder mehr ist, zwei oder mehr Ar₁₁(s) identisch oder voneinander verschieden sind; wenn c12 zwei oder mehr ist, zwei oder mehr Ar₁₂(s) identisch oder voneinander verschieden sind; wenn c13 zwei oder mehr ist, zwei oder mehr Ar₁₃(s) identisch oder voneinander verschieden sein können; wenn c14 zwei oder mehr ist, zwei oder mehr Ar₁₄(s) identisch oder voneinander verschieden sind; wenn c21 zwei oder mehr ist, zwei oder mehr Ar₂₁(s) identisch oder voneinander verschieden sind; wenn c22 zwei oder mehr ist, zwei oder mehr Ar₂₂(s) identisch oder voneinander verschieden sind; wenn c23 zwei oder mehr ist, zwei oder mehr Ar₂₃(S) identisch oder voneinander verschieden sind; und wenn c24 zwei oder mehr ist, zwei oder mehr Ar₂₄(s) identisch oder voneinander verschieden sind,
in Formel 1b mindestens eine Gruppe von Ar₁, Ar₂, Ar₁₁ bis Ar₁₄und Ar₂₁ bis Ar₂₄, durch eine vernetzbare Gruppe substituiert ist.

24. Auf Arylamin basierendes Polymer nach Anspruch 23, wobei in Formeln 11-1 und 11-2 Ar₁₁ₐ und Ar₁₃ₐ jeweils unabhängig aus den durch die Formeln 5-1 bis 5-8 dargestellten Gruppen ausgewählt sind: 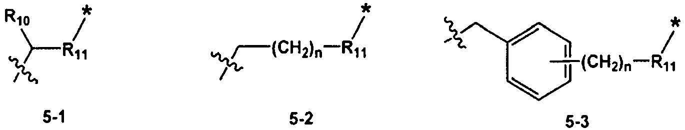 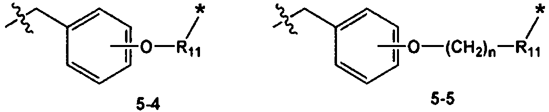 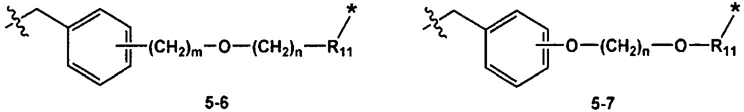 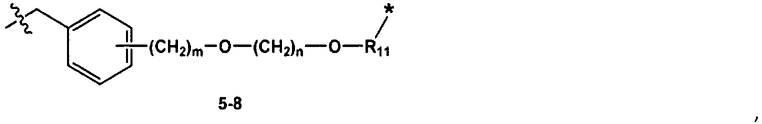 wobei in Formeln 5-1 bis 5-8,
R₁₀ ein Wasserstoffatom oder eine substituierte oder unsubstituierte C₁-C₂₀-Alkylgruppe ist,
R₁₁ ausgewählt ist aus einer Einfachbindung, einer substituierten oder unsubstituierten C₁-C₂₀-Alkylengruppe, einer substituierten oder unsubstituierten C₃-C₁₀-Cycloalkylengruppe, einer substituierten oder unsubstituierten C₁-C₁₀-Heterocycloalkylengruppe, einer substituierten oder unsubstituierten C₃-C₁₀-Cycloalkenylengruppe, einer substituierten oder unsubstituierten C₁-C₁₀-Heterocycloalkenylengruppe, einer substituierten oder unsubstituierten C₆-C₆₀-Arylengruppe, einer substituierten oder unsubstituierten C₁-C₆₀-Heteroarylengruppe, einer substituierten oder unsubstituierten zweiwertigen nicht-aromatischen kondensierten polycyclischen Gruppe und einer substituierten oder unsubstituierten zweiwertigen nicht-aromatischen kondensierten heteropolycyclischen Gruppe,
m und n jeweils unabhängig eine ganze Zahl von 1 bis 10 sind,
* eine Bindestelle an ein benachbartes Atom angibt, und 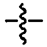
* eine Bindestelle an ein benachbartes Wiederholungseinheit angibt.

25. Auf Arylamin basierendes Polymer nach Anspruch 23 oder Anspruch 24, wobei in Formeln 11-1 und 11-2 Ar₁ eine Benzolgruppe ist und b1 4 oder 5 ist.

## Revendications

1. Dispositif électroluminescent organique (10, 20, 30, 40) comprenant :
une première électrode (110) ;
une seconde électrode (190) faisant face à la première électrode (110) ; et
une couche organique (150) entre la première électrode (110) et la seconde électrode (190) et comprenant une couche d'émission,
**caractérisé en ce que** la couche organique (150) comprend en outre au moins l'un d'un polymère à base d'arylamine formé par réticulation d'un composé à base d'arylamine réticulable représenté par la Formule 1a ou 1b :
**Formule 1a** **A₁-(B)ₚ-A₂,** 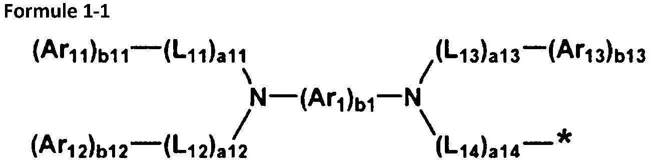 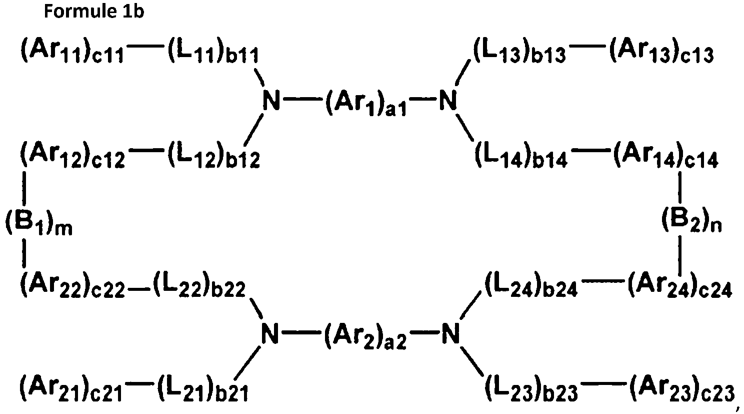
dans lequel, dans la Formule 1a, A₁ et A₂ sont chacun un groupe représenté par la Formule 1-1, et A₁ et A₂ sont identiques ou différents l'un de l'autre,
dans la Formule 1a, B est sélectionné parmi un groupe carbocyclique en C₅-C₆₀ substitué ou non substitué, un groupe hétérocyclique en C₁-C₆₀ substitué ou non substitué, et *'-Si(Q₄₁)(Q₄₂)-*" ; et p est un entier compris entre 1 et 10,
dans la Formule 1-1, * indique un site de liaison à (B)ₚ dans la Formule 1a,
dans la Formule 1-1, Ar₁ est sélectionné parmi un groupe benzène, un groupe pyridine, un groupe pyrimidine, un groupe pyrazine, un groupe triazine, un groupe furane, un groupe thiophène, un groupe imidazole, un groupe thiazole, un groupe isoxazole et un groupe oxazole,
dans la Formule 1-1, b1 est un entier supérieur ou égal à quatre, et quatre ou plus Ar₁ sont identiques ou différents les uns des autres,
dans la Formule 1-1, L₁₁ à L₁₄ sont chacun indépendamment un groupe carbocyclique en C₅-C₆₀ substitué ou non substitué ou un groupe hétérocyclique en C₁-C₆₀ substitué ou non substitué,
dans la Formule 1-1, a11 à a14 représentent chacun indépendamment 0, 1, 2, 3 ou 4,
dans la Formule 1-1, lorsque a11 représente 0, *'-(L₁₁)ₐ₁₁-*" est une liaison simple ; lorsque a12 représente 0, *'-(L₁₂)ₐ₁₂-*" est une liaison simple ; lorsque a13 représente 0, *'-(L₁₃)ₐ₁₃-*" est une liaison simple ; lorsque a14 représente 0, *'-(L₁₄)ₐ₁₄-*" est une liaison simple ; lorsque a11 représente deux ou plus, deux ou plusieurs L₁₁ sont identiques ou différents les uns des autres ; lorsque a12 représente deux ou plus, deux ou plusieurs L₁₂ sont identiques ou différents les uns des autres ; lorsque a13 représente deux ou plus, deux ou plusieurs L₁₃ sont identiques ou différents les uns des autres ; et lorsque a14 représente deux ou plus, deux ou plusieurs L₁₄ sont identiques ou différents les uns des autres,
dans la Formule 1-1, Ar₁₁ à Ar₁₃ sont chacun indépendamment sélectionnés parmi un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe hétéroaryle en C₁-C₆₀ substitué ou non substitué, un groupe polycyclique condensé non aromatique monovalent substitué ou non substitué, et un groupe hétéropolycyclique condensé non aromatique monovalent substitué ou non substitué, dans lequel au moins l'un de Ar₁₁ à Ar₁₃ est substitué par un groupe réticulable,
dans la Formule 1-1, b11 à b13 sont chacun indépendamment 1, 2, 3, 4 ou 5, dans lequel, lorsque b11 représente deux ou plus, deux ou plusieurs Ar₁₁ sont identiques ou différents les uns des autres ; lorsque b12 représente deux ou plus, deux ou plusieurs Ar₁₂ sont identiques ou différents les uns des autres ; et lorsque b13 représente deux ou plus, deux ou plusieurs Ar₁₃ sont identiques ou différents les uns des autres,
dans la Formule 1b, Ar₁ et Ar₂ sont chacun indépendamment un groupe carbocyclique en C₅-C₆₀ substitué ou non substitué ou un groupe hétérocyclique en C₁-C₆₀ substitué ou non substitué, dans lequel Ar₁ et Ar₂ sont identiques ou différents l'un de l'autre,
dans la Formule 1b, a1 et a2 sont chacun indépendamment un entier compris entre 1 et 5, dans lequel, lorsque a1 représente deux ou plus, deux ou plusieurs Ar₁ sont identiques ou différents les uns des autres ; et lorsque a2 représente deux ou plus, deux ou plusieurs Ar₂ sont identiques ou différents les uns des autres,
dans la Formule 1b, L₁₁ à L₁₄ et L₂₁ à L₂₄ sont chacun indépendamment un groupe carbocyclique en C₅-C₆₀ substitué ou non substitué ou un groupe hétérocyclique en C₁-C₆₀ substitué ou non substitué,
dans la Formule 1b, b11 à b14 et b21 à b24 sont chacun indépendamment un entier compris entre 0 et 3, dans lequel, lorsque b11 représente 0, *-(L₁₁)_{b11}-*' est une liaison simple ; lorsque b12 représente 0, *-(L₁₂)_{b12}-*' est une liaison simple ; lorsque b13 représente 0, *-(L₁₃)_{b13}-*' est une liaison simple ; lorsque b14 représente 0, *-(L₁₄)_{b14}-*' est une liaison simple ; lorsque b21 représente 0, *-(L₂₁)_{b21}-*' est une liaison simple ; lorsque b22 représente 0, *-(L₂₂)_{b22}-*' est une liaison simple ; lorsque b23 représente 0, *-(L₂₃)_{b23}-*' est une liaison simple ; lorsque b24 représente 0, *-(L₂₄)_{b24}-*' est une liaison simple ; lorsque b11 représente deux ou plus, deux ou plusieurs L₁₁ sont identiques ou différents les uns des autres ; lorsque b12 représente deux ou plus, deux ou plusieurs L₁₂ sont identiques ou différents les uns des autres ; lorsque b13 représente deux ou plus, deux ou plusieurs L₁₃ sont identiques ou différents les uns des autres ; lorsque b14 représente deux ou plus, deux ou plusieurs L₁₄ sont identiques ou différents les uns des autres ; lorsque b21 représente deux ou plus, deux ou plusieurs L₂₁ sont identiques ou différents les uns des autres ; lorsque b22 représente deux ou plus, deux ou plusieurs L₂₂ sont identiques ou différents les uns des autres ; lorsque b23 représente deux ou plus, deux ou plusieurs L₂₃ sont identiques ou différents les uns des autres ; et lorsque b24 représente deux ou plus, deux ou plusieurs L₂₄ sont identiques ou différents les uns des autres,
dans la Formule 1b, B₁ et B₂ sont chacun indépendamment sélectionnés parmi une liaison simple, un groupe alkylène en C₁-C₂₀ substitué ou non substitué, un groupe alcénylène en C₂-C₂₀ substitué ou non substitué et un groupe alcynylène en C₂-C₂₀ substitué ou non substitué,
dans la Formule 1b, m et n sont chacun indépendamment un entier compris entre 1 et 3, dans lequel, lorsque m représente deux ou plus, deux ou plusieurs B₁ sont identiques ou différents les uns des autres ; et lorsque n représente deux ou plus, deux ou plusieurs B₂ sont identiques ou différents les uns des autres,
dans la Formule 1b, Ar₁₁, Ar₁₃, Ar₂₁ et Ar₂₃ sont chacun indépendamment sélectionnés parmi un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe hétéroaryle en C₁-C₆₀ substitué ou non substitué, un groupe polycyclique condensé non aromatique monovalent substitué ou non substitué, et un groupe hétéropolycyclique condensé non aromatique monovalent substitué ou non substitué,
dans la Formule 1b, Ar₁₂, Ar₁₄, Ar₂₂ et Ar₂₄ sont chacun indépendamment sélectionnés parmi une liaison simple, un groupe cycloalkylène en C₃-C₁₀ substitué ou non substitué, un groupe hétérocycloalkylène en C₁-C₁₀ substitué ou non substitué, un groupe cycloalcénylène en C₃-C₁₀ substitué ou non substitué, un groupe hétérocycloalcénylène en C₁-C₁₀ substitué ou non substitué, un groupe arylène en C₆-C₆₀ substitué ou non substitué, un groupe hétéroarylène en C₁-C₆₀ substitué ou non substitué, un groupe polycyclique condensé divalent non aromatique substitué ou non substitué, et un groupe hétéropolycyclique condensé divalent non aromatique substitué ou non substitué,
dans la Formule 1b, c11 à c14 et c21 à c24 sont chacun indépendamment un entier compris entre 1 et 5, dans lequel, lorsque c11 représente deux ou plus, deux ou plusieurs Ar₁₁ sont identiques ou différents les uns des autres ; lorsque c12 représente deux ou plus, deux ou plusieurs Ar₁₂ sont identiques ou différents les uns des autres ; lorsque c13 représente deux ou plus, deux ou plusieurs Ar₁₃ peuvent être identiques ou différents les uns des autres ; lorsque c14 représente deux ou plus, deux ou plusieurs Ar₁₄ sont identiques ou différents les uns des autres ; lorsque c21 représente deux ou plus, deux ou plusieurs Ar₂₁ sont identiques ou différents les uns des autres ; lorsque c22 représente deux ou plus, deux ou plusieurs Ar₂₂ sont identiques ou différents les uns des autres ; lorsque c23 représente deux ou plus, deux ou plusieurs Ar₂₃ sont identiques ou différents les uns des autres ; et lorsque c24 représente deux ou plus, deux ou plusieurs Ar₂₄ sont identiques ou différents les uns des autres,
dans la Formule 1b, au moins un groupe sélectionné parmi Ar₁, Ar₂, Ar₁₁ à Ar₁₄et Ar₂₁ à Ar₂₄ est substitué par un groupe réticulable,
* et *' indiquent chacun un site de liaison à un atome voisin,
au moins un substituant du groupe carbocyclique en C₅-C₆₀ substitué, du groupe hétérocyclique en C₁-C₆₀ substitué, du groupe cycloalkylène en C₃-C₁₀ substitué, du groupe hétérocycloalkylène en C₁-C₁₀ substitué, du groupe cycloalcénylène en C₃-C₁₀ substitué, du groupe hétérocycloalcénylène en C₁-C₁₀ substitué, du groupe arylène en C₆-C₆₀ substitué, du groupe hétéroarylène en C₁-C₆₀ substitué, du groupe polycyclique condensé non aromatique divalent substitué, du groupe hétéropolycyclique condensé non aromatique divalent substitué, du groupe cycloalkyle en C₃-C₁₀ substitué, du groupe hétérocycloalkyle en C₁-C₁₀ substitué, du groupe cycloalcényle en C₃-C₁₀ substitué, du groupe hétérocycloalcényle en C₁-C₁₀ substitué, du groupe aryle en C₆-C₆₀ substitué, du groupe aryloxy en C₆-C₆₀ substitué, du groupe arylthio en C₆-C₆₀ substitué, du groupe hétéroaryle en C₁-C₆₀ substitué, du groupe polycyclique condensé non aromatique monovalent substitué, du groupe hétéropolycyclique condensé non aromatique monovalent substitué, du groupe alkylène en C₁-C₂₀ substitué, du groupe alcénylène en C₂-C₂₀ substitué et du groupe alcynylène en C₂-C₂₀ substitué est sélectionné parmi :
le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcényle en C₂-C₆₀, un groupe alcynyle en C₂-C₆₀ et un groupe alcoxy en C₁-C₆₀;
un groupe alkyle en C₁-C₆₀, un groupe alcényle en C₂-C₆₀, un groupe alcynyle en C₂-C₆₀ et un groupe alcoxy en C₁-C₆₀, chacun substitué par au moins un élément sélectionné parmi le deutérium, -F, -Cl, -Br, -I, un groupe hydroxyle, un groupe cyano, un groupe nitro, un groupe amidino, un groupe hydrazino, un groupe hydrazono, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, un groupe hétéropolycyclique condensé non aromatique monovalent, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), et -P(=O)(Q₁₁)(Q₁₂) ;
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, un groupe hétéropolycyclique condensé non aromatique monovalent, un groupe biphényle et un groupe terphényle ;
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, et un groupe hétéropolycyclique condensé non aromatique monovalent, chacun substitué par au moins un élément sélectionné parmi le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcényle en C₂-C₆₀, un groupe alcynyle en C₂-C₆₀, un groupe alcoxy en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, un groupe hétéropolycyclique condensé non aromatique monovalent, un groupe hétéropolycyclique condensé non aromatique monovalent, -Si(Q₂₁)(Q₂₂)(Q₂₃), -N(Q₂₁)(Q₂₂), -B(Q₂₁)(Q₂₂), -C(=O)(Q₂₁), -S(=O)2(Q₂₁), et - P(=O)(Q₂₁)(Q₂₂) ; et
-Si(Q₃₁)(Q₃₂)(Q₃₃), -N(Q₃₁)(Q₃₂), -B(Q₃₁)(Q₃₂), -C(=O)(Q₃₁), -S(=O)2(Q₃₁), et - P(=O)(Q₃₁)(Q₃₂), et
Q₁₁ à Q₁₃, Q₂₁ à Q₂₃, Q₃₁ à Q₃₃, et Q₄₁ à Q₄₂ sont chacun indépendamment sélectionnés parmi l'hydrogène, le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcényle en C₂-C₆₀, un groupe alcynyle en C₂-C₆₀, un groupe alcoxy en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe hétéroaryle en C₁-C₆₀, un groupe polycyclique condensé non aromatique monovalent, un groupe hétéropolycyclique condensé non aromatique monovalent, un groupe biphényle et un groupe terphényle.

2. Dispositif électroluminescent organique (10, 20, 30, 40) selon la revendication 1, dans lequel :
la première électrode (110) est une anode,
la seconde électrode (190) est une cathode, et
la couche organique (150) comprend en outre i) une région de transport de trous entre la première électrode et la couche d'émission et comprenant une couche d'injection de trous, une couche de transport de trous, une couche tampon, une couche de blocage d'électrons ou toute combinaison de celles-ci ; et ii) une région de transport d'électrons entre la couche d'émission et la seconde électrode et comprenant une couche de blocage de trous, une couche de transport d'électrons, une couche d'injection d'électrons ou toute combinaison de celles-ci.

3. Dispositif électroluminescent organique (10, 20, 30, 40) selon la revendication 2, dans lequel la région de transport de trous comprend au moins l'un du polymère à base d'arylamine.

4. Dispositif électroluminescent organique (10, 20, 30, 40) selon la revendication 2, dans lequel :
la région de transport de trous comprend une couche d'injection de trous et une couche de transport de trous, et
la couche de transport de trous comprend au moins l'un du polymère à base d'arylamine.

5. Appareil électronique comprenant :
un transistor à couches minces ; et
le dispositif électroluminescent organique (10, 20, 30, 40) selon l'une quelconque des revendications 1 à 4,
dans lequel le transistor à couches minces comprend une électrode source, une électrode drain, une couche active et une électrode de grille, et
la première électrode du dispositif électroluminescent organique est électriquement raccordée à l'une parmi l'électrode source et l'électrode drain du transistor à couches minces.

6. Composé à base d'arylamine réticulable représenté par la Formule 1a ou 1b :
**Formule 1a** A₁-(B)ₚ-A₂ 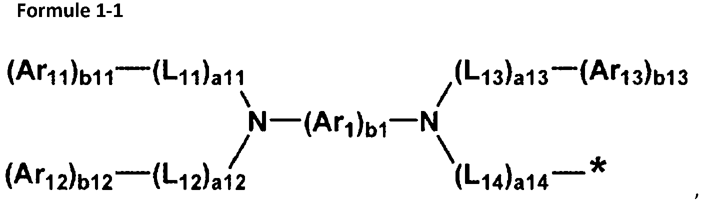 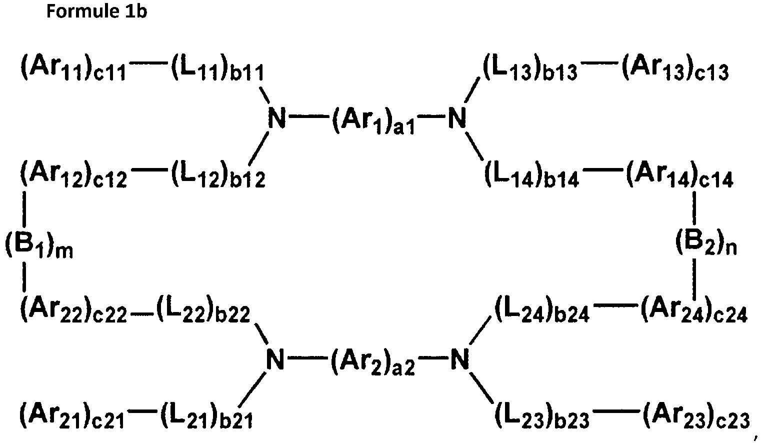
dans lequel, dans la Formule 1a, A₁ et A₂ sont chacun un groupe représenté par la Formule 1-1, et A₁ et A₂ sont identiques ou différents l'un de l'autre,
dans la Formule 1a, B est sélectionné parmi un groupe carbocyclique en C₅-C₆₀ substitué ou non substitué, un groupe hétérocyclique en C₁-C₆₀ substitué ou non substitué, et *'-Si(Q₄₁)(Q₄₂)-*" ; et p est un entier compris entre 1 et 10,
dans la Formule 1-1, * indique un site de liaison à (B)ₚ dans la Formule 1a,
dans la Formule 1-1, Ar₁ est sélectionné parmi un groupe benzène, un groupe pyridine, un groupe pyrimidine, un groupe pyrazine, un groupe triazine, un groupe furane, un groupe thiophène, un groupe imidazole, un groupe thiazole, un groupe isoxazole et un groupe oxazole ; dans la Formule 1-1, b1 est un entier supérieur ou égal à quatre, et quatre ou plusieurs Ar₁ sont identiques ou différents les uns des autres,
dans la Formule 1-1, L₁₁ à L₁₄ sont chacun indépendamment un groupe carbocyclique en C₅-C₆₀ substitué ou non substitué ou un groupe hétérocyclique en C₁-C₆₀ substitué ou non substitué,
dans la Formule 1-1, a11 à a14 représentent chacun indépendamment 0, 1, 2, 3 ou 4,
dans la Formule 1-1, lorsque a11 représente 0, *'-(L₁₁)ₐ₁₁-*" est une liaison simple ; lorsque a12 représente 0, *'-(L₁₂)ₐ₁₂-*" est une liaison simple ; lorsque a13 représente 0, *'-(L₁₃)ₐ₁₃-*" est une liaison simple ; lorsque a14 représente 0, *'-(L₁₄)ₐ₁₄-*" est une liaison simple ; lorsque a11 représente deux ou plus, deux ou plusieurs L₁₁ sont identiques ou différents les uns des autres ; lorsque a12 représente deux ou plus, deux ou plusieurs L₁₂ sont identiques ou différents les uns des autres ; lorsque a13 représente deux ou plus, deux ou plusieurs L₁₃ sont identiques ou différents les uns des autres ; et lorsque a14 représente deux ou plus, deux ou plusieurs L₁₄ sont identiques ou différents les uns des autres,
dans la Formule 1-1, Ar₁₁ à Ar₁₃ sont chacun indépendamment sélectionnés parmi un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe hétéroaryle en C₁-C₆₀ substitué ou non substitué, un groupe polycyclique condensé non aromatique monovalent substitué ou non substitué, et un groupe hétéropolycyclique condensé non aromatique monovalent substitué ou non substitué, dans lequel au moins l'un de Ar₁₁ à Ar₁₃ est substitué par un groupe réticulable,
dans la Formule 1-1, b11 à b13 sont chacun indépendamment 1, 2, 3, 4 ou 5, dans lequel, lorsque b11 représente deux ou plus, deux ou plusieurs Ar₁₁ sont identiques ou différents les uns des autres ; lorsque b12 représente deux ou plus, deux ou plusieurs Ar₁₂ sont identiques ou différents les uns des autres ; et lorsque b13 représente deux ou plus, deux ou plusieurs Ar₁₃ sont identiques ou différents les uns des autres,
dans la Formule 1b, Ar₁ et Ar₂ sont chacun indépendamment un groupe carbocyclique en C₅-C₆₀ substitué ou non substitué ou un groupe hétérocyclique en C₁-C₆₀ substitué ou non substitué, dans lequel Ar₁ et Ar₂ sont identiques ou différents l'un de l'autre,
dans la Formule 1b, a1 et a2 sont chacun indépendamment un entier compris entre 1 et 5, dans lequel, lorsque a1 représente deux ou plus, deux ou plusieurs Ar₁ sont identiques ou différents les uns des autres ; et lorsque a2 représente deux ou plus, deux ou plusieurs Ar₂ sont identiques ou différents les uns des autres,
dans la Formule 1b, L₁₁ à L₁₄ et L₂₁ à L₂₄ sont chacun indépendamment un groupe carbocyclique en C₅-C₆₀ substitué ou non substitué ou un groupe hétérocyclique en C₁-C₆₀ substitué ou non substitué,
dans la Formule 1b, b11 à b14 et b21 à b24 sont chacun indépendamment un entier compris entre 0 et 3, dans lequel, lorsque b11 représente 0, *-(L₁₁)_{b11}-*' est une liaison simple ; lorsque b12 représente 0, *-(L₁₂)_{b12}-*' est une liaison simple ; lorsque b13 représente 0, *-(L₁₃)_{b13}-*' est une liaison simple ; lorsque b14 représente 0, *-(L₁₄)_{b14}-*' est une liaison simple ; lorsque b21 représente 0, *-(L₂₁)_{b21}-*' est une liaison simple ; lorsque b22 représente 0, *-(L₂₂)_{b22}-*' est une liaison simple ; lorsque b23 représente 0, *-(L₂₃)_{b23}-*' est une liaison simple ; lorsque b24 représente 0, *-(L₂₄)_{b24}-*' est une liaison simple ; lorsque b11 représente deux ou plus, deux ou plusieurs L₁₁ sont identiques ou différents les uns des autres ; lorsque b12 représente deux ou plus, deux ou plusieurs L₁₂ sont identiques ou différents les uns des autres ; lorsque b13 représente deux ou plus, deux ou plusieurs L₁₃ sont identiques ou différents les uns des autres ; lorsque b14 représente deux ou plus, deux ou plusieurs L₁₄ sont identiques ou différents les uns des autres ; lorsque b21 représente deux ou plus, deux ou plusieurs L₂₁ sont identiques ou différents les uns des autres ; lorsque b22 représente deux ou plus, deux ou plusieurs L₂₂ sont identiques ou différents les uns des autres ; lorsque b23 représente deux ou plus, deux ou plusieurs L₂₃ sont identiques ou différents les uns des autres ; et lorsque b24 représente deux ou plus, deux ou plusieurs L₂₄ sont identiques ou différents les uns des autres,
dans la Formule 1b, B₁ et B₂ sont chacun indépendamment sélectionnés parmi une liaison simple, un groupe alkylène en C₁-C₂₀ substitué ou non substitué, un groupe alcénylène en C₂-C₂₀ substitué ou non substitué et un groupe alcynylène en C₂-C₂₀ substitué ou non substitué,
dans la Formule 1b, m et n sont chacun indépendamment un entier compris entre 1 et 3, dans lequel, lorsque m représente deux ou plus, deux ou plusieurs B₁ sont identiques ou différents les uns des autres ; et lorsque n représente deux ou plus, deux ou plusieurs B₂ sont identiques ou différents les uns des autres,
dans la Formule 1b, Ar₁₁, Ar₁₃, Ar₂₁ et Ar₂₃ sont chacun indépendamment sélectionnés parmi un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe hétéroaryle en C₁-C₆₀ substitué ou non substitué, un groupe polycyclique condensé non aromatique monovalent substitué ou non substitué, et un groupe hétéropolycyclique condensé non aromatique monovalent substitué ou non substitué,
dans la Formule 1b, Ar₁₂, Ar₁₄, Ar₂₂ et Ar₂₄ sont chacun indépendamment sélectionnés parmi une liaison simple, un groupe cycloalkylène en C₃-C₁₀ substitué ou non substitué, un groupe hétérocycloalkylène en C₁-C₁₀ substitué ou non substitué, un groupe cycloalcénylène en C₃-C₁₀ substitué ou non substitué, un groupe hétérocycloalcénylène en C₁-C₁₀ substitué ou non substitué, un groupe arylène en C₆-C₆₀ substitué ou non substitué, un groupe hétéroarylène en C₁-C₆₀ substitué ou non substitué, un groupe polycyclique condensé divalent non aromatique substitué ou non substitué, et un groupe hétéropolycyclique condensé divalent non aromatique substitué ou non substitué,
dans la Formule 1b, c11 à c14 et c21 à c24 sont chacun indépendamment un entier compris entre 1 et 5, dans lequel, lorsque c11 représente deux ou plus, deux ou plusieurs Ar₁₁ sont identiques ou différents les uns des autres ; lorsque c12 représente deux ou plus, deux ou plusieurs Ar₁₂ sont identiques ou différents les uns des autres ; lorsque c13 représente deux ou plus, deux ou plusieurs Ar₁₃ peuvent être identiques ou différents les uns des autres ; lorsque c14 représente deux ou plus, deux ou plusieurs Ar₁₄ sont identiques ou différents les uns des autres ; lorsque c21 représente deux ou plus, deux ou plusieurs Ar₂₁ sont identiques ou différents les uns des autres ; lorsque c22 représente deux ou plus, deux ou plusieurs Ar₂₂ sont identiques ou différents les uns des autres ; lorsque c23 représente deux ou plus, deux ou plusieurs Ar₂₃ sont identiques ou différents les uns des autres ; et lorsque c24 représente deux ou plus, deux ou plusieurs Ar₂₄ sont identiques ou différents les uns des autres,
dans la Formule 1b, au moins un groupe parmi Ar₁, Ar₂, Ar₁₁ à Ar₁₄et Ar₂₁ à Ar₂₄ est substitué par un groupe réticulable,
* et *' indiquent chacun un site de liaison à un atome voisin,
au moins un substituant du groupe carbocyclique en C₅-C₆₀ substitué, du groupe hétérocyclique en C₁-C₆₀ substitué, du groupe cycloalkylène en C₃-C₁₀ substitué, du groupe hétérocycloalkylène en C₁-C₁₀ substitué, du groupe cycloalcénylène en C₃-C₁₀ substitué, du groupe hétérocycloalcénylène en C₁-C₁₀ substitué, du groupe arylène en C₆-C₆₀ substitué, du groupe hétéroarylène en C₁-C₆₀ substitué, du groupe polycyclique condensé non aromatique divalent substitué, du groupe hétéropolycyclique condensé non aromatique divalent substitué, du groupe cycloalkyle en C₃-C₁₀ substitué, du groupe hétérocycloalkyle en C₁-C₁₀ substitué, du groupe cycloalcényle en C₃-C₁₀ substitué, du groupe hétérocycloalcényle en C₁-C₁₀ substitué, du groupe aryle en C₆-C₆₀ substitué, du groupe aryloxy en C₆-C₆₀ substitué, du groupe arylthio en C₆-C₆₀ substitué, du groupe hétéroaryle en C₁-C₆₀ substitué, du groupe polycyclique condensé non aromatique monovalent substitué, du groupe hétéropolycyclique condensé non aromatique monovalent substitué, du groupe alkylène en C₁-C₂₀ substitué, du groupe alcénylène en C₂-C₂₀ substitué et du groupe alcynylène en C₂-C₂₀ substitué est sélectionné parmi :
le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcényle en C₂-C₆₀, un groupe alcynyle en C₂-C₆₀ et un groupe alcoxy en C₁-C₆₀;
un groupe alkyle en C₁-C₆₀, un groupe alcényle en C₂-C₆₀, un groupe alcynyle en C₂-C₆₀ et un groupe alcoxy en C₁-C₆₀, chacun substitué par au moins un élément sélectionné parmi le deutérium, -F, -Cl, -Br, -I, un groupe hydroxyle, un groupe cyano, un groupe nitro, un groupe amidino, un groupe hydrazino, un groupe hydrazono, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, un groupe hétéropolycyclique condensé non aromatique monovalent, -Si(Q₁₁)(Q₁₂)(Q₁₃), - N(Q₁₁)(Q₁₂), -B(Q₁₁)(Q₁₂), - C(=O)(Q₁₁), -S(=O)₂(Q₁₁), et -P(=O)(Q₁₁)(Q₁₂) ;
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, un groupe hétéropolycyclique condensé non aromatique monovalent, un groupe biphényle et un groupe terphényle ;
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, et un groupe hétéropolycyclique condensé non aromatique monovalent, chacun substitué par au moins un élément sélectionné parmi le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcényle en C₂-C₆₀, un groupe alcynyle en C₂-C₆₀, un groupe alcoxy en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, un groupe hétéropolycyclique condensé non aromatique monovalent, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), et -P(=O)(Q₂₁)(Q₂₂) ; et
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), et - P(=O)(Q₃₁)(Q₃₂), et
Q₁₁ à Q₁₃, Q₂₁ à Q₂₃, Q₃₁ à Q₃₃, et Q₄₁ à Q₄₂ sont chacun indépendamment sélectionnés parmi l'hydrogène, le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcényle en C₂-C₆₀, un groupe alcynyle en C₂-C₆₀, un groupe alcoxy en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe hétéroaryle en C₁-C₆₀, un groupe polycyclique condensé non aromatique monovalent, un groupe hétéropolycyclique condensé non aromatique monovalent, un groupe biphényle et un groupe terphényle.

7. Composé à base d'arylamine réticulable selon la revendication 6, dans lequel dans la Formule 1a, A₁ et A₂ sont identiques l'un à l'autre.

8. Composé à base d'arylamine réticulable selon la revendication 6 ou la revendication 7, dans lequel le composé à base d'arylamine représenté par la Formule 1a est symétrique par rapport à *'-(B)ₚ-*".

9. Composé à base d'arylamine réticulable selon l'une quelconque des revendications 6 à 8, dans lequel :
dans la Formule 1b, Ar₁ et Ar₂ sont chacun indépendamment sélectionnés parmi :
un groupe arylène en C₆-C₆₀ et un groupe hétéroarylène en C₁-C₆₀; et
un groupe arylène en C₆-C₆₀ et un groupe hétéroarylène en C₁-C₆₀, chacun substitué par au moins l'un sélectionné parmi le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcoxy en C₁-C₆₀, -N(Q₃₁)(Q₃₂), et un groupe réticulable,
dans lequel Q₃₁ et Q₃₂ sont identiques à ceux définis dans l'une quelconque des revendications 6 à 8.

10. Composé à base d'arylamine réticulable selon l'une quelconque des revendications 6 à 9, dans lequel :
dans la Formule 1-1, Ar₁ est un groupe benzène, et b1 représente 4 ou 5, et
dans la Formule 1b, Ar₁ et Ar₂ sont chacun indépendamment sélectionnés parmi :
un groupe phénylène et un groupe naphtylène ; et
un groupe phénylène et un groupe naphtylène, chacun substitué par au moins l'un sélectionné parmi le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcoxy en C₁-C₆₀, -N(Q₃₁)(Q₃₂), et un groupe réticulable,
dans lequel Q₃₁ et Q₃₂ sont identiques à ceux définis dans l'une quelconque des revendications 6 à 8.

11. Composé à base d'arylamine réticulable selon l'une quelconque des revendications 6 à 10, dans lequel :
dans la Formule 1-1, les quatre ou plusieurs Ar₁ sont identiques les uns aux autres, et
dans la Formule 1b, lorsque a1 représente deux ou plus, deux ou plusieurs Ar₁ sont identiques les uns aux autres, et lorsque a2 représente deux ou plus, deux ou plusieurs Ar₂ sont identiques les uns aux autres.

12. Composé à base d'arylamine réticulable selon l'une quelconque des revendications 6 à 11, dans lequel, dans la Formule 1b, *-(Ar₁)ₐ₁-*' et *-(Ar₂)ₐ₂-*' sont identiques l'un à l'autre.

13. Composé à base d'arylamine réticulable selon l'une quelconque des revendications 6 à 12, dans lequel :
L₁₁ à L₁₄ dans la Formule 1-1 et L₁₁ à L₁₄ et L₂₁ à L₂₄ dans la Formule 1b sont chacun indépendamment sélectionnés parmi les groupes représentés par les Formules 2-1 à 2-37 : 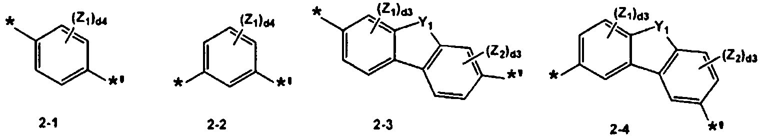 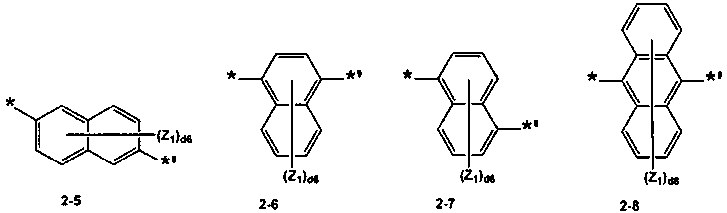 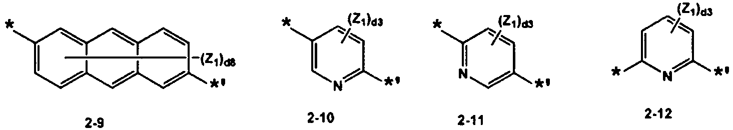 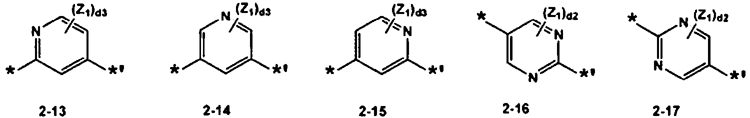 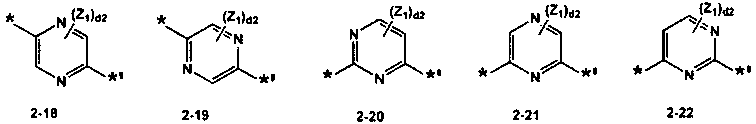 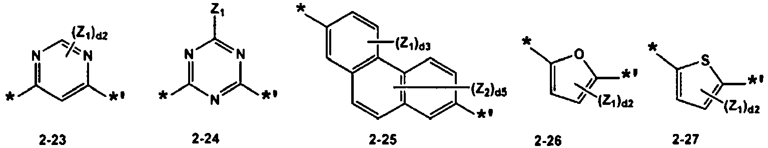 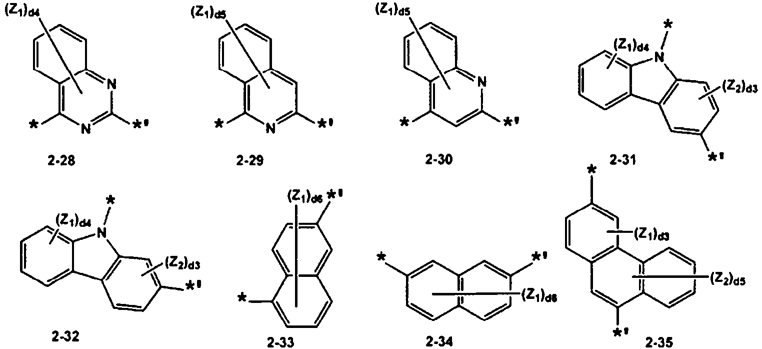 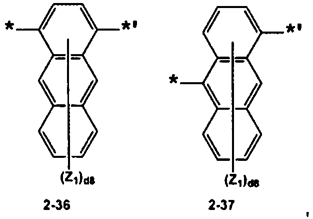 dans lequel, dans les Formules 2-1 et 3-37,
Y1 est O, S, C(Z₃)(Z₄), N(Z₅), ou Si(Z₆)(Z₇),
Z₁ à Z₇ sont chacun indépendamment sélectionnés parmi :
l'hydrogène, le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₂₀, un groupe alcoxy en C₁-C₂₀, un groupe cyclopentyle, un groupe cyclohexyle, un groupe cycloheptyle, un groupe cyclopentényle, un groupe cyclohexényle, un groupe phényle, un groupe biphényle, un groupe terphényle, un groupe pentalényle, un groupe indényle, un groupe naphtyle, un groupe azulényle, un groupe heptalényle, un groupe indacényle, un groupe acénaphtyle, un groupe fluorényle, un groupe spirofluorényle, un groupe benzofluorényle, un groupe dibenzofluorényle, un groupe phénalényle, un groupe phénanthrényle, un groupe anthracényle, un groupe fluoranthényle, un groupe triphénylényle, un groupe pyrényle, un groupe chrysényle, un groupe naphtacényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe indolyle, un groupe isoindolyle, un groupe indazolyle, un groupe purinyle, un groupe quinolinyle, un groupe isoquinolinyle, un groupe benzoquinolinyle, un groupe phtalazinyle, un groupe naphtyridinyle, un groupe quinoxalinyle, un groupe quinazolinyle, un groupe cinnolinyle, un groupe carbazolyle, un groupe phénanthridinyle, un groupe acridinyle, un groupe phénanthrolinyle, un groupe phénazinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe thiadiazolyle et un groupe imidazopyridinyle,
d2 est un entier compris entre 0 et 2,
d3 est un entier compris entre 0 et 3,
d4 est un entier compris entre 0 et 4,
d5 est un entier compris entre 0 et 5,
d6 est un entier compris entre 0 et 6,
d8 est un entier compris entre 0 et 8, et
* et *' indiquent chacun un site de liaison à un atome voisin.

14. Composé à base d'arylamine réticulable selon l'une quelconque des revendications 6 à 13, dans lequel :
dans la Formule 1a, *-(B)ₚ-*' est sélectionné parmi les groupes représentés par les Formules 3-1 à 3-19 : 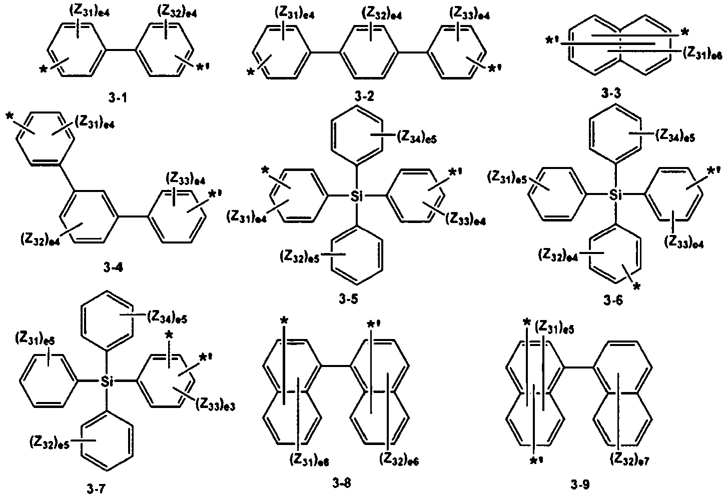 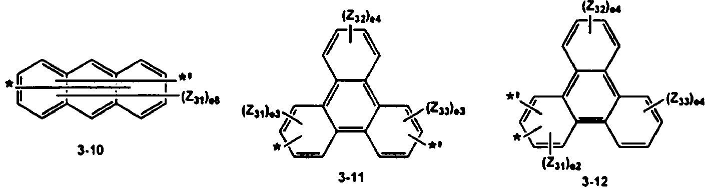 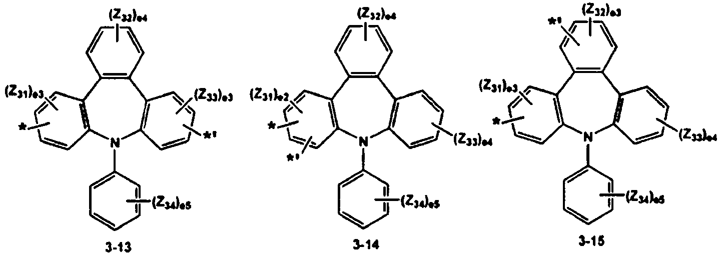 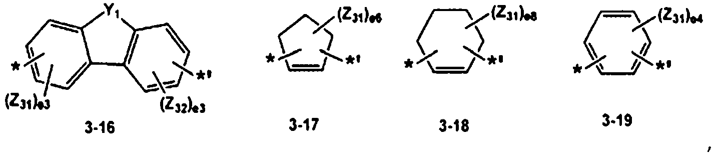 dans lequel, dans les Formules 3-1 à 3-19,
Y₁ est O, S, C(Z₃₅)(Z₃₆), N(Z₃₅), ou Si(Z₃₅)(Z₃₆),
Z₃₁ à Z₃₆ sont chacun indépendamment sélectionnés parmi l'hydrogène, le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₂₀, un groupe alcoxy en C₁-C₂₀, un groupe cyclopropényle, un groupe cyclobutényle, un groupe cyclopentyle, un groupe cyclohexyle, un groupe cycloheptyle, un groupe cyclopentényle, un groupe cyclohexényle, un groupe phényle, un groupe biphényle, un groupe terphényle, un groupe pentalényle, un groupe indényle, un groupe naphtyle, un groupe azulényle, un groupe indacényle, un groupe acénaphtyle, un groupe fluorényle, un groupe spiro-bifluorényle, un groupe spiro-benzofluorène-fluorényle, un groupe benzofluorényle, un groupe dibenzofluorényle, un groupe phénalényle, un groupe phénanthrényle, un groupe anthracényle, un groupe fluoranthényle, un groupe triphénylényle, un groupe pyrényle, un groupe chrysényle, un groupe pérylényle, un groupe pentacényle, un groupe pyrrolyle, un groupe thiophényle, un groupe furanyle, un groupe silolyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe indolyle, un groupe isoindolyle, un groupe indazolyle, un groupe purinyle, un groupe quinolinyle, un groupe isoquinolinyle, un groupe benzoquinolinyle, un groupe isoquinolinyle, un groupe phtalazinyle, un groupe naphtyridinyle, un groupe quinoxalinyle, un groupe benzoquinoxalinyle, un groupe quinazolinyle, un groupe benzoquinazolinyle, un groupe cinnolinyle, un groupe phénanthridinyle, un groupe acridinyle, un groupe phénanthrolinyle, un groupe phénazinyle, un groupe benzimidazolyle, un groupe benzofuranyle, un groupe benzothiophényle, un groupe benzosilolyle, un groupe benzothiazolyle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe carbazolyle, un groupe dibenzofuranyle, un groupe dibenzothiophényle, un groupe dibenzosilolyle, un groupe benzocarbazolyle, un groupe naphtobenzofuranyle, un groupe naphtobenzothiophényle, un groupe naphtobenzosilolyle, un groupe dibenzocarbazolyle, un groupe dinaphtofuranyle, un groupe dinaphtothiophényle, un groupe dinaphtosilolyle, un groupe thiadiazolyle, un groupe imidazopyridinyle, un groupe imidazopyrimidinyle, un groupe oxazolopyridinyle, un groupe thiazolopyridinyle, un groupe benzonaphthyridinyle, un groupe azafluorényle, un groupe azaspiro-bifluorényle, un groupe azacarbazolyle, un groupe azadibenzofuranyle, un groupe azadibenzothiophényle, un groupe azadibenzosilolyle, un groupe indénopyrrolyle, un groupe indolopyrrolyle, un groupe indénocarbazolyle, un groupe indolocarbazolyle, -Si(Q₃₁)(Q₃₂)(Q₃₃), et -N(Q₃₁)(Q₃₂),
Q₃₁ à Q₃₃ sont chacun indépendamment sélectionnés parmi :
un groupe alkyle en C₁-C₁₀, un groupe alcoxy en C₁-C₁₀, un groupe phényle, un groupe biphényle, un groupe terphényle et un groupe naphtyle ; et
un groupe phényle, un groupe biphényle, un groupe terphényle et un groupe naphtyle, chacun substitué par au moins l'un sélectionné parmi un groupe alkyle en C₁-C₁₀, un groupe alcoxy en C₁-C₁₀ et un groupe phényle,
e2 est un entier compris entre 0 et 2,
e3 est un entier compris entre 0 et 3,
e4 est un entier compris entre 0 et 4,
e5 est un entier compris entre 0 et 5,
e6 est un entier compris entre 0 et 6,
e7 est un entier compris entre 0 et 7,
e8 est un entier compris entre 0 et 8, et
* et *' indiquent chacun un site de liaison à un atome voisin.

15. Composé à base d'arylamine réticulable selon l'une quelconque des revendications 6 à 14, dans lequel :
dans la Formule 1-1, Ar₁₁ à Ar₁₃ sont chacun indépendamment sélectionnés parmi :
un groupe aryle en C₆-C₆₀ ; et
un groupe aryle en C₆-C₆₀ substitué par au moins l'un sélectionné parmi le deutérium, -F, -Cl, -Br, -I, un groupe hydroxyle, un groupe cyano, un groupe nitro, un groupe amidino, un groupe hydrazino, un groupe hydrazono, un groupe alcényle en C₂-C₆₀, un groupe alcynyle en C₂-C₆₀ et un groupe alcoxy en C₁-C₆₀, et
dans la Formule 1b, Ar₁₁, Ar₁₃, Ar₂₁ et Ar₂₃ sont chacun indépendamment sélectionnés parmi :
un groupe phényle, un groupe biphényle, un groupe terphényle et un groupe naphtyle ; et
un groupe phényle, un groupe biphényle, un groupe terphényle et un groupe naphtyle, chacun substitué par au moins l'un sélectionné parmi le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₂₀, un groupe alcoxy en C₁-C₂₀, un groupe phényle, un groupe biphényle, un groupe terphényle, un groupe naphtyle, un groupe réticulable et -N(Q₃₁)(Q₃₂),
dans lequel Q₃₁ et Q₃₂ sont identiques à ceux définis dans l'une quelconque des revendications 6 à 14.

16. Composé à base d'arylamine réticulable selon l'une quelconque des revendications 6 à 15, dans lequel, dans la Formule 1b, Ar₁₂, Ar₁₄, Ar₂₂ et Ar₂₄ sont chacun indépendamment sélectionnés parmi :
une liaison unique ;
un groupe phénylène et un groupe naphtylène ; et
un groupe phénylène et un groupe naphtylène, chacun substitué par au moins l'un sélectionné parmi le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcoxy en C₁-C₆₀, -N(Q₃₁)(Q₃₂), et un groupe réticulable,
dans lequel Q₃₁ et Q₃₂ sont identiques à ceux définis dans l'une quelconque des revendications 6 à 14.

17. Composé à base d'arylamine réticulable selon l'une quelconque des revendications 6 à 16, dans lequel dans la Formule 1b, Ar₁₁ et Ar₁₃ sont identiques l'un à l'autre, Ar₂₁ et Ar₂₃ sont identiques l'un à l'autre, Ar₁₂ et Ar₁₄ sont identiques l'un à l'autre, et Ar₂₂ et Ar₂₄ sont identiques l'un à l'autre.

18. Composé à base d'arylamine réticulable selon l'une quelconque des revendications 6 à 17, dans lequel dans la Formule 1b, les substituants de Ar₁₁, Ar₁₃, Ar₂₁ et Ar₂₃ comprennent un groupe réticulable.

19. Composé à base d'arylamine réticulable selon l'une quelconque des revendications 6 à 18, dans lequel, dans la Formule 1b,
les substituants d'au moins deux groupes sélectionnés parmi Ar₁, Ar₂, Ar₁₂, Ar₁₄, Ar₂₂, et Ar₂₄ comprennent un groupe réticulable, et B₁ et B₂ sont chacun une liaison simple.

20. Composé à base d'arylamine réticulable selon l'une quelconque des revendications 6 à 19, dans lequel le groupe réticulable comprend au moins l'un sélectionné parmi un fragment vinylène, un fragment styrène, un fragment cyclobutane et un fragment époxy.

21. Composé à base d'arylamine réticulable selon l'une quelconque des revendications 6 à 20, dans lequel le groupe réticulable est sélectionné parmi les groupes représentés par les Formules 4-1 à 4-14 : 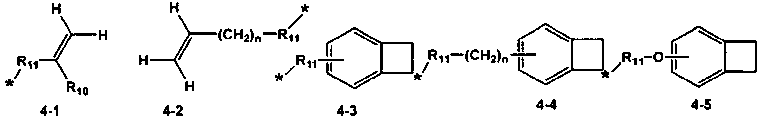 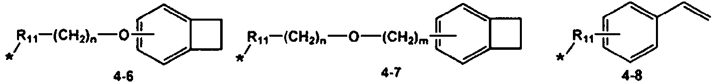 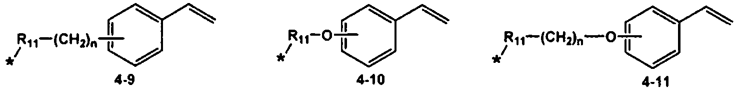 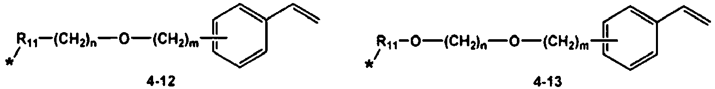 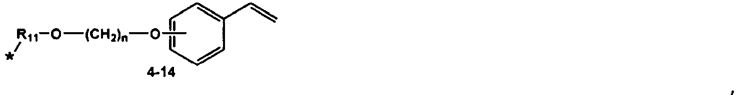 dans lequel, dans les Formules 4-1 à 4-14,
R₁₀ est un atome d'hydrogène ou un groupe alkyle en C₁-C₂₀ substitué ou non substitué,
R₁₁ est sélectionné parmi une liaison simple, un groupe alkylène en C₁-C₂₀ substitué ou non substitué, un groupe cycloalkylène en C₃-C₁₀ substitué ou non substitué, un groupe hétérocycloalkylène en C₁-C₁₀ substitué ou non substitué, un groupe cycloalcénylène en C₃-C₁₀ substitué ou non substitué, un groupe hétérocycloalcénylène en C₁-C₁₀ substitué ou non substitué, un groupe arylène en C₆-C₆₀ substitué ou non substitué, un groupe hétéroarylène en C₁-C₆₀ substitué ou non substitué, un groupe polycyclique condensé non aromatique divalent substitué ou non substitué, et un groupe hétéropolycyclique condensé non aromatique divalent substitué ou non substitué,
m et n représentent chacun indépendamment un entier compris entre 1 et 10, et * indique un site de liaison à un atome voisin.

22. Composé à base d'arylamine réticulable selon la revendication 6, dans lequel le composé à base d'arylamine réticulable est sélectionné parmi les Composés 1 à 40 :

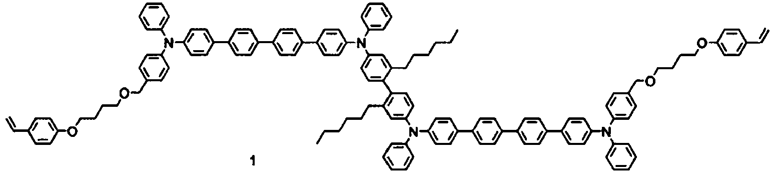

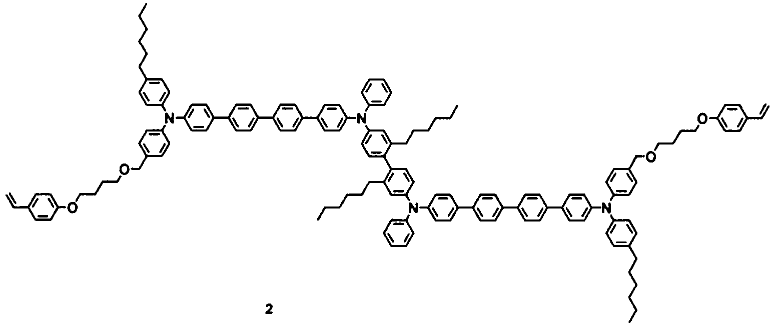

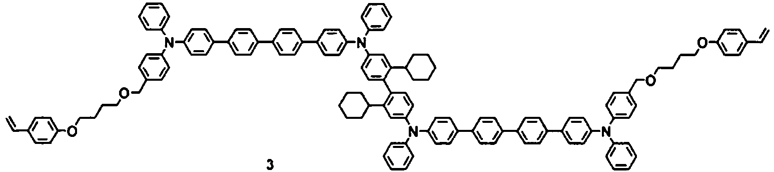

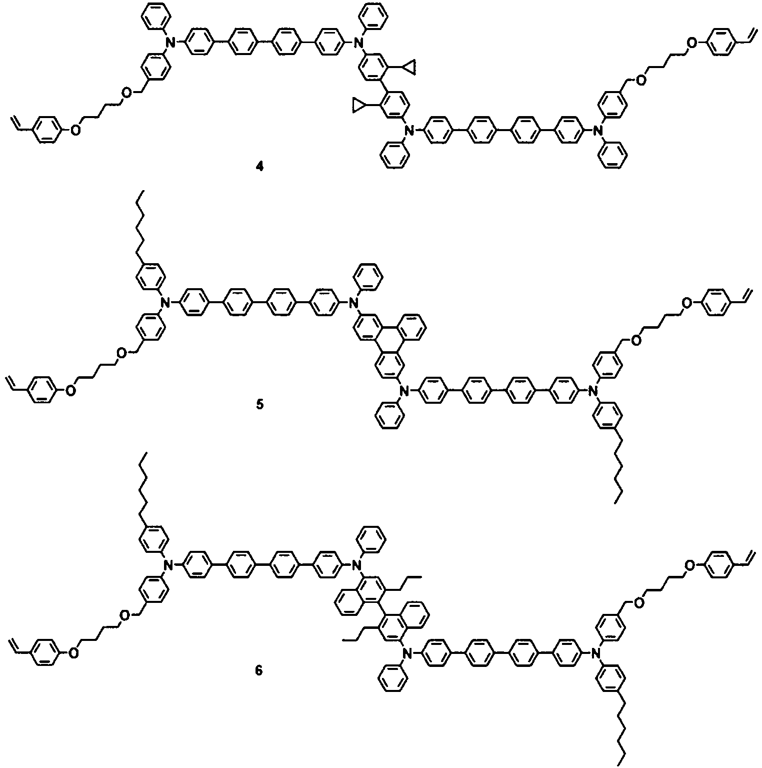

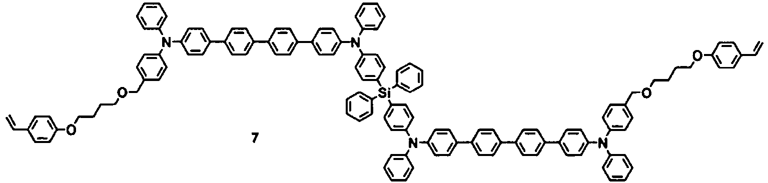

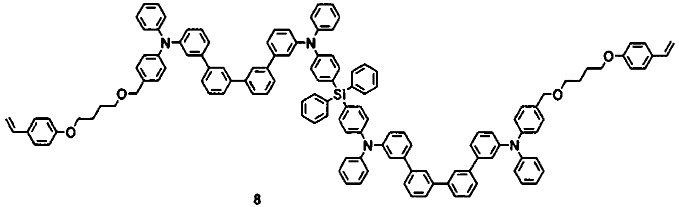

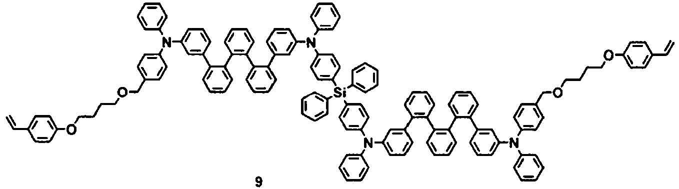

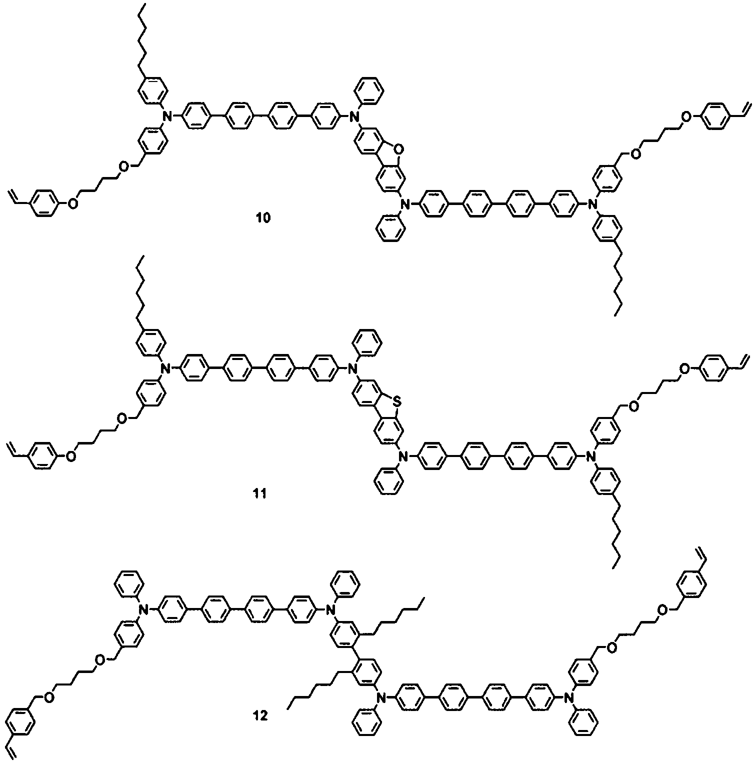

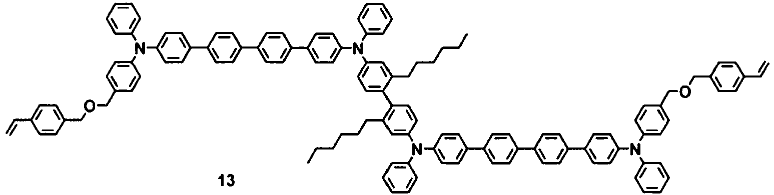

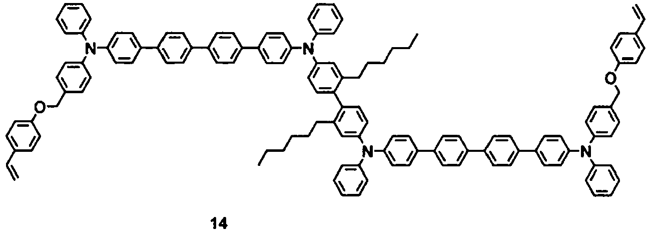

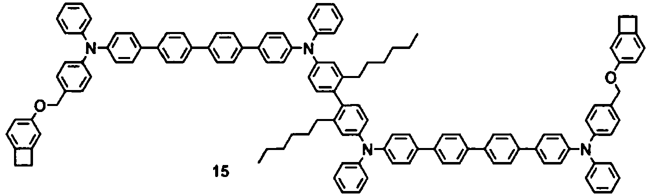

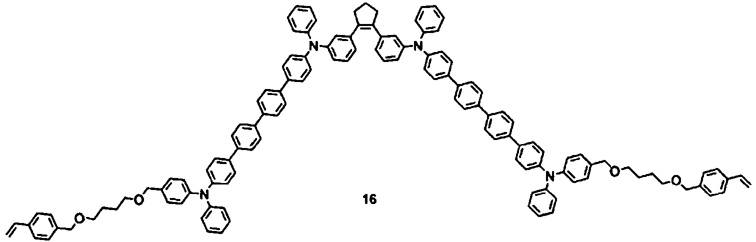

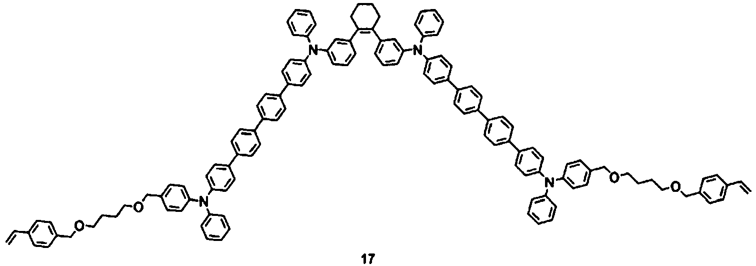

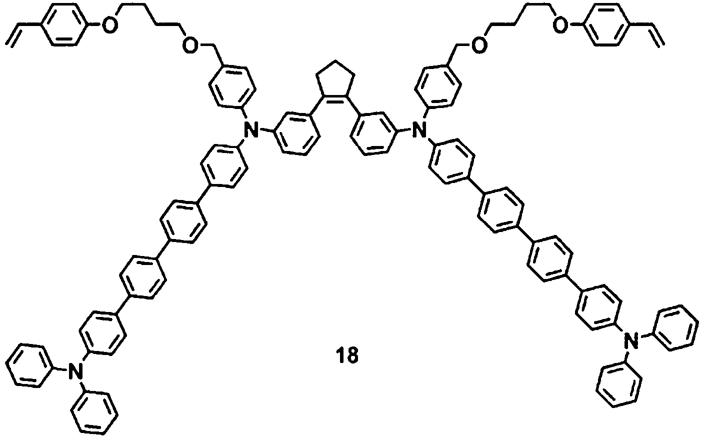

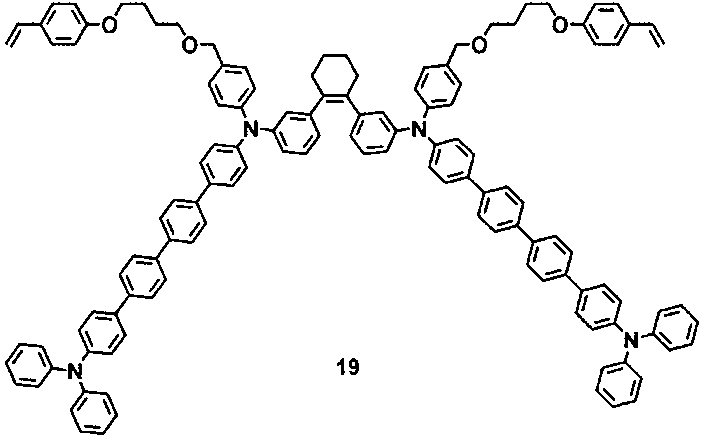

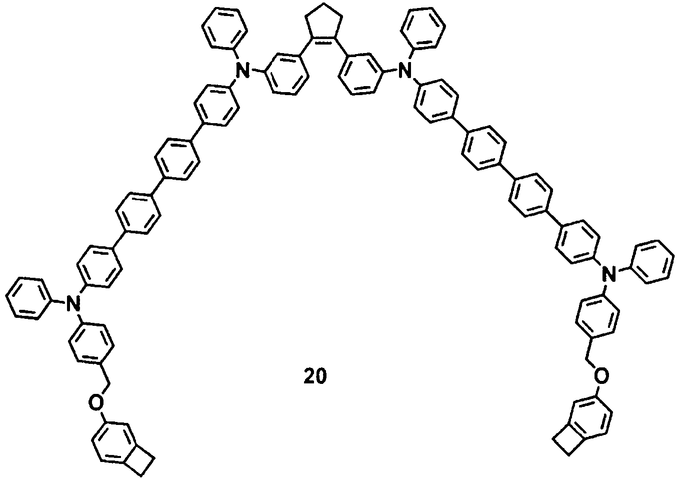

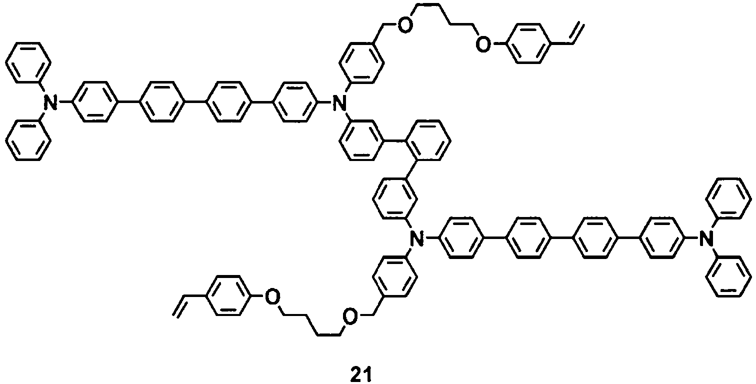

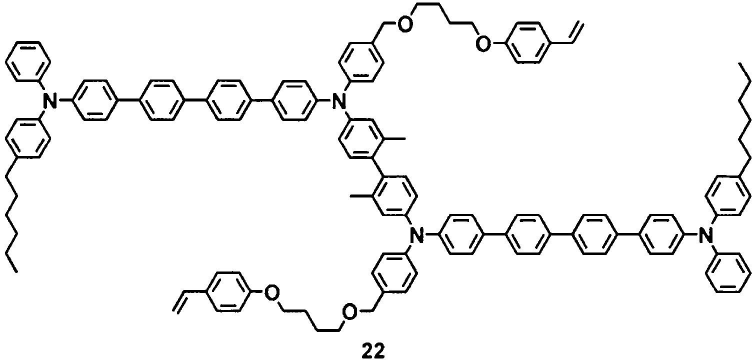

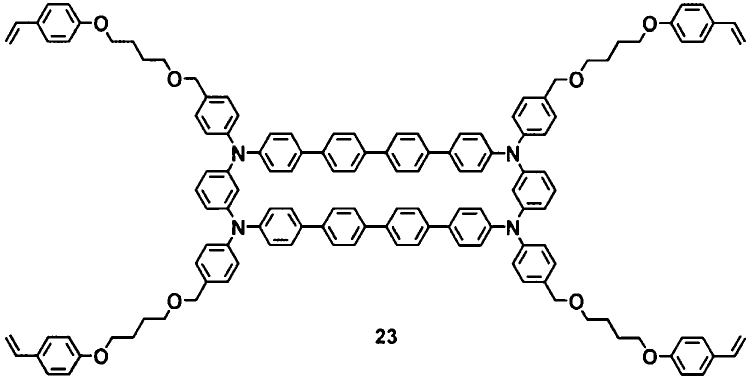

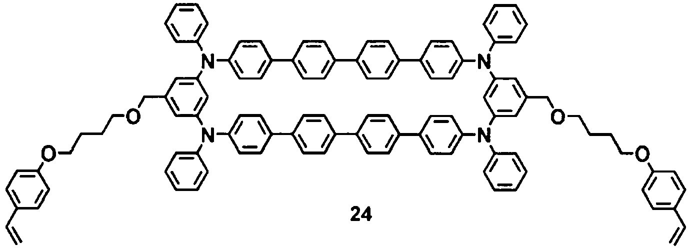

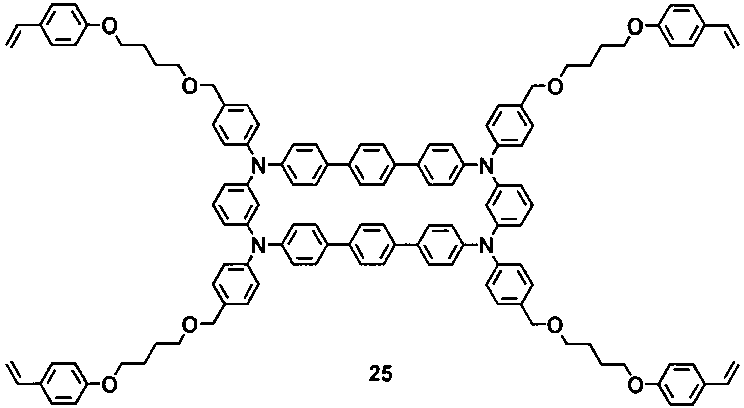

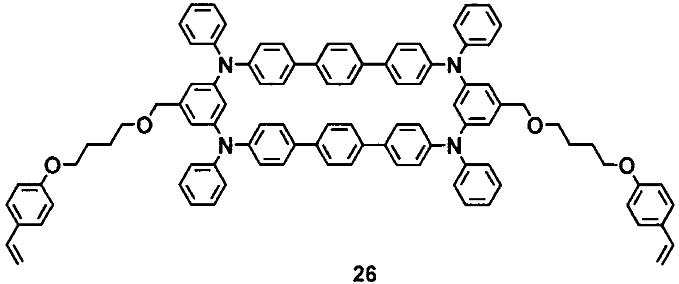

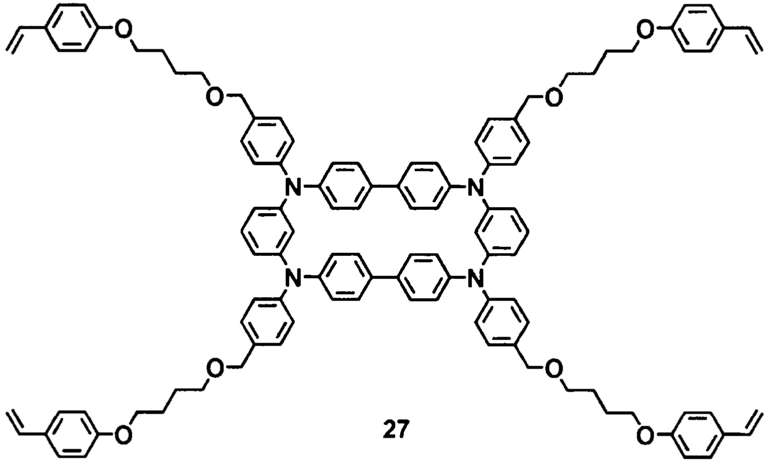

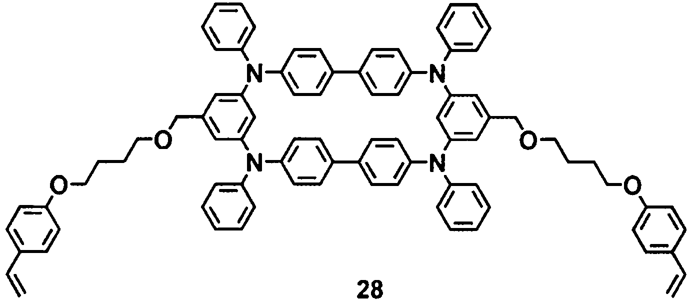

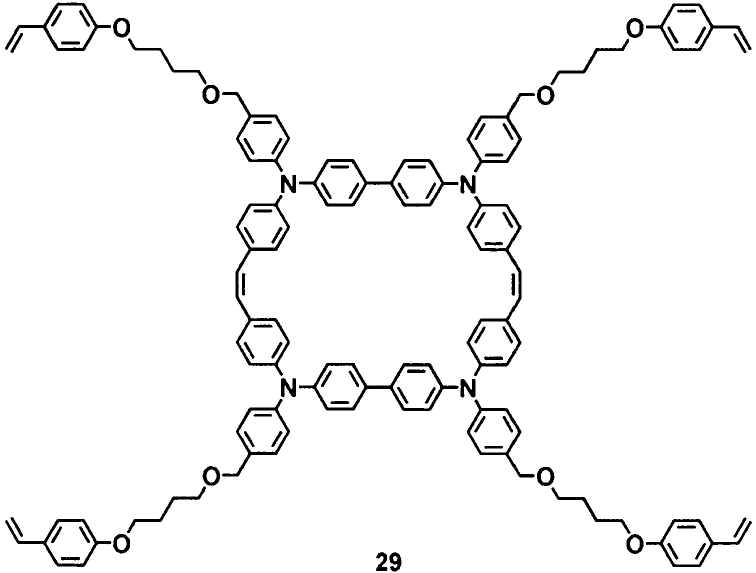

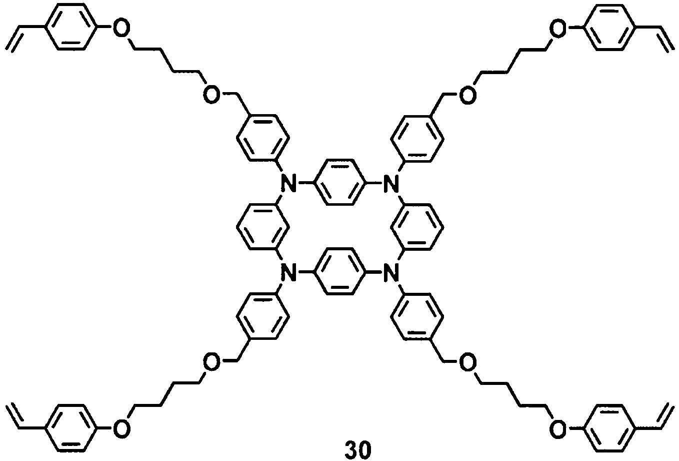

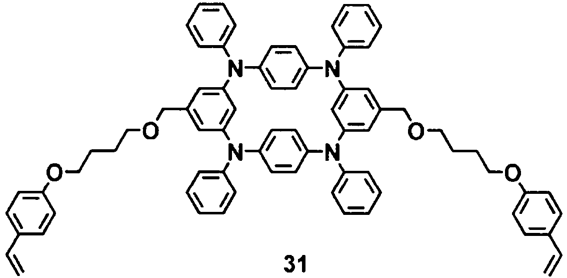

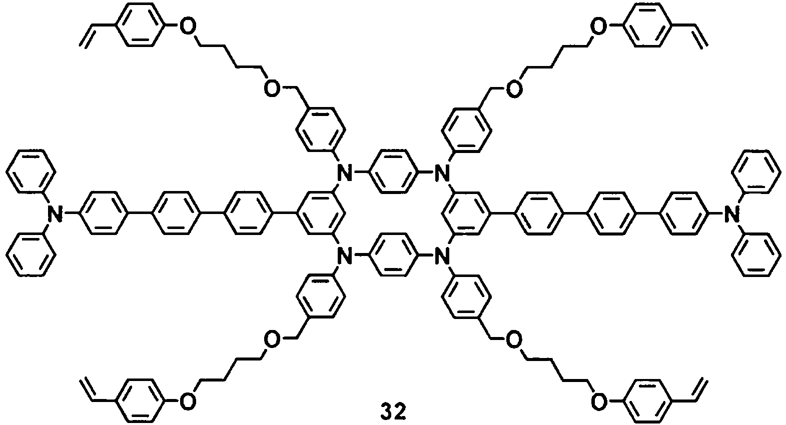

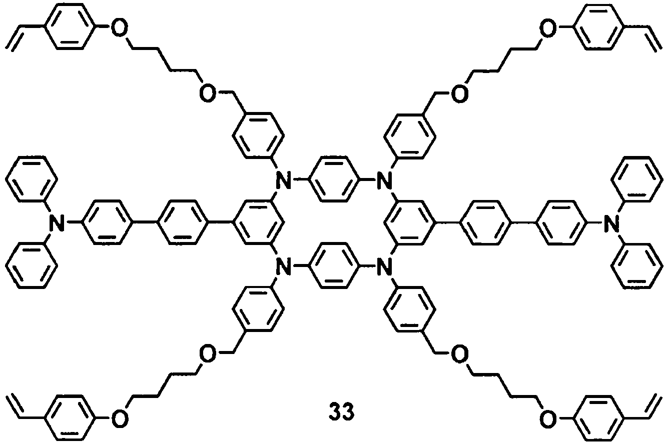

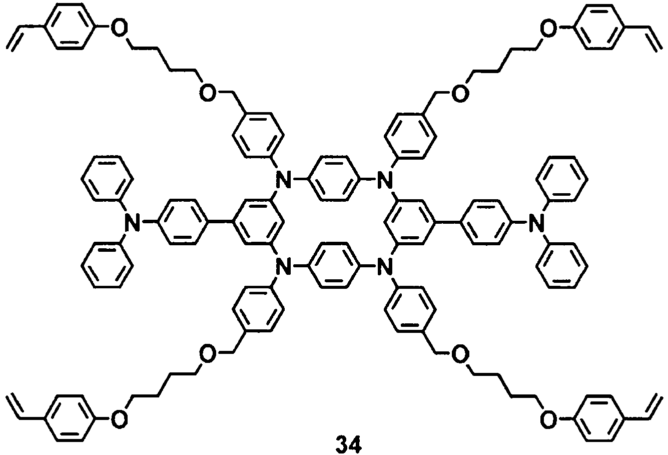

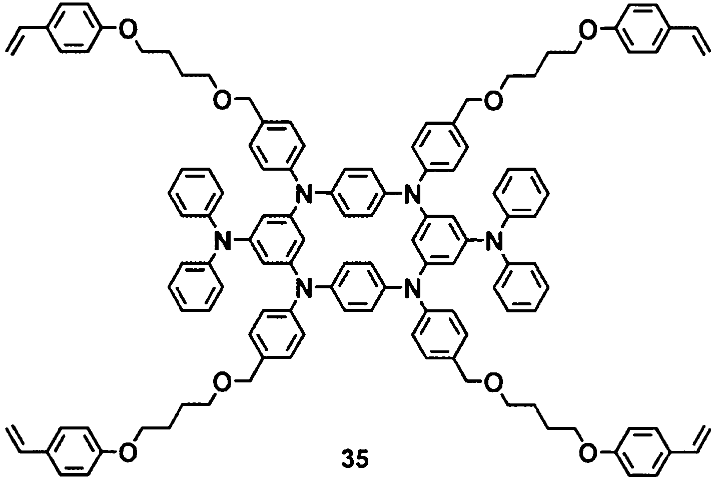

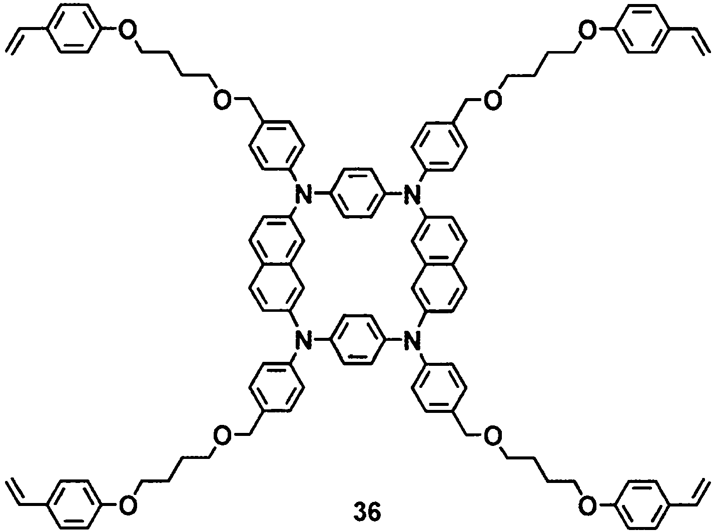

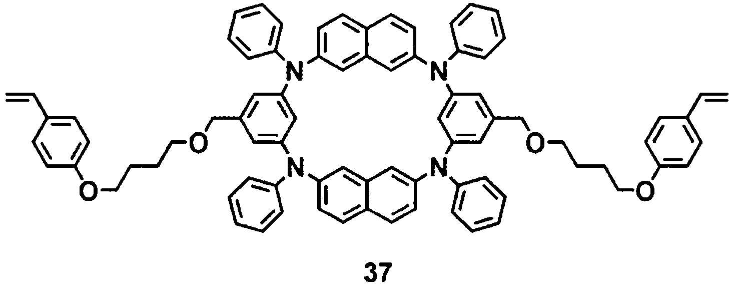

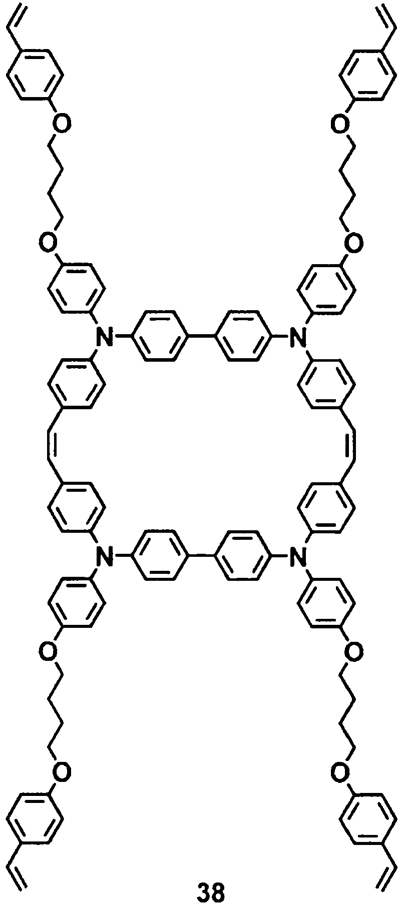

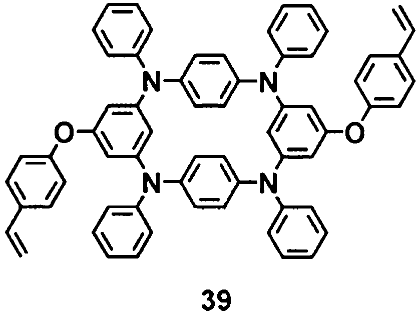

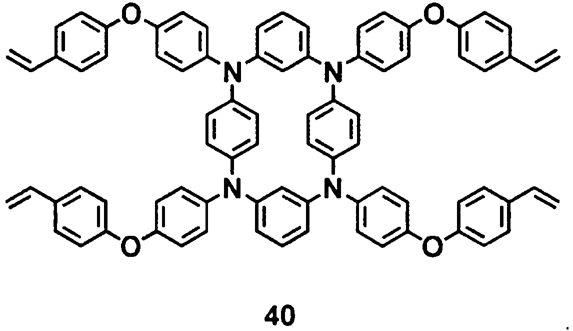

23. Polymère à base d'arylamine comprenant une unité répétitive représentée par la Formule 11 ou formé par réticulation d'un composé à base d'arylamine réticulable représenté par la Formule 1b : 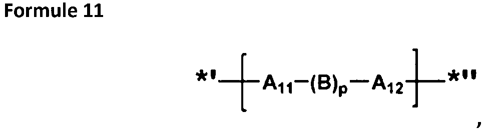 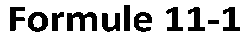 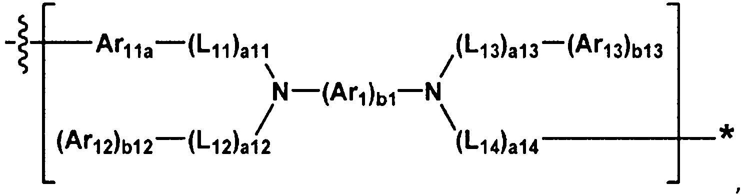 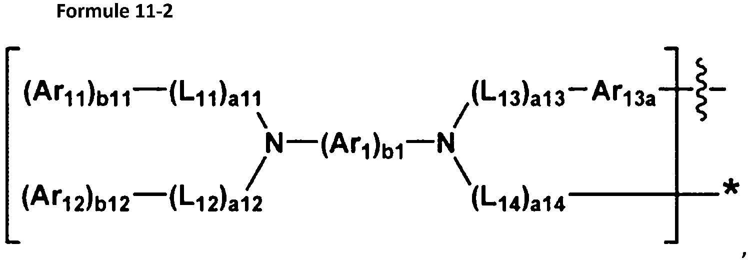 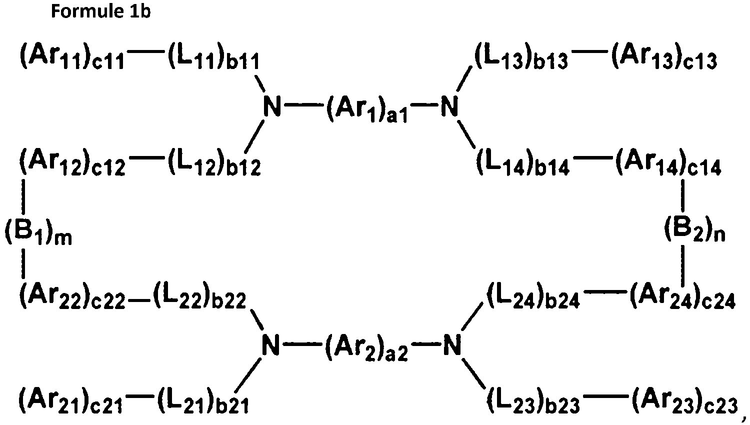
dans lequel, dans la Formule 11, A₁₁ et A₁₂ sont chacun indépendamment sélectionnés parmi les groupes représentés par les Formules 11-1 et 11-2, et A₁₁ et A₁₂ sont identiques ou différents l'un de l'autre,
dans la Formule 11-1, * indique un site de liaison à (B)ₚ dans la Formule 11,
dans les Formules 11, 11-1 et 11-2, *', *", et 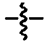 indiquent chacun un site de liaison à une unité répétitive voisine,
dans les Formules 11-1 et 11-2, Ar₁₁ₐ et Ar₁₃ₐ sont chacun un résidu formé après réticulation, et les unités répétitives sont liées par un groupe cyclobutane divalent,
dans la Formule 11, B est sélectionné parmi un groupe carbocyclique en C₅-C₆₀ substitué ou non substitué, un groupe hétérocyclique en C₁-C₆₀ substitué ou non substitué, et *'-Si(Q₄₁)(Q₄₂)-*", p est un entier compris entre 1 et 10,
dans les Formules 11-1 et 11-2, Ar1 est sélectionné parmi un groupe benzène, un groupe pyridine, un groupe pyrimidine, un groupe pyrazine, un groupe triazine, un groupe furane, un groupe thiophène, un groupe imidazole, un groupe thiazole, un groupe isoxazole et un groupe oxazole,
b1 est un entier supérieur ou égal à quatre, et quatre ou plus Ar₁ sont identiques ou différents les uns des autres,
dans les Formules 11-1 et 11-2, L₁₁ à L₁₄ sont chacun indépendamment un groupe carbocyclique en C₅-C₆₀ substitué ou non substitué ou un groupe hétérocyclique en C₁-C₆₀ substitué ou non substitué,
dans les Formules 11-1 et 11-2, a11 à a14 représentent chacun indépendamment 0, 1, 2, 3 ou 4, dans lequel, lorsque a11 représente 0, *'-(L₁₁)ₐ₁₁-*" est une liaison simple ; lorsque a12 représente 0, *'-(L₁₂)ₐ₁₂-*" est une liaison simple ; lorsque a13 représente 0, *'-(L₁₃)ₐ₁₃-*" est une liaison simple ; lorsque a14 représente 0, *'-(L₁₄)ₐ₁₄-*" est une liaison simple ; lorsque a11 représente deux ou plus, deux ou plusieurs L₁₁ sont identiques ou différents les uns des autres ; lorsque a12 représente deux ou plus, deux ou plusieurs L₁₂ sont identiques ou différents les uns des autres ; lorsque a13 représente deux ou plus, deux ou plusieurs L₁₃ sont identiques ou différents les uns des autres ; et lorsque a14 représente deux ou plus, deux ou plusieurs L₁₄ sont identiques ou différents les uns des autres,
dans les Formules 11-1 et 11-2, Ar₁₁ à Ar₁₃ sont chacun indépendamment sélectionnés parmi un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe hétéroaryle en C₁-C₆₀ substitué ou non substitué, un groupe polycyclique condensé non aromatique monovalent substitué ou non substitué, et un groupe hétéropolycyclique condensé non aromatique monovalent substitué ou non substitué, dans lequel au moins l'un de Ar₁₁ à Ar₁₃ est substitué par un groupe réticulable,
dans les Formules 11-1 et 11-2, b11 à b13 sont chacun indépendamment 1, 2, 3, 4 ou 5, dans lequel, lorsque b11 représente deux ou plus, deux ou plusieurs Ar₁₁ sont identiques ou différents les uns des autres ; lorsque b12 représente deux ou plus, deux ou plusieurs Ar₁₂ sont identiques ou différents les uns des autres ; et lorsque b13 représente deux ou plus, deux ou plusieurs Ar₁₃ sont identiques ou différents les uns des autres,
dans les Formules 11-1 et 11-2, Ar₁ et Ar₂ sont chacun indépendamment un groupe carbocyclique en C₅-C₆₀ substitué ou non substitué ou un groupe hétérocyclique en C₁-C₆₀ substitué ou non substitué, dans lequel Ar₁ et Ar₂ sont identiques ou différents l'un de l'autre,
dans la Formule 1b, Ar₁ et Ar₂ sont chacun indépendamment un groupe carbocyclique en C₅-C₆₀ substitué ou non substitué ou un groupe hétérocyclique en C₁-C₆₀ substitué ou non substitué, dans lequel Ar₁ et Ar₂ sont identiques ou différents l'un de l'autre,
dans la Formule 1b, a1 et a2 sont chacun indépendamment un entier compris entre 1 et 5, dans lequel, lorsque a1 représente deux ou plus, deux ou plusieurs Ar₁ sont identiques ou différents les uns des autres ; et lorsque a2 représente deux ou plus, deux ou plusieurs Ar₂ sont identiques ou différents les uns des autres,
dans la Formule 1b, L₁₁ à L₁₄ et L₂₁ à L₂₄ sont chacun indépendamment un groupe carbocyclique en C₅-C₆₀ substitué ou non substitué ou un groupe hétérocyclique en C₁-C₆₀ substitué ou non substitué,
dans la Formule 1b, b11 à b14 et b21 à b24 sont chacun indépendamment un entier compris entre 0 et 3, dans lequel, lorsque b11 représente 0, *-(L₁₁)_{b11}-*' est une liaison simple ; lorsque b12 représente 0, *-(L₁₂)_{b12}-*' est une liaison simple ; lorsque b13 représente 0, *-(L₁₃)_{b13}-*' est une liaison simple ; lorsque b14 représente 0, *-(L₁₄)_{b14}-*' est une liaison simple ; lorsque b21 représente 0, *-(L₂₁)_{b21}-*' est une liaison simple ; lorsque b22 représente 0, *-(L₂₂)_{b22}-*' est une liaison simple ; lorsque b23 représente 0, *-(L₂₃)_{b23}-*' est une liaison simple ; lorsque b24 représente 0, *-(L₂₄)_{b24}-*' est une liaison simple ; lorsque b11 représente deux ou plus, deux ou plusieurs L₁₁ sont identiques ou différents les uns des autres ; lorsque b12 représente deux ou plus, deux ou plusieurs L₁₂ sont identiques ou différents les uns des autres ; lorsque b13 représente deux ou plus, deux ou plusieurs L₁₃ sont identiques ou différents les uns des autres ; lorsque b14 représente deux ou plus, deux ou plusieurs L₁₄ sont identiques ou différents les uns des autres ; lorsque b21 représente deux ou plus, deux ou plusieurs L₂₁ sont identiques ou différents les uns des autres ; lorsque b22 représente deux ou plus, deux ou plusieurs L₂₂ sont identiques ou différents les uns des autres ; lorsque b23 représente deux ou plus, deux ou plusieurs L₂₃ sont identiques ou différents les uns des autres ; et lorsque b24 représente deux ou plus, deux ou plusieurs L₂₄ sont identiques ou différents les uns des autres,
dans la Formule 1b, B₁ et B₂ sont chacun indépendamment sélectionnés parmi une liaison simple, un groupe alkylène en C₁-C₂₀ substitué ou non substitué, un groupe alcénylène en C₂-C₂₀ substitué ou non substitué et un groupe alcynylène en C₂-C₂₀ substitué ou non substitué,
dans la Formule 1b, m et n sont chacun indépendamment un entier compris entre 1 et 3, dans lequel, lorsque m représente deux ou plus, deux ou plusieurs B₁ sont identiques ou différents les uns des autres ; et lorsque n représente deux ou plus, deux ou plusieurs B₂ sont identiques ou différents les uns des autres,
dans la Formule 1b, Ar₁₁, Ar₁₃, Ar₂₁ et Ar₂₃ sont chacun indépendamment sélectionnés parmi un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe hétéroaryle en C₁-C₆₀ substitué ou non substitué, un groupe polycyclique condensé non aromatique monovalent substitué ou non substitué, et un groupe hétéropolycyclique condensé non aromatique monovalent substitué ou non substitué,
dans la Formule 1b, Ar₁₂, Ar₁₄, Ar₂₂ et Ar₂₄ sont chacun indépendamment sélectionnés parmi une liaison simple, un groupe cycloalkylène en C₃-C₁₀ substitué ou non substitué, un groupe hétérocycloalkylène en C₁-C₁₀ substitué ou non substitué, un groupe cycloalcénylène en C₃-C₁₀ substitué ou non substitué, un groupe hétérocycloalcénylène en C₁-C₁₀ substitué ou non substitué, un groupe arylène en C₆-C₆₀ substitué ou non substitué, un groupe hétéroarylène en C₁-C₆₀ substitué ou non substitué, un groupe polycyclique condensé divalent non aromatique substitué ou non substitué, et un groupe hétéropolycyclique condensé divalent non aromatique substitué ou non substitué,
dans la Formule 1b, c11 à c14 et c21 à c24 sont chacun indépendamment un entier compris entre 1 et 5, dans lequel, lorsque c11 représente deux ou plus, deux ou plusieurs Ar₁₁ sont identiques ou différents les uns des autres ; lorsque c12 représente deux ou plus, deux ou plusieurs Ar₁₂ sont identiques ou différents les uns des autres ; lorsque c13 représente deux ou plus, deux ou plusieurs Ar₁₃ peuvent être identiques ou différents les uns des autres ; lorsque c14 représente deux ou plus, deux ou plusieurs Ar₁₄ sont identiques ou différents les uns des autres ; lorsque c21 représente deux ou plus, deux ou plusieurs Ar₂₁ sont identiques ou différents les uns des autres ; lorsque c22 représente deux ou plus, deux ou plusieurs Ar₂₂ sont identiques ou différents les uns des autres ; lorsque c23 représente deux ou plus, deux ou plusieurs Ar₂₃ sont identiques ou différents les uns des autres ; et lorsque c24 représente deux ou plus, deux ou plusieurs Ar₂₄ sont identiques ou différents les uns des autres,
dans la Formule 1b, au moins un groupe parmi Ar₁, Ar₂, Ar₁₁ à Ar₁₄et Ar₂₁ à Ar₂₄ est substitué par un groupe réticulable.

24. Polymère à base d'arylamine selon la revendication 23, dans lequel, dans les Formules 11-1 et 11-2, Ar₁₁ₐ et Ar₁₃ₐ sont chacun indépendamment sélectionnés parmi les groupes représentés par les Formules 5-1 à 5-8 : 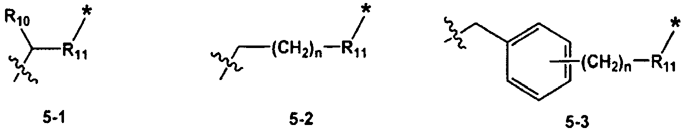 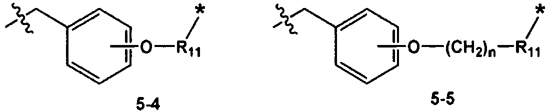 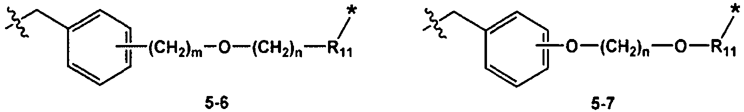 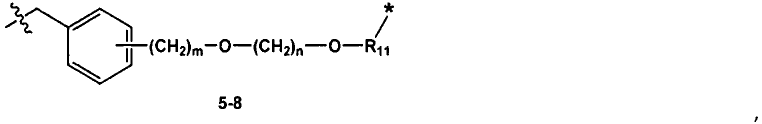 dans lequel, dans les Formules 5-1 à 5-8,
R₁₀ est un atome d'hydrogène ou un groupe alkyle en C₁-C₂₀ substitué ou non substitué,
R₁₁ est sélectionné parmi une liaison simple, un groupe alkylène en C₁-C₂₀ substitué ou non substitué, un groupe cycloalkylène en C₃-C₁₀ substitué ou non substitué, un groupe hétérocycloalkylène en C₁-C₁₉ substitué ou non substitué, un groupe cycloalcénylène en C₃-C₁₀ substitué ou non substitué, un groupe hétérocycloalcénylène en C₁-C₁₀ substitué ou non substitué, un groupe arylène en C₆-C₆₀ substitué ou non substitué, un groupe hétéroarylène en C₁-C₆₀ substitué ou non substitué, un groupe polycyclique condensé non aromatique divalent substitué ou non substitué, et un groupe hétéropolycyclique condensé non aromatique divalent substitué ou non substitué,
m et n représentent chacun indépendamment un entier compris entre 1 et 10,
* indique un site de liaison à un atome voisin, et 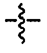
indique un site de liaison à une unité répétitive voisine.

25. Polymère à base d'arylamine selon la revendication 23 ou la revendication 24, dans lequel dans les Formules 11-1 et 11-2, Ar₁ est un groupe benzène, et b1 représente 4 ou 5.
